# EUROPEAN PATENT SPECIFICATION

(11) **EP 4 214 220 B1**
(45) Date of publication and mention of the grant of the patent: **02.09.2026**
(21) Application number: 21762607.6
(22) Date of filing: 05.08.2021
(51) Int. Cl.: C07K 7/08, C07K 14/47, A61P 31/04, A61K 38/00

(54) **ANTIMICROBIAL PEPTIDOMIMETICS**
ANTIMIKROBIELLE PEPTIDOMIMETIKA
PEPTIDOMIMÉTIQUES À ACTIVITÉ ANTIMICROBIENNE

(30) Priority: 05.08.2020 EP 20020353
(43) Date of publication of application: 26.07.2023
(73) Proprietor: Basilea Pharmaceutica International AG, Allschwil, 4123 Allschwil (CH); Universität Zürich, 8006 Zürich (CH)
(72) Inventor: OBRECHT, Daniel, 4112 Bättwil (CH); LUTHER, Anatol, 79589 Binzen (DE); UPERT, Grégory, 68680 Kembs (FR); DESJONQUERES, Nicolas, 68680 Kembs (FR); BRABET, Emile, 68300 Saint Louis (FR); ZBINDEN, Peter, 4312 Magden (CH); ZERBE, Oliver, 8057 Zürich (CH); MÖHLE, Kerstin, 8907 Wettswil (CH)
(74) Representative: Thwaite, Jonathan Simon
(86) International application number: PCT/EP2021/025301
(87) International publication number: WO 2022/028737

(56) References cited:
- CN-A- 101 173 005
- JP-A- 2011 072 294
- IMAMURA T ET AL: "NMR based structure-activity relationship analysis of an antimicrobial peptide, thanatin, engineered by site-specific chemical modification: Activity improvement and spectrum alteration", BIOCHEMICAL AND BIOPHYSICAL RESEARCH COMMUNICATIONS, ELSEVIER, AMSTERDAM NL, vol. 369, no. 2, 2 May 2008 (2008-05-02), pages 609 - 615, XP022624984, ISSN: 0006-291X, [retrieved on 20080221], DOI: 10.1016/J.BBRC.2008.02.057
- LEE MYUNG-KYU ET AL: "Role of Amino Acid Residues within the Disulfide Loop of Thanatin, a Potent Antibiotic Peptide", BMB REPORTS, vol. 35, no. 3, 31 May 2002 (2002-05-31), KR, pages 291 - 296, XP055864141, ISSN: 1976-6696, Retrieved from the Internet <URL:http://dx.doi.org/10.5483/BMBRep.2002.35.3.291> DOI: 10.5483/BMBRep.2002.35.3.291
- VETTERLI STEFAN U. ET AL: "Thanatin targets the intermembrane protein complex required for lipopolysaccharide transport in Escherichia coli", SCIENCE ADVANCES, vol. 4, no. 11, 21 November 2018 (2018-11-21), pages 2634 - 14, XP055863989, DOI: 10.1126/sciadv.aau2634
- MOURA ELISABETE C. C. M. ET AL: "Thanatin Impairs Lipopolysaccharide Transport Complex Assembly by Targeting LptC-LptA Interaction and Decreasing LptA Stability", FRONTIERS IN MICROBIOLOGY, vol. 11, no. Article 909, 13 May 2020 (2020-05-13), XP055864006, DOI: 10.3389/fmicb.2020.00909

## Description

The present invention is directed to peptidomimetics having antimicrobial activity, especially against Gram-negative bacteria. The peptidomimetics of the invention are compounds of the general formula (**I**),

P¹-P²-P³-P⁴-P⁵-P⁶-P⁷-P⁸-P⁹-P¹⁰-P¹¹-P¹²-P¹³-P¹⁴-P¹⁵-P¹⁶ (**I**)

and pharmaceutically acceptable salts thereof, as described herein below. The invention is also directed to therapeutic uses of the peptidomimetics for the treatment or prevention of bacterial infections and diseases related to bacterial infections and to non-therapeutic uses of the peptidomimetics for preserving or disinfecting foodstuffs, cosmetics, medicaments or other nutrient-containing materials. In addition, the present invention provides an efficient synthetic process by which these compounds can, if desired, be made in parallel library-format. Moreover, the peptidomimetics of the invention show improved antimicrobial activity, low or no hemolysis of red blood cells and reduced cytotoxicity.

There are limited treatment options for carbapenem-resistant *Enterobacteriaceae* (CRE) infections. Antibiotics that more frequently show in vitro activity against CRE include colistin, tigecycline and fosfomycin. However, the data on their effectiveness and clinical experience is limited. There are also more frequent adverse effects, rapid development of resistance during treatment, and increasing resistance globally. Colistin is frequently being used to treat CRE infections, but colistin resistance may develop in CRE-infected patients treated with colistin. Since 2015, the discovery of transferable plasmid-mediated colistin resistance genes (*mcr* 1-5) that can transmit colistin resistance more easily between bacteria has further increased the risk of colistin resistance spreading (Giamarellou H. et al., Antimicrob Agents Chemother. 2013, 57(5), 2388-90).

None of the recently approved antibiotics or those in late stage development have a satisfactory coverage of CRE. Notably, new beta-lactam combinations lack activity against metallo-beta-lactamase (MBL) producing organisms. Ceftazidime / Avibactam (CAZ-AVI), most commonly used novel antibiotic against CREs is not active against MBL organisms. Furthermore, reports of CAZ-AVI-resistant CRE strains that have developed resistance during treatment with CAZ-AVI, alone or in combination with other antibiotics, soon after the launch of CAZ-AVI. After these reports of concern, ECDC has issued a rapid risk assessment report regarding this issue in June 12, 2018. The new aminoglycoside plazomicin has safety warnings (nephrotoxicity, ototoxicity, neuromuscular blockade and fetal harm) in the prescribing information.

Thus, there is an on-going need for the development of antibiotics that can be used for the effective treatment of CRE infections.

The natural antimicrobial peptide thanatin, a 21-residue inducible insect defense peptide (Fehlbaum P. et al., Proc. Natl. Acad. Sci. USA 1996, 93, 1221-1225), is targeting the lipopolysaccharide transport protein LptA of Gram-negative bacteria, which leads to inhibition of LPS transport and outer membrane (OM) biogenesis (Vetterli S. U. et al., Sci. Adv. 2018; 4:eaau2634). Thanatin is active against carbapenem-resistant *Enterobacteriaceae* including pan resistant strains. These highly resistant organisms can cause a variety of infections including complicated urinary tract infections (cUTI), complicated intra-abdominal infections (clAI), hospital- or ventilator-associated pneumonia (HAP/VAP), or bloodstream infections (BSI).

CN101173005A discloses antimicrobial peptide thanatin derivatives.

The present invention embraces a novel class of thanatin-derived peptidomimetics having 16 amino acid or amino acid derived residues and showing a narrow antimicrobial spectrum focused on *Enterobacteriaceae.* Despite their shorter sequences compared to thanatin, these novel thanatin-derived peptidomimetics surprisingly exhibit an improved antimicrobial activity, low or no hemolysis of red blood cells and reduced cytotoxicity.

In a first aspect, the invention provides a peptidomimetic compound of the general formula (**I**),

P¹-P²-P³-P⁴-P⁵-P⁶-P⁷-P⁸-P⁹-P¹⁰-P¹¹-P¹²-P¹³-P¹⁴-P¹⁵-P¹⁶ **(I)**

wherein
- P¹ is: 2OHVal, Val, NMeVal, Abu, tBuGly;
wherein the amino group of P¹ is optionally replaced by a guanidino group (Gua) or by a tetramethyl guanidino (TMG) group;
- P² is: Pro, Pro(4R)F, Pro(4R)OMe, Pro(4R)OPhe, Pro(3,4dehydro), Pr(4,4F2), Pic;
Agb, Agp, Dab, Dab(iPr), Dap, Dap(iPr), Har, Lys, Lys(iPr), Narg, Ndab, Nlys, Norn, Orn, Orn(iPr), Pro((4R)guanidine), Pro((4R)NH₂), Pro((4S)NH₂), Arg, NMeLys;
Hyp, N*allo*Thr;
- P³ is: Hle, Ile, Leu, Nle, Cyg;
- P⁴ is: Ala, Ala(cPr), Ala(tetrahydropyran4yl), Abu, allolle, Cha, Cpa, Cpg, Cyg, Dea, Hle, Ile, Leu, Met, Nle, OctGly, tBuGly, tBuAla, Val, NMeAla, NMeVal, Nva;
Phe, His, Phe(3OH), Phe(4F), Phe(4OCF3), Trp(6Cl), Tyr(3Cl), Tyr(3F), Tyr(Phenyl), Trp, Tyr, 4Thz, Phe(4(4hydroxyphenoxy)), Phe(4NH2), Tyr(Me), Ntyr, Nphe;
Agb, Agp, Dab, Dab(iPr), Dap, Dap(iPr), Har, Lys, Lys(iPr), Narg, Ndab, Nlys, Norn, Orn, Orn(iPr), Arg, NMeLys, Lys(Me);
alloThr, Cit, Hgn, Hse, Leu((3R)OH), Asn, Gln, Ser, Thr, Asp, Glu, Hgl, Val(3OH);
- P⁵ is: Phe, His, Trp, Tyr;
- P⁶ is: Pra, Abu(4N₃);
Dab, Dap;
Cys, Hcy, NMeCys, Pen;
Asp, Glu, Hgl;
- P⁷ is: Ala, Ala(cPr), Ala(tetrahydropyran4yl), Abu, allolle, Cha, Cpa, Cpg, Cyg, Dea, Hle, Ile, Leu, Met, Nle, OctGly, tBuGly, tBuAla, Val, NMeAla, NMeVal, Nva;
Phe, His, Phe(3OH), Phe(4F), Phe(4OCF3), Trp(6Cl), Tyr(3Cl), Tyr(3F), Tyr(Phenyl), Trp, Tyr, 4Thz, Phe(4(4hydroxyphenoxy)), Phe(4NH2), Tyr(Me), Ntyr, Nphe;
Agb, Agp, Dab, Dab(iPr), Dap, Dap(iPr), Har, Lys, Lys(iPr), Narg, Ndab, Nlys, Norn, Orn, Orn(iPr), Arg, NMeLys;
alloThr, Cit, Hgn, Hse, Leu((3R)OH), Asn, Gln, Ser, Thr, Asp, Glu, Hgl;
- P⁸ is: Agb, Har, Arg, Lys, Orn;
- P⁹ is: Dab, Dap, Har, Lys, Orn, Arg, Dab(iPr);
Cit;
- P¹⁰ is: alloThr, Hse, Ser, Thr, Hyp, Gln, Asn, Glu, Asp;
- P¹¹ is: ^{D}Dab, ^{D}Dap, ^{D}Lys, ^{D}Orn, ^{D}Arg, ^{D}Dab(iPr);
- P¹² is: Ala, Ala(cPr), Ala(tetrahydropyran4yl), Abu, allolle, Cha, Cpa, Cpg, Cyg, Dea, Hle, Ile, Leu, Met, Nle, OctGly, tBuGly, tBuAla, Val, NMeAla, NMeVal, Nva;
Phe, His, Phe(3OH), Phe(4F), Phe(4OCF3), Trp(6Cl), Tyr(3Cl), Tyr(3F), Tyr(Phenyl), Trp, Tyr, 4Thz, Phe(4(4hydroxyphenoxy)), Phe(4NH2), Tyr(Me), Ntyr, Nphe;
Agb, Agp, Dab, Dab(iPr), Dap, Dap(iPr), Har, Lys, Lys(iPr), Narg, Ndab, Nlys, Norn, Orn, Orn(iPr), Arg, NMeLys;
alloThr, Cit, Hgn, Hse, Leu((3R)OH), Asn, Gln, Ser, Thr, Asp, Glu, Hgl;
- P¹³ is: Pra, Abu(4N₃);
Dab, Dap;
Cys, Hcy, NMeCys, Pen;
Asp, Glu, Hgl;
- p¹⁴ is: Phe, His, Phe(3OH), Phe(4F), Phe(4OCF3), Trp(6Cl), Tyr(3Cl), Tyr(3F), Tyr(Phenyl), Trp, Tyr, 4Thz, Phe(4(4hydroxyphenoxy)), Phe(4NH2), Tyr(Me), Ntyr, Nphe;
Agb, Agp, Dab, Dab(iPr); Dap, Dap(iPr), Har, Lys, Lys(iPr), Narg, Ndab, Nlys, Norn, Orn, Orn(iPr), Arg, NMeLys;
alloThr, Cit, Hgn, Hse, Leu((3R)OH), Asn, Gln, Ser, Thr, Asp, Glu, Hgl;
- P¹⁵ is: Ala, Ala(cPr), Ala(tetrahydropyran4yl), Abu, allolle, Cha, Cpa, Cpg, Cyg, Dea, Hle, Ile, Leu, Met, Nle, OctGly, tBuGly, tBuAla, Val, NMeAla, NMeVal, Nva;
Agb, Agp, Dab, Dab(iPr), Dap, Dap(iPr), Har, Lys, Lys(iPr), Narg, Ndab, Nlys, Norn, Orn, Orn(iPr), Arg, NMeLys;
alloThr, Cit, Hgn, Hse, Leu((3R)OH), Asn, Gln, Ser, Thr, Asp, Glu, Hgl;
- P¹⁶ is: Ala, Ala(cPr), Ala(tetrahydropyran4yl), Abu, allolle, Cha, Cpa, Cpg, Cyg, Dea, Hle, Ile, Leu, Met, Nle, OctGly, tBuGly, tBuAla, Val, NMeAla, NMeVal, Nva;
Phe, His, Phe(3OH), Phe(4F), Phe(4OCF3), Trp(6Cl), Tyr(3Cl), Tyr(3F), Tyr(Phenyl), Trp, Tyr, 4Thz, Phe(4(4hydroxyphenoxy)), Phe(4NH2), Tyr(Me), Ntyr, Nphe;

or a tautomer, a rotamer, a salt, a hydrate or a solvate thereof,
wherein
Cys, Hcy, NMeCys or Pen at P⁶, if present, and Cys, Hcy, NMeCys or Pen at P¹³, if present, optionally form a disulfide bridge between P⁶ and P¹³; or wherein
Dab or Dap at P⁶, if present, and Asp, Glu or Hgl at P¹³, if present, optionally form a lactam bridge between P⁶ and P¹³; or wherein
Asp, Glu or Hgl at P⁶, if present, and Dab or Dap at P¹³, if present, optionally form a lactam bridge between P⁶ and P¹³; or wherein
Pra at P⁶, if present, and Abu(4N₃) at P¹³, if present, optionally form a 1,2,3-triazole bridge between P⁶ and P¹³; or wherein
Abu(4N₃) at P⁶, if present, and Pra at P¹³, if present, optionally form a 1,2,3-triazole bridge between P⁶ and P¹³;
with the proviso that at least three amino acid residues among the four amino acid residues at positions P⁹, P¹², P¹⁴ and P¹⁵ are basic amino acid residues selected from Agb, Agp, Dab, Dab(iPr), Dap, Dap(iPr), Har, Lys, Lys(iPr), Narg, Ndab, Nlys, Norn, Orn, Orn(iPr), Arg, NMeLys.

A preferred embodiment of the first aspect relates to a compound, wherein
- P¹ is: 2OHVal, Val, or NMeVal;
wherein the N-terminal amino group of P¹ is optionally replaced by a guanidino group (Gua) or by a tetramethyl guanidino (TMG) group;
- P² is: Pro,
Agb, Agp, Dab, Dap, Har, Lys, Narg, Ndab, Nlys, Norn, Orn, Pro((4R)guanidine), Pro((4R)NH₂), Pro((4S)NH₂), Arg, NMeLys, or
Hyp;
- P³ is: *Hle,* Ile, Leu, or Nle;
- P⁴ is: Ala, Ala(cPr), Ala(tetrahydropyran4yl), Abu, allolle, Cha, Cpa, Cpg, Cyg, Dea, Hle, Ile, Leu, Met, Nle, OctGly, tBuGly, tBuAla, Val, NMeAla, NMeVal, Nva,
Phe, His, Phe(3OH), Phe(4F), Phe(4OCF₃), Trp(6Cl), Tyr(3Cl), Tyr(3F), Tyr(Phenyl), Trp, Tyr, 4Thz, Phe(4(4hydroxyphenoxy)), Phe(4NH2), Tyr(Me), Ntyr, Nphe,
Agb, Agp, Dab, Dap, Har, Lys, Narg, Ndab, Nlys, Norn, Orn, Arg, NMeLys,
alloThr, Cit, Hgn, Hse, Leu((3R)OH), Asn, Gln, Ser, Thr, Asp, Glu, or Hgl;
- P⁵ is: Phe, His, Trp, or Tyr;
- P⁶ is: Pra, Abu(4N₃),
Dab, Dap,
Cys, Hcy, NMeCys, Pen,
Asp, Glu, or Hgl;
- P⁷ is: Ala, Ala(cPr), Ala(tetrahydropyran4yl), Abu, allolle, Cha, Cpa, Cpg, Cyg, Dea, Hle, Ile, Leu, Met, Nle, OctGly, tBuGly, tBuAla, Val, NMeAla, NMeVal, Nva,
Phe, His, Phe(3OH), Phe(4F), Phe(4OCF₃), Trp(6Cl), Tyr(3Cl), Tyr(3F), Tyr(Phenyl), Trp, Tyr, 4Thz, Phe(4(4hydroxyphenoxy)), Phe(4NH₂), Tyr(Me), Ntyr, Nphe,
Agb, Agp, Dab, Dap, Har, Lys, Narg, Ndab, Nlys, Norn, Orn, Arg, NMeLys,
alloThr, Cit, Hgn, Hse, Leu((3R)OH), Asn, Gln, Ser, Thr, Asp, Glu, or Hgl;
- P⁸ is: Har, or Arg;
- P⁹ is: Dab, Dap, Har, Lys, Orn, or Arg;
- P¹⁰ is: alloThr, Hse, Ser, or Thr;
- P¹¹ is: ^{D}Dab, ^{D}Dap, ^{D}Lys, ^{D}Orn, ^{D}Agp, ^{D}Agb, or ^{D}Arg;
- P¹² is: Ala, Ala(cPr), Ala(tetrahydropyran4yl), Abu, allolle, Cha, Cpa, Cpg, Cyg, Dea, Hle, Ile, Leu, Met, Nle, OctGly, tBuGly, tBuAla, Val, NMeAla, NMeVal, Nva,
Phe, His, Phe(3OH), Phe(4F), Phe(4OCF₃), Trp(6Cl), Tyr(3Cl), Tyr(3F), Tyr(Phenyl), Trp, Tyr, 4Thz, Phe(4(4hydroxyphenoxy)), Phe(4NH₂), Tyr(Me), Ntyr, Nphe,
Agb, Agp, Dab, Dap, Har, Lys, Narg, Ndab, Nlys, Norn, Orn, Arg, NMeLys,
alloThr, Cit, Hgn, Hse, Leu((3R)OH), Asn, Gln, Ser, Thr, Asp, Glu, or Hgl;
- P¹³ is: Pra, Abu(4N₃),
Dab, Dap,
Cys, Hcy, NMeCys, Pen,
Asp, Glu, or Hgl;
- P¹⁴ is: Phe, His, Phe(3OH), Phe(4F), Phe(4OCF₃), Trp(6Cl), Tyr(3Cl), Tyr(3F), Tyr(Phenyl), Trp, Tyr, 4Thz, Phe(4(4hydroxyphenoxy)), Phe(4NH₂), Tyr(Me), Ntyr, Nphe,
Agb, Agp, Dab, Dap, Har, Lys, Narg, Ndab, Nlys, Norn, Orn, Arg, NMeLys,
alloThr, Cit, Hgn, Hse, Leu((3R)OH), Asn, Gln, Ser, Thr, Asp, Glu, or Hgl;
- P¹⁵ is: Ala, Ala(cPr), Ala(tetrahydropyran4yl), Abu, allolle, Cha, Cpa, Cpg, Cyg, Dea, Hle, Ile, Leu, Met, Nle, OctGly, tBuGly, tBuAla, Val, NMeAla, NMeVal, Nva,
Agb, Agp, Dab, Dap, Har, Lys, Narg, Ndab, Nlys, Norn, Orn, Arg, NMeLys,
alloThr, Cit, Hgn, Hse, Leu((3R)OH), Asn, Gln, Ser, Thr, Asp, Glu, or Hgl;
- P¹⁶ is: Ala, Ala(cPr), Ala(tetrahydropyran4yl), Abu, allolle, Cha, Cpa, Cpg, Cyg, Dea, Hle, Ile, Leu, Met, Nle, OctGly, tBuGly, tBuAla, Val, NMeAla, NMeVal, Nva,
Phe, His, Phe(3OH), Phe(4F), Phe(4OCF₃), Trp(6Cl), Tyr(3Cl), Tyr(3F), Tyr(Phenyl), Trp, Tyr, 4Thz, Phe(4(4hydroxyphenoxy)), Phe(4NH₂), Tyr(Me), Ntyr, or Nphe;

or a tautomer, a rotamer, a salt, a hydrate or a solvate thereof;
wherein
Cys, Hcy, NMeCys or Pen at P⁶, if present, and Cys, Hcy, NMeCys or Pen at P¹³, if present, optionally form a disulfide bridge between P⁶ and P¹³; or wherein Dab or Dap at P⁶, if present, and Asp, Glu or Hgl at P¹³, if present, optionally form a lactam bridge between P⁶ and P¹³; or wherein
Asp, Glu or Hgl at P⁶, if present, and Dab or Dap at P¹³, if present, optionally form a lactam bridge between P⁶ and P¹³; or wherein
Pra at P⁶, if present, and Abu(4N₃) at P¹³, if present, optionally form a 1,2,3-triazole bridge between P⁶ and P¹³; or wherein
Abu(4N₃) at P⁶, if present, and Pra at P¹³, if present, optionally form a 1,2,3-triazole bridge between P⁶ and P¹³;
with the proviso that at least two amino acid residues among the three amino acid residues at positions P¹², P¹⁴ and P¹⁵ are basic amino acid residues selected from Agb, Agp, Dab, Dap, Har, Lys, Narg, Ndab, Nlys, Norn, Orn, Arg, or NMeLys.

A more preferred embodiment of the first aspect relates to a compound, wherein
- P¹ is: 2OHVal, Val, NMeVal, Abu, tBuGly;
wherein the amino group of P¹ is optionally replaced by a guanidino group (Gua) or by a tetramethyl guanidino (TMG) group;
- P² is: Pro, Pro(4R)F, Pro(4R)OMe, Pro(4R)OPhe, Pro(3,4dehydro), Pr(4,4F2), Pic;
Agb, Agp, Dab, Dab(iPr), Dap, Dap(iPr), Har, Lys, Lys(iPr), Narg, Ndab, Nlys, Norn, Orn, Orn(iPr), Pro((4R)guanidine), Pro((4R)NH₂), Pro((4S)NH₂), Arg, NMeLys;
Hyp, N*allo*Thr;
- P³ is: Ile;
- P⁴ is: Ala, Ala(cPr), Ala(tetrahydropyran4yl), Abu, allolle, Cha, Cpa, Cpg, Cyg, Dea, Hle, Ile, Leu, Met, Nle, OctGly, tBuGly, tBuAla, Val, NMeAla, NMeVal, Nva;
Phe, His, Phe(3OH), Phe(4F), Phe(4OCF3), Trp(6Cl), Tyr(3Cl), Tyr(3F), Tyr(Phenyl), Trp, Tyr, 4Thz, Phe(4(4hydroxyphenoxy)), Phe(4NH2), Tyr(Me), Ntyr, Nphe;
Agb, Agp, Dab, Dab(iPr), Dap, Dap(iPr), Har, Lys, Lys(iPr), Narg, Ndab, Nlys, Norn, Orn, Orn(iPr), Arg, NMeLys, Lys(Me);
alloThr, Cit, Hgn, Hse, Leu((3R)OH), Asn, Gln, Ser, Thr, Asp, Glu, Hgl, Val(3OH);
- P⁵ is: Phe, His, Trp, Tyr;
- P⁶ is: Dab, Dap;
Cys, Pen;
Asp, Glu;
- P⁷ is: Ala, Ala(cPr), Ala(tetrahydropyran4yl), Abu, allolle, Cha, Cpa, Cpg, Cyg, Dea, Hle, Ile, Leu, Met, Nle, OctGly, tBuGly, tBuAla, Val, NMeAla, NMeVal, Nva;
Phe, His, Phe(3OH), Phe(4F), Phe(4OCF3), Trp(6Cl), Tyr(3Cl), Tyr(3F), Tyr(Phenyl), Trp, Tyr, 4Thz, Phe(4(4hydroxyphenoxy)), Phe(4NH2), Tyr(Me), Ntyr, Nphe;
Agb, Agp, Dab, Dab(iPr), Dap, Dap(iPr), Har, Lys, Lys(iPr), Narg, Ndab, Nlys, Norn, Orn, Orn(iPr), Arg, NMeLys;
alloThr, Cit, Hgn, Hse, Leu((3R)OH), Asn, Gln, Ser, Thr, Asp, Glu, Hgl;
- P⁸ is: Arg;
- P⁹ is: Dab, Dap, Har, Lys, Orn, Arg, Dab(iPr);
Cit;
- P¹⁰ is: alloThr, Hse, Ser, Thr;
- P¹¹ is: ^{D}Dab, ^{D}Dap, ^{D}Lys, ^{D}Orn, ^{D}Arg, ^{D}Dab(iPr);
- P¹² is: Ala, Ala(cPr), Ala(tetrahydropyran4yl), Abu, allolle, Cha, Cpa, Cpg, Cyg, Dea, Hle, Ile, Leu, Met, Nle, OctGly, tBuGly, tBuAla, Val, NMeAla, NMeVal, Nva;
Phe, His, Phe(3OH), Phe(4F), Phe(4OCF3), Trp(6Cl), Tyr(3Cl), Tyr(3F), Tyr(Phenyl), Trp, Tyr, 4Thz, Phe(4(4hydroxyphenoxy)), Phe(4NH2), Tyr(Me), Ntyr, Nphe;
Agb, Agp, Dab, Dab(iPr), Dap, Dap(iPr), Har, Lys, Lys(iPr), Narg, Ndab, Nlys, Norn, Orn, Orn(iPr), Arg, NMeLys;
alloThr, Cit, Hgn, Hse, Leu((3R)OH), Asn, Gln, Ser, Thr, Asp, Glu, Hgl;
- P¹³ is: Dab, Dap;
Cys, Pen;
Asp, Glu;
- P¹⁴ is: Phe, His, Phe(3OH), Phe(4F), Phe(4OCF3), Trp(6Cl), Tyr(3Cl), Tyr(3F), Tyr(Phenyl), Trp, Tyr, 4Thz, Phe(4(4hydroxyphenoxy)), Phe(4NH2), Tyr(Me), Ntyr, Nphe;
Agb, Agp, Dab, Dab(iPr); Dap, Dap(iPr), Har, Lys, Lys(iPr), Narg, Ndab, Nlys, Norn, Orn, Orn(iPr), Arg, NMeLys;
alloThr, Cit, Hgn, Hse, Leu((3R)OH), Asn, Gln, Ser, Thr, Asp, Glu, or Hgl;
- P¹⁵ is: Ala, Ala(cPr), Ala(tetrahydropyran4yl), Abu, allolle, Cha, Cpa, Cpg, Cyg, Dea, Hle, Ile, Leu, Met, Nle, OctGly, tBuGly, tBuAla, Val, NMeAla, NMeVal, Nva;
Agb, Agp, Dab, Dab(iPr), Dap, Dap(iPr), Har, Lys, Lys(iPr), Narg, Ndab, Nlys, Norn, Orn, Orn(iPr), Arg, NMeLys;
alloThr, Cit, Hgn, Hse, Leu((3R)OH), Asn, Gln, Ser, Thr, Asp, Glu, or Hgl;
- P¹⁶ is: Ala, Ala(cPr), Ala(tetrahydropyran4yl), Abu, allolle, Cha, Cpa, Cpg, Cyg, Dea, Hle, Ile, Leu, Met, Nle, OctGly, tBuGly, tBuAla, Val, NMeAla, NMeVal, Nva;
Phe, His, Phe(3OH), Phe(4F), Phe(4OCF3), Trp(6Cl), Tyr(3Cl), Tyr(3F), Tyr(Phenyl), Trp, Tyr, 4Thz, Phe(4(4hydroxyphenoxy)), Phe(4NH2), Tyr(Me), Ntyr, Nphe;

or a tautomer, a rotamer, a salt, a hydrate or a solvate thereof,
wherein
Cys or Pen at P⁶, if present, and Cys or Pen at P¹³, if present, optionally form a disulfide bridge between P⁶ and P¹³; or wherein
Dab or Dap at P⁶, if present, and Asp or Glu at P¹³, if present, optionally form a lactam bridge between P⁶ and P¹³; or wherein
Asp or Glu at P⁶, if present, and Dab or Dap at P¹³, if present, optionally form a lactam bridge between P⁶ and P¹³;
with the proviso that at least three amino acid residues among the four amino acid residues at positions P⁹, P¹², P¹⁴ and P¹⁵ are basic amino acid residues selected from Agb, Agp, Dab, Dab(iPr), Dap, Dap(iPr), Har, Lys, Lys(iPr), Narg, Ndab, Nlys, Norn, Orn, Orn(iPr), Arg, NMeLys.

Another more preferred embodiment of the first aspect relates to a compound, wherein
- P¹ is: 2OHVal, Val, or NMeVal;
wherein the N-terminal amino group of P¹ is optionally replaced by a guanidino group (Gua) or by a tetramethyl guanidino (TMG) group;
- P² is: Pro,
Agb, Agp, Dab, Dap, Har, Lys, Narg, Ndab, Nlys, Norn, Orn, Pro((4R)guanidine), Pro((4R)NH₂), Pro((4S)NH₂), Arg, NMeLys, or
Hyp;
- P³ is: Ile;
- P⁴ is: Ala, Ala(cPr), Ala(tetrahydropyran4yl), Abu, allolle, Cha, Cpa, Cpg, Cyg, Dea, Hle, Ile, Leu, Met, Nle, OctGly, tBuGly, tBuAla, Val, NMeAla, NMeVal, Nva,
Phe, His, Phe(3OH), Phe(4F), Phe(4OCF₃), Trp(6Cl), Tyr(3Cl), Tyr(3F), Tyr(Phenyl), Trp, Tyr, 4Thz, Phe(4(4hydroxyphenoxy)), Phe(4NH₂), Tyr(Me), Ntyr, Nphe,
Agb, Agp, Dab, Dap, Har, Lys, Narg, Ndab, Nlys, Norn, Orn, Arg, NMeLys,
alloThr, Cit, Hgn, Hse, Leu((3R)OH), Asn, Gln, Ser, Thr, Asp, Glu, or Hgl;
- P⁵ is: Phe, His, Trp, or Tyr;
- P⁶ is: Dab, Dap,
Cys, Pen,
Asp, or Glu;
- P⁷ is: Ala, Ala(cPr), Ala(tetrahydropyran4yl), Abu, allolle, Cha, Cpa, Cpg, Cyg, Dea, Hle, Ile, Leu, Met, Nle, OctGly, tBuGly, tBuAla, Val, NMeAla, NMeVal, Nva,
Phe, His, Phe(3OH), Phe(4F), Phe(4OCF₃), Trp(6Cl), Tyr(3Cl), Tyr(3F), Tyr(Phenyl), Trp, Tyr, 4Thz, Phe(4(4hydroxyphenoxy)), Phe(4NH₂), Tyr(Me), Ntyr, Nphe,
Agb, Agp, Dab, Dap, Har, Lys, Narg, Ndab, Nlys, Norn, Orn, Arg, NMeLys,
alloThr, Cit, Hgn, Hse, Leu((3R)OH), Asn, Gln, Ser, Thr, Asp, Glu, or Hgl;
- P⁸ is: Arg;
- P⁹ is: Dab, Dap, Har, Lys, Orn, or Arg;
- P¹⁰ is: alloThr, Hse, Ser, or Thr;
- P¹¹ is: ^{D}Dab, ^{D}Dap, ^{D}Lys, ^{D}Orn, ^{D}Agp, ^{D}Agb, or ^{D}Arg;
- P¹² is: Ala, Ala(cPr), Ala(tetrahydropyran4yl), Abu, allolle, Cha, Cpa, Cpg, Cyg, Dea, Hle, Ile, Leu, Met, Nle, OctGly, tBuGly, tBuAla, Val, NMeAla, NMeVal, Nva,
Phe, His, Phe(3OH), Phe(4F), Phe(4OCF₃), Trp(6Cl), Tyr(3Cl), Tyr(3F), Tyr(Phenyl), Trp, Tyr, 4Thz, Phe(4(4hydroxyphenoxy)), Phe(4NH₂), Tyr(Me), Ntyr, Nphe,
Agb, Agp, Dab, Dap, Har, Lys, Narg, Ndab, Nlys, Norn, Orn, Arg, NMeLys,
alloThr, Cit, Hgn, Hse, Leu((3R)OH), Asn, Gln, Ser, Thr, Asp, Glu, or Hgl;
- P¹³ is: Dab, Dap,
Cys, Pen,
Asp, or Glu;
- P¹⁴ is: Phe, His, Phe(3OH), Phe(4F), Phe(4OCF₃), Trp(6Cl), Tyr(3Cl), Tyr(3F), Tyr(Phenyl), Trp, Tyr, 4Thz, Phe(4(4hydroxyphenoxy)), Phe(4NH₂), Tyr(Me), Ntyr, Nphe,
Agb, Agp, Dab, Dap, Har, Lys, Narg, Ndab, Nlys, Norn, Orn, Arg, NMeLys,
alloThr, Cit, Hgn, Hse, Leu((3R)OH), Asn, Gln, Ser, Thr, Asp, Glu, or Hgl;
- P¹⁵ is: Ala, Ala(cPr), Ala(tetrahydropyran4yl), Abu, allolle, Cha, Cpa, Cpg, Cyg, Dea, Hle, Ile, Leu, Met, Nle, OctGly, tBuGly, tBuAla, Val, NMeAla, NMeVal, Nva,
Agb, Agp, Dab, Dap, Har, Lys, Narg, Ndab, Nlys, Norn, Orn, Arg, NMeLys,
alloThr, Cit, Hgn, Hse, Leu((3R)OH), Asn, Gln, Ser, Thr, Asp, Glu, or Hgl;
- P¹⁶ is: Ala, Ala(cPr), Ala(tetrahydropyran4yl), Abu, allolle, Cha, Cpa, Cpg, Cyg, Dea, Hle, Ile, Leu, Met, Nle, OctGly, tBuGly, tBuAla, Val, NMeAla, NMeVal, Nva,
Phe, His, Phe(3OH), Phe(4F), Phe(4OCF₃), Trp(6Cl), Tyr(3Cl), Tyr(3F), Tyr(Phenyl), Trp, Tyr, 4Thz, Phe(4(4hydroxyphenoxy)), Phe(4NH₂), Tyr(Me), Ntyr, or Nphe;

or a tautomer, a rotamer, a salt, a hydrate or a solvate thereof;
wherein
Cys or Pen at P⁶, if present, and Cys or Pen at P¹³, if present, optionally form a disulfide bridge between P⁶ and P¹³; or wherein
Dab or Dap at P⁶, if present, and Asp or Glu at P¹³, if present, optionally form a lactam bridge between P⁶ and P¹³; or wherein
Asp or Glu at P⁶, if present, and Dab or Dap at P¹³, if present, optionally form a lactam bridge between P⁶ and P¹³;
with the proviso that at least two amino acid residues among the three amino acid residues at positions P¹², P¹⁴ and P¹⁵ are basic amino acid residues selected from Agb, Agp, Dab, Dap, Har, Lys, Narg, Ndab, Nlys, Norn, Orn, Arg, or NMeLys.

A further preferred embodiment of the first aspect relates to a compound, wherein
- P¹ is: 2OHVal, Val, NMeVal, Abu, tBuGly;
wherein the amino group of P¹ is optionally replaced by a guanidino group (Gua) or by a tetramethyl guanidino (TMG) group;
- P² is: Pro, Pro(4R)F, Pro(4R)OMe, Pro(4R)OPhe, Pro(3,4dehydro), Pr(4,4F2), Pic;
Agb, Agp, Dab, Dab(iPr), Dap, Dap(iPr), Lys, Lys(iPr), Narg, Ndab, Nlys, Norn, Orn, Orn(iPr), Pro((4R)NH₂), Arg, NMeLys;
Hyp, N*allo*Thr;
- P³ is: *Hle*, Ile, Leu, Nle;
- P⁴ is: Ala, Ala(cPr), Ala(tetrahydropyran4yl), Abu, allolle, Cyg, Dea, Ile, Leu, Nle, tBuGly, tBuAla, Val, NMeAla, NMeVal, Nva;
Phe, His, Phe(3OH), Phe(4F), Trp(6Cl), Trp, Tyr, 4Thz, Phe(4(4hydroxyphenoxy)), Phe(4NH2), Tyr(Me), Ntyr, Nphe;
Agb, Agp, Dab, Dab(iPr), Dap, Dap(iPr), Lys, Lys(iPr), Narg, Ndab, Nlys, Norn, Orn, Orn(iPr), Arg, NMeLys, Lys(Me);
alloThr, Cit, Hgn, Hse, Leu((3R)OH), Asn, Gln, Ser, Thr, Asp, Glu, Hgl, Val(3OH);
- P⁵ is: Phe, His, Trp, Tyr;
- P⁶ is: Pra, Abu(4N₃);
Dab, Dap;
Cys, Hcy, NMeCys, Pen;
Asp, Glu, Hgl;
- P⁷ is: Ala, Ala(cPr), Ala(tetrahydropyran4yl), Abu, allolle, Cyg, Dea, Ile, Leu, Nle, tBuGly, tBuAla, Val, NMeAla, NMeVal, Nva;
Phe, His, Phe(3OH), Phe(4F), Trp(6Cl), Trp, Tyr, 4Thz, Phe(4(4hydroxyphenoxy)), Phe(4NH2), Tyr(Me), Ntyr, Nphe;
Agb, Agp, Dab, Dab(iPr), Dap, Dap(iPr), Lys, Lys(iPr), Narg, Ndab, Nlys, Norn, Orn, Orn(iPr), Arg, NMeLys;
alloThr, Cit, Hgn, Hse, Leu((3R)OH), Asn, Gln, Ser, Thr, Asp, Glu, Hgl;
- P⁸ is: Agb, Har, Arg;
- P⁹ is: Dab, Dap, Har, Lys, Orn, Arg, Dab(iPr);
Cit;
- P¹⁰ is: alloThr, Hse, Ser, Thr;
- P¹¹ is: ^{D}Dab, ^{D}Dap, ^{D}Lys, ^{D}Orn, ^{D}Arg, ^{D}Dab(iPr);
- P¹² is: Ala, Ala(cPr), Ala(tetrahydropyran4yl), Abu, allolle, Cyg, Dea, Ile, Leu, Nle, tBuGly, tBuAla, Val, NMeAla, NMeVal, Nva;
Phe, His, Phe(3OH), Phe(4F), Trp(6Cl), Tyr(Phenyl), Trp, Tyr, 4Thz, Phe(4(4hydroxyphenoxy)), Phe(4NH2), Tyr(Me), Ntyr, Nphe;
Agb, Agp, Dab, Dab(iPr), Dap, Dap(iPr), Lys, Lys(iPr), Narg, Ndab, Nlys, Norn, Orn, Orn(iPr), Arg, NMeLys;
alloThr, Cit, Hgn, Hse, Leu((3R)OH), Asn, Gln, Ser, Thr, Asp, Glu, Hgl;
- P¹³ is: Pra, Abu(4N₃);
Dab, Dap;
Cys, Hcy, NMeCys, Pen;
Asp, Glu, Hgl;
- p¹⁴ is: Phe, His, Phe(3OH), Phe(4F), Trp(6Cl), Trp, Tyr, 4Thz, Phe(4(4hydroxyphenoxy)), Phe(4NH2), Tyr(Me), Ntyr, Nphe;
Agb, Agp, Dab, Dab(iPr); Dap, Dap(iPr), Lys, Lys(iPr), Narg, Ndab, Nlys, Norn, Orn, Orn(iPr), Arg, NMeLys;
alloThr, Cit, Hgn, Hse, Leu((3R)OH), Asn, Gln, Ser, Thr, Asp, Glu, Hgl;
- P¹⁵ is: Ala, Ala(cPr), Ala(tetrahydropyran4yl), Abu, allolle, Cyg, Dea, Ile, Leu, Nle, tBuGly, tBuAla, Val, NMeAla, NMeVal, Nva;
Agb, Agp, Dab, Dab(iPr), Dap, Dap(iPr), Lys, Lys(iPr), Narg, Ndab, Nlys, Norn, Orn, Orn(iPr), Arg, NMeLys;
alloThr, Cit, Hgn, Hse, Leu((3R)OH), Asn, Gln, Ser, Thr, Asp, Glu, Hgl;
- P¹⁶ is: Ala, Ala(cPr), Ala(tetrahydropyran4yl), Abu, allolle, Cyg, Dea, Ile, Leu, Nle, tBuGly, tBuAla, Val, NMeAla, NMeVal, Nva;
Phe, His, Phe(3OH), Phe(4F), Trp(6Cl), Trp, Tyr, 4Thz, Phe(4(4hydroxyphenoxy)), Phe(4NH2), Tyr(Me), Ntyr, Nphe;

or a tautomer, a rotamer, a salt, a hydrate or a solvate thereof,
wherein
Cys, Hcy, NMeCys or Pen at P⁶, if present, and Cys, Hcy, NMeCys or Pen at P¹³, if present, optionally form a disulfide bridge between P⁶ and P¹³; or wherein
Dab or Dap at P⁶, if present, and Asp, Glu or Hgl at P¹³, if present, optionally form a lactam bridge between P⁶ and P¹³; or wherein
Asp, Glu or Hgl at P⁶, if present, and Dab or Dap at P¹³, if present, optionally form a lactam bridge between P⁶ and P¹³; or wherein
Pra at P⁶, if present, and Abu(4N₃) at P¹³, if present, optionally form a 1,2,3-triazole bridge between P⁶ and P¹³; or wherein
Abu(4N₃) at P⁶, if present, and Pra at P¹³, if present, optionally form a 1,2,3-triazole bridge between P⁶ and P¹³;
with the proviso that at least three amino acid residues among the four amino acid residues at positions P⁹, P¹², P¹⁴ and P¹⁵ are basic amino acid residues selected from Agb, Agp, Dab, Dab(iPr), Dap, Dap(iPr), Lys, Lys(iPr), Narg, Ndab, Nlys, Norn, Orn, Orn(iPr), Arg, NMeLys.

A further preferred embodiment of
the first aspect relates to a compound, wherein
   - P¹ is: 2OHVal, Val, or NMeVal;
   wherein the N-terminal amino group of P¹ is optionally replaced by a guanidino group (Gua) or by a tetramethyl guanidino (TMG) group;
   - P² is: Pro,
   Agb, Agp, Dab, Dap, Lys, Narg, Ndab, Nlys, Norn, Orn, Pro((4R)NH₂), Arg, NMeLys, or
   Hyp;
   - P³ is: *Hle,* Ile, Leu, or Nle;
   - P⁴ is: Ala, Ala(cPr), Ala(tetrahydropyran4yl), Abu, allolle, Cyg, Dea, Ile, Leu, Nle, tBuGly, tBuAla, Val, NMeAla, NMeVal, Nva,
   Phe, His, Phe(3OH), Phe(4F), Trp(6Cl), Trp, Tyr, 4Thz, Phe(4(4hydroxyphenoxy)), Phe(4NH₂), Tyr(Me), Ntyr, Nphe,
   Agb, Agp, Dab, Dap, Lys, Narg, Ndab, Nlys, Norn, Orn, Arg, NMeLys,
   alloThr, Cit, Hgn, Hse, Leu((3R)OH), Asn, Gln, Ser, Thr, Asp, Glu, or Hgl;
   - P⁵ is: Phe, His, Trp, or Tyr;
   - P⁶ is: Pra, Abu(4N₃),
   Dab, Dap,
   Cys, Hcy, NMeCys, Pen,
   Asp, Glu, or Hgl;
   - P⁷ is: Ala, Ala(cPr), Ala(tetrahydropyran4yl), Abu, allolle, Cyg, Dea, Ile, Leu, Nle, tBuGly, tBuAla, Val, NMeAla, NMeVal, Nva,
   Phe, His, Phe(3OH), Phe(4F), Trp(6Cl), Trp, Tyr, 4Thz, Phe(4(4hydroxyphenoxy)), Phe(4NH₂), Tyr(Me), Ntyr, Nphe,
   Agb, Agp, Dab, Dap, Lys, Narg, Ndab, Nlys, Norn, Orn, Arg, NMeLys,
   alloThr, Cit, Hgn, Hse, Leu((3R)OH), Asn, Gln, Ser, Thr, Asp, Glu, or Hgl;
   - P⁸ is: Har, or Arg;
   - P⁹ is: Dab, Dap, Har, Lys, Orn, or Arg;
   - P¹⁰ is: alloThr, Hse, Ser, or Thr;
   - P¹¹ is: ^{D}Dab, ^{D}Dap, ^{D}Lys, ^{D}Orn, or ^{D}Arg;
   - P¹² is: Ala, Ala(cPr), Ala(tetrahydropyran4yl), Abu, allolle, Cyg, Dea, Ile, Leu, Nle, tBuGly, tBuAla, Val, NMeAla, NMeVal, Nva,
   Phe, His, Phe(3OH), Phe(4F), Trp(6Cl), Tyr(Phenyl), Trp, Tyr, 4Thz, Phe(4(4hydroxyphenoxy)), Phe(4NH₂), Tyr(Me), Ntyr, Nphe,
   Agb, Agp, Dab, Dap, Lys, Narg, Ndab, Nlys, Norn, Orn, Arg, NMeLys,
   alloThr, Cit, Hgn, Hse, Leu((3R)OH), Asn, Gln, Ser, Thr, Asp, Glu, or Hgl;
   - P¹³ is: Pra, Abu(4N₃),
   Dab, Dap,
   Cys, Hcy, NMeCys, Pen,
   Asp, Glu, or Hgl;
   - p¹⁴ is: Phe, His, Phe(3OH), Phe(4F), Trp(6Cl), Trp, Tyr, 4Thz, Phe(4(4hydroxyphenoxy)), Phe(4NH₂), Tyr(Me), Ntyr, Nphe,
   Agb, Agp, Dab, Dap, Lys, Narg, Ndab, Nlys, Norn, Orn, Arg, NMeLys,
   alloThr, Cit, Hgn, Hse, Leu((3R)OH), Asn, Gln, Ser, Thr, Asp, Glu,or Hgl;
   - P¹⁵ is: Ala, Ala(cPr), Ala(tetrahydropyran4yl), Abu, allolle, Cyg, Dea, Ile, Leu, Nle, tBuGly, tBuAla, Val, NMeAla, NMeVal, Nva,
   Agb, Agp, Dab, Dap, Lys, Narg, Ndab, Nlys, Norn, Orn, Arg, NMeLys,
   alloThr, Cit, Hgn, Hse, Leu((3R)OH), Asn, Gln, Ser, Thr, Asp, Glu, or Hgl;
   - P¹⁶ is: Ala, Ala(cPr), Ala(tetrahydropyran4yl), Abu, allolle, Cyg, Dea, Ile, Leu, Nle, tBuGly, tBuAla, Val, NMeAla, NMeVal, Nva,
   Phe, His, Phe(3OH), Phe(4F), Trp(6Cl), Trp, Tyr, 4Thz, Phe(4(4hydroxyphenoxy)), Phe(4NH₂), Tyr(Me), Ntyr, or Nphe;
or a tautomer, a rotamer, a salt, a hydrate or a solvate thereof;
wherein
Cys, Hcy, NMeCys or Pen at P⁶, if present, and Cys, Hcy, NMeCys or Pen at P¹³, if present, optionally form a disulfide bridge between P⁶ and P¹³; or wherein
Dab or Dap at P⁶, if present, and Asp, Glu or Hgl at P¹³, if present, optionally form a lactam bridge between P⁶ and P¹³; or wherein
Asp, Glu or Hgl at P⁶, if present, and Dab or Dap at P¹³, if present, optionally form a lactam bridge between P⁶ and P¹³; or wherein
Pra at P⁶, if present, and Abu(4N₃) at P¹³, if present, optionally form a 1,2,3-triazole bridge between P⁶ and P¹³; or wherein
Abu(4N₃) at P⁶, if present, and Pra at P¹³, if present, optionally form a 1,2,3-triazole bridge between P⁶ and P¹³;
with the proviso that at least two amino acid residues among the three amino acid residues at positions P¹², P¹⁴ and P¹⁵ are basic amino acid residues selected from Agb, Agp, Dab, Dap, Lys, Narg, Ndab, Nlys, Norn, Orn, Arg, or NMeLys.

A more preferred embodiment of
the first aspect relates to a compound, wherein
   - P¹ is: 2OHVal, Val, NMeVal, Abu, tBuGly;
   wherein the amino group of P¹ is optionally replaced by a guanidino group (Gua) or by a tetramethyl guanidino (TMG) group;
   - P² is: Pro, Pro(4R)F, Pro(4R)OMe, Pro(4R)OPhe, Pro(3,4dehydro), Pr(4,4F2), Pic;
   Agb, Agp, Dab, Dab(iPr), Dap, Dap(iPr), Lys, Lys(iPr), Narg, Ndab, Nlys, Norn, Orn, Orn(iPr), Pro((4R)NH₂), Arg, NMeLys;
   Hyp, N*allo*Thr;
   - P³ is: Ile;
   - P⁴ is: Ala, Ala(cPr), Ala(tetrahydropyran4yl), Abu, allolle, Cyg, Dea, Ile, Leu, Nle, tBuGly, tBuAla, Val, NMeAla, NMeVal, Nva;
   Phe, His, Phe(3OH), Phe(4F), Trp(6Cl), Trp, Tyr, 4Thz, Phe(4(4hydroxyphenoxy)), Phe(4NH2), Tyr(Me), Ntyr, Nphe;
   Agb, Agp, Dab, Dab(iPr), Dap, Dap(iPr), Lys, Lys(iPr), Narg, Ndab, Nlys, Norn, Orn, Orn(iPr), Arg, NMeLys; Lys(Me);
   alloThr, Cit, Hgn, Hse, Leu((3R)OH), Asn, Gln, Ser, Thr, Asp, Glu, Hgl, Val(3OH);
   - P⁵ is: Phe, His, Trp, Tyr;
   - P⁶ is: Dab, Dap;
   Cys, Pen;
   Asp, Glu;
   - P⁷ is: Ala, Ala(cPr), Ala(tetrahydropyran4yl), Abu, allolle, Cyg, Dea, Ile, Leu, Nle, tBuGly, tBuAla, Val, NMeAla, NMeVal, Nva;
   Phe, His, Phe(3OH), Phe(4F), Trp(6Cl), Trp, Tyr, 4Thz, Phe(4(4hydroxyphenoxy)), Phe(4NH2), Tyr(Me), Ntyr, Nphe;
   Agb, Agp, Dab, Dab(iPr), Dap, Dap(iPr), Lys, Lys(iPr), Narg, Ndab, Nlys, Norn, Orn, Orn(iPr), Arg, NMeLys;
   alloThr, Cit, Hgn, Hse, Leu((3R)OH), Asn, Gln, Ser, Thr, Asp, Glu, Hgl;
   - P⁸ is: Arg;
   - P⁹ is: Dab, Dap, Har, Lys, Orn, Arg, Dab(iPr);
   Cit;
   - P¹⁰ is: alloThr, Hse, Ser, Thr;
   - P¹¹ is: ^{D}Dab, ^{D}Dap, ^{D}Lys, ^{D}Orn, ^{D}Arg, ^{D}Dab(iPr);
   - P¹² is: Ala, Ala(cPr), Ala(tetrahydropyran4yl), Abu, allolle, Cyg, Dea, Ile, Leu, Nle, tBuGly, tBuAla, Val, NMeAla, NMeVal, Nva;
   Phe, His, Phe(3OH), Phe(4F), Trp(6Cl), Tyr(Phenyl), Trp, Tyr, 4Thz, Phe(4(4hydroxyphenoxy)), Phe(4NH2), Tyr(Me), Ntyr, Nphe;
   Agb, Agp, Dab, Dab(iPr), Dap, Dap(iPr), Lys, Lys(iPr), Narg, Ndab, Nlys, Norn, Orn, Orn(iPr), Arg, NMeLys;
   alloThr, Cit, Hgn, Hse, Leu((3R)OH), Asn, Gln, Ser, Thr, Asp, Glu, Hgl;
   - P¹³ is: Dab, Dap;
   Cys, Pen;
   Asp, Glu;
   - p¹⁴ is: Phe, His, Phe(3OH), Phe(4F), Trp(6Cl), Trp, Tyr, 4Thz, Phe(4(4hydroxyphenoxy)), Phe(4NH2), Tyr(Me), Ntyr, Nphe;
   Agb, Agp, Dab, Dab(iPr); Dap, Dap(iPr), Lys, Lys(iPr), Narg, Ndab, Nlys, Norn, Orn, Orn(iPr), Arg, NMeLys;
   alloThr, Cit, Hgn, Hse, Leu((3R)OH), Asn, Gln, Ser, Thr, Asp, Glu, or Hgl;
   - P¹⁵ is: Ala, Ala(cPr), Ala(tetrahydropyran4yl), Abu, allolle, Cyg, Dea, Ile, Leu, Nle, tBuGly, tBuAla, Val, NMeAla, NMeVal, Nva;
   Agb, Agp, Dab, Dab(iPr), Dap, Dap(iPr), Lys, Lys(iPr), Narg, Ndab, Nlys, Norn, Orn, Orn(iPr), Arg, NMeLys;
   alloThr, Cit, Hgn, Hse, Leu((3R)OH), Asn, Gln, Ser, Thr, Asp, Glu, Hgl;
   - P¹⁶ is: Ala, Ala(cPr), Ala(tetrahydropyran4yl), Abu, allolle, Cyg, Dea, Ile, Leu, Nle, tBuGly, tBuAla, Val, NMeAla, NMeVal, Nva;
   Phe, His, Phe(3OH), Phe(4F), Trp(6Cl), Trp, Tyr, 4Thz, Phe(4(4hydroxyphenoxy)), Phe(4NH2), Tyr(Me), Ntyr, Nphe;
or a tautomer, a rotamer, a salt, a hydrate or a solvate thereof,
wherein
Cys or Pen at P⁶, if present, and Cys or Pen at P¹³, if present, optionally form a disulfide bridge between P⁶ and P¹³; or wherein
Dab or Dap at P⁶, if present, and Asp or Glu at P¹³, if present, optionally form a lactam bridge between P⁶ and P¹³; or wherein
Asp or Glu at P⁶, if present, and Dab or Dap at P¹³, if present, optionally form a lactam bridge between P⁶ and P¹³;
with the proviso that at least three amino acid residues among the four amino acid residues at positions P⁹, P¹², P¹⁴ and P¹⁵ are basic amino acid residues selected from Agb, Agp, Dab, Dab(iPr), Dap, Dap(iPr), Lys, Lys(iPr), Narg, Ndab, Nlys, Norn, Orn, Orn(iPr), Arg, NMeLys.

A further more preferred embodiment of
the first aspect relates to a compound, wherein
   - P¹ is: 2OHVal, Val, or NMeVal;
   wherein the N-terminal amino group of P¹ is optionally replaced by a guanidino group (Gua) or by a tetramethyl guanidino (TMG) group;
   - P² is: Pro,
   Agb, Agp, Dab, Dap, Lys, Narg, Ndab, Nlys, Norn, Orn, Pro((4R)NH₂), Arg, NMeLys, or
   Hyp;
   - P³ is: Ile;
   - P⁴ is: Ala, Ala(cPr), Ala(tetrahydropyran4yl), Abu, allolle, Cyg, Dea, Ile, Leu, Nle, tBuGly, tBuAla, Val, NMeAla, NMeVal, Nva
   Phe, His, Phe(3OH), Phe(4F), Trp(6Cl), Trp, Tyr, 4Thz, Phe(4(4hydroxyphenoxy)), Phe(4NH₂), Tyr(Me), Ntyr, Nphe,
   Agb, Agp, Dab, Dap, Lys, Narg, Ndab, Nlys, Norn, Orn, Arg, NMeLys,
   alloThr, Cit, Hgn, Hse, Leu((3R)OH), Asn, Gln, Ser, Thr, Asp, Glu, or Hgl;
   - P⁵ is: Phe, His, Trp, or Tyr;
   - P⁶ is: Dab, Dap,
   Cys, Pen,
   Asp, or Glu;
   - P⁷ is: Ala, Ala(cPr), Ala(tetrahydropyran4yl), Abu, allolle, Cyg, Dea, Ile, Leu, Nle, tBuGly, tBuAla, Val, NMeAla, NMeVal, Nva,
   Phe, His, Phe(3OH), Phe(4F), Trp(6Cl), Trp, Tyr, 4Thz, Phe(4(4hydroxyphenoxy)), Phe(4NH₂), Tyr(Me), Ntyr, Nphe,
   Agb, Agp, Dab, Dap, Lys, Narg, Ndab, Nlys, Norn, Orn, Arg, NMeLys,
   alloThr, Cit, Hgn, Hse, Leu((3R)OH), Asn, Gln, Ser, Thr, Asp, Glu, or Hgl;
   - P⁸ is: Arg;
   - P⁹ is: Dab, Dap, Har, Lys, Orn, or Arg;
   - P¹⁰ is: alloThr, Hse, Ser, or Thr;
   - P¹¹ is: ^{D}Dab, ^{D}Dap, ^{D}Lys, ^{D}Orn, or ^{D}Arg;
   - P¹² is: Ala, Ala(cPr), Ala(tetrahydropyran4yl), Abu, allolle, Cyg, Dea, Ile, Leu, Nle, tBuGly, tBuAla, Val, NMeAla, NMeVal, Nva,
   Phe, His, Phe(3OH), Phe(4F), Trp(6Cl), Tyr(Phenyl), Trp, Tyr, 4Thz, Phe(4(4hydroxyphenoxy)), Phe(4NH₂), Tyr(Me), Ntyr, Nphe,
   Agb, Agp, Dab, Dap, Lys, Narg, Ndab, Nlys, Norn, Orn, Arg, NMeLys,
   alloThr, Cit, Hgn, Hse, Leu((3R)OH), Asn, Gln, Ser, Thr, Asp, Glu, or Hgl;
   - P¹³ is: Dab, Dap,
   Cys, Pen,
   Asp, or Glu;
   - p¹⁴ is: Phe, His, Phe(3OH), Phe(4F), Trp(6Cl), Trp, Tyr, 4Thz, Phe(4(4hydroxyphenoxy)), Phe(4NH₂), Tyr(Me), Ntyr, Nphe,
   Agb, Agp, Dab, Dap, Lys, Narg, Ndab, Nlys, Norn, Orn, Arg, NMeLys, alloThr, Cit, Hgn, Hse, Leu((3R)OH), Asn, Gln, Ser, Thr, Asp, Glu, or Hgl;
   - P¹⁵ is: Ala, Ala(cPr), Ala(tetrahydropyran4yl), Abu, allolle, Cyg, Dea, Ile, Leu, Nle, tBuGly, tBuAla, Val, NMeAla, NMeVal, Nva,
   Agb, Agp, Dab, Dap, Lys, Narg, Ndab, Nlys, Norn, Orn, Arg, NMeLys,
   alloThr, Cit, Hgn, Hse, Leu((3R)OH), Asn, Gln, Ser, Thr, Asp, Glu, or Hgl;
   - P¹⁶ is: Ala, Ala(cPr), Ala(tetrahydropyran4yl), Abu, allolle, Cyg, Dea, Ile, Leu, Nle, tBuGly, tBuAla, Val, NMeAla, NMeVal, Nva,
   Phe, His, Phe(3OH), Phe(4F), Trp(6Cl), Trp, Tyr, 4Thz, Phe(4(4hydroxyphenoxy)), Phe(4NH₂), Tyr(Me), Ntyr, or Nphe;
or a tautomer, a rotamer, a salt, a hydrate or a solvate thereof;
wherein
Cys or Pen at P⁶, if present, and Cys or Pen at P¹³, if present, optionally form a disulfide bridge between P⁶ and P¹³; or wherein
Dab or Dap at P⁶, if present, and Asp or Glu at P¹³, if present, optionally form a lactam bridge between P⁶ and P¹³; or wherein
Asp or Glu at P⁶, if present, and Dab or Dap at P¹³, if present, optionally form a lactam bridge between P⁶ and P¹³;
with the proviso that at least two amino acid residues among the three amino acid residues at positions P¹², P¹⁴ and P¹⁵ are basic amino acid residues selected from Agb, Agp, Dab, Dap, Lys, Narg, Ndab, Nlys, Norn, Orn, Arg, or NMeLys.

A particular embodiment of the first aspect relates to a compound, wherein
- P¹ is: Val, 2OHVal, NMeVal, Gua-Val, TMG-Val, Abu, or tBuGly;
- P² is: Pro, Pro(4R)F, Pro(4R)OMe, Pro(4R)OPhe, Pro(3,4dehydro), Pr(4,4F2), Pic, Pro((4R)NH₂), Ndab, N*allo*Thr, or Hyp;
- P³ is: Ile;
- P⁴ is: Ile, Thr, Phe, Dab, Orn, Arg, Tyr, Leu, Asn, Lys, Lys(Me), Dap, Val(3OH), or alloThr;
- P⁵ is: Trp or Tyr;
- P⁶ is: Cys, Pen, Asp, or Pra;
- P⁷ is: Asn, Ala, Leu, Ile, Ser, Thr, Lys, Dap, Glu, or His;
- P⁸ is: Arg;
- P⁹ is: Arg, Dab, Dab(iPr), Lys, or Cit;
- P¹⁰ is: Ser or Thr;
- P¹¹ is: ^{D}Dab, ^{D}Dap, ^{D}Orn, ^{D}Lys, ^{D}Arg, or ^{D}Dab(iPr);
- P¹² is: Lys, Ile, Ser, Tyr, Trp, Asn, Dab, Orn, or Cit;
- P¹³ is: Cys, Pen, Dab, or Abu(4N₃);
- P¹⁴ is: Dab, Dab(iPr), *Lys,* Gln, *Ser,* or Tyr;
- P¹⁵ is: Arg, Thr, Leu, Ser, Dab, Lys, Orn, or Orn(iPr); and
- P¹⁶ is: Nle, Cha, or Tyr;

or a tautomer, a rotamer, a salt, a hydrate or a solvate thereof,
wherein Cys or Pen at P⁶, if present, and Cys or Pen at P¹³, if present, optionally form a disulfide bridge between P⁶ and P¹³, or wherein
Asp at P⁶, if present, and Dab at P¹³, if present, optionally form a lactam bridge between P⁶ and P¹³, or wherein
Pra at P⁶, if present, and Abu(4N₃) at P¹³, if present, optionally form a 1,2,3-triazole bridge between P⁶ and P¹³;
with the proviso that at least three amino acid residues among the four amino acid residues at positions P⁹, P¹², P¹⁴ and P¹⁵ are basic amino acid residues selected from Dab, Lys, Arg, or Dab(iPr) at P⁹, Lys, Dab, or Orn at P¹², Dab, Dab(iPr), or *Lys* at P¹⁴ and Arg, Dab, Lys, Orn or Orn(iPr) at P¹⁵.

Another particular embodiment of the first aspect relates to a compound, wherein
- P¹ is: Val, 2OHVal, NMeVal, Gua-Val, or TMG-Val;
- P² is: Pro, Pro((4R)NH₂), Ndab, or Hyp;
- P³ is: Ile;
- P⁴ is: Ile, Thr, Phe, Dab, Arg, Tyr, Leu, Asn, Lys, Dap, or alloThr;
- P⁵ is: Trp or Tyr;
- P⁶ is: Cys, Pen, Asp, or Pra;
- P⁷ is: Asn, Ala, Leu, Ile, Ser, Thr, Lys, Dap, Glu, or His;
- P⁸ is: Arg;
- P⁹ is: Arg, Dab, or Lys;
- P¹⁰ is: Ser or Thr;
- P¹¹ is: ^{D}Dab, ^{D}Dap, ^{D}Orn, ^{D}Lys, or ^{D}Arg;
- P¹² is: Lys, Ile, Ser, Tyr, Trp, Asn, Dab, or Cit;
- P¹³ is: Cys, Pen, Dab, or Abu(4N₃);
- P¹⁴ is: Dab, Gln, or Tyr;
- P¹⁵ is: Arg, Thr, Leu, Ser, Dab, Lys, or Orn; and
- P¹⁶ is: Nle, Cha, or Tyr;

or a tautomer, a rotamer, a salt, a hydrate or a solvate thereof;
wherein Cys or Pen at P⁶, if present, and Cys or Pen at P¹³, if present, optionally form a disulfide bridge between P⁶ and P¹³; or wherein
Asp at P⁶, if present, and Dab at P¹³, if present, optionally form a lactam bridge between P⁶ and P¹³; or wherein
Pra at P⁶, if present, and Abu(4N₃) at P¹³, if present, optionally form a 1,2,3-triazole bridge between P⁶ and P¹³;
with the proviso that at least two amino acid residues among the three amino acid residues at positions P¹², P¹⁴ and P¹⁵ are basic amino acid residues selected from Lys or Dab at P¹², Dab at P¹⁴ and Arg, Dab, Lys, or Orn at P¹⁵,

A particularly preferred embodiment of the first aspect relates to a compound, wherein
- P¹ is: Val, NMeVal, Gua-Val, TMG-Val, or Abu;
- P² is: Pro, Pro(4R)OMe, Pro(3,4dehydro), Pic, Pro((4R)NH₂), Ndab, N*allo*Thr, or Hyp;
- P³ is: Ile;
- P⁴ is: Ile, Thr, Phe, Dab, Arg, Val(3OH), or Tyr;
- P⁵ is: Tyr;
- P⁶ is: Cys, Pen, or Asp;
- P⁷ is: Asn, Leu, Ile, Ser, Dap, or His;
- P⁸ is: Arg;
- P⁹ is: Arg, Lys, Dab, or Dab(iPr);
- P¹⁰ is: Ser or Thr;
- P¹¹ is: ^{D}Dab, ^{D}Dab(iPr), or ^{D}Arg;
- P¹² is: Lys, Ile, Ser, Dab, Orn, or *Cit*;
- P¹³ is: Cys, Pen, or Dab;
- P¹⁴ is: Dab, Dab(iPr), *Lys,* Gln, *Ser,* or Tyr;
- P¹⁵ is: Arg, Dab, Orn, Orn(iPr), Ser, or Thr;
- P¹⁶ is: Nle, Cha, or Tyr;

or a tautomer, a rotamer, a salt, a hydrate or a solvate thereof,
wherein Cys or Pen at P⁶, if present, and Cys or Pen at P¹³, if present, optionally form a disulfide bridge between P⁶ and P¹³, or wherein
Asp at P⁶, if present, and Dab at P¹³, if present, optionally form a lactam bridge between P⁶ and P¹³;
with the proviso that at least three amino acid residues among the four amino acid residues at positions P⁹, P¹², P¹⁴ and P¹⁵ are basic amino acid residues selected from Dab, Lys, Arg, or Dab(iPr) at P⁹, Lys, Orn, or Dab at P¹², Lys, Dab or Dab(iPr) at P¹⁴ and Arg, Dab, Orn, or Orn(iPr) at P¹⁵.

Another particularly preferred embodiment of the first aspect relates to a compound, wherein
- P¹ is: Val, NMeVal, Gua-Val, or TMG-Val;
- P² is: Pro, Pro((4R)NH₂), Ndab, or Hyp;
- P³ is: Ile;
- P⁴ is: Ile, Thr, Phe, Arg, or Tyr;
- P⁵ is: Tyr;
- P⁶ is: Cys, Pen, or Asp;
- P⁷ is: Asn, Leu, Ser, Dap, or His;
- P⁸ is: Arg;
- P⁹ is: Arg, or Dab;
- P¹⁰ is: Ser or Thr;
- P¹¹ is: ^{D}Dab, or ^{D}Arg;
- P¹² is: Lys, or Dab;
- P¹³ is: Cys, Pen, or Dab;
- P¹⁴ is: Dab, Gln, or Tyr;
- P¹⁵ is: Arg, Dab, or Orn;
- P¹⁶ is: Nle, Cha, or Tyr;

or a tautomer, a rotamer, a salt, a hydrate or a solvate thereof;
wherein Cys or Pen at P⁶, if present, and Cys or Pen at P¹³, if present, optionally form a disulfide bridge between P⁶ and P¹³; or wherein
Asp at P⁶, if present, and Dab at P¹³, if present, optionally form a lactam bridge between P⁶ and P¹³;
with the proviso that at least two amino acid residues among the three amino acid residues at positions P¹², P¹⁴ and P¹⁵ are basic amino acid residues selected from Lys or Dab at P¹², Dab at P¹⁴ and Arg, Dab, or Orn at P¹⁵.

Another particularly preferred embodiment of the first aspect relates to a compound, wherein
- P¹ is: 2OHVal, Gua-Val, or TMG-Val;
- P² is: Pro, Pro((4R)NH₂), or Hyp;
- P³ is: Ile;
- P⁴ is: Ile, Thr, Dab, or Orn;
- P⁵ is: Tyr;
- P⁶ is: Cys, Pen, Asp or Pra;
- P⁷ is: Ile, Asn, or Thr;
- P⁸ is: Arg;
- P⁹ is: Lys, Arg, Dab, or Dab(iPr);
- P¹⁰ is: Ser, or Thr;
- P¹¹ is: ^{D}Dab, ^{D}Dab(iPr), or ^{D}Dap;
- P¹² is: Lys, Ile, Asn, Ser, Tyr, Orn, or Cit;
- P¹³ is: Cys, Dab, or Abu(4N₃);
- P¹⁴ is: Dab, Dab(iPr), Lys, or Ser;
- P¹⁵ is: Arg, Dab, Orn; Orn(iPr), Ser, or Thr;
- P¹⁶ is: Nle, or Tyr;

or a tautomer, a rotamer, a salt, a hydrate or a solvate thereof,
wherein Cys or Pen at P⁶, if present, and Cys at P¹³, if present, optionally form a disulfide bridge between P⁶ and P¹³, or wherein
Asp at P⁶, if present, and Dab at P¹³, if present, optionally form a lactam bridge between P⁶ and P¹³; or wherein
Pra at P⁶, if present, and Abu(4N₃) at P¹³, if present, optionally form a 1,2,3-triazole bridge between P⁶ and P¹³;
with the proviso that at least three amino acid residues among the four amino acid residues at positions P⁹, P¹², P¹⁴ and P¹⁵ are basic amino acid residues selected from Dab, Lys, Arg, or Dab(iPr) at P⁹, Lys, or Orn at P¹², Lys, Dab or Dab(iPr) at P¹⁴ and Arg, Dab, Orn, or Orn(iPr) at P¹⁵.

Still another particularly preferred embodiment of the first aspect relates to a compound, wherein
- P¹ is: 2OHVal, Gua-Val, or TMG-Val;
- P² is: Pro, or Hyp;
- P³ is: Ile;
- P⁴ is: Ile, Thr, or Dab;
- P⁵ is: Tyr;
- P⁶ is: Cys, Pen, Asp or Pra;
- P⁷ is: Asn, or Thr;
- P⁸ is: Arg;
- P⁹ is: Lys, Arg, or Dab;
- P¹⁰ is: Thr;
- P¹¹ is: ^{D}Dab, or ^{D}Dap;
- P¹² is: Lys, Ile, Asn, Ser, or Tyr;
- P¹³ is: Cys, Dab, or Abu(4N₃);
- P¹⁴ is: Dab;
- P¹⁵ is: Arg, Dab, Orn; Ser, or Thr;
- P¹⁶ is: Nle, or Tyr;

or a tautomer, a rotamer, a salt, a hydrate or a solvate thereof;
wherein Cys or Pen at P⁶, if present, and Cys at P¹³, if present, optionally form a disulfide bridge between P⁶ and P¹³; or wherein
Asp at P⁶, if present, and Dab at P¹³, if present, optionally form a lactam bridge between P⁶ and P¹³; or wherein
Pra at P⁶, if present, and Abu(4N₃) at P¹³, if present, optionally form a 1,2,3-triazole bridge between P⁶ and P¹³;
with the proviso that at least two amino acid residues among the three amino acid residues at positions P¹², P¹⁴ and P¹⁵ are basic amino acid residues selected from Lys at P12, Dab at P¹⁴ and Arg, Dab, or Orn at P¹⁵.

A more particularly preferred embodiment of the first aspect relates to a compound, wherein the compound is selected from the group consisting of
Val-Pro-Ile-Ile-Tyr-Cys-Asn-Arg-Arg-Thr-^{D}Dab-Lys-Cys-Dab-Arg-Nle;
Val-Pro((4R)NH₂)-Ile-Ile-Tyr-Pen-Asn-Arg-Arg-Thr-^{D}Dab-Lys-Cys-Dab-Arg-Nle;
Val-Pro((4R)NH₂)-Ile-Ile-Tyr-Pen-Asn-Arg-Dab-Thr-^{D}Dab-Lys-Cys-Dab-Arg-Nle;
Val-Ndab-Ile-Ile-Tyr-Pen-Asn-Arg-Dab-Thr-^{D}Dab-Lys-Cys-Dab-Arg-Nle;
Val-Pro((4R)NH₂)-Ile-Thr-Tyr-Pen-Asn-Arg-Dab-Thr-^{D}Dab-Lys-Cys-Dab-Arg-Nle;
Val-Pro-Ile-Ile-Tyr-Cys-Asn-Arg-Dab-Thr-^{D}Dab-Lys-Cys-Dab-Arg-Cha;
Val-Pro-Ile-Ile-Tyr-Pen-Asn-Arg-Dab-Thr-^{D}Dab-Lys-Cys-Dab-Arg-Tyr;
Val-Pro-Ile-Ile-Tyr-Cys-Asn-Arg-Dab-Thr-^{D}Dab-Lys-Pen-Dab-Arg-Tyr;
Val-Pro((4R)NH₂)-Ile-Ile-Tyr-Cys-Asn-Arg-Dab-Thr-^{D}Dab-Lys-Cys-Dab-Arg-Tyr;
Val-Hyp-Ile-Ile-Tyr-Cys-Asn-Arg-Dab-Thr-^{D}Dab-Lys-Cys-Dab-Arg-Tyr;
Val-Pro-Ile-Thr-Tyr-Cys-Asn-Arg-Dab-Thr-^{D}Dab-Lys-Cys-Dab-Arg-Tyr;
Val-Pro-Ile-Phe-Tyr-Cys-Asn-Arg-Dab-Thr-^{D}Dab-Lys-Cys-Dab-Arg-Tyr;
2OHVal-Pro-Ile-Ile-Tyr-Cys-Asn-Arg-Dab-Thr-^{D}Dab-Lys-Cys-Dab-Arg-Tyr;
Val-Hyp-Ile-Ile-Tyr-Pen-Asn-Arg-Dab-Thr-^{D}Dab-Lys-Cys-Dab-Arg-Tyr;
Val-Hyp-Ile-Thr-Tyr-Pen-Asn-Arg-Dab-Thr-^{D}Dab-Lys-Cys-Dab-Arg-Tyr;
Val-Hyp-Ile-Thr-Tyr-Pen-Asn-Arg-Dab-Thr-^{D}Dap-Lys-Cys-Dab-Arg-Tyr;
Val-Hyp-Ile-Thr-Tyr-Pen-Asn-Arg-Dab-Thr-^{D}Orn-Lys-Cys-Dab-Arg-Tyr;
Val-Hyp-Ile-Thr-Tyr-Pen-Asn-Arg-Dab-Thr-^{D}Lys-Lys-Cys-Dab-Arg-Tyr;
Va I-Hyp-I le-Th r-Tyr-Pe n-Asn-Arg-Da b-Th r-Arg-Lys-Cys-Da b-Arg-Tyr;
Val-Hyp-Ile-Dab-Tyr-Pen-Asn-Arg-Dab-Thr-^{D}Dab-Lys-Cys-Dab-Thr-Tyr;
Val-Hyp-Ile-Arg-Tyr-Pen-Asn-Arg-Dab-Thr-^{D}Dab-Lys-Cys-Dab-Thr-Tyr;
Val-Hyp-Ile-Tyr-Tyr-Pen-Asn-Arg-Dab-Thr-^{D}Dab-Lys-Cys-Dab-Arg-Tyr;
Val-Hyp-Ile-Leu-Tyr-Pen-Asn-Arg-Dab-Thr-^{D}Dab-Lys-Cys-Dab-Arg-Tyr;
Val-Hyp-Ile-Asn-Tyr-Pen-Asn-Arg-Dab-Thr-^{D}Dab-Lys-Cys-Dab-Arg-Tyr;
Val-Hyp-Ile-Dab-Tyr-Pen-Asn-Arg-Dab-Thr-^{D}Dab-Lys-Cys-Dab-Arg-Tyr;
Val-Hyp-Ile-Lys-Tyr-Pen-Asn-Arg-Dab-Thr-^{D}Dab-Lys-Cys-Dab-Arg-Tyr;
Val-Hyp-Ile-Thr-Trp-Pen-Asn-Arg-Dab-Thr-^{D}Dab-Lys-Cys-Dab-Arg-Tyr;
Val-Hyp-Ile-Thr-Tyr-Pen-Ala-Arg-Dab-Thr-^{D}Dab-Lys-Cys-Dab-Arg-Tyr;
Val-Hyp-Ile-Thr-Tyr-Pen-Leu-Arg-Dab-Thr-^{D}Dab-Lys-Cys-Dab-Arg-Tyr;
Val-Hyp-Ile-Thr-Tyr-Pen-Ile-Arg-Dab-Thr-^{D}Dab-Lys-Cys-Dab-Arg-Tyr;
Val-Hyp-Ile-Thr-Tyr-Pen-Ser-Arg-Dab-Thr-^{D}Dab-Lys-Cys-Dab-Arg-Tyr;
Val-Hyp-Ile-Thr-Tyr-Pen-Thr-Arg-Dab-Thr-^{D}Dab-Lys-Cys-Dab-Arg-Tyr;
Val-Hyp-Ile-Thr-Tyr-Pen-Lys-Arg-Dab-Thr-^{D}Dab-Lys-Cys-Dab-Arg-Tyr;
Val-Hyp-Ile-Thr-Tyr-Pen-Asn-Arg-Lys-Thr-^{D}Dab-Lys-Cys-Dab-Arg-Tyr;
Val-Hyp-Ile-Thr-Tyr-Pen-Asn-Arg-Arg-Thr-^{D}Dab-Lys-Cys-Dab-Arg-Tyr;
Val-Hyp-Ile-Thr-Tyr-Pen-Asn-Arg-Dab-Thr-^{D}Dab-Ile-Cys-Dab-Arg-Tyr;
Val-Hyp-Ile-Thr-Tyr-Pen-Asn-Arg-Dab-Thr-^{D}Dab-Ser-Cys-Dab-Arg-Tyr;
Val-Hyp-Ile-Thr-Tyr-Pen-Asn-Arg-Dab-Thr-^{D}Dab-Tyr-Cys-Dab-Arg-Tyr;
Val-Hyp-Ile-Thr-Tyr-Pen-Asn-Arg-Dab-Thr-^{D}Dab-Trp-Cys-Dab-Arg-Tyr;
Val-Hyp-Ile-Thr-Tyr-Pen-Asn-Arg-Dab-Thr-^{D}Dab-Asn-Cys-Dab-Arg-Tyr;
Va-Hyp-Ile-Thr-Tyr-Pen-Asn-Arg-Dab-Thr-^{D}Dab-Dab-Cys-Dab-Arg-Tyr;
Val-Hyp-Ile-Thr-Tyr-Pen-Asn-Arg-Dab-Thr-^{D}Dab-Lys-Cys-Dab-Leu-Tyr;
Val-Hyp-Ile-Thr-Tyr-Pen-Asn-Arg-Dab-Thr-^{D}Dab-Lys-Cys-Dab-Ser-Tyr;
Val-Hyp-Ile-Thr-Tyr-Pen-Asn-Arg-Dab-Thr-^{D}Dab-Lys-Cys-Dab-Dab-Tyr;
Val-Hyp-Ile-Thr-Tyr-Pen-Asn-Arg-Dab-Thr-^{D}Dab-Lys-Cys-Dab-Lys-Tyr;
Val-Hyp-Ile-Thr-Tyr-Pen-Asn-Arg-Dab-Thr-^{D}Dab-Lys-Cys-Dab-Orn- Tyr;
Val-Hyp-Ile-Thr-Tyr-Pen-Asn-Arg-Dab-Thr-^{D}Dab-Cit-Cys-Dab-Arg-Tyr;
NMeVal-Hyp-Ile-Thr-Tyr-Pen-Asn-Arg-Dab-Thr-^{D}Dab-Lys-Cys-Dab-Arg-Tyr;
Val-Hyp-Ile-Thr-Tyr-Pen-Dap-Arg-Dab-Thr-^{D}Dab-Lys-Cys-Dab-Arg-Tyr;
Val-Hyp-Ile-Dap-Tyr-Pen-Asn-Arg-Dab-Thr-^{D}Dab-Lys-Cys-Dab-Arg-Tyr;
Val-Hyp-Ile-Arg-Tyr-Pen-Asn-Arg-Dab-Thr-^{D}Dab-Lys-Cys-Dab-Arg-Tyr;
Val-Pro((4R)NH₂)-Ile-Thr-Tyr-Pen-Asn-Arg-Dab-Thr-^{D}Dab-Lys-Cys-Tyr-Arg-Tyr;
Gua-Val-Pro-Ile-Ile-Tyr-Cys-Asn-Arg-Dab-Thr-^{D}Dab-Lys-Cys-Dab-Arg-Tyr;
Gua-Val-Pro-Ile-Ile-Tyr-Pen-Asn-Arg-Dab-Thr-^{D}Dab-Lys-Cys-Dab-Arg-Tyr;
Gua-Val-Hyp-Ile-Thr-Tyr-Pen-Asn-Arg-Dab-Thr-^{D}Dab-Lys-Cys-Dab-Arg-Tyr;
Gua-Val-Hyp-Ile-Ile-Tyr-Pen-Asn-Arg-Dab-Thr-^{D}Dab-Lys-Cys-Dab-Arg-Tyr;
Gua-Val-Pro((4R)NH₂)-Ile-Thr-Tyr-Pen-Asn-Arg-Dab-Thr-^{D}Dab-Lys-Cys-Dab-Arg-Tyr;
Gua-Val-Pro((4R)NH₂)-Ile-Ile-Tyr-Pen-Asn-Arg-Dab-Thr-^{D}Dab-Lys-Cys-Dab-Arg-Tyr;
Gua-Val-Hyp-Ile-Tyr-Tyr-Pen-Asn-Arg-Dab-Thr-^{D}Dab-Lys-Cys-Gln-Arg-Tyr;
Gua-Val-Hyp-Ile-Thr-Tyr-Pen-Glu-Arg-Dab-Thr-^{D}Dab-Lys-Cys-Dab-Arg-Tyr;
Gua-Val-Hyp-Ile-Thr-Tyr-Pen-His-Arg-Dab-Thr-^{D}Dab-Lys-Cys-Dab-Arg-Tyr;
Gua-Val-Hyp-Ile-Thr-Tyr-Pen-Asn-Arg-Dab-Thr-^{D}Dab-Dab-Cys-Dab-Arg-Tyr;
Gua-Val-Hyp-Ile-Thr-Tyr-Pen-Asn-Arg-Dab-Ser-^{D}Dab-Lys-Cys-Dab-Arg-Tyr;
Gua-Val-Hyp-Ile-Thr-Tyr-Pen-Asn-Arg-Dab-Ser-^{D}Dab-Lys-Cys-Dab-Dab-Tyr;
Gua-Val-Hyp-Ile-Thr-Tyr-Pen-Asn-Arg-Dab-Ser-^{D}Dab-Lys-Cys-Dab-Orn- Tyr;
Gua-Val-Pro((4R)NH₂)-Ile-Thr-Tyr-Pen-Asn-Arg-Dab-Thr-^{D}Dab-Lys-Cys-Tyr-Arg-Tyr;
Gua-Val-Pro((4R)NH₂)-Ile-Ile-Tyr-Pen-Asn-Arg-Dab-Thr-^{D}Dab-Lys-Cys-Tyr-Arg-Tyr;
Gua-Val-Hyp-Ile-alloThr-Tyr-Pen-Asn-Arg-Dab-Thr-^{D}Dab-Lys-Cys-Tyr-Arg-Tyr;
TMG-Val-Pro-Ile-Ile-Tyr-Cys-Asn-Arg-Dab-Thr-^{D}Dab-Lys-Cys-Dab-Arg-Tyr;
TMG-Val-Hyp-Ile-Thr-Tyr-Pen-Asn-Arg-Dab-Thr-^{D}Dab-Lys-Cys-Dab-Arg-Tyr;
Val-Hyp-Ile-Ile-Tyr-Pra-Asn-Arg-Dab-Thr-^{D}Dab-Lys-Abu(4N₃)-Dab-Arg-Tyr;
Val-Hyp-Ile-Ile-Tyr-Asp-Asn-Arg-Dab-Thr-^{D}Dab-Lys-Dab-Dab-Arg-Tyr;
Val-Hyp-Ile-Ile-Tyr-Pen-Asn-Arg-Dab-Thr-^{D}Dab-Lys-Cys-Dab-Cit-Tyr;
Val-Hyp-Ile-Ile-Tyr-Pen-Asn-Arg-Dab-Thr-^{D}Dab-Lys-Cys-Dab-Dab-Tyr;
Val-Hyp-Ile-Ile-Tyr-Pen-Asn-Arg-Dab-Ser-^{D}Dab-Lys-Cys-Dab-Arg-Tyr;
Val-Hyp-Ile-Ile-Tyr-Pen-Asn-Arg-Dab-Ser-^{D}Dab-Lys-Cys-Dab-Dab-Tyr;
Val-Hyp-Ile-Thr-Tyr-Pen-Asn-Arg-Dab-Ser-^{D}Dab-Lys-Cys-Dab-Dab-Tyr;
Val-Hyp-Ile-Ile-Tyr-Pen-Asn-Arg-Dab-Thr-^{D}Dab-Orn-Cys-Dab-Arg-Tyr;
Abu-Hyp-Ile-Ile-Tyr-Pen-Asn-Arg-Dab-Thr-^{D}Dab-Lys-Cys-Dab-Arg-Tyr;
Val-Pro-Ile-Thr-Tyr-Pen-Asn-Arg-Dab-Thr-^{D}Dab-Lys-Cys-Dab-Arg-Tyr;
Val-Hyp-Ile-Dab-Tyr-Pen-Asn-Arg-Dab-Ser-^{D}Dab-Lys-Cys-Dab-Dab-Tyr;
Val-Hyp-Ile-Ile-Tyr-Pen-Asn-Arg-Lys-Thr-^{D}Dab-Lys-Cys-Lys-Arg-Tyr;
Val-Hyp-Ile-Ile-Tyr-Pen-Asn-Arg-Dab-Thr-^{D}Dab-Ile-Cys-Dab-Dab-Tyr;
Val-Hyp-Ile-Ile-Tyr-Pen-Asn-Arg-Dab-Ser-^{D}Dab-IIe-Cys-Dab-Dab-Tyr;
Val-Hyp-Ile-Ile-Tyr-Pen-Asn-Arg-Dab-Thr-^{D}Dab-Lys-Cys-Dab-Orn-Tyr;
Val-Hyp-Ile-Ile-Tyr-Pen-Asn-Arg-Dab-Ser-^{D}Dab-Lys-Cys-Dab-Orn-Tyr;
Val-Hyp-Ile-Ile-Tyr-Pen-Asn-Arg-Dab-Thr-^{D}Dab-Ile-Cys-Dab-Arg-Tyr;
Val-Hyp-Ile-Thr-Tyr-Pen-Ile-Arg-Dab-Thr-^{D}Dab-Lys-Cys-Dab-Orn-Tyr;
Val-Hyp-Ile-Dab-Tyr-Pen-Asn-Arg-Dab-Thr-^{D}Dab-Lys-Cys-Dab-Orn-Tyr;
Val-Pro((4R)NH₂)-Ile-Ile-Tyr-Pen-Asn-Arg-Dab-Thr-^{D}Dab-Ile-Cys-Dab-Arg-Tyr;
Val-Pro((4R)NH₂)-Ile-Ile-Tyr-Pen-Asn-Arg-Dab-Thr-^{D}Dab-Cit-Cys-Dab-Arg-Tyr;
Val-Hyp-Ile-Thr-Tyr-Pen-Ile-Arg-Dab-Thr-^{D}Dab-Lys-Cys-Ser-Orn-Tyr;)
Val-Pro((4R)NH₂)-Ile-Ile-Tyr-Pen-Asn-Arg-Dab-Thr-^{D}Dab-Lys-Cys-Ser-Arg-Tyr;
Val-Hyp-Ile-Thr-Tyr-Pen-Asn-Arg-Dab-Ser-^{D}Dab-Lys-Cys-Dab-Orn- Tyr;
tBuGly-Hyp-Ile-Thr-Tyr-Pen-Ile-Arg-Dab-Thr-^{D}Dab-Lys-Cys-Dab-Orn-Tyr;
Val-Hyp-Ile-Ile-Tyr-Pen-Asn-Arg-Dab-Ser-^{D}Dab-Ile-Cys-Dab-Orn-Tyr;
Val-Hyp-Ile-Ile-Tyr-Pen-Asn-Arg-Dab-Thr-^{D}Dab-Ile-Cys-Dab-Orn-Tyr;
Val-Hyp-Ile-Ile-Tyr-Pen-Asn-Arg-Dab-Thr-^{D}Dab-Cit-Cys-Dab-Arg-Tyr;
Val-Hyp-Ile-Thr-Tyr-Pen-Ile-Arg-Dab-Thr-^{D}Dab-Lys-Cys-Dab-Ser-Tyr;
Val-Pro((4R)NH₂)-Ile-Ile-Tyr-Pen-Asn-Arg-Dab-Thr-^{D}Dab-Lys-Cys-Dab-Ser-Tyr;
Val-Hyp-Ile-Ile-Tyr-Pen-Asn-Arg-Dab-Thr-DDab-Ser-Cys-Dab-Arg-Tyr;
Val-Pro((4R)NH₂)-Ile-Thr-Tyr-Pen-Asn-Arg-Dab-Thr-^{D}Dab-Cit-Cys-Dab-Ser-Tyr;
Val-Pro((4R)NH₂)-Ile-Thr-Tyr-Pen-Asn-Arg-Dab-Thr-^{D}Dab-Lys-Cys-Ser-Ser-Tyr;
Val-Hyp-Ile-Thr-Tyr-Pen-Ile-Arg-Dab-Thr-^{D}Dab-Lys-Cys-Ser-Arg-Tyr;
Val-Hyp-Ile-Ile-Tyr-Pen-Asn-Arg-Dab-Thr-^{D}Dab-Lys-Cys-Dab-Ser-Tyr;
Val-Pro((4R)NH₂)-Ile-Ile-Tyr-Pen-Asn-Arg-Dab-Thr-^{D}Dab-Cit-Cys-Dab-Ser-Tyr;
Val-Hyp-Ile-Ile-Tyr-Pen-Asn-Arg-Dab-Thr-^{D}Dab-Lys-Cys-Ser-Arg-Tyr;
Val-Hyp-Ile-Dab-Tyr-Pen-Asn-Arg-Dab-Thr-^{D}Dab-Cit-Cys-Dab-Ser-Tyr;
Val-Pro((4R)NH₂)-Ile-Ile-Tyr-Pen-Asn-Arg-Dab-Thr-^{D}Dab-Lys-Cys-Dab-Thr-Tyr;
Val-Pro((4R)NH₂)-lle-Thr-Tyr-Pen-Asn-Arg-Dab-Thr-^{D}Dab-Lys-Cys-Ser-Arg-Tyr;
Val-Hyp-Ile-Ile-Tyr-Pen-Asn-Arg-Dab-Thr-^{D}Dab-Lys-Cys-Dab-Thr-Tyr;
Val-Hyp-Ile-Ile-Tyr-Pen-Asn-Arg-Dab-Ser-^{D}Dab-Lys-Cys-Dab-Thr-Tyr;
Val-Hyp-Ile-Dab-Tyr-Pen-Asn-Arg-Dab-Thr-^{D}Dab-Cit-Cys-Dab-Thr-Tyr;
tBuGly-Hyp-Ile-Ile-Tyr-Pen-Asn-Arg-Dab-Thr-^{D}Dab-Lys-Cys-Dab-Arg-Tyr;
Val-Pro((4R)NH₂)-Ile-Ile-Tyr-Pen-Asn-Arg-Dab-Thr-^{D}Dab-Ile-Cys-Dab-Thr-Tyr;
Val-Pro((4R)NH₂)-Ile-Thr-Tyr-Pen-Asn-Arg-Dab-Thr-^{D}Dab-Cit-Cys-Dab-Thr-Tyr;
Val-Pro((4R)NH₂)-Ile-Thr-Tyr-Pen-Asn-Arg-Dab-Thr-^{D}Dab-Ile-Cys-Dab-Thr-Tyr;
Val-Hyp-Ile-Ile-Tyr-Pen-Asn-Arg-Dab-Ser-^{D}Dab-Ile-Cys-Dab-Arg-Tyr;
Val-Pro((4R)NH₂)-Ile-Thr-Tyr-Pen-Asn-Arg-Dab-Thr-^{D}Dab-Lys-Cys-Ser-Thr-Tyr;
Val-Pro((4R)NH₂)-Ile-Thr-Tyr-Pen-Asn-Arg-Dab-Thr-^{D}Dab-Lys-Cys-Dab-Thr-Tyr;
Val-Hyp-Ile-Ile-Tyr-Pen-Asn-Arg-Orn-Thr-^{D}Dab-Ile-Cys-Dab-Arg-Tyr;
Val-Hyp-Ile-Orn-Tyr-Pen-Asn-Arg-Dab-Thr-^{D}Dab-Lys-Cys-Dab-Thr-Tyr;
Val-Hyp-Ile-Thr-Tyr-Pen-Ile-Arg-Dab-Thr-^{D}Dab-Orn-Cys-Dab-Arg-Tyr;
Val-Hyp-Ile-Ile-Tyr-Pen-Asn-Arg-Cit-Thr-^{D}Dab-Lys-Cys-Dab-Arg-Tyr;
Val-Hyp-Ile-Lys(Me)-Tyr-Pen-Asn-Arg-Dab-Thr-^{D}Dab-Lys-Cys-Dab-Arg-Tyr;
Val-Hyp-Ile-Val(3OH)-Tyr-Pen-Asn-Arg-Dab-Thr-^{D}Dab-Lys-Cys-Dab-Arg-Tyr;
Val-Pro(4,4F₂)-Ile-Thr-Tyr-Pen-Asn-Arg-Dab-Thr-^{D}Dab-Lys-Cys-Dab-Arg-Tyr;
Val-Pro(3,4dehydro)-Ile-Thr-Tyr-Pen-Asn-Arg-Dab-Thr-^{D}Dab-Lys-Cys-Dab-Arg-Tyr;
Val-Pro(4,4F₂)-Ile-Thr-Tyr-Pen-Asn-Arg-Dab-Thr-^{D}Dab-Lys-Cys-Dab-Arg-Tyr;
Val-Pro((4R)OPh)-Ile-Thr-Tyr-Pen-Asn-Arg-Dab-Thr-^{D}Dab-Lys-Cys-Dab-Arg-Tyr
Val-Pro((4R)F)-Ile-Thr-Tyr-Pen-Asn-Arg-Dab-Thr-^{D}Dab-Lys-Cys-Dab-Arg-Tyr;
Val-Pic-Ile-Thr-Tyr-Pen-Asn-Arg-Dab-Thr-^{D}Dab-Lys-Cys-Dab-Arg-Tyr;
Val-NalIloThr-Ile-Thr-Tyr-Pen-Asn-Arg-Dab-Thr-^{D}Dab-Lys-Cys-Dab-Arg-Tyr;
Hyp-Ile-Thr-Tyr-Pen-Asn-Arg-Dab(iPr)-Thr-^{D}Dab-Lys-Cys-Dab-Orn(iPr)-Tyr;
Hyp-Ile-Thr-Tyr-Pen-Asn-Arg-Dab(iPr)-Thr-^{D}Dab-Lys-Cys-Dab(iPr)-Arg-Tyr; (
Hyp-Ile-Thr-Tyr-Pen-Asn-Arg-Dab-Thr-^{D}Dab-Lys-Cys-Dab-Orn(iPr)-Tyr;
Hyp-Ile-Thr-Tyr-Pen-Ile-Arg-Dab-Thr-^{D}Dab-Lys-Cys-Dab-Orn(iPr)-Tyr;
Hyp-Ile-Thr-Tyr-Pen-Asn-Arg-Dab-Thr-^{D}Dab(iPr)-Lys-Cys-Dab-Arg-Tyr;
or a tautomer, a rotamer, a salt, a hydrate or a solvate thereof;
wherein Cys or Pen at P⁶, if present, and Cys or Pen at P¹³, if present, optionally form a disulfide bridge between P⁶ and P¹³; or wherein
Asp at P⁶, if present, and Dab at P¹³, if present, optionally form a lactam bridge between P⁶ and P¹³; or wherein
Pra at P⁶, if present, and Abu(4N₃) at P¹³, if present, optionally form a 1,2,3-triazole bridge between P⁶ and P¹³.

Another more particularly preferred embodiment of the first aspect relates to a compound, wherein the compound is selected from the group consisting of
Val-Pro-Ile-Ile-Tyr-Cys-Asn-Arg-Arg-Thr-^{D}Dab-Lys-Cys-Dab-Arg-Nle;
Val-Pro((4R)NH₂)-Ile-Ile-Tyr-Pen-Asn-Arg-Arg-Thr-^{D}Dab-Lys-Cys-Dab-Arg-Nle;
Val-Pro((4R)NH₂)-Ile-Ile-Tyr-Pen-Asn-Arg-Dab-Thr-^{D}Dab-Lys-Cys-Dab-Arg-Nle;
Val-Ndab-Ile-Ile-Tyr-Pen-Asn-Arg-Dab-Thr-^{D}Dab-Lys-Cys-Dab-Arg-Nle;
Val-Pro((4R)NH₂)-Ile-Thr-Tyr-Pen-Asn-Arg-Dab-Thr-^{D}Dab-Lys-Cys-Dab-Arg-Nle;
Val-Pro-Ile-Ile-Tyr-Cys-Asn-Arg-Dab-Thr-^{D}Dab-Lys-Cys-Dab-Arg-Cha;
Val-Pro-Ile-Ile-Tyr-Pen-Asn-Arg-Dab-Thr-^{D}Dab-Lys-Cys-Dab-Arg-Tyr;
Val-Pro-Ile-Ile-Tyr-Cys-Asn-Arg-Dab-Thr-^{D}Dab-Lys-Pen-Dab-Arg-Tyr;
Val-Pro((4R)NH₂)-Ile-Ile-Tyr-Cys-Asn-Arg-Dab-Thr-^{D}Dab-Lys-Cys-Dab-Arg-Tyr;
Val-Hyp-Ile-Ile-Tyr-Cys-Asn-Arg-Dab-Thr-^{D}Dab-Lys-Cys-Dab-Arg-Tyr;
Val-Pro-Ile-Thr-Tyr-Cys-Asn-Arg-Da b-Thr-^{D}Dab-Lys-Cys-Dab-Arg-Tyr;
Val-Pro-Ile-Phe-Tyr-Cys-Asn-Arg-Dab-Thr-^{D}Dab-Lys-Cys-Dab-Arg-Tyr;
2OHVal-Pro-Ile-Ile-Tyr-Cys-Asn-Arg-Dab-Thr-^{D}Dab-Lys-Cys-Dab-Arg-Tyr;
Val-Hyp-Ile-Ile-Tyr-Pen-Asn-Arg-Dab-Thr-^{D}Dab-Lys-Cys-Dab-Arg-Tyr;
Val-Hyp-Ile-Thr-Tyr-Pen-Asn-Arg-Dab-Thr-^{D}Dab-Lys-Cys-Dab-Arg-Tyr;
Val-Hyp-Ile-Thr-Tyr-Pen-Asn-Arg-Dab-Thr-^{D}Dap-Lys-Cys-Dab-Arg-Tyr;
Val-Hyp-Ile-Thr-Tyr-Pen-Asn-Arg-Dab-Thr-^{D}Orn-Lys-Cys-Dab-Arg-Tyr;
Val-Hyp-Ile-Thr-Tyr-Pen-Asn-Arg-Dab-Thr-^{D}Lys-Lys-Cys-Dab-Arg-Tyr;
Val-Hyp-Ile-Thr-Tyr-Pen-Asn-Arg-Dab-Thr-^{D}Arg-Lys-Cys-Dab-Arg-Tyr;
Val-Hyp-Ile-Dab-Tyr-Pen-Asn-Arg-Dab-Thr-^{D}Dab-Lys-Cys-Dab-Thr-Tyr;
Val-Hyp-Ile-Arg-Tyr-Pen-Asn-Arg-Dab-Thr-^{D}Dab-Lys-Cys-Dab-Thr-Tyr;
Val-Hyp-Ile-Tyr-Tyr-Pen-Asn-Arg-Dab-Thr-^{D}Dab-Lys-Cys-Dab-Arg-Tyr;
Val-Hyp-Ile-Leu-Tyr-Pen-Asn-Arg-Dab-Thr-^{D}Dab-Lys-Cys-Dab-Arg-Tyr;
Val-Hyp-Ile-Asn-Tyr-Pen-Asn-Arg-Dab-Thr-^{D}Dab-Lys-Cys-Dab-Arg-Tyr;
Val-Hyp-Ile-Dab-Tyr-Pen-Asn-Arg-Dab-Thr-^{D}Dab-Lys-Cys-Dab-Arg-Tyr;
Val-Hyp-Ile-Lys-Tyr-Pen-Asn-Arg-Dab-Thr-^{D}Dab-Lys-Cys-Dab-Arg-Tyr;
VaI-Hyp-Ile-Thr-Trp-Pen-Asn-Arg-Dab-Thr-^{D}Dab-Lys-Cys-Dab-Arg-Tyr;
Val-Hyp-Ile-Thr-Tyr-Pen-Ala-Arg-Dab-Thr-^{D}Dab-Lys-Cys-Dab-Arg-Tyr;
Val-Hyp-Ile-Thr-Tyr-Pen-Leu-Arg-Dab-Thr-^{D}Dab-Lys-Cys-Dab-Arg-Tyr;
Val-Hyp-Ile-Thr-Tyr-Pen-Ile-Arg-Dab-Thr-^{D}Dab-Lys-Cys-Dab-Arg-Tyr;
Val-Hyp-Ile-Thr-Tyr-Pen-Ser-Arg-Dab-Thr-^{D}Dab-Lys-Cys-Dab-Arg-Tyr;
Val-Hyp-Ile-Thr-Tyr-Pen-Thr-Arg-Dab-Thr-^{D}Dab-Lys-Cys-Dab-Arg-Tyr;
Val-Hyp-Ile-Thr-Tyr-Pen-Lys-Arg-Dab-Thr-^{D}Dab-Lys-Cys-Dab-Arg-Tyr;
Val-Hyp-Ile-Thr-Tyr-Pen-Asn-Arg-Lys-Thr-^{D}Dab-Lys-Cys-Dab-Arg-Tyr;
Val-Hyp-Ile-Thr-Tyr-Pen-Asn-Arg-Arg-Thr-^{D}Dab-Lys-Cys-Dab-Arg-Tyr;
Val-Hyp-Ile-Thr-Tyr-Pen-Asn-Arg-Dab-Thr-^{D}Dab-Ile-Cys-Dab-Arg-Tyr;
Val-Hyp-Ile-Thr-Tyr-Pen-Asn-Arg-Dab-Thr-^{D}Dab-Ser-Cys-Dab-Arg-Tyr;
Val-Hyp-Ile-Thr-Tyr-Pen-Asn-Arg-Dab-Thr-^{D}Dab-Tyr-Cys-Dab-Arg-Tyr;
Val-Hyp-Ile-Thr-Tyr-Pen-Asn-Arg-Dab-Thr-^{D}Dab-Trp-Cys-Dab-Arg-Tyr;
Val-Hyp-Ile-Thr-Tyr-Pen-Asn-Arg-Dab-Thr-^{D}Dab-Asn-Cys-Dab-Arg-Tyr;
Va-Hyp-Ile-Thr-Tyr-Pen-Asn-Arg-Dab-Thr-^{D}Dab-Dab-Cys-Dab-Arg-Tyr;
Val-Hyp-Ile-Thr-Tyr-Pen-Asn-Arg-Dab-Thr-^{D}Dab-Lys-Cys-Dab-Leu-Tyr;
Val-Hyp-Ile-Thr-Tyr-Pen-Asn-Arg-Dab-Thr-^{D}Dab-Lys-Cys-Dab-Ser-Tyr;
Val-Hyp-Ile-Thr-Tyr-Pen-Asn-Arg-Dab-Thr-^{D}Dab-Lys-Cys-Dab-Dab-Tyr;
Val-Hyp-Ile-Thr-Tyr-Pen-Asn-Arg-Dab-Thr-^{D}Dab-Lys-Cys-Dab-Lys-Tyr;
Val-Hyp-Ile-Thr-Tyr-Pen-Asn-Arg-Dab-Thr-^{D}Dab-Lys-Cys-Dab-Orn-Tyr;
Val-Hyp-Ile-Thr-Tyr-Pen-Asn-Arg-Dab-Thr-^{D}Dab-Cit-Cys-Dab-Arg-Tyr;
NMeVal-Hyp-Ile-Thr-Tyr-Pen-Asn-Arg-Dab-Thr-^{D}Dab-Lys-Cys-Dab-Arg-Tyr;
Val-Hyp-Ile-Thr-Tyr-Pen-Dap-Arg-Dab-Thr-^{D}Dab-Lys-Cys-Dab-Arg-Tyr;
Val-Hyp-Ile-Dap-Tyr-Pen-Asn-Arg-Dab-Thr-^{D}Dab-Lys-Cys-Dab-Arg-Tyr;
Val-Hyp-Ile-Arg-Tyr-Pen-Asn-Arg-Dab-Thr-^{D}Dab-Lys-Cys-Dab-Arg-Tyr;
Val-Pro((4R)NH₂)-Ile-Thr-Tyr-Pen-Asn-Arg-Dab-Thr-^{D}Dab-Lys-Cys-Tyr-Arg-Tyr;
Gua-Val-Pro-Ile-Ile-Tyr-Cys-Asn-Arg-Dab-Thr-^{D}Dab-Lys-Cys-Dab-Arg-Tyr;
Gua-Val-Pro-Ile-Ile-Tyr-Pen-Asn-Arg-Dab-Thr-^{D}Dab-Lys-Cys-Dab-Arg-Tyr;
Gua-Val-Hyp-Ile-Thr-Tyr-Pen-Asn-Arg-Dab-Thr-^{D}Dab-Lys-Cys-Dab-Arg-Tyr;
Gua-Val-Hyp-Ile-Ile-Tyr-Pen-Asn-Arg-Dab-Thr-^{D}Dab-Lys-Cys-Dab-Arg-Tyr;
Gua-Val-Pro((4R)NH₂)-Ile-Thr-Tyr-Pen-Asn-Arg-Dab-Thr-^{D}Dab-Lys-Cys-Dab-Arg-Tyr;
Gua-Val-Pro((4R)NH₂)-Ile-Ile-Tyr-Pen-Asn-Arg-Dab-Thr-^{D}Dab-Lys-Cys-Dab-Arg-Tyr;
Gua-Val-Hyp-Ile-Tyr-Tyr-Pen-Asn-Arg-Dab-Thr-^{D}Dab-Lys-Cys-Gln-Arg-Tyr;
Gua-Val-Hyp-Ile-Thr-Tyr-Pen-Glu-Arg-Dab-Thr-^{D}Dab-Lys-Cys-Dab-Arg-Tyr;
Gua-Val-Hyp-Ile-Thr-Tyr-Pen-His-Arg-Dab-Thr-^{D}Dab-Lys-Cys-Dab-Arg-Tyr;
Gua-Val-Hyp-Ile-Thr-Tyr-Pen-Asn-Arg-Dab-Thr-^{D}Dab-Dab-Cys-Dab-Arg-Tyr;
Gua-Val-Hyp-Ile-Thr-Tyr-Pen-Asn-Arg-Dab-Ser-^{D}Dab-Lys-Cys-Dab-Arg-Tyr;
Gua-Val-Hyp-Ile-Thr-Tyr-Pen-Asn-Arg-Dab-Ser-^{D}Dab-Lys-Cys-Dab-Dab-Tyr;
Gua-Val-Hyp-Ile-Thr-Tyr-Pen-Asn-Arg-Dab-Ser-^{D}Dab-Lys-Cys-Dab-Orn- Tyr;
Gua-Val-Pro((4R)NH₂)-Ile-Thr-Tyr-Pen-Asn-Arg-Dab-Thr-^{D}Dab-Lys-Cys-Tyr-Arg-Tyr;
Gua-Val-Pro((4R)NH₂)-Ile-Ile-Tyr-Pen-Asn-Arg-Dab-Thr-^{D}Dab-Lys-Cys-Tyr-Arg-Tyr;
Gua-Val-Hyp-Ile-alloThr-Tyr-Pen-Asn-Arg-Dab-Thr-^{D}Dab-Lys-Cys-Tyr-Arg-Tyr;
TMG-Val-Pro-Ile-Ile-Tyr-Cys-Asn-Arg-Dab-Thr-^{D}Dab-Lys-Cys-Dab-Arg-Tyr;
TMG-Val-Hyp-Ile-Thr-Tyr-Pen-Asn-Arg-Dab-Thr-^{D}Dab-Lys-Cys-Dab-Arg-Tyr;
Val-Hyp-Ile-Ile-Tyr-Pra-Asn-Arg-Dab-Thr-^{D}Dab-Lys-Abu(4N₃)-Dab-Arg-Tyr;
Val-Hyp-Ile-Ile-Tyr-Asp-Asn-Arg-Dab-Thr-^{D}Dab-Lys-Dab-Dab-Arg-Tyr;
or a tautomer, a rotamer, a salt, a hydrate or a solvate thereof;
wherein Cys or Pen at P⁶, if present, and Cys or Pen at P¹³, if present, optionally form a disulfide bridge between P⁶ and P¹³; or wherein
Asp at P⁶, if present, and Dab at P¹³, if present, optionally form a lactam bridge between P⁶ and P¹³; or wherein
Pra at P⁶, if present, and Abu(4N₃) at P¹³, if present, optionally form a 1,2,3-triazole bridge between P⁶ and P¹³.

A further embodiment of the first aspect relates to compounds, which are identical to the compounds of formula (I), except that one or more atoms are replaced by an atom having an atomic mass number or mass different from the atomic mass number or mass usually found in nature, e.g. compounds enriched in ²H (D), ³H, ¹¹C, ¹⁴C, ¹²⁷I etc. These isotopic analogs and their pharmaceutical salts and formulations are considered useful agents in the therapy and/or diagnostic, for example, but not limited to, where a fine-tuning of *in vivo* half-life time could lead to an optimized dosage regimen.

In a second aspect, the invention relates to an enantiomer of a compound of formula (I) according to the first aspect.

Hereinafter follows a list of abbreviations, corresponding to generally adopted usual practice, of amino acids or derivatives thereof which, or the residues of which, are suitable for the purposes of the present invention and referred to in this document.

In spite of this specific determination of amino acids or derivatives thereof, it is noted that, for a person skilled in the art, it is obvious that derivatives of these amino acids or derivatives thereof, resembling alike structural and physico-chemical properties, lead to functional analogs with similar biological activity, and therefore still form part of the gist of the present invention.

| | | |
|---|---|---|
| Ala | A | L-Alanine |
| Arg | R | L-Arginine |
| Asn | N | L-Asparagine |
| Asp | D | L-Aspartic acid |
| Cys | C Q | L-Cysteine |
| Gln | | L-Glutamine |
| Glu | E | L-Glutamic acid |
| Gly | G | Glycine |
| His | H | L-Histidine |
| Ile | I | L-Isoleucine |
| Leu | L | L-Leucine |
| Lys | K M | L-Lysine |
| Met | | L-Methionine |
| Phe | F | L-Phenylalanine |
| Pro | P | L-Proline |
| Ser | S | L-Serine |
| Thr | T W | L-Threonine |
| Trp | | L-Tryptophan |
| Tyr | Y | L-Tyrosine |
| Val | V | L-Valine |

- 2OHVal: (*S*)-2-hydroxy-3-methylbutanoic acid
- Ala(cPr): (*S*)-2-amino-3-cyclopropylpropanoic acid
- Ala(tetrahydropyran4yl): (*S*)-2-amino-3-(tetrahydro-2H-pyran-4-yl)propanoic acid
- Abu: (*S*)-2-aminobutanoic acid
- *allo*lle: L-alloisoleucine
- betaGly: 3-aminopropanoic acid
- Cha: (*S*)-2-amino-3-cyclohexylpropanoic acid
- Cpa: (*S*)-2-amino-3-cyclopentylpropanoic acid
- Cpg: (*S*)-2-amino-2-cyclopentylacetic acid
- Cyg: (*S*)-2-amino-2-cyclopropylacetic acid
- Dea: (*S*)-2-amino-3-ethylpentanoic acid
- Hle: (*S*)-2-amino-5-methylhexanoic acid
- Nle: (*S*)-2-aminohexanoic acid
- OctGly: (*S*)-2-aminodecanoic acid
- Sar: *N*-methylglycine
- tBuGly: (*S*)-2-amino-3,3-dimethylbutanoic acid
- tBuAla: (*S*)-2-amino-4,4-dimethylpentanoic acid
- NMeAla: *N*-methyl-L-alanine
- NMeVal: *N*-methyl-L-valine
- Gua-Val: *N*-amidino-L-valine
- TMG-Val: (*S*)-2-(*N,N,N',N*'-tetramethylguanidino)-propanoic acid
- Nva: (*S*)-2-aminopentanoic acid

- Pic: (*S*)-piperidine-2-carboxylic acid
- Pro((4R)F): (2*S*,4*R*)-4-fluoropyrrolidine-2-carboxylic acid
- Pro((4S)F): (2*S*,4*S*)-4-fluoropyrrolidine-2-carboxylic acid
- Pro(4,4F₂): (*S*)-4,4-difluoropyrrolidine-2-carboxylic acid
- Pro((4R)OMe): (2*S*,4*R*)-4-methoxypyrrolidine-2-carboxylic acid
- Pro(3,4dehydro): (*S*)-2,5-dihydro-1H-pyrrole-2-carboxylic acid

- Phe(3OH): (*S*)-2-amino-3-(3-hydroxyphenyl)propanoic acid
- Phe(4F): (*S*)-2-amino-3-(4-fluorophenyl)propanoic acid
- Phe(4OCF₃): (*S*)-2-amino-3-(4-(trifluoromethoxy)phenyl)propanoic acid
- Trp(6Cl): (*S*)-2-amino-3-(6-chloro-1H-indol-3-yl)propanoic acid
- Tyr(3Cl): (*S*)-2-amino-3-(3-chloro-4-hydroxyphenyl)propanoic acid
- Tyr(3F): (*S*)-2-amino-3-(3-fluoro-4-hydroxyphenyl)propanoic acid
- Tyr(Phenyl): (*S*)-2-amino-3-(4-phenoxyphenyl)propanoic acid
- 4Thz: (*R*)-thiazolidine-4-carboxylic acid
- Phe(4(4hydroxyphenoxy)): (*S*)-2-amino-3-(4-(4-hydroxyphenoxy)phenyl)propanoic acid
- Phe(4NH₂): (*S*)-2-amino-3-(4-aminophenyl)propanoic acid
- Tyr(Me): (*S*)-2-amino-3-(4-methoxyphenyl)propanoic acid
- Ntyr: *N*-(4-Hydroxybenzyl)glycine
- Nphe: *N*-(benzyl)glycine

- Agb: (*S*)-2-amino-4-guanidinobutanoic acid
- Agp: (*S*)-2-amino-3-guanidinopropanoic acid
- Dab: (*S*)-2,4-diaminobutanoic acid
- 2OHDab: (*S*)-2-hydroxy-4-aminobutanoic acid
- Dab(iPr): (*S*)-2-amino-4-(isopropylamino)butanoic acid
- ^{D}Dab(iPr): (*R*)-2-amino-4-(isopropylamino)butanoic acid
- Dap: (*S*)-2,3-diaminopropanoic acid
- Dap(iPr): (*S*)-2-amino-3-(isopropylamino)propanoic acid
- ^{D}Dap(iPr): (*R*)-2-amino-3-(isopropylamino)propanoic acid
- Har: *N*6-carbamimidoyl-L-lysine
- Lys(iPr): *N*6-isopropyl-L-lysine
- ^{D}Lys(iPr): *N*6-isopropyl-D-lysine
- Narg: *N*-(3-guanidinopropyl)glycine
- Ndab: *N*-(2-aminoethyl)glycine
- Nlys: *N*-(4-aminobutyl)glycine
- Norn: *N*-(3-aminopropyl)glycine
- NalloThr: *N*-((*S*)-Hydroxyethyl)glycine
- Orn: (*S*)-2,5-diaminopentanoic acid
- Orn(iPr): (*S*)-2-amino-5-(isopropylamino)pentanoic acid
- ^{D}Orn(iPr): (*R*)-2-amino-5-(isopropylamino)pentanoic acid
- Pro((4R)guanidine: (2*S*,4*R*)-4-guanidinopyrrolidine-2-carboxylic acid
- Pro((4R)NH₂): (2*S*,4*R*)-4-aminopyrrolidine-2-carboxylic acid
- Pro((4S)NH₂): (2*S*,4*S*)-4-aminopyrrolidine-2-carboxylic acid
- NMeLys: *N*-methyl-L-lysine
- Lys(Me): N⁶-Methyl-L-lysine

- alloThr: L-*allo*threonine
- Cit: (*S*)-2-amino-5-ureidopentanoic acid
- Hgn: (*S*)-2,6-diamino-6-oxohexanoic acid
- Hse: L-homoserine
- Hyp: (2*S*,4*R*)-4-hydroxypyrrolidine-2-carboxylic acid
- Leu((3R)OH): (2*S*,3*R*)-2-amino-3-hydroxy-4-methylpentanoic acid
- Hgl: (*S*)-2-aminohexanedioic acid
- Val(3OH): 3-Hydroxy-L-valine

- Pra: L-propargylglycine
- Abu(4N₃): (*S*)-2-amino-4-azidobutanoic acid
- Hcy: L-homocysteine
- NMeCys: *N*-methyl-L-cysteine
- Pen: (*R*)-2-amino-3-mercapto-3-methylbutanoic acid

The abbreviation of D-isomers, e.g. ^{D}Lys corresponds to the epimer at the 2-position of the appropriate amino acid described above.

The abbreviation "Gua-" followed by an abbreviation of an amino acid, or amino acid residue, as listed above, corresponds to the N-amidinylated amino acid, or amino acid residue, having the N-terminal amino group replaced by a guanidino (Gua) group, like for example:
- Gua-Glu: *N*-amidino-L-glutamic acid
(*S*)-2-guanidino-pentanedioic acid

The abbreviation "TMG-" followed by an abbreviation of an amino acid, or amino acid residue, as listed above, corresponds to the amino acid, or amino acid residue, having the N-terminal amino group replaced by a *N,N,N',N*'-tetramethylguanidino (TMG) group, like for example:
- TMG-Trp: (*S*)-2-(*N,N,N',N'*-tetramethylguanidino)-3-(1*H*-indol-3-yl)propanoic acid.

In a third aspect, the invention relates to a pharmaceutical composition containing a compound or a mixture of compounds according to the first aspect and at least one pharmaceutically inert carrier.

In one embodiment of the third aspect, the pharmaceutical composition is in a form suitable for oral, topical, transdermal, injection, buccal, transmucosal, rectal, pulmonary or inhalation administration. In a further embodidment of the third aspect, the pharmaceutical composition is in the form of a tablet, a dragee, a capsule, a solution, a liquid, a gel, a plaster, a cream, an ointment, a syrup, a slurry, a suspension, a spray, a nebulizer, an aerosol, or a suppository.

In a fourth aspect, the invention relates to a compound of formula (I) according to the first aspect or a pharmaceutically acceptable salt thereof.

In a fifth aspect, the invention relates to a compound of formula (I) according to the first aspect or a pharmaceutically acceptable salt thereof for use as a medicament.

In a sixth aspect, the invention relates to a compound according to the first aspect for use as a pharmaceutically active substance having antibiotic activity.

In a seventh aspect, the invention relates to a compound according to the first aspect for use in the treatment or prevention of infections or diseases related to such infections; particularly infections related to respiratory diseases or skin or soft tissue diseases or gastrointestinal diseases or eye diseases or ear diseases or CNS diseases or bone diseases or cardiovascular diseases or genitourinary diseases, or nosocomial infections, or catheter-related and non-catheter-related infections, or urinary tract infections, or bloodstream infections; or infection-induced sepsis. The infection may be selected from nosocomial infections, catheter-related and non-catheter-related infections, urinary tract infections, bloodstream infections, or a disease or disorder associated with an infection, especially diseases or disorders such as ventilator-associated pneumonia (VAP), ventilator-associated bacterial pneumonia (VABP), hospital-acquired pneumonia (HAP), hospital-acquired bacterial pneumonia (HABP), healthcare-associated pneumonia (HCAP), cystic fibrosis, emphysema, asthma, pneumonia, epidemic diarrhea, necrotizing enterocolitis, typhlitis, gastroenteritis, pancreatitis, keratitis, endophthalmitis, otitis, brain abscess, meningitis, encephalitis, osteochondritis, pericarditis, epididymitis, prostatitis, urethritis, sepsis; surgical wounds, traumatic wounds, and burns.

In an eighth aspect, the invention relates to a use of a compound according to the first aspect as a disinfectant or preservative for foodstuffs, cosmetics, medicaments, and/or other nutrient-containing materials.

In a ninth aspect, the invention relates to a use of a compound according to the first aspect as a pharmaceutically active substance having antibiotic activity.

In a tenth aspect, the invention relates to a use of a compound according to the first aspect or a composition according to the third aspect as a disinfectant or preservative for foodstuffs, cosmetics, medicaments and/or other nutrient-containing materials.

In an eleventh aspect, the invention relates to a process for the preparation of a compound according to the first aspect which comprises the following steps:
(a) coupling an appropriately functionalized solid support with an appropriately N-protected derivative of that amino acid which in the desired end-product is in position P¹⁶; any functional group which may be present in said N-protected amino acid derivative being likewise appropriately protected;
(b) removing the N-protecting group from the product thus obtained;
(c) coupling the product thus obtained with an appropriately N-protected derivative of that amino acid which in the desired end-product is in position P¹⁵; any functional group which may be present in said N-protected amino acid derivative being likewise appropriately protected;
(d) effecting steps substantially corresponding to steps (b) and (c) using appropriately N-protected derivatives of amino acids which in the desired end-product are in positions P¹⁴ to P⁶, any functional group(s) which may be present in said N-protected amino acid derivatives being likewise appropriately protected;
(e) optionally selectively deprotecting one or several protected functional group(s) present in the molecule and chemically transforming the reactive group(s) thus liberated;
(f) effecting steps substantially corresponding to steps (b) and (c) using appropriately N-protected derivatives of amino acids which in the desired end-product are in positions P⁵ to P², any functional group(s) which may be present in said N-protected amino acid derivatives being likewise appropriately protected; and, optionally, following each coupling, selectively deprotecting one or several protected functional group(s) present in the molecule and chemically transforming the reactive group(s) thus liberated;
(g) further effecting steps substantially corresponding to steps (b) and (c) using an appropriately N-protected derivative of an amino acid, or optionally, an appropriately protected derivative of a hydroxy acid, which in the desired end-product is in position P¹, any functional group(s) which may be present in said N-protected amino acid derivative, or hydroxy acid derivative, being likewise appropriately protected; and, optionally, following the coupling, selectively deprotecting one or several protected functional group(s) present in the molecule and chemically transforming the reactive group(s) thus liberated;
(h) optionally selectively deprotecting one or several protected functional group(s) present in the molecule and chemically transforming the reactive group(s) thus liberated;
(i) optionally, removing the N-protecting group at position P¹;
(j) detaching the product thus obtained from the solid support;
(k) optionally selectively deprotecting one or several protected functional group(s) present in the molecule and chemically transforming the reactive group(s) thus liberated;
(l) removing any protecting groups present on functional groups of any members of the chain of residues and, optionally, any protecting group(s) which may in addition be present in the molecule;
(m) optionally implementing additional chemical transformations of one or more reactive group(s) present in the molecule;
(n) if required, removing any protecting groups present on functional groups of any members of the chain of residues and, optionally, any protecting group(s) which may in addition be present in the molecule; and
(o) optionally converting the product thus obtained into a pharmaceutically acceptable salt; or
   optionally converting a pharmaceutically acceptable or unacceptable salt thus obtained into the corresponding free compound of formula (I); or
   optionally converting a pharmaceutically acceptable or unacceptable salt thus obtained into a different, pharmaceutically acceptable salt.

Enantiomers of the compounds defined herein before form also part of the present invention. These enantiomers can be prepared by a modification of the above process wherein enantiomers of all chiral starting materials are utilized.

The process of the invention can advantageously be carried out as parallel array synthesis to yield libraries of peptidomimetics of the invention. Such parallel syntheses allow one to obtain arrays of numerous (normally 12 to 576, typically 96) compounds as described above in moderate to high yields and defined purities, minimizing the formation of dimeric and polymeric by-products.

The functionalized solid support is conveniently derived from polystyrene crosslinked with, preferably 1-5%, divinylbenzene; polystyrene coated with polyethyleneglycol spacers (Tentagel^{™}); and polyacrylamide resins (see also D. Obrecht, J.-M. Villalgordo, "Solid-Supported Combinatorial and Parallel Synthesis of Small-Molecular-Weight Compound Libraries", Tetrahedron Organic Chemistry Series, Vol. 17, Pergamon, Elsevier Science, 1998).

The solid support is functionalized by means of a linker, i.e. a bifunctional spacer molecule which contains on one end an anchoring group for attachment to the solid support and on the other end a selectively cleavable functional group used for the subsequent chemical transformations and cleavage procedures. For the purposes of the present invention two types of linkers are used:
Type 1 linkers are designed to release the amide group under acidic conditions (H. Rink, Tetrahedron Lett. 1987, 28, 3783-3790). Linkers of this kind form amides of the carboxyl group of the amino acids; examples of resins functionalized by such linker structures include 4-[(((2,4-dimethoxyphenyl)Fmoc-aminomethyl) phenoxyacetamido) aminomethyl] PS resin, 4-[(((2,4-dimethoxyphenyl) Fmoc-aminomethyl)phenoxyacetamido)-aminomethyl]-4-methyl-benzydrylamine PS resin (Rink amide MBHA PS Resin), and 4-[(((2,4-dimethoxy-phenyl) Fmoc-aminomethyl)phenoxyacetamido) aminomethyl] benzhydrylamine PS-resin (Rink amide BHA PS resin), and Fmoc-amino-xanthen-3-yloxy PS resin, Sieber linker resin). Preferably, the support is derived from polystyrene crosslinked with, most preferably 1-5%, divinylbenzene and functionalized by means of the 4-(((2,4-dimethoxy-phenyl) Fmoc-aminomethyl)phenoxyacetamido) linker.
Type 2 linkers are designed to eventually release the carboxyl group under acidic conditions. Linkers of this kind form acid-labile esters with the carboxyl group of the amino acids, usually acid-labile benzyl, benzhydryl and trityl esters; examples of such linker structures include 2-methoxy-4-hydroxymethylphenoxy (Sasrin^{™} linker), 4-(2,4-dimethoxyphenyl-hydroxy-methyl)-phenoxy (Rink linker), 4-(4-hydroxymethyl-3-methoxyphenoxy)butyric acid (HMPB linker), trityl and 2-chlorotrityl. Preferably, the support is derived from polystyrene crosslinked with, most preferably 1-5%, divinylbenzene and functionalized by means of the 2-chlorotrityl linker.

When carried out as parallel array synthesis the process of the invention can be advantageously carried out as described herein below but it will be immediately apparent to those skilled in the art how these procedures will have to be modified in case it is desired to synthesize one single compound of the invention.

A number of reaction vessels (normally 12 to 576, typically 96) equal to the total number of compounds to be synthesized by the parallel method are loaded with 10 to 1000 mg, preferably 40 mg, of the appropriate functionalized solid support, preferably 1 to 5% cross-linked polystyrene.

The solvent to be used must be capable of swelling the resin and includes, but is not limited to, dichloromethane (DCM), dimethylformamide (DMF), N-methylpyrrolidone (NMP), dioxane, toluene, tetrahydrofuran (THF), ethanol (EtOH), trifluoroethanol (TFE), isopropylalcohol and the like. Solvent mixtures containing as at least one component a polar solvent (e.g. 20% TFE/DCM, 35% THF/NMP) are beneficial for ensuring high reactivity and solvation of the resin-bound peptide chains (G.B. Fields, C.G. Fields, J. Am. Chem. Soc. 1991, 113, 4202-4207).

With the development of various linkers that release the C-terminal carboxylic acid group under mild acidic conditions, not affecting acid-labile groups protecting functional groups in the side chain(s), considerable progresses have been made in the synthesis of protected peptide fragments. The 2-methoxy-4-hydroxybenzylalcohol-derived linker (Sasrin^{™} linker, Mergler et al., Tetrahedron Lett. 1988, 29 4005-4008) is cleavable with diluted trifluoroacetic acid (0.5-1% TFA in DCM) and is stable to Fmoc deprotection conditions during the peptide synthesis, Boc/tBu-based additional protecting groups being compatible with this protection scheme. Other linkers which are suitable for the process of the invention include the super acid labile 4-(2,4-dimethoxyphenyl-hydroxymethyl)-phenoxy linker (Rink linker, H. Rink, Tetrahedron Lett. 1987, 28, 3787-3790), where the removal of the peptide requires 10% acetic acid in DCM or 0.2% trifluoroacetic acid in DCM; the 4-(4-hydroxymethyl-3-methoxyphenoxy)butyric acid-derived linker (HMPB-linker, Flörsheimer & Riniker, 1991, Peptides 1990: Proceedings of the Twenty-First European Peptide Symposium, 131) which is also cleaved with 1% TFA/DCM in order to yield a peptide fragment containing all acid labile side-chain protective groups; and, in addition, the 2-chlorotritylchloride linker (Barlos et al., Tetrahedron Lett. 1989, 30, 3943-3946), which allows the peptide detachment using a mixture of glacial acetic acid/trifluoroethanol/DCM (1:2:7) for 30 min.

Suitable protecting groups for amino acids and, respectively, for their residues are, for example,
- for the amino group (as is present e.g. also in the side-chain of lysine)
   - Cbz: benzyloxycarbonyl
   - Boc: tert.-butyloxycarbonyl
   - Fmoc: 9-fluorenylmethoxycarbonyl
   - Alloc: allyloxycarbonyl
   - Teoc: trimethylsilylethoxycarbonyl
   - Tcc: trichloroethoxycarbonyl
   - Nps: o-nitrophenylsulfonyl
   - Trt: triphenylmethyl or trityl
   - ivDe: 1-(4,4-dimethyl-2,6-dioxocyclohex-1-ylidene)-3-methylbutyl;

- for the carboxyl group (as is present e.g. also in the side-chain of aspartic and glutamic acid) by conversion into esters with the alcohol components
   - tBu: *tert*.-butyl
   - Bn: benzyl
   - Me: methyl
   - Ph: phenyl
   - Pac: phenacyl
   allyl
   - Tse: trimethylsilylethyl
   - Tce: trichloroethyl
   - Dmab: 4-N-(1-[ dimethyl-2,6-dioxocyclohexylidene]-3-methylbutyl)-amino benzyl;
   - 2-PhiPr: 2-phenyl-isopropyl;
- for the guanidino group (as is present e.g. in the side-chain of arginine)
   - Pmc: 2,2,5,7,8-pentamethylchroman-6-sulfonyl
   - Ts: tosyl (i.e. p-toluenesulfonyl)
   - Cbz: benzyloxycarbonyl
   - Pbf: pentamethyldihydrobenzofuran-5-sulfonyl;
- and for the hydroxy group (as is present e.g. in the side-chain of threonine and serine)
   - tBu: *tert*.-butyl
   - Bn: benzyl
   - Trt: trityl
   - Alloc: allyloxycarbonyl.

The 9-fluorenylmethoxycarbonyl-(Fmoc)-protected amino acid derivatives are preferably used as the building blocks for the construction of the peptidomimetics of the invention. For the deprotection, i.e. cleaving off of the Fmoc group, 20% piperidine in DMF or 2% DBU/2% piperidine in DMF can be used as well as 25% hexafluoroisopropanol in CH₂Cl₂.

The quantity of the reactant, i.e. of the amino acid derivative, is usually 1 to 20 equivalents (eq) based on the milliequivalents per gram (meq/g) loading of the functionalized solid support (typically 0.1 to 2.85 meq/g for polystyrene resins) originally weighed into the reaction tube. Additional equivalents of reactants can be used, if required, to drive the reaction to completion in a reasonable time. The preferred workstations (without, however, being limited thereto) are Protein Technologies' Symphony X and MultiSynTech's-Syro synthesizer, the latter additionally equipped with a transfer unit and a reservoir box during the process of detachment of the fully protected linear peptide from the solid support. All synthesizers are able to provide a controlled environment, for example, reactions can be accomplished at temperatures different from room temperature as well as under inert gas atmosphere, if desired.

Amide bond formation requires the activation of the α-carboxyl group for the acylation step. When this activation is being carried out by means of the commonly used carbodiimides such as dicyclohexylcarbodiimide (DCC, Sheehan & Hess, J. Am. Chem. Soc. 1955, 77, 1067-1068) or diisopropylcarbodiimide (DIC, Sarantakis et al Biochem. Biophys. Res. Commun. 1976, 73, 336-342), the resulting dicyclohexylurea and, respectively, diisopropylurea is insoluble and, respectively, soluble in the solvents generally used. In a variation of the carbodiimide method, 1-hydroxy benzotriazole (HOBt, König & Geiger, Chem. Ber. 1970, 103, 788-798) or HOAt (ref) or ethyl cyano(hydroxyimino) acetate (Oxyma, (R. Subirós-Funosas, et al, Chem. Eur. J. 2009, 15, 9394-9403)) is included as an additive to the coupling mixture. HOBt, HOAt and Oxyma prevent dehydration, suppresses racemization of the activated amino acids and acts as a catalyst to improve the sluggish coupling reactions. Certain phosphonium reagents have been used as direct coupling reagents, such as benzotriazol-1-yl-oxy-tris-(dimethyl-amino)-phosphonium hexafluorophosphate (BOP, Castro et al., Tetrahedron Lett. 1975, 14, 1219-1222; Synthesis 1976, 751-752), or benzotriazol-1-yl-oxy-tris-pyrrolidino-phosphonium hexaflurophoshate (Py-BOP, Coste et al., Tetrahedron Lett. 1990, 31, 205-208), or 2-(1H-benzotriazol-1-yl-)1,1,3,3-tetramethyluronium tetrafluoroborate (TBTU), or hexafluorophosphate (HBTU, Knorr et al., Tetrahedron Lett. 1989, 30, 1927-1930); these phosphonium reagents are also suitable for *in situ* formation of HOBt esters with the protected amino acid derivatives. Diphenoxyphosphoryl azide (DPPA) or O-(7-aza-benzotriazol-1-yl)-N,N,N',N'-tetramethyluronium tetrafluoro borate (TATU) or O-(7-aza-benzotriazol-1-yl)-N,N,N',N'-tetramethyluronium hexa fluorophosphate (HATU)/7-aza-1-hydroxybenzotriazole (HOAt, Carpino et al., Tetrahedron Lett. 1994, 35, 2279-2281) or -(6-Chloro-1H-benzotriazol-1-yl-)- N,N,N',N'-1,1,3,3-tetramethyl uronium tetrafluoroborate (TCTU), or hexafluoro phosphate (HCTU, Marder, Shivo and Albericio: HCTU and TCTU: New Coupling Reagents: Development and Industrial Applications, Poster Presentation, Gordon Conference February 2002) can be used as coupling reagents as well as 1,1,3,3-bis(tetramethylene)chlorouronium hexafluorophosphate (PyCIU) especially for coupling of N-methylated amino acids (J. Coste, E. Frérot, P. Jouin, B. Castro, Tetrahedron Lett. 1991, 32, 1967) or pentafluorophenyl diphenyl-phosphinate (S. Chen, J. Xu, Tetrahedron Lett. 1991, 32, 6711). More recently, new coupling reagents based on Oxyma have been introduced e.g ([(1-(cyano-2-ethoxy-2-oxoethyl-ideneaminooxy) dimethylaminomorpholino)] uronium hexafluorophosphate (COMU, A. El-Faham, et al. Chem. Eur. J 2009, 15, 9404-9416))

Due to the fact that near-quantitative coupling reactions are essential, it is desirable to have experimental evidence for completion of the reactions. The ninhydrin test (Kaiser et al., Anal. Biochemistry 1970, 34, 595) and the 2,4,6-trinitrobenzene sulfonic (TNBS) test (Hancook W.S. et al, Anal. Biochem 1976, 71, 260), where a positive colorimetric response to an aliquot of resin-bound peptide or peptide indicates qualitatively the presence of the primary amine, can easily and quickly be performed after each coupling step. For the secondary amine detection e.g for proline derivatives, the chloranil test (Vojkovsky T., Pept. Res. 1995, 68, 236) can be used. Fmoc chemistry allows the spectrophotometric detection of the Fmoc chromophore when it is released with the base (Meienhofer et al., Int. J. Peptide Protein Res. 1979, 13, 35-42).

The resin-bound intermediate within each reaction vessel is washed free of excess of retained reagents, of solvents, and of by-products by repetitive exposure to pure solvent(s).

Washing procedures are repeated up to about 30 times (preferably about 5 times), monitoring the efficiency of reagent, solvent, and by-product removal by methods such as TLC, GC, LC-MS or inspection of the washings.

The above described procedure of reacting the resin-bound compound with reagents within the reaction wells followed by removal of excess reagents, by-products, and solvents is repeated with each successive transformation until the final resin-bound fully protected linear peptide has been obtained.

Before this fully protected linear peptide is detached from the solid support, it is possible, if desired, to selectively deprotect one or several protected functional group(s) present in the molecule and to appropriately substitute the reactive group(s) thus liberated. To this effect, the functional group(s) in question must initially be protected by a protecting group which can be selectively removed without affecting the remaining protecting groups present. Alloc (allyloxycarbonyl) is an example for such an amino protecting group which can be selectively removed, e.g. by means of Pd° and dimethylbarbituric acid (DMBA) in DCM/DMSO, without affecting the remaining protecting groups, such as Fmoc, present in the molecule. The reactive group thus liberated can then be treated with an agent suitable for further functionalization or for cyclization of the peptide on-solid support using the well-established lactam bridge. This bridge is formed by linking e.g. the amino group-bearing side chains of 2,4-diaminobutyric acid (Dab), ornithine and lysine, respectively, with the carboxyl group-bearing side chains of glutamic and aspartic acid residues located at opposite positions in the structure by means of an amide bond formation. Preferred protective groups for the side chain amino-groups side chains are allyloxycarbonyl (alloc) and for the side chain carboxyl-groups of aspartic and glutamic acid allylesters (allyl). For instance, the formation of a lactam bridge on solid support can be carried out after assembly of the linear peptide on resin by applying 0.2 eq tetrakis(triphenyl-phosphine)palladium(0) (10 mM) in dry DCM and 10 eq dimethylbarbituric acic in DMSO to selectively remove alloc- and allyl-protecting groups from amino and carboxyl functional groups of the side chains of amino acid residues to be linked. After repetition of the above procedure, the lactam bridge is formed on solid support by adding 4 eq of DIPEA in NMP and subsequent addition of 2 eq PyBOP in DMF or using 2 eq of Oxyma and 4 eq. of DIC in DCM. Finally, after the on support synthesis including elongation and modification e.g N-terminal functionalization or cyclization, the concomitant detachment and full deprotection of the peptide derivative can be performed with 95% TFA, 2.5% H₂O, 2.5% TIS, or 82.5% TFA, 5% anisole, 5% thioanisole, 5% H₂O and 2.5% TIS or another combination of scavengers for effecting the cleavage of the protected peptide and removal of protecting groups. The deprotection reaction time is commonly 30 minutes to 12 hours, preferably about 2.5 hours. The deprotected linear or cyclic peptide can be precipitated and washed using cold Et₂O or isopropyl ether (IPE).

For some compounds of the present invention according general formula (I) additional synthetic steps are required. These transformations can be applied either on a fully protected or partially deprotected linear or cyclic peptide, attached to or already released from the solid support or on the final deprotected molecule.

In addition to the lactam bridge described above, various methods are known to form interstrand linkages including those described by: J.P. Tam et al., Synthesis 1979, 955-957; J.M. Stewart et al., Solid Phase Peptide Synthesis, 2d Ed., Pierce Chemical Company, Rockford, IL, 1984**;** A.K. Ahmed et al., J. Biol. Chem. 1975, 250, 8477-8482; and M.W. Pennington et al., Peptides, pages 164-166, Giralt and Andreu, Eds., ESCOM Leiden, The Netherlands, 1990**;** C.E. Schafmeister et al., J. Am. Chem. Soc. 2000, 122, 5891.

A widely known linkage is the disulfide bridge formed by e.g. cysteines, homo-cysteines or penicillamine (Pen).

Recently, a further type of interstrand linkages based on 1,4-disubstituted 1,2,3-triazole-containing alkanediyl groups have been introduced (copper(I)-catalyzed alkyne-azide cycloaddition (CuAAC) "click" reaction). The linkage is obtained through a 1,3-dipolar cycloaddition between the ω-yne group of the side chain of an amino acid residue like e.g. L-propargylglycine and the ω-azido group of the side chain of an amino acid residue like e.g. (*S*)-2-amino-4-azidobutanoic acid, both residues located at opposite positions in the structure. This cycloaadition is favored in presence of copper(I). For instance, the formation of such a triazole-containing bridge is performed by stirring the purified fully deprotected linear peptide in a buffer containing copper(II) sulfate pentahydrate (CuSO₄. 5 H₂O) and L(+)-ascorbic acid used for the *in situ* generation of copper(I).

Depending on its purity, the final product as obtained following the procedures above can be used directly for biological assays, or has to be further purified, for example by preparative HPLC.

It is thereafter possible, if desired, to convert the fully deprotected product thus obtained into a pharmaceutically acceptable salt or to convert a pharmaceutically acceptable, or unacceptable, salt thus obtained into the corresponding free compound or into a different, pharmaceutically acceptable, salt. Any of these operations can be carried out by methods well known in the art.

In general the building blocks for the peptide derivatives of the present invention can be synthesized according to the literature methods, which are known to a person skilled in the art or are commercially available. All other corresponding amino acids have been described either as unprotected or as Boc- or Fmoc-protected racemates, (D)- or (L)-isomers. It will be appreciated that unprotected amino acid building blocks can be easily transformed into the corresponding Fmoc-protected amino acid building blocks required for the present invention by standard protecting group manipulations. Reviews describing general methods for the synthesis of α-amino acids include: R. Duthaler, Tetrahedron (Report) 1994, 349, 1540-1650; R.M. Williams, "Synthesis of optically active α-amino acids", Tetrahedron Organic Chemistry Series, Vol.7, J.E. Baldwin, P.D. Magnus (Eds.), Pergamon Press., Oxford 1989**.** An especially useful method for the synthesis of optically active α-amino acids relevant for this invention includes kinetic resolution using hydrolytic enzymes (M.A. Verhovskaya, I.A. Yamskov, Russian Chem. Rev. 1991, 60, 1163-1179; R.M. Williams, "Synthesis of optically active α-amino acids", Tetrahedron Organic Chemistry Series, Vol.7, J.E. Baldwin, P.D. Magnus (Eds.), Pergamon Press., Oxford 1989, Chapter 7, p.257-279). Kinetic resolution using hydrolytic enzymes involves hydrolysis of amides and nitriles by aminopeptidases or nitrilases, cleavage of N-acyl groups by acylases, and ester hydrolysis by lipases or proteases. It is well documented that certain enzymes will lead specifically to pure (L)-enantiomers whereas others yield the corresponding (D)-enantiomers (e.g.: R. Duthaler, Tetrahedron Report 1994, 349, 1540-1650; R.M. Williams, "Synthesis of optically active α-amino acids", Tetrahedron Organic Chemistry Series, Vol.7, J.E. Baldwin, P.D. Magnus (Eds.), Pergamon Press., Oxford 1989).

The references to methods of treatment in the subsequent paragraphs of this description are to be interpreted as references to the compounds, pharamceutical compositions and medicaments of the present invention for use in a method for treatment of the human (or animal) body by therapy.

The peptidomimetics of the invention can be used in a wide range of applications in order to inhibit the growth of or to kill microorganisms leading to the desired therapeutic effect in man or, due to their similar etiology, in other mammals. In particular they can be used to inhibit the growth of or to kill Gram-negative bacteria, in particular *Enterobacteriaceae,* and even more particular *Klebsiella pneumoniae* and/or *Escherichia coli.*

They can be used for example as disinfectants or as preservatives for materials such as foodstuffs, cosmetics, medicaments and other nutrient-containing materials.

The peptidomimetics of the invention can also be used to treat or prevent diseases related to microbial infection in plants and animals.

For use as disinfectants or preservatives the peptidomimetics can be added to the desired material singly, as mixtures of several peptidomimetics or in combination with other antimicrobial agents.

The peptidomimetics of the invention can be used to treat or prevent infections or diseases related to such infections, particularly nosocomial infections caused by Gram-negative bacteria related to diseases such as ventilator-associated pneumonia (VAP), hospital-acquired pneumonia (HAP), healthcare-associated pneumonia (HCAP); catheter-related and non-catheter-related infections such as urinary tract infections (UTIs) or bloodstream infections (BSIs); infections related to respiratory diseases such as cystic fibrosis, emphysema, asthma or pneumonia; infections related to skin or soft tissue diseases such as surgical wounds, traumatic wounds or burn; infections related to gastrointestinal diseases such as epidemic diarrhea, necrotizing enterocolitis, typhlitis, gastroenteritis or pancreatitis; infections related to eye diseases such as keratitis and endophthalmitis; infections related to ear diseases such as otitis; infections related to CNS diseases such as brain abscess and meningitis or encephalitis; infections related to bone diseases such as osteochondritis and osteomyelitis; infections related to cardiovascular diseases such as endocartitis and pericarditis; or infections related to genitourinary diseases such as epididymitis, prostatitis and urethritis. They can be administered singly, as mixtures of several peptidomimetics, in combination with other antimicrobial or antibiotic agents, or anti cancer agents, or antiviral (e.g. anti-HIV) agents, or in combination with other pharmaceutically active agents. The peptidomimetics can be administered per se or as pharmaceutical compositions.

The peptidomimetics of the invention may be administered per se or may be applied as an appropriate formulation together with carriers, diluents or excipients well known in the art.

Pharmaceutical compositions comprising peptidomimetics of the invention may be manufactured by means of conventional mixing, dissolving, granulating, coated tablet-making, levigating, emulsifying, encapsulating, entrapping or lyophilizing processes. Pharmaceutical compositions may be formulated in conventional manner using one or more physiologically acceptable carriers, diluents, excipients or auxiliaries which facilitate processing of the active peptidomimetics into preparations which can be used pharmaceutically. Proper formulation depends upon the method of administration chosen.

For topical administration the peptidomimetics of the invention may be formulated as solutions, gels, ointments, creams, suspensions, etc. as are well-known in the art.

Systemic formulations include those designed for administration by injection, e.g. subcutaneous, intravenous, intramuscular, intrathecal or intraperitoneal injection, as well as those designed for transdermal, transmucosal, oral or pulmonary administration.

For injections, the peptidomimetics of the invention may be formulated in adequate solutions, preferably in physiologically compatible buffers such as Hink's solution, Ringer's solution, or physiological saline buffer. The solutions may contain formulatory agents such as suspending, stabilizing and/or dispersing agents. Alternatively, the peptidomimetics of the invention may be in powder form for combination with a suitable vehicle, e.g., sterile pyrogen-free water, before use.

For transmucosal administration, penetrants appropriate to the barrier to be permeated are used in the formulation as known in the art.

For oral administration, the compounds can be readily formulated by combining the active peptidomimetics of the invention with pharmaceutically acceptable carriers well known in the art. Such carriers enable the peptidomimetics of the invention to be formulated as tablets, pills, dragees, capsules, liquids, gels, syrups, slurries, suspensions etc., for oral ingestion by a patient to be treated. For oral formulations such as, for example, powders, capsules and tablets, suitable excipients include fillers such as sugars, such as lactose, sucrose, mannitol and sorbitol; cellulose preparations such as maize starch, wheat starch, rice starch, potato starch, gelatin, gum tragacanth, methyl cellulose, hydroxypropylmethyl cellulose, sodium carboxymethyl-cellulose, and/or polyvinylpyrrolidone (PVP); granulating agents; and binding agents. If desired, desintegrating agents may be added, such as cross-linked polyvinylpyrrolidones, agar, or alginic acid or a salt thereof, such as sodium alginate. If desired, solid dosage forms may be sugar-coated or enteric-coated using standard techniques.

For oral liquid preparations such as, for example, suspensions, elixirs and solutions, suitable carriers, excipients or diluents include water, glycols, oils, alcohols, etc. In addition, flavoring agents, preservatives, coloring agents and the like may be added.

For buccal administration, the composition may take the form of tablets, lozenges, etc. formulated as usual.

For administration by inhalation, the peptidomimetics of the invention are conveniently delivered in form of an aerosol spray from pressurized packs or a nebulizer, with the use of a suitable propellant, e.g. dichlorodifluoromethane, trichlorofluromethane, carbon dioxide or another suitable gas. In the case of a pressurized aerosol the dose unit may be determined by providing a valve to deliver a metered amount. Capsules and cartridges of e.g. gelatin for use in an inhaler or insufflator may be formulated containing a powder mix of the peptidomimetics of the invention and a suitable powder base such as lactose or starch.

The compounds may also be formulated in rectal or vaginal compositions such as suppositories together with appropriate suppository bases such as cocoa butter or other glycerides.

In addition to the formulations described above, the peptidomimetics of the invention may also be formulated as depot preparations. Such long acting formulations may be administered by implantation (e.g. subcutaneously or intramuscularly) or by intramuscular injection. For the manufacture of such depot preparations the peptidomimetics of the invention may be formulated with suitable polymeric or hydrophobic materials (e.g. as an emulsion in an acceptable oil) or ion exchange resins, or as sparingly soluble salts.

In addition, other pharmaceutical delivery systems may be employed such as liposomes and emulsions well known in the art. Certain organic solvents such as dimethylsulfoxide may also be employed. Additionally, the peptidomimetics of the invention may be delivered using a sustained-release system, such as semipermeable matrices of solid polymers containing the therapeutic agent (e.g. for coated stents). Various sustained-release materials have been established and are well known by those skilled in the art. Sustained-release capsules may, depending on their chemical nature, release the compounds for a few weeks up to over 100 days. Depending on the chemical nature and the biological stability of the therapeutic agent, additional strategies for protein stabilization may be employed.

As the peptidomimetics of the invention may contain charged residues, they may be included in any of the above-described formulations as such or as pharmaceutically acceptable salts. Pharmaceutically acceptable salts tend to be more soluble in aqueous and other protic solvents than are the corresponding free forms.

The peptidomimetics of the invention, or compositions thereof, will generally be used in an amount effective to achieve the intended purpose. It is to be understood that the amount used will depend on a particular application.

For example, for use as a disinfectant or preservative, an antimicrobially effective amount of a peptidomimetic of the invention, or a composition thereof, is applied or added to the material to be desinfected or preserved. By antimicrobially effective amount is meant an amount of a peptidomimetic of the invention, or a composition thereof, that inhibits the growth of, or is lethal to, a target microbe population. While the antimicrobially effective amount will depend on a particular application, for use as disinfectants or preservatives the peptidomimetics of the invention, or compositions thereof, are usually added or applied to the material to be desinfected or preserved in relatively low amounts. Typically, the peptidomimetics of the invention comprise less than about 5% by weight of a disinfectant solution or material to be preserved, preferably less than 1% by weight and more preferably less than 0.1% by weight. An ordinary skilled expert will be able to determine antimicrobially effective amounts of particular peptidomimetics of the invention for particular applications without undue experimentation using, for example, the results of the in vitro assays provided in the examples.

For use to treat or prevent microbial infections or diseases related to such infections, the peptidomimetics of the invention, or compositions thereof, are administered or applied in a therapeutically effective amount. By therapeutically effective amount is meant an amount effective in ameliorating the symptoms of, or in ameliorating, treating or preventing microbial infections or diseases related thereto. Determination of a therapeutically effective amount is well within the capacities of those skilled in the art, especially in view of the detailed disclosure provided herein.

As in the case of disinfectants and preservatives, for topical administration to treat or prevent bacterial infections and/or viral infections a therapeutically effective dose can be determined using, for example, the results of the in vitro assays provided in the examples. The treatment may be applied while the infection is visible, or even when it is not visible. An ordinary skilled expert will be able to determine therapeutically effective amounts to treat topical infections without undue experimentation.

For systemic administration, a therapeutically effective dose can be estimated initially from in vitro assays. For example, a dose can be formulated in animal models to achieve a circulating peptidomimetic concentration range that includes the IC₅₀ as determined in the cell culture (i.e. the concentration of a test compound that is lethal to 50% of a cell culture). Such information can be used to more accurately determine useful doses in humans.

Initial dosages can also be determined from in vivo data, e.g. animal models, using techniques that are well known in the art. One having ordinary skill in the art could readily optimize administration to humans based on animal data.

Dosage amounts for applications as anti-infective agents may be adjusted individually to provide plasma levels of the peptidomimetics of the invention which are sufficient to maintain the therapeutic effect. Therapeutically effective serum levels may be achieved by administering multiple doses each day.

In cases of local administration or selective uptake, the effective local concentration of the peptidomimetics of the invention may not be related to plasma concentration. One having the ordinary skill in the art will be able to optimize therapeutically effective local dosages without undue experimentation.

The amount of peptidomimetics administered will, of course, be dependent on the subject being treated, on the subject's weight, the severity of the affliction, the manner of administration and the judgement of the prescribing physician.

The antimicrobial therapy may be repeated intermittently while infections are detectable or even when they are not detectable. The therapy may be provided alone or in combination with other drugs, such as for example anti-HIV agents or anti-cancer agents, or other antimicrobial agents.

Normally, a therapeutically effective dose of the peptidomimetics described herein will provide therapeutic benefit without causing substantial toxicity.

Toxicity of the peptidomimetics of the invention can be determined by standard pharmaceutical procedures in cell cultures or experimental animals, e.g., by determining the LD₅₀ (the dose lethal to 50% of the population) or the LD₁₀₀ (the dose lethal to 100% of the population). The dose ratio between toxic and therapeutic effect is the therapeutic index. Compounds which exhibit high therapeutic indices are preferred. The data obtained from these cell culture assays and animal studies can be used in formulating a dosage range that is not toxic for use in humans. The dosage of the peptidomimetics of the invention lies preferably within a range of circulating concentrations that include the effective dose with little or no toxicity. The dosage may vary within the range depending upon the dosage form employed and the route of administration utilized. The exact formulation, route of administration and dose can be chosen by the individual physician in view of the patient's condition (see, e.g. Fingl et al. 1975, In: The Pharmacological Basis of Therapeutics, Ch.1, p.1).

The following Examples illustrate the present invention but are not to be construed as limiting its scope in any way.

### Abbreviations:

- Ac: Acetyl;
- BSA: Bovine serum albumin;
- Boc: *tert*-Butyloxycarbonyl;
- DCHA: Dicyclohexylamine;
- DCM: Dichloromethane;
- DEAD: Diethyl azodicarboxylate;
- DIC: Diisopropylcarbodiimid;
- DIPEA: Diisopropylethylamine;
- DMF: Dimethylformamide;
- DMEM: Dulbecco's Modified Eagle's Medium;
- DODT: 3,6-dioxa-1,8-octanedithiol;
- FCS: Fetal Calf Serum;
- Fmoc: Fluorenylmethyloxycarbonyl;
- HATU: O-(7-Aza-benzotriazole-1-yl)-N,N,N',N'-tetramethyluronoium hexafluorophosphate;
- HBSS: Hank's Buffered Salt Solution;
- HBTU: O-(Benzotriazol-1-yl)-N,N,N',N'-tetramethyluronium hexafluorophosphate;
- HCTU: O-(6-Chlorobenzotriazol-1-yl)-N,N,N',N'-tetramethyluronium hexafluorophosphate;
- Hepes: 4-(2-hydroxyethyl)-1-piperazineethanesulfonic acid;
- HFIP: Hexafluoroisopropanol
- HOAt: 1-Hydroxy-7-azabenzotriazole;
- IMDM: Iscove's Modified Dulbecco's Media;
- IPE: Isopropylether;
- iPrOH: Isopropanol
- NMP: N-Methyl-2-pyrrolidone;
- NMM: N-Methylmorpholine;
- Oxyma: Ethylcyanohydroxyiminoacetate;
- PyBop^{®}: (Benzotriazol-1-yloxy)tripyrrolidinophosphonium hexafluorophosphate;
- TIS: Triisopropylsilane;
- TPP: Triphenylphosphine;
- RPMI: Roswell Park Memorial Institute medium;
- rt: Room temperature.

### Examples

### 1. Peptide synthesis

### 1.1 General synthetic procedures

A general method for the synthesis of the peptidomimetics of the present invention is exemplified in the following. This is to demonstrate the principal concept and does not limit or restrict the present invention in any way. A person skilled in the art is easily able to modify these procedures.

### Procedure A. Coupling of the first protected amino acid residue to the resin

In a dried flask, 2-chlorotritylchloride resin (polystyrene, 1% crosslinked; loading: 1.4 mMol/g) was swollen in dry DCM for 30 min (7 mL DCM per g resin). A solution of 0.8 eq of the Fmoc-protected amino acid and 6 eq of DIPEA in dry DCM/DMF (4/1) (10 mL per g resin) was added. After shaking for 2-4 h at rt the resin was filtered off and washed successively with DCM, DMF, DCM, DMF and DCM. Then a solution of dry DCM/MeOH/DIPEA (17:2:1) was added (10 mL per g resin). After shaking for 3 x 30 min the resin was filtered off in a pre-weighed sinter funnel and washed successively with DCM, DMF, DCM, MeOH, DCM, MeOH, DCM (2x) and Et₂O (2x). The resin was dried under high vacuum overnight. The final mass of resin was calculated before qualitative control.

Loading was typically 0.6 - 0.7 mMol/g.

### Procedure B. Synthesis of the fully protected peptide fragment

The synthesis was carried out on a Syro-peptide synthesizer (MultiSynTech GmbH) using 24 to 576 reaction vessels. Depending on the scale used (0.005 to 0.25 mmol), the above resin was placed into the size corresponding reactor and the resin was swollen in DCM and DMF for 15 min, respectively.

The following reaction cycles were programmed and carried out:

| **Step** | **Reagent Time** | |
|---|---|---|
| 1 | DCM, wash and swell | 1 x 3 min |
| 2 | NMP, wash and swell | 2 x 30 min |
| 3 | 20% piperidine/DMF | 1 x 5 min and 1 x 15 min |
| 4 | NMP, wash | 5 x 1 min |
| 5 | 7.2 eq Fmoc amino acid in NMP | |
| | + 6.8 eq HATU | |
| | + 21.6 eq NMM | 1 x 15 min |
| 6 | 7.2 eq Fmoc amino acid in NMP | |
| | + 6.8 eq HATU | |
| | + 21.6 eq NMM | 1 x 15 min |
| 7 | NMP, wash | 5 x 1 min |
| 8 | 12 eq acetic anhydride | |
| | + 12 eq NMM | 1x5 min |
| 9 | 20% piperidine/DMF | 2 x 2 min |
| 10 | NMP, wash | 5 x 1 min |
| 11 | DCM, wash (at the end of the synthesis) | 3 x 1 min |

Steps **5** to **10** are repeated to add each amino-acid residue. In case the N-terminal residue is a hydroxy acid residue, the same steps **5** to **9** are performed.

Standard Fmoc/tBu amino acids building blocks were used except for example 72 where Allyl/Alloc side chain protected amino acids were used in P⁶ and P¹³ and example 71 where alkyne and azido side chain derivatives were used in P⁶ and P¹³, and example 13 where a hydroxy acid was used in P¹.

### Procedure C. Cleavage/deprotection

After assembly of the protected peptide, the resin was suspended for 1 minutes in the cocktail cleaveage/deprotection TFA/anisole/thioanisole/water/TIS 82.5/5/5/5/2.5 v/v/v/v/v (20 mL/mmol of resin). After filtration, the cleavage/deprotection step was repeated twice. The combined filtrates were shaken for 3h at room temperature. The linear peptide was precipitated in cold Et₂O/pentane 1/1 v/v and wash three times with the same solvent mixtures. The solid was air dried.

### Procedure D. Purification procedure (preparative reverse phase LC-MS)

Compounds were purified by reverse phase chromatography using two column Waters BEH XBridge C8 OBD column, 30 x 150 mm , 5 µm (Cat No. 186003083) in series.

Mobile phases used were:
A: 0.1% TFA in Water/Acetonitrile 98/2 v/v
B: 0.1% TFA in Acetonitrile

Gradient slopes in the preparative runs were adapted each time based on analytical LC-MS analysis of the crude product. As an example, a typical run was executed with a flow rate of 35 mL/min running a gradient as follows:

| **T (min)** | **Flow (ml/min)** | **%B** |
|---|---|---|
| 0 | 10 | 0 |
| 0.3 | 10 | 0 |
| 0.5 | 35 | 0 |
| 1 | 35 | 0 |
| 1.1 | 35 | 10 |
| 13 | 35 | 20 |
| 13.1 | 35 | 100 |
| 19.3 | 35 | 100 |
| 19.4 | 0.1 | 100 |

In this example purification the retention time of the targeted compound was 10.4 min Detection: MS (ESI positive 60V profile mode) and UV @ 220 nm and 254 nm Fractions collected were evaporated using a Genevac HT4 evaporator or a Büchi system.

### 1.2 Analytical Method

Analytical HPLC retention times (RT, in minutes) were determined on HPLC system: Thermo Scientific Ultimate 3000RS, MS: Thermo Scientific MSQ plus utilizing a Ascentis Express C8 column, 100x3 mm, 2.7 µm, with the following solvents A (H₂O+0.1% TFA) and B (CH₃CN + 0.085% TFA) and the gradient was run at 55°C as follows:

| **T (min)** | **Flow (ml/min)** | **%B** |
|---|---|---|
| 0 | 1,4 | 5 |
| 0.1 | 1,4 | 5 |
| 7 | 1,4 | 55 |
| 7.02 | 1,4 | 97 |
| 7.5 | 1,4 | 97 |
| 7.52 | 1,4 | 5 |
| 8.8 | 1,4 | 5 |

Detection: MS (ESI positive 60V profile mode) and UV @ 220 nm and 254 nm

### 1.3 Synthesis of peptide sequences

### Examples 1-52, 73-138:

The protected peptide was synthesized from C- to N-terminus. The starting amino acid functionalized resin (obtained following procedure A) used for the synthesis corresponds to P¹⁶ in ***Table 1*.** The protected linear peptide immobilized on resin (Resin-P¹⁶-P¹⁵-P¹⁴-P¹³-P¹²-P¹¹-P¹⁰-P⁹-P⁸-P⁷-P⁶-P⁵-P⁴-P³-P²-P¹) was synthesized following procedure B. Cleavage/deprotection of the modified peptide was performed as described in procedure C. The global deprotected linear peptide was solubilized in ammonium acetate buffer 1M at pH 6 containing 5% DMSO v/v (140 mL/mmol). The peptide solution was stirred 48 h in an opened flask. The crude was purified according procedure D. Analytical data for each example are summarized in ***Table 1.***

### Examples 53-68:

The protected peptide was synthesized from C- to N-terminus. The starting amino acid functionalized resin (obtained following procedure A) used for the synthesis corresponds to P¹⁶ in ***Table 1*.** The protected linear peptide immobilized on resin (Resin-P¹⁶-P¹⁵-P¹⁴-P¹³-P¹²-P¹¹-P¹⁰-P⁹-P⁸-P⁷-P⁶-P⁵-P⁴-P³-P²-P¹) was synthesized following procedure B. Subsequently, the resin was swollen in DMF and N,N'-bis-Boc-guanylpyrazole (10 eq) in DMSO/DMF 1/1 v/v was added to the resin. The reaction was shaken overnight and the resin was thoroughly washed with DMF and DCM. Cleavage/deprotection of the modified peptide was performed as described in procedure C. The deprotected linear peptide was solubilized in ammonium acetate buffer 1M at pH 6 containing 5% DMSO v/v (140 mL/mmol). The peptide solution was stirred 48 h in an opened flask. The crude was purified according procedure D. Analytical data for each example are summarized in ***Table 1.***

### Example 69-70:

The protected peptide was synthesized from C- to N-terminus. The starting amino acid functionalized resin (obtained following procedure A) used for the synthesis corresponds to P¹⁶ in ***Table 1*.** The protected linear peptide immobilized on resin (Resin-P¹⁶-P¹⁵-P¹⁴-P¹³-P¹²-P¹¹-P¹⁰-P⁹-P⁸-P⁷-P⁶-P⁵-P⁴-P³-P²-P¹) was synthesized following procedure B. Subsequently, the resin was swollen in NMP and HATU (11.4 eq) and NMM (24 eq) were added to the resin. The reaction was shaken 1h and the resin was thoroughly washed with DMF and DCM. Cleavage/deprotection of the modified peptide was performed as described in procedure C. The deprotected linear peptide obtained was solubilized in ammonium acetate buffer 1M at pH 6 containing 5% DMSO v/v (140 mL/mmol). The peptide solution was stirred 48 h in an opened flask. The crude was purified according procedure D. Analytical data for each example are summarized in ***Table 1.***

### Example 71:

The protected peptide was synthesized from C to N-terminus. The amino acid functionalized resin (obtained following procedure A) used for the synthesis corresponds to P¹⁶ in ***Table 1*.** The protected linear peptide immobilized on resin (Resin-P¹⁶-P¹⁵-P¹⁴-P¹³-P¹²-P¹¹-P¹⁰-P⁹-P⁸-P⁷-P⁶-P⁵-P⁴-P³-P²-P¹) was synthesized following procedure B. Cleavage/deprotection of the modified peptide was performed as described in procedure C. The linear peptide was purified according procedure D. The purified peptide containing a side chain alkyne moiety and a side chain azido moiety was dissolved in degassed ammonium acetate buffer 1M pH 8 and added dropwise over a freshly prepared solution of CuSO₄.5 H₂O (4.4 eq) and L(+)-ascorbic acid (5.8 eq). After 20 min, the solution was acidified with TFA to pH 4 and directly purified according procedure D. Analytical data for each example are summarized in ***Table 1.***

### Example 72:

The protected peptide was synthesized from C to N-terminus. The amino acid functionalized resin (obtained following procedure A) used for the synthesis corresponds to P¹⁶ in ***Table 1*.** Following assembly of the protected peptide following procedure B until P⁶ bearing the N-terminus Fmoc protection (Resin-P¹⁶-P¹⁵-P¹⁴-P¹³-P¹²-P¹¹-P¹⁰-P⁹-P⁸-P⁷-P⁶-Fmoc), the resin was swollen in DCM for at least 15 min. To selectively remove alloc- and allyl-protecting groups in P⁶ and P¹³ from amino and carboxyl functional groups, respectively, 0.2 eq tetrakis(triphenyl-phosphine)palladium(0) (10 mM) in dry DCM and 10 eq DMBA were added. After shaking the reaction mixture for 5 min at rt, the resin was filtered off and wash NMP, iPrOH, IPE and DCM. A fresh solution of reagents was added to repeat the procedure. Following subsequent washing of the resin with NMP, iPrOH, IPE and DCM, the resin was swollen DCM. 2 eq of Oxyma solubilized in dry DCM were added to the resin followed by 4 eq of DIC in dry DCM. After 1h, 2 eq of DIC were added in dry DCM. After stirring the reaction mixture overnight, the resin was filtered and washed thoroughly with DCM and NMP. The elongation of the peptide was continued following procedure A (P⁵ to P¹). Cleavage/deprotection of the modified peptide was performed as described in procedure C and purified following procedure D. Analytical data for each example are summarized in ***Table 1.***

### 1.4 Sequence data

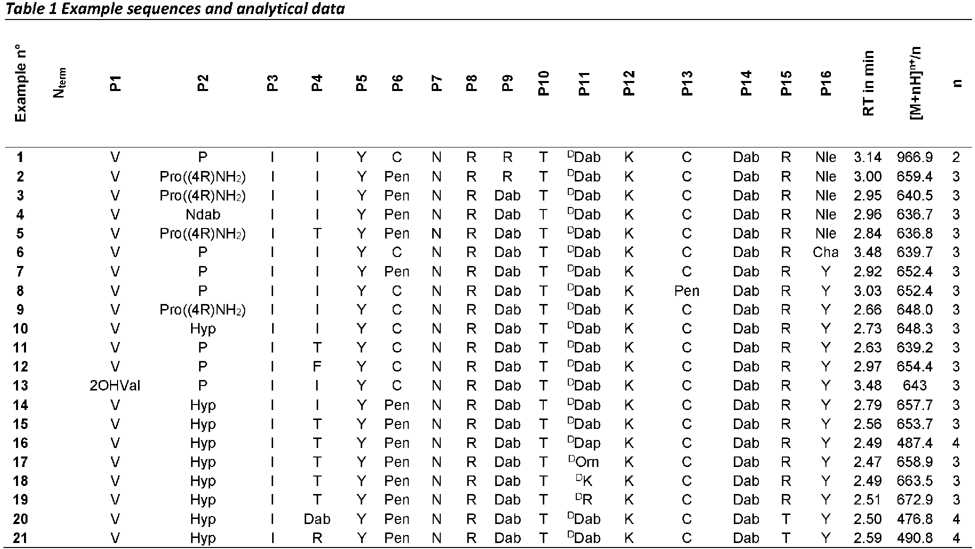

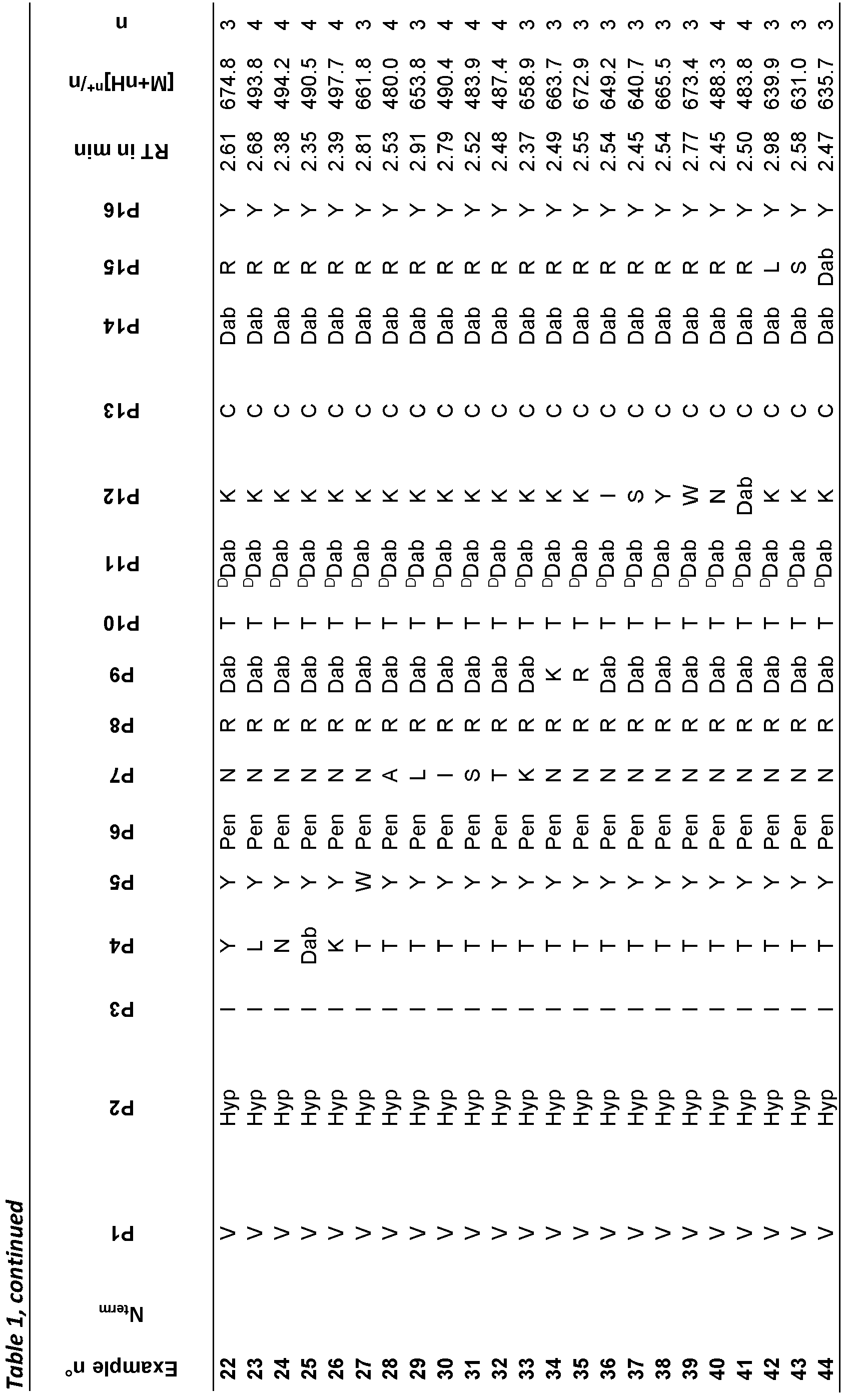

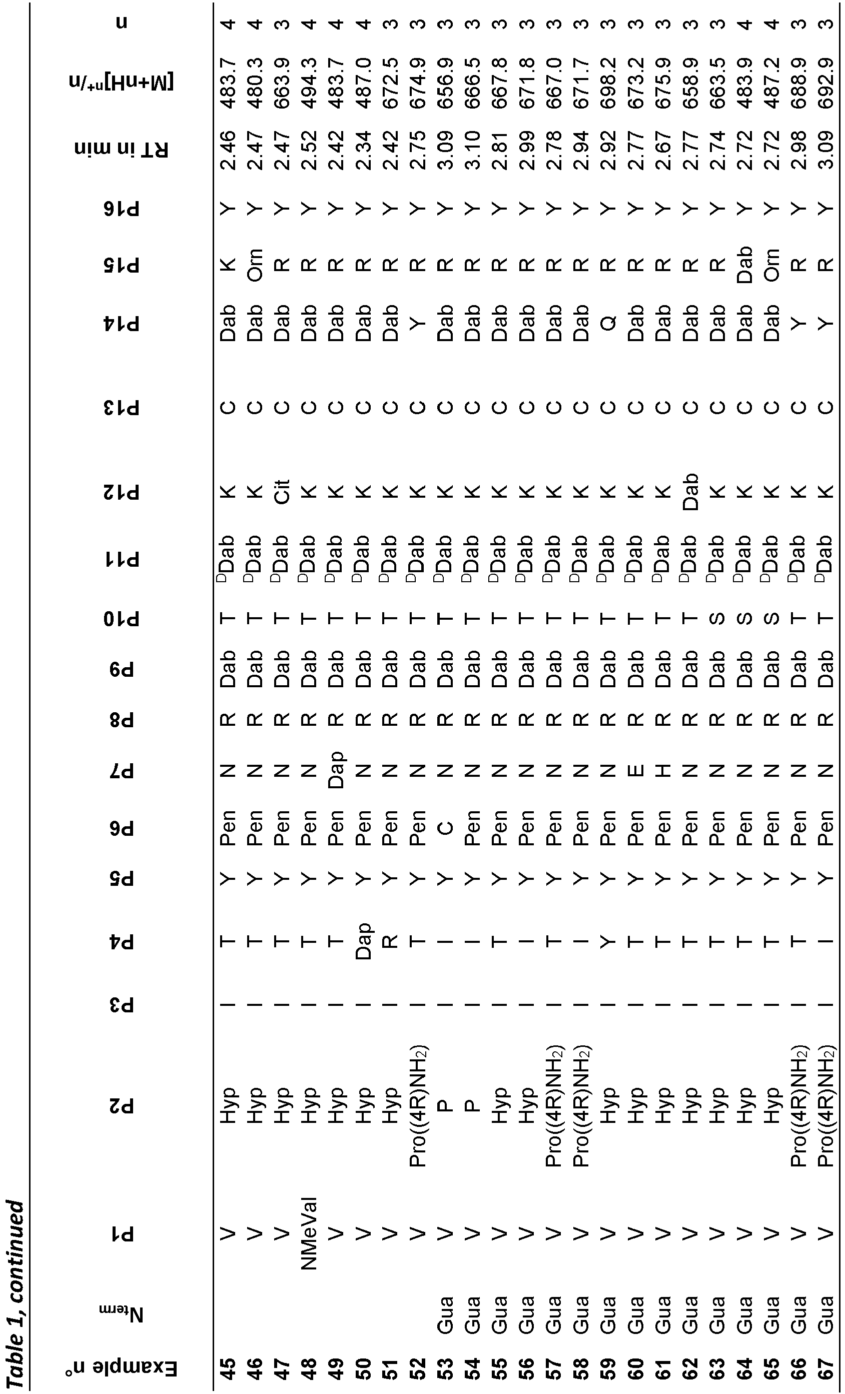

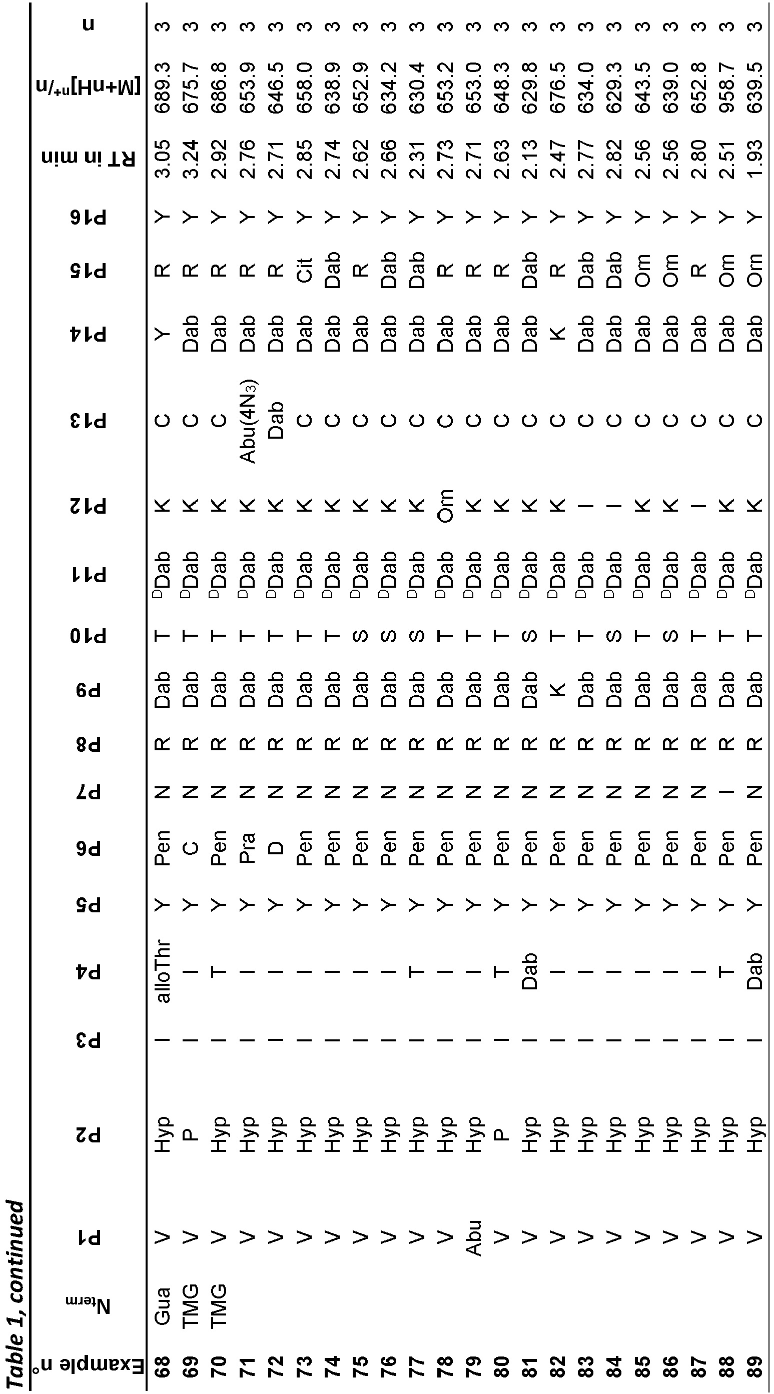

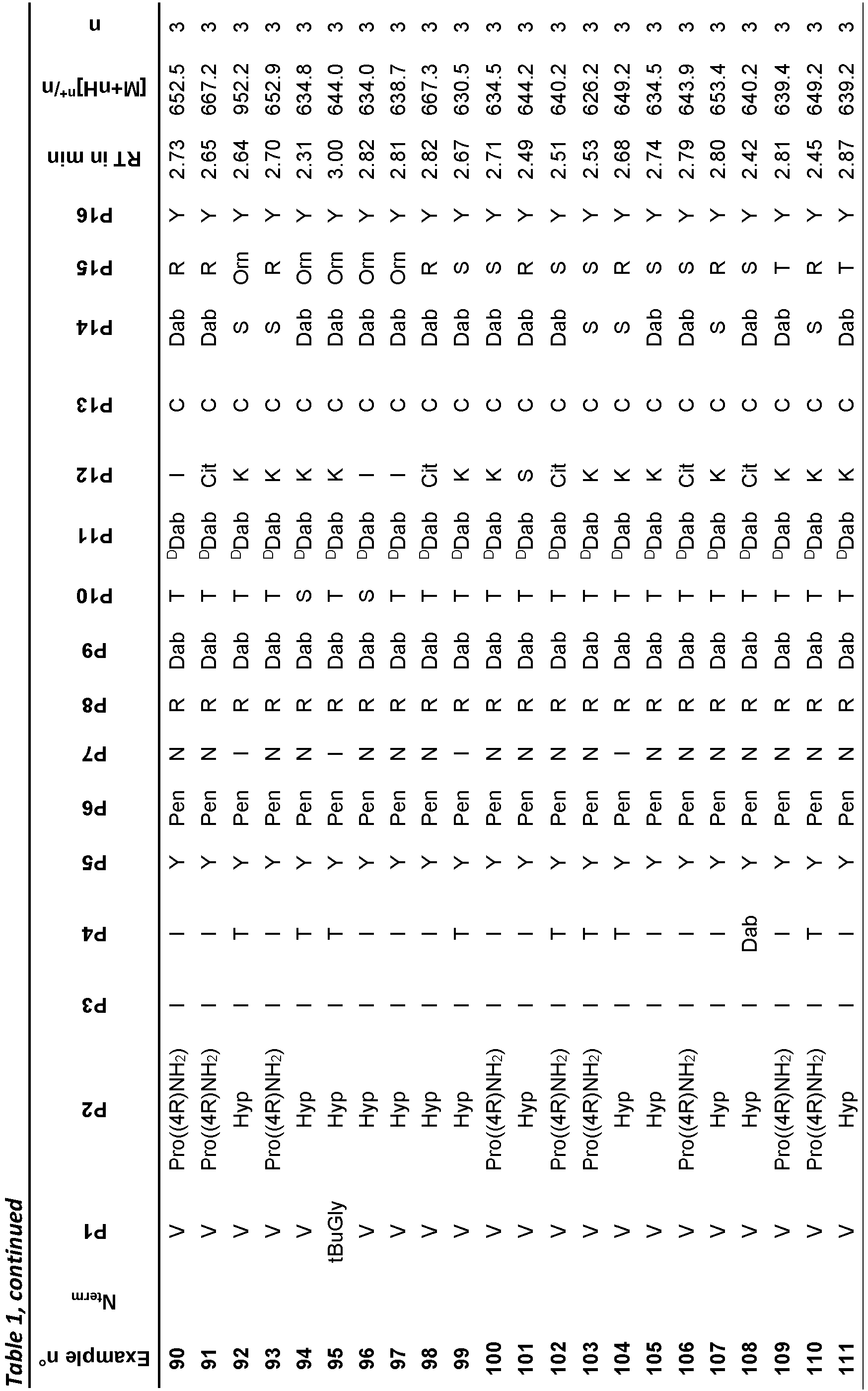

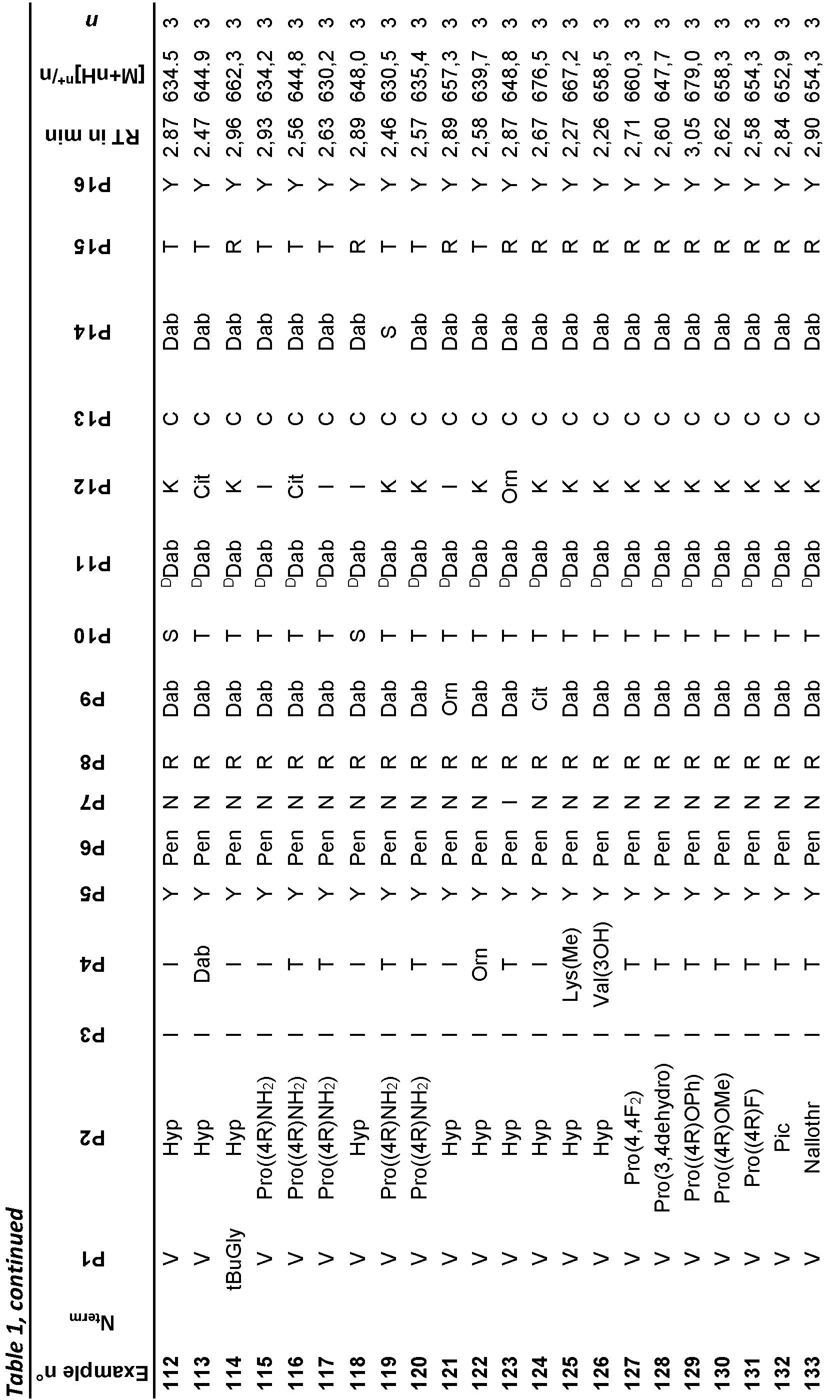

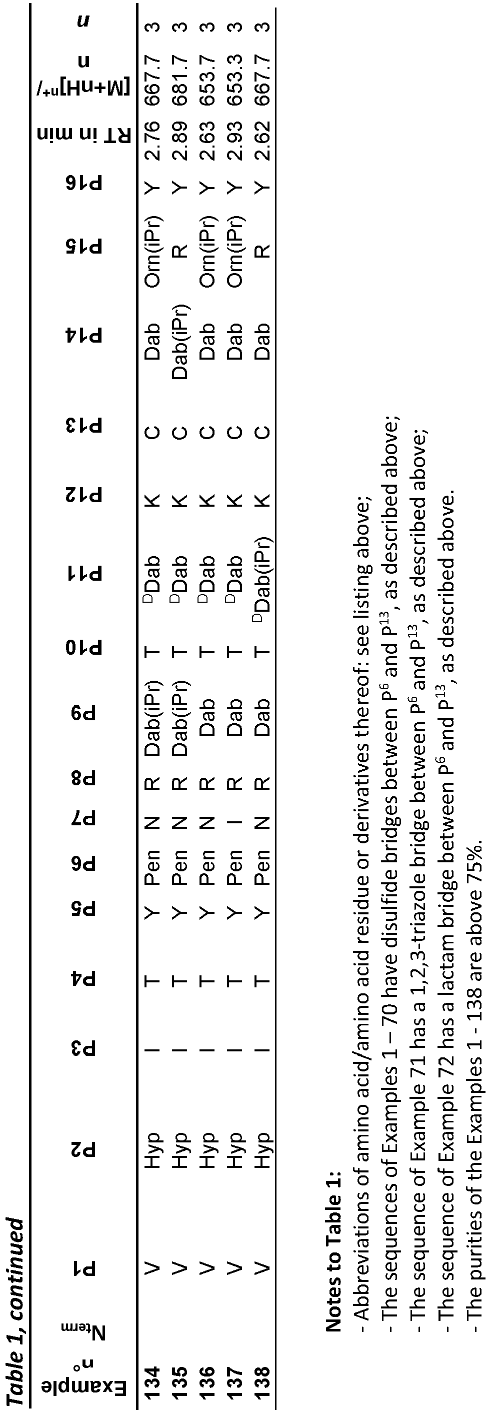

### 2. Biological methods

### 2.1. Preparation of the peptides

Lyophilized peptides were weighed on a Microbalance (Mettler MT5) and dissolved in sterile water to a final concentration of 1 mg/mL. Stock solutions were kept at +4 °C, light protected.

### 2.2. Antimicrobial activity of the peptides

The selective antimicrobial activities of the peptides were determined in 96-well plates (Greiner, polystyrene) by the standard CLSI broth microdilution method (Clinical and Laboratory Standards Institute. *Methods for Dilution Antimicrobial Susceptibility Tests for Bacteria That Grow Aerobically; Approved Standard-Ninth Edition.* CLSI document M07-A9 (ISBN 1-56238-783-9 [Print]; ISBN 1-56238-784-7 [Electronic]). Clinical and Laboratory Standards Institute, 950 West Valley Road, Suite 2500, Wayne, Pennsylvania 19087, USA, 2012) with slight modifications.

Colonies of the microorganisms were diluted in saline (0.85%, NaCl) and and adjusted using a McFarland reader (bioMérieux SA, Marcy-I'Etoile, France) to 0.5 McFarland standard. Subsequently, the bacterial suspension was diluted in Mueller-Hinton II (MHII, cation adjusted) broth to give approximately 5x 10⁵ colony forming units (CFU/mL).

Inocula of the microorganisms were diluted into Mueller-Hinton II (MH, cation adjusted) broth and compared with a 0.5 McFarland standard to give appr. 10⁶ colony forming units (CFU)/mL. Aliquots (90 µl) of inoculate were added to 10 µl of water + P-80 (Polysorbate 80, 0.002% final concentration) containing the peptide in serial two-fold dilutions at 10 fold final concentration. The following microorganisms were used to determine antibiotic selectivity of the peptides: *Escherichia coli* ATCC 25922, *Escherichia coli* MCR-1 Af 45 and *Klebsiella pneumoniae* SSI3010. Antimicrobial activities of the peptides were expressed as the minimal inhibitory concentration (MIC) in µg/mL at which no visible growth was observed after 18-20 hours of incubation at 35 °C.

### 2.3. Hemolysis

The peptides were tested for their hemolytic activity against human red blood cells (hRBC). Fresh hRBC were washed three times with phosphate buffered saline (PBS) and centrifuged for 5 min at 3000 x g. Compounds (200 µg/mL) were incubated with 20% hRBC (v/v) for 1 h at 37 °C and shaking at 300 rpm. A value of 0% and 100% cell lyses, respectively, was determined by incubation of hRBC in the presence of PBS and 2.5% Triton X-100 in H₂O respectively. The samples were centrifuged, the supernatants were 8-fold diluted in PBS buffer and the optical densities (OD) were measured at 540 nm. The 100% lyses value (OD₅₄₀H₂O gave an OD₅₄₀ of approximately 0.5-1.0.

Percent hemolysis was calculated as follows: (OD₅₄₀peptide/OD₅₄₀H₂O) x100%.

The results of the experiments described in **2.2 - 2.3** are indicated in ***Table 2*** herein below.

**Table 2: Minimal inhibitory concentrations (MIC) in Mueller-Hinton broth II and hemolysis**

| **Example n°** | ***Escherichia coli* ATCC 25922 MIC [mg/L]** | ***Escherichia coli* MCR-1 Af 45 MIC [mg/L]** | ***Klebsiella pneumoniae* SSI3010 MIC [mg/L]** | **Hemolysis at 0.2 g/L [%]** |
|---|---|---|---|---|
| **Thanatin** | 2 | 1 | 2 | 0 |
| **1** | 0.0625 | 0.0625 | 0.125 | 0 |
| **2** | 0.0625 | 0.0625 | 0.125 | 0 |
| **3** | 0.125 | 0.0625 | 0.125 | 0 |
| **4** | 0.125 | 0.0625 | 0.25 | 0 |
| **5** | 0.125 | 0.0625 | 0.25 | 0 |
| **6** | 0.0625 | 0.0625 | 0.125 | 0 |
| **7** | 0.0625 | 0.0625 | 0.125 | 0 |
| **8** | 0.0625 | 0.0625 | 0.25 | 0 |
| **9** | 0.125 | 0.0625 | 0.0625 | 0 |
| **10** | 0.0625 | 0.03125 | 0.125 | 0 |
| **11** | 0.0625 | 0.25 | 0.125 | 0 |
| **12** | 0.0625 | 0.0625 | 0.125 | 0 |
| **13** | 0.5 | 0.5 | 0.5 | 0 |
| **14** | 0.125 | 0.0625 | 0.25 | 0 |
| **15** | ≤0.125 | 0.125 | 0.25 | 0 |
| **16** | 0.25 | 0.25 | 0.5 | 0 |
| **17** | 0.25 | 0.125 | 0.5 | 0 |
| **18** | 0.25 | 0.25 | 0.5 | 0 |
| **19** | 0.25 | 0.25 | 0.25 | 0 |
| **20** | 0.125 | 0.5 | 0.5 | 0 |
| **21** | 0.25 | 0.25 | 0.5 | 0 |
| **22** | 0.125 | 0.0625 | 0.25 | 0 |
| **23** | 0.25 | 0.25 | 0.5 | 0 |
| **24** | 0.125 | 0.125 | 0.5 | 0 |
| **25** | 0.125 | 0.125 | 0.5 | 0 |
| **26** | 0.125 | 0.25 | 0.5 | 0 |
| **27** | 0.25 | 0.25 | 0.5 | 0 |
| **28** | 0.25 | 0.125 | 0.5 | 0 |
| **29** | 0.25 | 0.125 | 0.25 | 0 |
| **30** | 0.125 | 0.125 | 0.5 | 0 |
| **31** | 0.125 | 0.25 | 0.25 | 0 |
| **32** | 0.125 | 0.125 | 0.5 | 0 |
| **33** | 0.25 | 0.125 | 0.5 | 0 |
| **34** | 0.25 | 0.125 | 0.5 | 0 |
| **35** | 0.125 | 0.5 | 0.25 | 0 |
| **36** | 0.5 | 0.25 | 0.5 | 0 |
| **37** | 0.25 | 0.5 | 0.5 | 0 |
| **38** | 0.5 | 0.5 | 0.25 | 0 |
| **39** | 0.5 | 0.5 | 0.5 | 0 |
| **40** | 0.25 | 0.25 | 0.5 | 0 |
| **41** | 0.125 | 0.0625 | 0.25 | 0 |
| **42** | 0.25 | 0.25 | 0.5 | 0 |
| **43** | 0.25 | 0.25 | 0.5 | 0 |
| **44** | 0.125 | 0.125 | 0.25 | 0 |
| **45** | 0.25 | 0.125 | 0.5 | 0 |
| **46** | 0.125 | 0.0625 | 0.25 | 0 |
| **47** | 0.25 | 0.25 | 0.5 | 1 |
| **48** | 0.125 | 0.125 | 0.25 | 0 |
| **49** | 0.0625 | 0.125 | 0.25 | 0 |
| **50** | 0.25 | 0.125 | 0.5 | 0 |
| **51** | 0.25 | 0.0625 | 0.25 | 0 |
| **52** | 0.25 | 0.125 | 0.5 | 0 |
| **53** | 0.125 | 0.125 | ≤0.25 | 0 |
| **54** | 0.125 | 0.125 | 0.125 | 0 |
| **55** | 0.125 | ≤0.125 | 0.25 | 0 |
| **56** | 0.03125 | 0.03125 | 0.0625 | 0 |
| **57** | 0.0625 | 0.0625 | 0.125 | 0 |
| **58** | 0.0625 | 0.0625 | 0.0625 | 0 |
| **59** | 0.25 | 0.25 | 0.25 | 0 |
| **60** | 0.25 | 0.25 | 0.5 | 0 |
| **61** | 0.25 | 0.125 | 0.25 | 0 |
| **62** | 0.25 | 0.0625 | 0.125 | 0 |
| **63** | 0.125 | 0.0625 | 0.125 | 0 |
| **64** | 0.0625 | 0.0625 | 0.125 | 0 |
| **65** | 0.125 | 0.0625 | 0.125 | 0 |
| **66** | 0.25 | 0.125 | 0.25 | 0 |
| **67** | 0.5 | 0.125 | 0.125 | 0 |
| **68** | 0.5 | 0.25 | 0.5 | 0 |
| **69** | 0.0625 | 0.0625 | 0.25 | 0 |
| **70** | 0.25 | 0.125 | 0.5 | 0 |
| **71** | 0.25 | 0.25 | 2 | 0 |
| **72** | 0.125 | 0.125 | 0.25 | 0 |
| **73** | 0.5 | 0.25 | 0.5 | <1 |
| **74** | 0.125 | 0.0625 | 0.25 | <1 |
| **75** | 0.0625 | 0.0625 | 0.125 | <1 |
| **76** | 0.0625 | 0.0625 | 0.25 | <1 |
| **77** | 0.0625 | 0.0625 | 0.125 | <1 |
| **78** | 0.0625 | 0.0625 | 0.125 | <1 |
| **79** | 0.125 | 0.125 | 0.5 | <1 |
| **80** | 0.125 | 0.0625 | 0.25 | <1 |
| **81** | 0.125 | 0.0625 | 0.5 | <1 |
| **82** | 0.125 | 0.125 | 0.25 | <1 |
| **83** | 0.25 | 0.25 | 0.5 | <1 |
| **84** | 0.25 | 0.25 | 0.5 | <1 |
| **85** | 0.125 | 0.0625 | 0.25 | <1 |
| **86** | 0.125 | 0.0625 | 0.25 | <1 |
| **87** | 0.125 | 0.125 | 0.25 | <1 |
| **88** | 0.125 | 0.0625 | 0.25 | <1 |
| **89** | 0.0625 | 0.0625 | 0.25 | <1 |
| **90** | 0.125 | 0.0625 | 0.25 | <1 |
| **91** | 0.25 | 0.03125 | 0.25 | <1 |
| **92** | 0.25 | 0.125 | 0.5 | <1 |
| **93** | 0.25 | 0.0625 | 0.25 | <1 |
| **94** | 0.0625 | 0.0625 | 0.25 | <1 |
| **95** | 0.125 | 0.125 | 0.25 | <1 |
| **96** | 0.25 | 0.25 | 0.5 | <1 |
| **97** | 0.25 | 0.25 | 0.5 | <1 |
| **98** | 0.25 | 0.25 | 0.5 | <1 |
| **99** | 0.125 | 0.125 | 0.25 | <1 |
| **100** | 0.25 | 0.0625 | 0.25 | <1 |
| **101** | 0.125 | 0.125 | 0.25 | <1 |
| **102** | 0.25 | 0.125 | 0.25 | <1 |
| **103** | 0.5 | 0.125 | 0.5 | <1 |
| **104** | 0.125 | 0.125 | 0.25 | <1 |
| **105** | 0.25 | 0.125 | 0.5 | <1 |
| **106** | 0.25 | 0.25 | 0.25 | <1 |
| **107** | 0.25 | 0.25 | 0.5 | <1 |
| **108** | 0.25 | 0.125 | 0.25 | <1 |
| **109** | 0.125 | 0.125 | 0.25 | <1 |
| **110** | 0.5 | 0.125 | 0.5 | <1 |
| **111** | 0.25 | 0.125 | 0.5 | <1 |
| **112** | 0.25 | 0.0625 | 0.25 | <1 |
| **113** | 0.125 | 0.125 | 0.25 | <1 |
| **114** | 0.125 | 0.125 | 0.5 | <1 |
| **115** | 0.5 | 0.125 | 0.5 | <1 |
| **116** | 0.25 | 0.25 | 0.5 | <1 |
| **117** | 0.125 | 0.125 | 0.25 | <1 |
| **118** | 0.25 | 0.25 | 0.25 | <1 |
| **119** | 0.25 | 0.25 | 0.25 | <1 |
| **120** | 0.25 | 0.125 | 0.5 | <1 |
| **121** | 0.25 | 0.25 | 0.5 | <1 |
| **122** | 0.25 | 0.125 | 0.5 | <1 |
| **123** | 0.0625 | 0.125 | 0.125 | <1 |
| **124** | 0.25 | 0.25 | 0.5 | <1 |
| **125** | 0.125 | 0.03125 | 0.5 | <1 |
| **126** | 0.0625 | 0.0625 | 0.125 | <1 |
| **127** | 0.25 | 0.125 | 0.5 | <1 |
| **128** | 0.125 | 0.125 | 0.25 | <1 |
| **129** | 0.125 | 0.0625 | 0.5 | <1 |
| **130** | 0.125 | 0.125 | 0.25 | <1 |
| **131** | 0.25 | 0.125 | 0.5 | <1 |
| **132** | 0.125 | 0.125 | 0.25 | <1 |
| **133** | 0.25 | 0.25 | 0.5 | <1 |
| **134** | 0,125 | 0,25 | 0,5 | 1 |
| **135** | 0,25 | 0,25 | 0,5 | <1 |
| **136** | 0,125 | 0,25 | 0,5 | <1 |
| **137** | 0,125 | 0,125 | 0,25 | <1 |
| **138** | 0,125 | 0,125 | 0,5 | <1 |

| | | | | |
|---|---|---|---|---|
| **Note to Table 2:** - Thanatin was synthesized according to a standard solid phase peptide synthesis (SPPS) procedure. | | | | |

## Claims

1. A peptidomimetic compound of the general formula (I),
P¹-P²-P³-P⁴-P⁵-P⁶-P⁷-P⁸-P⁹-P¹⁰-P¹¹-P¹²-P¹³-P¹⁴-P¹⁵-P¹⁶ (I)
P¹ is 2OHVal, Val, NMeVal, Abu, tBuGly;
wherein the amino group of P¹ is optionally replaced by a guanidino group (Gua) or by a tetramethyl guanidino (TMG) group;
P² is Pro, Pro(4R)F, Pro(4R)OMe, Pro(4R)OPhe, Pro(3,4dehydro), Pr(4,4F2), Pic;
Agb, Agp, Dab, Dab(iPr), Dap, Dap(iPr), Har, Lys, Lys(iPr), Narg, Ndab, Nlys, Norn, Orn, Orn(iPr), Pro((4R)guanidine), Pro((4R)NH₂), Pro((4S)NH₂), Arg, NMeLys; Hyp, N*allo*Thr;
P³ is Hle, Ile, Leu, Nle, Cyg;
P⁴ is Ala, Ala(cPr), Ala(tetrahydropyran4yl), Abu, allolle, Cha, Cpa, Cpg, Cyg, Dea, Hle, Ile, Leu, Met, Nle, OctGly, tBuGly, tBuAla, Val, NMeAla, NMeVal, Nva;
Phe, His, Phe(3OH), Phe(4F), Phe(4OCF3), Trp(6Cl), Tyr(3Cl), Tyr(3F), Tyr(Phenyl), Trp, Tyr, 4Thz, Phe(4(4hydroxyphenoxy)), Phe(4NH2), Tyr(Me), Ntyr, Nphe;
Agb, Agp, Dab, Dab(iPr), Dap, Dap(iPr), Har, Lys, Lys(iPr), Narg, Ndab, Nlys, Norn, Orn, Orn(iPr), Arg, NMeLys, Lys(Me);
alloThr, Cit, Hgn, Hse, Leu((3R)OH), Asn, Gln, Ser, Thr, Asp, Glu, Hgl, Val(3OH);
P⁵ is Phe, His, Trp, Tyr;
P⁶ is Pra, Abu(4N₃);
Dab, Dap;
Cys, Hcy, NMeCys, Pen;
Asp, Glu, Hgl;
P⁷ is Ala, Ala(cPr), Ala(tetrahydropyran4yl), Abu, allolle, Cha, Cpa, Cpg, Cyg, Dea, Hle, Ile, Leu, Met, Nle, OctGly, tBuGly, tBuAla, Val, NMeAla, NMeVal, Nva;
Phe, His, Phe(3OH), Phe(4F), Phe(4OCF3), Trp(6Cl), Tyr(3Cl), Tyr(3F), Tyr(Phenyl), Trp, Tyr, 4Thz, Phe(4(4hydroxyphenoxy)), Phe(4NH2), Tyr(Me), Ntyr, Nphe;
Agb, Agp, Dab, Dab(iPr), Dap, Dap(iPr), Har, Lys, Lys(iPr), Narg, Ndab, Nlys, Norn, Orn, Orn(iPr), Arg, NMeLys;
alloThr, Cit, Hgn, Hse, Leu((3R)OH), Asn, Gln, Ser, Thr, Asp, Glu, Hgl;
P⁸ is Agb, Har, Arg. Lys, Orn;
P⁹ is Dab, Dap, Har, Lys, Orn, Arg, Dab(iPr);
Cit;
P¹⁰ is alloThr, Hse, Ser, Thr, Hyp, Gln, Asn, Glu, Asp;
P¹¹ is ^{D}Dab, ^{D}Dap, ^{D}Lys, ^{D}Orn, ^{D}Arg, ^{D}Dab(iPr);
P¹² is Ala, Ala(cPr), Ala(tetrahydropyran4yl), Abu, allolle, Cha, Cpa, Cpg, Cyg, Dea, Hle, Ile, Leu, Met, Nle, OctGly, tBuGly, tBuAla, Val, NMeAla, NMeVal, Nva;
Phe, His, Phe(3OH), Phe(4F), Phe(4OCF3), Trp(6Cl), Tyr(3Cl), Tyr(3F), Tyr(Phenyl), Trp, Tyr, 4Thz, Phe(4(4hydroxyphenoxy)), Phe(4NH2), Tyr(Me), Ntyr, Nphe;
Agb, Agp, Dab, Dab(iPr), Dap, Dap(iPr), Har, Lys, Lys(iPr), Narg, Ndab, Nlys, Norn, Orn, Orn(iPr), Arg, NMeLys;
alloThr, Cit, Hgn, Hse, Leu((3R)OH), Asn, Gln, Ser, Thr, Asp, Glu, Hgl;
P¹³ is Pra, Abu(4N₃);
Dab, Dap;
Cys, Hcy, NMeCys, Pen;
Asp, Glu, Hgl;
P¹⁴ is Phe, His, Phe(3OH), Phe(4F), Phe(4OCF3), Trp(6Cl), Tyr(3Cl), Tyr(3F), Tyr(Phenyl), Trp, Tyr, 4Thz, Phe(4(4hydroxyphenoxy)), Phe(4NH2), Tyr(Me), Ntyr, Nphe;
Agb, Agp, Dab, Dab(iPr); Dap, Dap(iPr), Har, Lys, Lys(iPr), Narg, Ndab, Nlys, Norn, Orn, Orn(iPr), Arg, NMeLys;
alloThr, Cit, Hgn, Hse, Leu((3R)OH), Asn, Gln, Ser, Thr, Asp, Glu, Hgl;
P¹⁵ is Ala, Ala(cPr), Ala(tetrahydropyran4yl), Abu, allolle, Cha, Cpa, Cpg, Cyg, Dea, Hle, Ile, Leu, Met, Nle, OctGly, tBuGly, tBuAla, Val, NMeAla, NMeVal, Nva;
Agb, Agp, Dab, Dab(iPr), Dap, Dap(iPr), Har, Lys, Lys(iPr), Narg, Ndab, Nlys, Norn, Orn, Orn(iPr), Arg, NMeLys;
alloThr, Cit, Hgn, Hse, Leu((3R)OH), Asn, Gln, Ser, Thr, Asp, Glu, Hgl;
P¹⁶ is Ala, Ala(cPr), Ala(tetrahydropyran4yl), Abu, allolle, Cha, Cpa, Cpg, Cyg, Dea, Hle, Ile, Leu, Met, Nle, OctGly, tBuGly, tBuAla, Val, NMeAla, NMeVal, Nva;
Phe, His, Phe(3OH), Phe(4F), Phe(4OCF3), Trp(6Cl), Tyr(3Cl), Tyr(3F), Tyr(Phenyl), Trp, Tyr, 4Thz, Phe(4(4hydroxyphenoxy)), Phe(4NH2), Tyr(Me), Ntyr, Nphe;
or a tautomer, a rotamer, a salt, a hydrate or a solvate thereof,
wherein
Cys, Hcy, NMeCys or Pen at P⁶, if present, and Cys, Hcy, NMeCys or Pen at P¹³, if present, optionally form a disulfide bridge between P⁶ and P¹³; or wherein
Dab or Dap at P⁶, if present, and Asp, Glu or Hgl at P¹³, if present, optionally form a lactam bridge between P⁶ and P¹³; or wherein
Asp, Glu or Hgl at P⁶, if present, and Dab or Dap at P¹³, if present, optionally form a lactam bridge between P⁶ and P¹³; or wherein
Pra at P⁶, if present, and Abu(4N₃) at P¹³, if present, optionally form a 1,2,3-triazole bridge between P⁶ and P¹³; or wherein
Abu(4N₃) at P⁶, if present, and Pra at P¹³, if present, optionally form a 1,2,3-triazole bridge between P⁶ and P¹³;
with the proviso that at least three amino acid residues among the four amino acid residues at positions P⁹, P¹², P¹⁴ and P¹⁵ are basic amino acid residues selected from Agb, Agp, Dab, Dab(iPr), Dap, Dap(iPr), Har, Lys, Lys(iPr), Narg, Ndab, Nlys, Norn, Orn, Orn(iPr), Arg, NMeLys.

2. The compound according to claim 1, wherein
P¹ is 2OHVal, Val, NMeVal, Abu, tBuGly;
wherein the amino group of P¹ is optionally replaced by a guanidino group (Gua) or by a tetramethyl guanidino (TMG) group;
P² is Pro, Pro(4R)F, Pro(4R)OMe, Pro(4R)OPhe, Pro(3,4dehydro), Pr(4,4F2), Pic;
Agb, Agp, Dab, Dab(iPr), Dap, Dap(iPr), Har, Lys, Lys(iPr), Narg, Ndab, Nlys, Norn, Orn, Orn(iPr), Pro((4R)guanidine), Pro((4R)NH₂), Pro((4S)NH₂), Arg, NMeLys;
Hyp, N*allo*Thr;
P³ is Ile;
P⁴ is Ala, Ala(cPr), Ala(tetrahydropyran4yl), Abu, allolle, Cha, Cpa, Cpg, Cyg, Dea, Hle, Ile, Leu, Met, Nle, OctGly, tBuGly, tBuAla, Val, NMeAla, NMeVal, Nva;
Phe, His, Phe(3OH), Phe(4F), Phe(4OCF3), Trp(6Cl), Tyr(3Cl), Tyr(3F), Tyr(Phenyl), Trp, Tyr, 4Thz, Phe(4(4hydroxyphenoxy)), Phe(4NH2), Tyr(Me), Ntyr, Nphe;
Agb, Agp, Dab, Dab(iPr), Dap, Dap(iPr), Har, Lys, Lys(iPr), Narg, Ndab, Nlys, Norn, Orn, Orn(iPr), Arg, NMeLys, Lys(Me);
alloThr, Cit, Hgn, Hse, Leu((3R)OH), Asn, Gln, Ser, Thr, Asp, Glu, Hgl, Val(3OH);
P⁵ is Phe, His, Trp, Tyr;
P⁶ is Dab, Dap;
Cys, Pen;
Asp, Glu;
P⁷ is Ala, Ala(cPr), Ala(tetrahydropyran4yl), Abu, allolle, Cha, Cpa, Cpg, Cyg, Dea, Hle, Ile, Leu, Met, Nle, OctGly, tBuGly, tBuAla, Val, NMeAla, NMeVal, Nva;
Phe, His, Phe(3OH), Phe(4F), Phe(4OCF3), Trp(6Cl), Tyr(3Cl), Tyr(3F), Tyr(Phenyl), Trp, Tyr, 4Thz, Phe(4(4hydroxyphenoxy)), Phe(4NH2), Tyr(Me), Ntyr, Nphe;
Agb, Agp, Dab, Dab(iPr), Dap, Dap(iPr), Har, Lys, Lys(iPr), Narg, Ndab, Nlys, Norn, Orn, Orn(iPr), Arg, NMeLys;
alloThr, Cit, Hgn, Hse, Leu((3R)OH), Asn, Gln, Ser, Thr, Asp, Glu, Hgl;
P⁸ is Arg;
P⁹ is Dab, Dap, Har, Lys, Orn, Arg, Dab(iPr);
Cit;
P¹⁰ is alloThr, Hse, Ser, Thr;
P¹¹ is ^{D}Dab, ^{D}Dap, ^{D}Lys, ^{D}Orn, ^{D}Arg, ^{D}Dab(iPr);
P¹² is Ala, Ala(cPr), Ala(tetrahydropyran4yl), Abu, allolle, Cha, Cpa, Cpg, Cyg, Dea, Hle, Ile, Leu, Met, Nle, OctGly, tBuGly, tBuAla, Val, NMeAla, NMeVal, Nva;
Phe, His, Phe(3OH), Phe(4F), Phe(4OCF3), Trp(6Cl), Tyr(3Cl), Tyr(3F), Tyr(Phenyl), Trp, Tyr, 4Thz, Phe(4(4hydroxyphenoxy)), Phe(4NH2), Tyr(Me), Ntyr, Nphe;
Agb, Agp, Dab, Dab(iPr), Dap, Dap(iPr), Har, Lys, Lys(iPr), Narg, Ndab, Nlys, Norn, Orn, Orn(iPr), Arg, NMeLys;
alloThr, Cit, Hgn, Hse, Leu((3R)OH), Asn, Gln, Ser, Thr, Asp, Glu, Hgl;
P¹³ is Dab, Dap;
Cys, Pen;
Asp, Glu;
P¹⁴ is Phe, His, Phe(3OH), Phe(4F), Phe(4OCF3), Trp(6Cl), Tyr(3Cl), Tyr(3F), Tyr(Phenyl), Trp, Tyr, 4Thz, Phe(4(4hydroxyphenoxy)), Phe(4NH2), Tyr(Me), Ntyr, Nphe;
Agb, Agp, Dab, Dab(iPr); Dap, Dap(iPr), Har, Lys, Lys(iPr), Narg, Ndab, Nlys, Norn, Orn, Orn(iPr), Arg, NMeLys;
alloThr, Cit, Hgn, Hse, Leu((3R)OH), Asn, Gln, Ser, Thr, Asp, Glu, or Hgl;
P¹⁵ is Ala, Ala(cPr), Ala(tetrahydropyran4yl), Abu, allolle, Cha, Cpa, Cpg, Cyg, Dea, Hle, Ile, Leu, Met, Nle, OctGly, tBuGly, tBuAla, Val, NMeAla, NMeVal, Nva;
Agb, Agp, Dab, Dab(iPr), Dap, Dap(iPr), Har, Lys, Lys(iPr), Narg, Ndab, Nlys, Norn, Orn, Orn(iPr), Arg, NMeLys;
alloThr, Cit, Hgn, Hse, Leu((3R)OH), Asn, Gln, Ser, Thr, Asp, Glu, or Hgl;
P¹⁶ is Ala, Ala(cPr), Ala(tetrahydropyran4yl), Abu, allolle, Cha, Cpa, Cpg, Cyg, Dea, Hle, Ile, Leu, Met, Nle, OctGly, tBuGly, tBuAla, Val, NMeAla, NMeVal, Nva;
Phe, His, Phe(3OH), Phe(4F), Phe(4OCF3), Trp(6Cl), Tyr(3Cl), Tyr(3F), Tyr(Phenyl), Trp, Tyr, 4Thz, Phe(4(4hydroxyphenoxy)), Phe(4NH2), Tyr(Me), Ntyr, Nphe;
or a tautomer, a rotamer, a salt, a hydrate or a solvate thereof,
wherein
Cys or Pen at P⁶, if present, and Cys or Pen at P¹³, if present, optionally form a disulfide bridge between P⁶ and P¹³; or wherein
Dab or Dap at P⁶, if present, and Asp or Glu at P¹³, if present, optionally form a lactam bridge between P⁶ and P¹³; or wherein
Asp or Glu at P⁶, if present, and Dab or Dap at P¹³, if present, optionally form a lactam bridge between P⁶ and P¹³;
with the proviso that at least three amino acid residues among the four amino acid residues at positions P⁹, P¹², P¹⁴ and P¹⁵ are basic amino acid residues selected from Agb, Agp, Dab, Dab(iPr), Dap, Dap(iPr), Har, Lys, Lys(iPr), Narg, Ndab, Nlys, Norn, Orn, Orn(iPr), Arg, NMeLys.

3. The compound according to claim 1 or claim 2 , wherein
P¹ is 2OHVal, Val, NMeVal, Abu, tBuGly;
wherein the amino group of P¹ is optionally replaced by a guanidino group (Gua) or by a tetramethyl guanidino (TMG) group;
P² is Pro, Pro(4R)F, Pro(4R)OMe, Pro(4R)OPhe, Pro(3,4dehydro), Pr(4,4F2), Pic;
Agb, Agp, Dab, Dab(iPr), Dap, Dap(iPr), Lys, Lys(iPr), Narg, Ndab, Nlys, Norn, Orn, Orn(iPr), Pro((4R)NH₂), Arg, NMeLys;
Hyp, N*allo*Thr;
P³ is *Hle,* Ile, Leu, Nle;
P⁴ is Ala, Ala(cPr), Ala(tetrahydropyran4yl), Abu, allolle, Cyg, Dea, Ile, Leu, Nle, tBuGly, tBuAla, Val, NMeAla, NMeVal, Nva;
Phe, His, Phe(3OH), Phe(4F), Trp(6Cl), Trp, Tyr, 4Thz, Phe(4(4hydroxyphenoxy)), Phe(4NH2), Tyr(Me), Ntyr, Nphe;
Agb, Agp, Dab, Dab(iPr), Dap, Dap(iPr), Lys, Lys(iPr), Narg, Ndab, Nlys, Norn, Orn, Orn(iPr), Arg, NMeLys, Lys(Me);
alloThr, Cit, Hgn, Hse, Leu((3R)OH), Asn, Gln, Ser, Thr, Asp, Glu, Hgl, Val(3OH);
P⁵ is Phe, His, Trp, Tyr;
P⁶ is Pra, Abu(4N₃);
Dab, Dap;
Cys, Hcy, NMeCys, Pen;
Asp, Glu, Hgl;
P⁷ is Ala, Ala(cPr), Ala(tetrahydropyran4yl), Abu, allolle, Cyg, Dea, Ile, Leu, Nle, tBuGly, tBuAla, Val, NMeAla, NMeVal, Nva;
Phe, His, Phe(3OH), Phe(4F), Trp(6Cl), Trp, Tyr, 4Thz, Phe(4(4hydroxyphenoxy)), Phe(4NH2), Tyr(Me), Ntyr, Nphe;
Agb, Agp, Dab, Dab(iPr), Dap, Dap(iPr), Lys, Lys(iPr), Narg, Ndab, Nlys, Norn, Orn, Orn(iPr), Arg, NMeLys;
alloThr, Cit, Hgn, Hse, Leu((3R)OH), Asn, Gln, Ser, Thr, Asp, Glu, Hgl;
P⁸ is Agb, Har, Arg;
P⁹ is Dab, Dap, Har, Lys, Orn, Arg, Dab(iPr);
Cit;
P¹⁰ is alloThr, Hse, Ser, Thr;
P¹¹ is ^{D}Dab, ^{D}Dap, ^{D}Lys, ^{D}Orn, ^{D}Arg, ^{D}Dab(iPr);
P¹² is Ala, Ala(cPr), Ala(tetrahydropyran4yl), Abu, allolle, Cyg, Dea, Ile, Leu, Nle, tBuGly, tBuAla, Val, NMeAla, NMeVal, Nva;
Phe, His, Phe(3OH), Phe(4F), Trp(6Cl), Tyr(Phenyl), Trp, Tyr, 4Thz, Phe(4(4hydroxyphenoxy)), Phe(4NH2), Tyr(Me), Ntyr, Nphe;
Agb, Agp, Dab, Dab(iPr), Dap, Dap(iPr), Lys, Lys(iPr), Narg, Ndab, Nlys, Norn, Orn, Orn(iPr), Arg, NMeLys;
alloThr, Cit, Hgn, Hse, Leu((3R)OH), Asn, Gln, Ser, Thr, Asp, Glu, Hgl;
P¹³ is Pra, Abu(4N₃);
Dab, Dap;
Cys, Hcy, NMeCys, Pen;
Asp, Glu, Hgl;
P¹⁴ is Phe, His, Phe(3OH), Phe(4F), Trp(6Cl), Trp, Tyr, 4Thz, Phe(4(4hydroxyphenoxy)), Phe(4NH2), Tyr(Me), Ntyr, Nphe;
Agb, Agp, Dab, Dab(iPr); Dap, Dap(iPr), Lys, Lys(iPr), Narg, Ndab, Nlys, Norn, Orn, Orn(iPr), Arg, NMeLys;
alloThr, Cit, Hgn, Hse, Leu((3R)OH), Asn, Gln, Ser, Thr, Asp, Glu, Hgl;
P¹⁵ is Ala, Ala(cPr), Ala(tetrahydropyran4yl), Abu, allolle, Cyg, Dea, Ile, Leu, Nle, tBuGly, tBuAla, Val, NMeAla, NMeVal, Nva;
Agb, Agp, Dab, Dab(iPr), Dap, Dap(iPr), Lys, Lys(iPr), Narg, Ndab, Nlys, Norn, Orn, Orn(iPr), Arg, NMeLys;
alloThr, Cit, Hgn, Hse, Leu((3R)OH), Asn, Gln, Ser, Thr, Asp, Glu, Hgl;
P¹⁶ is Ala, Ala(cPr), Ala(tetrahydropyran4yl), Abu, allolle, Cyg, Dea, Ile, Leu, Nle, tBuGly, tBuAla, Val, NMeAla, NMeVal, Nva;
Phe, His, Phe(3OH), Phe(4F), Trp(6Cl), Trp, Tyr, 4Thz, Phe(4(4hydroxyphenoxy)), Phe(4NH2), Tyr(Me), Ntyr, Nphe;
or a tautomer, a rotamer, a salt, a hydrate or a solvate thereof,
wherein
Cys, Hcy, NMeCys or Pen at P⁶, if present, and Cys, Hcy, NMeCys or Pen at P¹³, if present, optionally form a disulfide bridge between P⁶ and P¹³; or wherein
Dab or Dap at P⁶, if present, and Asp, Glu or Hgl at P¹³, if present, optionally form a lactam bridge between P⁶ and P¹³; or wherein
Asp, Glu or Hgl at P⁶, if present, and Dab or Dap at P¹³, if present, optionally form a lactam bridge between P⁶ and P¹³; or wherein
Pra at P⁶, if present, and Abu(4N₃) at P¹³, if present, optionally form a 1,2,3-triazole bridge between P⁶ and P¹³; or wherein
Abu(4N₃) at P⁶, if present, and Pra at P¹³, if present, optionally form a 1,2,3-triazole bridge between P⁶ and P¹³;
with the proviso that at least three amino acid residues among the four amino acid residues at positions P⁹, P¹², P¹⁴ and P¹⁵ are basic amino acid residues selected from Agb, Agp, Dab, Dab(iPr), Dap, Dap(iPr), Lys, Lys(iPr), Narg, Ndab, Nlys, Norn, Orn, Orn(iPr), Arg, NMeLys.

4. The compound according to any one of claims 1 to 3, wherein
P¹ is 2OHVal, Val, NMeVal, Abu, tBuGly;
wherein the amino group of P¹ is optionally replaced by a guanidino group (Gua) or by a tetramethyl guanidino (TMG) group;
P² is Pro, Pro(4R)F, Pro(4R)OMe, Pro(4R)OPhe, Pro(3,4dehydro), Pr(4,4F2), Pic;
Agb, Agp, Dab, Dab(iPr), Dap, Dap(iPr), Lys, Lys(iPr), Narg, Ndab, Nlys, Norn, Orn, Orn(iPr), Pro((4R)NH₂), Arg, NMeLys;
Hyp, N*allo*Thr;
P³ is Ile;
P⁴ is Ala, Ala(cPr), Ala(tetrahydropyran4yl), Abu, allolle, Cyg, Dea, Ile, Leu, Nle, tBuGly, tBuAla, Val, NMeAla, NMeVal, Nva;
Phe, His, Phe(3OH), Phe(4F), Trp(6Cl), Trp, Tyr, 4Thz, Phe(4(4hydroxyphenoxy)), Phe(4NH2), Tyr(Me), Ntyr, Nphe;
Agb, Agp, Dab, Dab(iPr), Dap, Dap(iPr), Lys, Lys(iPr), Narg, Ndab, Nlys, Norn, Orn, Orn(iPr), Arg, NMeLys; Lys(Me);
alloThr, Cit, Hgn, Hse, Leu((3R)OH), Asn, Gln, Ser, Thr, Asp, Glu, Hgl, Val(3OH);
P⁵ is Phe, His, Trp, Tyr;
P⁶ is Dab, Dap;
Cys, Pen;
Asp, Glu;
P⁷ is Ala, Ala(cPr), Ala(tetrahydropyran4yl), Abu, allolle, Cyg, Dea, Ile, Leu, Nle, tBuGly, tBuAla, Val, NMeAla, NMeVal, Nva;
Phe, His, Phe(3OH), Phe(4F), Trp(6Cl), Trp, Tyr, 4Thz, Phe(4(4hydroxyphenoxy)), Phe(4NH2), Tyr(Me), Ntyr, Nphe;
Agb, Agp, Dab, Dab(iPr), Dap, Dap(iPr), Lys, Lys(iPr), Narg, Ndab, Nlys, Norn, Orn, Orn(iPr), Arg, NMeLys;
alloThr, Cit, Hgn, Hse, Leu((3R)OH), Asn, Gln, Ser, Thr, Asp, Glu, Hgl;
P⁸ is Arg;
P⁹ is Dab, Dap, Har, Lys, Orn, Arg, Dab(iPr);
Cit;
P¹⁰ is alloThr, Hse, Ser, Thr;
P¹¹ is ^{D}Dab, ^{D}Dap, ^{D}Lys, ^{D}Orn, ^{D}Arg, ^{D}Dab(iPr);
P¹² is Ala, Ala(cPr), Ala(tetrahydropyran4yl), Abu, allolle, Cyg, Dea, Ile, Leu, Nle, tBuGly, tBuAla, Val, NMeAla, NMeVal, Nva;
Phe, His, Phe(3OH), Phe(4F), Trp(6Cl), Tyr(Phenyl), Trp, Tyr, 4Thz, Phe(4(4hydroxyphenoxy)), Phe(4NH2), Tyr(Me), Ntyr, Nphe;
Agb, Agp, Dab, Dab(iPr), Dap, Dap(iPr), Lys, Lys(iPr), Narg, Ndab, Nlys, Norn, Orn, Orn(iPr), Arg, NMeLys;
alloThr, Cit, Hgn, Hse, Leu((3R)OH), Asn, Gln, Ser, Thr, Asp, Glu, Hgl;
P¹³ is Dab, Dap;
Cys, Pen;
Asp, Glu;
P¹⁴ is Phe, His, Phe(3OH), Phe(4F), Trp(6Cl), Trp, Tyr, 4Thz, Phe(4(4hydroxyphenoxy)), Phe(4NH2), Tyr(Me), Ntyr, Nphe;
Agb, Agp, Dab, Dab(iPr); Dap, Dap(iPr), Lys, Lys(iPr), Narg, Ndab, Nlys, Norn, Orn, Orn(iPr), Arg, NMeLys;
alloThr, Cit, Hgn, Hse, Leu((3R)OH), Asn, Gln, Ser, Thr, Asp, Glu, or Hgl;
P¹⁵ is Ala, Ala(cPr), Ala(tetrahydropyran4yl), Abu, allolle, Cyg, Dea, Ile, Leu, Nle, tBuGly, tBuAla, Val, NMeAla, NMeVal, Nva;
Agb, Agp, Dab, Dab(iPr), Dap, Dap(iPr), Lys, Lys(iPr), Narg, Ndab, Nlys, Norn, Orn, Orn(iPr), Arg, NMeLys;
alloThr, Cit, Hgn, Hse, Leu((3R)OH), Asn, Gln, Ser, Thr, Asp, Glu, Hgl;
P¹⁶ is Ala, Ala(cPr), Ala(tetrahydropyran4yl), Abu, allolle, Cyg, Dea, Ile, Leu, Nle, tBuGly, tBuAla, Val, NMeAla, NMeVal, Nva;
Phe, His, Phe(3OH), Phe(4F), Trp(6Cl), Trp, Tyr, 4Thz, Phe(4(4hydroxyphenoxy)), Phe(4NH2), Tyr(Me), Ntyr, Nphe;
or a tautomer, a rotamer, a salt, a hydrate or a solvate thereof,
wherein
Cys or Pen at P⁶, if present, and Cys or Pen at P¹³, if present, optionally form a disulfide bridge between P⁶ and P¹³; or wherein
Dab or Dap at P⁶, if present, and Asp or Glu at P¹³, if present, optionally form a lactam bridge between P⁶ and P¹³; or wherein
Asp or Glu at P⁶, if present, and Dab or Dap at P¹³, if present, optionally form a lactam bridge between P⁶ and P¹³;
with the proviso that at least three amino acid residues among the four amino acid residues at positions P⁹, P¹², P¹⁴ and P¹⁵ are basic amino acid residues selected from Agb, Agp, Dab, Dab(iPr), Dap, Dap(iPr), Lys, Lys(iPr), Narg, Ndab, Nlys, Norn, Orn, Orn(iPr), Arg, NMeLys.

5. The compound according to any one of claims 1 to 4, wherein
P¹ is Val, 2OHVal, NMeVal, Gua-Val, TMG-Val, Abu, or tBuGly;
P² is Pro, Pro(4R)F, Pro(4R)OMe, Pro(4R)OPhe, Pro(3,4dehydro), Pr(4,4F2), Pic, Pro((4R)NH₂), Ndab, N*allo*Thr, or Hyp;
P³ is Ile;
P⁴ is Ile, Thr, Phe, Dab, Orn, Arg, Tyr, Leu, Asn, Lys, Lys(Me), Dap, Val(3OH), or alloThr;
P⁵ is Trp or Tyr;
P⁶ is Cys, Pen, Asp, or Pra;
P⁷ is Asn, Ala, Leu, Ile, Ser, Thr, Lys, Dap, Glu, or His;
P⁸ is Arg;
P⁹ is Arg, Dab, Dab(iPr), Lys, or Cit;
P¹⁰ is Ser or Thr;
P¹¹ is ^{D}Dab, ^{D}Dap, ^{D}Orn, ^{D}Lys, ^{D}Arg, or ^{D}Dab(iPr);
P¹² is Lys, Ile, Ser, Tyr, Trp, Asn, Dab, Orn, or Cit;
P¹³ is Cys, Pen, Dab, or Abu(4N₃);
P¹⁴ is Dab, Dab(iPr), Lys, Gln, Ser, or Tyr;
P¹⁵ is Arg, Thr, Leu, Ser, Dab, Lys, Orn, or Orn(iPr); and
P¹⁶ is Nle, Cha, or Tyr;
or a tautomer, a rotamer, a salt, a hydrate or a solvate thereof,
wherein Cys or Pen at P⁶, if present, and Cys or Pen at P¹³, if present, optionally form a disulfide bridge between P⁶ and P¹³, or wherein
Asp at P⁶, if present, and Dab at P¹³, if present, optionally form a lactam bridge between P⁶ and P¹³, or wherein
Pra at P⁶, if present, and Abu(4N₃) at P¹³, if present, optionally form a 1,2,3-triazole bridge between P⁶ and P¹³;
with the proviso that at least three amino acid residues among the four amino acid residues at positions P⁹, P¹², P¹⁴ and P¹⁵ are basic amino acid residues selected from Dab, Lys, Arg, or Dab(iPr) at P⁹, Lys, Dab, or Orn at P¹², Dab, Dab(iPr), or *Lys* at P¹⁴ and Arg, Dab, Lys, Orn or Orn(iPr) at P¹⁵.

6. The compound according to any one of claims 1 to 5, wherein
P¹ is Val, NMeVal, Gua-Val, TMG-Val, or Abu;
P² is Pro, Pro(4R)OMe, Pro(3,4dehydro), Pic, Pro((4R)NH₂), Ndab, N*allo*Thr, or Hyp;
P³ is Ile;
P⁴ is Ile, Thr, Phe, Dab, Arg, Val(3OH), or Tyr;
P⁵ is Tyr;
P⁶ is Cys, Pen, or Asp;
P⁷ is Asn, Leu, Ile, Ser, Dap, or His;
P⁸ is Arg;
P⁹ is Arg, Lys, Dab, or Dab(iPr);
P¹⁰ is Ser or Thr;
P¹¹ is ^{D}Dab, ^{D}Dab(iPr), or ^{D}Arg;
P¹² is Lys, Ile, Ser, Dab, Orn, or *Cit;*
P¹³ is Cys, Pen, or Dab;
P¹⁴ is Dab, Dab(iPr), Lys, Gln, Ser, or Tyr;
P¹⁵ is Arg, Dab, Orn, Orn(iPr), Ser, or Thr;
P¹⁶ is Nle, Cha, or Tyr;
or a tautomer, a rotamer, a salt, a hydrate or a solvate thereof,
wherein Cys or Pen at P⁶, if present, and Cys or Pen at P¹³, if present, optionally form a disulfide bridge between P⁶ and P¹³, or wherein
Asp at P⁶, if present, and Dab at P¹³, if present, optionally form a lactam bridge between P⁶ and P¹³;
with the proviso that at least three amino acid residues among the four amino acid residues at positions P⁹, P¹², P¹⁴ and P¹⁵ are basic amino acid residues selected from Dab, Lys, Arg, or Dab(iPr) at P⁹, Lys, Orn, or Dab at P¹², Lys, Dab or Dab(iPr) at P¹⁴ and Arg, Dab, Orn, or Orn(iPr) at P¹⁵.

7. The compound according to any one of claims 1 to 6, wherein
P¹ is 2OHVal, Gua-Val, or TMG-Val;
P² is Pro, Pro((4R)NH₂), or Hyp;
P³ is Ile;
P⁴ is Ile, Thr, Dab, or Orn;
P⁵ is Tyr;
P⁶ is Cys, Pen, Asp or Pra;
P⁷ is Ile, Asn, or Thr;
P⁸ is Arg;
P⁹ is Lys, Arg, Dab, or Dab(iPr);
P¹⁰ is Ser, or Thr;
P¹¹ is ^{D}Dab, ^{D}Dab(iPr), or ^{D}Dap;
P¹² is Lys, Ile, Asn, Ser, Tyr, Orn, or Cit;
P¹³ is Cys, Dab, or Abu(4N₃);
P¹⁴ is Dab, Dab(iPr), Lys, or Ser;
P¹⁵ is Arg, Dab, Orn; Orn(iPr), Ser, or Thr;
P¹⁶ is Nle, or Tyr;
or a tautomer, a rotamer, a salt, a hydrate or a solvate thereof,
wherein Cys or Pen at P⁶, if present, and Cys at P¹³, if present, optionally form a disulfide bridge between P⁶ and P¹³, or wherein
Asp at P⁶, if present, and Dab at P¹³, if present, optionally form a lactam bridge between P⁶ and P¹³; or wherein
Pra at P⁶, if present, and Abu(4N₃) at P¹³, if present, optionally form a 1,2,3-triazole bridge between P⁶ and P¹³;
with the proviso that at least three amino acid residues among the four amino acid residues at positions P⁹, P¹², P¹⁴ and P¹⁵ are basic amino acid residues selected from Dab, Lys, Arg, or Dab(iPr) at P⁹, Lys, or Orn at P¹², Lys, Dab or Dab(iPr) at P¹⁴ and Arg, Dab, Orn, or Orn(iPr) at P¹⁵.

8. A compound according to claim 1, wherein the compound is selected from the group consisting of:
Val-Pro-Ile-Ile-Tyr-Cys-Asn-Arg-Arg-Thr-^{D}Dab-Lys-Cys-Dab-Arg-Nle;
Val-Pro((4R)NH₂)-Ile-Ile-Tyr-Pen-Asn-Arg-Arg-Thr-^{D}Dab-Lys-Cys-Dab-Arg-Nle;
Val-Pro((4R)NH₂)-Ile-Ile-Tyr-Pen-Asn-Arg-Dab-Thr-^{D}Dab-Lys-Cys-Dab-Arg-Nle;
Val-Ndab-Ile-Ile-Tyr-Pen-Asn-Arg-Dab-Thr-^{D}Dab-Lys-Cys-Dab-Arg-Nle;
Val-Pro((4R)NH₂)-Ile-Thr-Tyr-Pen-Asn-Arg-Dab-Thr-^{D}Dab-Lys-Cys-Dab-Arg-Nle;
Val-Pro-Ile-Ile-Tyr-Cys-Asn-Arg-Dab-Thr-^{D}Dab-Lys-Cys-Dab-Arg-Cha;
Val-Pro-Ile-Ile-Tyr-Pen-Asn-Arg-Dab-Thr-^{D}Dab-Lys-Cys-Dab-Arg-Tyr;
Val-Pro-Ile-Ile-Tyr-Cys-Asn-Arg-Dab-Thr-^{D}Dab-Lys-Pen-Dab-Arg-Tyr;
Val-Pro((4R)NH₂)-Ile-Ile-Tyr-Cys-Asn-Arg-Dab-Thr-^{D}Dab-Lys-Cys-Dab-Arg-Tyr;
Val-Hyp-Ile-Ile-Tyr-Cys-Asn-Arg-Dab-Thr-^{D}Dab-Lys-Cys-Dab-Arg-Tyr;
Val-Pro-Ile-Thr-Tyr-Cys-Asn-Arg-Dab-Thr-^{D}Dab-Lys-Cys-Dab-Arg-Tyr;
Val-Pro-Ile-Phe-Tyr-Cys-Asn-Arg-Dab-Thr-^{D}Dab-Lys-Cys-Dab-Arg-Tyr;
2OHVal-Pro-Ile-Ile-Tyr-Cys-Asn-Arg-Dab-Thr-^{D}Dab-Lys-Cys-Dab-Arg-Tyr;
Val-Hyp-Ile-Ile-Tyr-Pen-Asn-Arg-Dab-Thr-^{D}Dab-Lys-Cys-Dab-Arg-Tyr;
Val-Hyp-Ile-Thr-Tyr-Pen-Asn-Arg-Dab-Thr-^{D}Dab-Lys-Cys-Dab-Arg-Tyr;
Val-Hyp-Ile-Thr-Tyr-Pen-Asn-Arg-Dab-Thr-^{D}Dap-Lys-Cys-Dab-Arg-Tyr;
Val-Hyp-Ile-Thr-Tyr-Pen-Asn-Arg-Dab-Thr-^{D}Orn-Lys-Cys-Dab-Arg-Tyr;
Val-Hyp-Ile-Thr-Tyr-Pen-Asn-Arg-Dab-Thr-^{D}Lys-Lys-Cys-Dab-Arg-Tyr;
Val-Hyp-Ile-Thr-Tyr-Pen-Asn-Arg-Dab-Thr-^{D}Arg-Lys-Cys-Dab-Arg-Tyr;
Val-Hyp-Ile-Dab-Tyr-Pen-Asn-Arg-Dab-Thr-^{D}Dab-Lys-Cys-Dab-Thr-Tyr;
Val-Hyp-Ile-Arg-Tyr-Pen-Asn-Arg-Dab-Thr-^{D}Dab-Lys-Cys-Dab-Thr-Tyr;
Val-Hyp-Ile-Tyr-Tyr-Pen-Asn-Arg-Dab-Thr-^{D}Dab-Lys-Cys-Dab-Arg-Tyr;
Val-Hyp-Ile-Leu-Tyr-Pen-Asn-Arg-Dab-Thr-^{D}Dab-Lys-Cys-Dab-Arg-Tyr;
Val-Hyp-Ile-Asn-Tyr-Pen-Asn-Arg-Dab-Thr-^{D}Dab-Lys-Cys-Dab-Arg-Tyr;
Val-Hyp-Ile-Dab-Tyr-Pen-Asn-Arg-Dab-Thr-^{D}Dab-Lys-Cys-Dab-Arg-Tyr;
Val-Hyp-Ile-Lys-Tyr-Pen-Asn-Arg-Dab-Thr-^{D}Dab-Lys-Cys-Dab-Arg-Tyr;
Val-Hyp-Ile-Thr-Trp-Pen-Asn-Arg-Dab-Thr-^{D}Dab-Lys-Cys-Dab-Arg-Tyr;
Val-Hyp-Ile-Thr-Tyr-Pen-Ala-Arg-Dab-Thr-^{D}Dab-Lys-Cys-Dab-Arg-Tyr;
Val-Hyp-Ile-Thr-Tyr-Pen-Leu-Arg-Dab-Thr-^{D}Dab-Lys-Cys-Dab-Arg-Tyr;
Val-Hyp-Ile-Thr-Tyr-Pen-Ile-Arg-Dab-Thr-^{D}Dab-Lys-Cys-Dab-Arg-Tyr;
Val-Hyp-Ile-Thr-Tyr-Pen-Ser-Arg-Dab-Thr-^{D}Dab-Lys-Cys-Dab-Arg-Tyr;
Val-Hyp-Ile-Thr-Tyr-Pen-Thr-Arg-Dab-Thr-^{D}Dab-Lys-Cys-Dab-Arg-Tyr;
Val-Hyp-Ile-Thr-Tyr-Pen-Lys-Arg-Dab-Thr-^{D}Dab-Lys-Cys-Dab-Arg-Tyr;
Val-Hyp-Ile-Thr-Tyr-Pen-Asn-Arg-Lys-Thr-^{D}Dab-Lys-Cys-Dab-Arg-Tyr;
Val-Hyp-Ile-Thr-Tyr-Pen-Asn-Arg-Arg-Thr-^{D}Dab-Lys-Cys-Dab-Arg-Tyr;
Val-Hyp-Ile-Thr-Tyr-Pen-Asn-Arg-Dab-Thr-^{D}Dab-Ile-Cys-Dab-Arg-Tyr;
Val-Hyp-Ile-Thr-Tyr-Pen-Asn-Arg-Dab-Thr-^{D}Dab-Ser-Cys-Dab-Arg-Tyr;
Val-Hyp-Ile-Thr-Tyr-Pen-Asn-Arg-Dab-Thr-^{D}Dab-Tyr-Cys-Dab-Arg-Tyr;
Val-Hyp-Ile-Thr-Tyr-Pen-Asn-Arg-Dab-Thr-^{D}Dab-Trp-Cys-Dab-Arg-Tyr;
Val-Hyp-Ile-Thr-Tyr-Pen-Asn-Arg-Dab-Thr-^{D}Dab-Asn-Cys-Dab-Arg-Tyr;
Va-Hyp-Ile-Thr-Tyr-Pen-Asn-Arg-Dab-Thr-^{D}Dab-Dab-Cys-Dab-Arg-Tyr;
Val-Hyp-Ile-Thr-Tyr-Pen-Asn-Arg-Dab-Thr-^{D}Dab-Lys-Cys-Dab-Leu-Tyr
Val-Hyp-Ile-Thr-Tyr-Pen-Asn-Arg-Dab-Thr-^{D}Dab-Lys-Cys-Dab-Ser-Tyr;
Val-Hyp-Ile-Thr-Tyr-Pen-Asn-Arg-Dab-Thr-^{D}Dab-Lys-Cys-Dab-Dab-Tyr;
Val-Hyp-Ile-Thr-Tyr-Pen-Asn-Arg-Dab-Thr-^{D}Dab-Lys-Cys-Dab-Lys-Tyr;
Val-Hyp-Ile-Thr-Tyr-Pen-Asn-Arg-Dab-Thr-^{D}Dab-Lys-Cys-Dab-Orn- Tyr;
Val-Hyp-Ile-Thr-Tyr-Pen-Asn-Arg-Dab-Thr-^{D}Dab-Cit-Cys-Dab-Arg-Tyr;
NMeVal-Hyp-Ile-Thr-Tyr-Pen-Asn-Arg-Dab-Thr-^{D}Dab-Lys-Cys-Dab-Arg-Tyr;
Val-Hyp-Ile-Thr-Tyr-Pen-Dap-Arg-Dab-Thr-^{D}Dab-Lys-Cys-Dab-Arg-Tyr;
Val-Hyp-Ile-Dap-Tyr-Pen-Asn-Arg-Dab-Thr-^{D}Dab-Lys-Cys-Dab-Arg-Tyr;
Val-Hyp-Ile-Arg-Tyr-Pen-Asn-Arg-Dab-Thr-^{D}Dab-Lys-Cys-Dab-Arg-Tyr;
Val-Pro((4R)NH₂)-Ile-Thr-Tyr-Pen-Asn-Arg-Dab-Thr-^{D}Dab-Lys-Cys-Tyr-Arg-Tyr;
Gua-Val-Pro-Ile-Ile-Tyr-Cys-Asn-Arg-Dab-Thr-^{D}Dab-Lys-Cys-Dab-Arg-Tyr;
Gua-Val-Pro-Ile-Ile-Tyr-Pen-Asn-Arg-Dab-Thr-^{D}Dab-Lys-Cys-Dab-Arg-Tyr;
Gua-Val-Hyp-Ile-Thr-Tyr-Pen-Asn-Arg-Dab-Thr-^{D}Dab-Lys-Cys-Dab-Arg-Tyr;
Gua-Val-Hyp-Ile-Ile-Tyr-Pen-Asn-Arg-Dab-Thr-^{D}Dab-Lys-Cys-Dab-Arg-Tyr;
Gua-VaI-Pro((4R)NH₂)-Ile-Thr-Tyr-Pen-Asn-Arg-Dab-Thr-^{D}Dab-Lys-Cys-Dab-Arg-Tyr;
Gua-VaI-Pro((4R)NH₂)-Ile-Ile-Tyr-Pen-Asn-Arg-Dab-Thr-^{D}Dab-Lys-Cys-Dab-Arg-Tyr;
Gua-Val-Hyp-Ile-Tyr-Tyr-Pen-Asn-Arg-Dab-Thr-^{D}Dab-Lys-Cys-Gln-Arg-Tyr;
Gua-Val-Hyp-Ile-Thr-Tyr-Pen-Glu-Arg-Dab-Thr-^{D}Dab-Lys-Cys-Dab-Arg-Tyr;
Gua-Val-Hyp-Ile-Thr-Tyr-Pen-His-Arg-Dab-Thr-^{D}Dab-Lys-Cys-Dab-Arg-Tyr;
Gua-Val-Hyp-Ile-Thr-Tyr-Pen-Asn-Arg-Dab-Thr-^{D}Dab-Dab-Cys-Dab-Arg-Tyr;
Gua-Val-Hyp-Ile-Thr-Tyr-Pen-Asn-Arg-Dab-Ser-^{D}Dab-Lys-Cys-Dab-Arg-Tyr;
Gua-Val-Hyp-Ile-Thr-Tyr-Pen-Asn-Arg-Dab-Ser-^{D}Dab-Lys-Cys-Dab-Dab-Tyr;
Gua-Val-Hyp-Ile-Thr-Tyr-Pen-Asn-Arg-Dab-Ser-^{D}Dab-Lys-Cys-Dab-Orn-Tyr;
Gua-Val-Pro((4R)NH₂)-Ile-Thr-Tyr-Pen-Asn-Arg-Dab-Thr-^{D}Dab-Lys-Cys-Tyr-Arg-Tyr;
Gua-Val-Pro((4R)NH₂)-Ile-Ile-Tyr-Pen-Asn-Arg-Dab-Thr-^{D}Dab-Lys-Cys-Tyr-Arg-Tyr;
Gua-Val-Hyp-Ile-alloThr-Tyr-Pen-Asn-Arg-Dab-Thr-^{D}Dab-Lys-Cys-Tyr-Arg-Tyr;
TMG-Val-Pro-Ile-Ile-Tyr-Cys-Asn-Arg-Dab-Thr-^{D}Dab-Lys-Cys-Dab-Arg-Tyr;
TMG-Val-Hyp-Ile-Thr-Tyr-Pen-Asn-Arg-Dab-Thr-^{D}Dab-Lys-Cys-Dab-Arg-Tyr;
Val-Hyp-Ile-Ile-Tyr-Pra-Asn-Arg-Dab-Thr-^{D}Dab-Lys-Abu(4N₃)-Dab-Arg-Tyr;
Val-Hyp-Ile-Ile-Tyr-Asp-Asn-Arg-Dab-Thr-^{D}Dab-Lys-Dab-Dab-Arg-Tyr;
Val-Hyp-Ile-Ile-Tyr-Pen-Asn-Arg-Dab-Thr-^{D}Dab-Lys-Cys-Dab-Cit-Tyr;
Val-Hyp-Ile-Ile-Tyr-Pen-Asn-Arg-Dab-Thr-^{D}Dab-Lys-Cys-Dab-Dab-Tyr;
Val-Hyp-Ile-Ile-Tyr-Pen-Asn-Arg-Dab-Ser-^{D}Dab-Lys-Cys-Dab-Arg-Tyr;
Val-Hyp-Ile-Ile-Tyr-Pen-Asn-Arg-Dab-Ser-^{D}Dab-Lys-Cys-Dab-Dab-Tyr;
Val-Hyp-Ile-Thr-Tyr-Pen-Asn-Arg-Dab-Ser-^{D}Dab-Lys-Cys-Dab-Dab-Tyr;
Val-Hyp-Ile-Ile-Tyr-Pen-Asn-Arg-Dab-Thr-^{D}Dab-Orn-Cys-Dab-Arg-Tyr;
Abu-Hyp-Ile-Ile-Tyr-Pen-Asn-Arg-Dab-Thr-^{D}Dab-Lys-Cys-Dab-Arg-Tyr;
Val-Pro-Ile-Thr-Tyr-Pen-Asn-Arg-Dab-Thr-^{D}Dab-Lys-Cys-Dab-Arg-Tyr;
Val-Hyp-Ile-Dab-Tyr-Pen-Asn-Arg-Dab-Ser-^{D}Dab-Lys-Cys-Dab-Dab-Tyr;
Val-Hyp-Ile-Ile-Tyr-Pen-Asn-Arg-Lys-Thr-^{D}Dab-Lys-Cys-Lys-Arg-Tyr;
Val-Hyp-Ile-Ile-Tyr-Pen-Asn-Arg-Dab-Thr-^{D}Dab-Ile-Cys-Dab-Dab-Tyr;
Val-Hyp-Ile-Ile-Tyr-Pen-Asn-Arg-Dab-Ser-^{D}Dab-Ile-Cys-Dab-Dab-Tyr;
Val-Hyp-Ile-Ile-Tyr-Pen-Asn-Arg-Dab-Thr-^{D}Dab-Lys-Cys-Dab-Orn-Tyr;
Val-Hyp-Ile-Ile-Tyr-Pen-Asn-Arg-Dab-Ser-^{D}Dab-Lys-Cys-Dab-Orn-Tyr;
Val-Hyp-Ile-Ile-Tyr-Pen-Asn-Arg-Dab-Thr-^{D}Dab-Ile-Cys-Dab-Arg-Tyr;
Val-Hyp-Ile-Thr-Tyr-Pen-Ile-Arg-Dab-Thr-^{D}Dab-Lys-Cys-Dab-Orn-Tyr;
Val-Hyp-Ile-Dab-Tyr-Pen-Asn-Arg-Dab-Thr-^{D}Dab-Lys-Cys-Dab-Orn-Tyr;
Val-Pro((4R)NH₂)-Ile-Ile-Tyr-Pen-Asn-Arg-Dab-Thr-^{D}Dab-Ile-Cys-Dab-Arg-Tyr;
Val-Pro((4R)NH₂)-Ile-Ile-Tyr-Pen-Asn-Arg-Dab-Thr-^{D}Dab-Cit-Cys-Dab-Arg-Tyr;
Val-Hyp-Ile-Thr-Tyr-Pen-Ile-Arg-Dab-Thr-^{D}Dab-Lys-Cys-Ser-Orn-Tyr;
Val-Pro((4R)NH₂)-Ile-Ile-Tyr-Pen-Asn-Arg-Dab-Thr-^{D}Dab-Lys-Cys-Ser-Arg-Tyr;
Val-Hyp-Ile-Thr-Tyr-Pen-Asn-Arg-Dab-Ser-^{D}Dab-Lys-Cys-Dab-Orn- Tyr;
tBuGly-Hyp-Ile-Thr-Tyr-Pen-Ile-Arg-Dab-Thr-^{D}Dab-Lys-Cys-Dab-Orn-Tyr;
Val-Hyp-Ile-Ile-Tyr-Pen-Asn-Arg-Dab-Ser-^{D}Dab-Ile-Cys-Dab-Orn-Tyr;
Val-Hyp-Ile-Ile-Tyr-Pen-Asn-Arg-Dab-Thr-^{D}Dab-Ile-Cys-Dab-Orn-Tyr;
Val-Hyp-Ile-Ile-Tyr-Pen-Asn-Arg-Dab-Thr-^{D}Dab-Cit-Cys-Dab-Arg-Tyr;
Val-Hyp-Ile-Thr-Tyr-Pen-Ile-Arg-Dab-Thr-^{D}Dab-Lys-Cys-Dab-Ser-Tyr;
Val-Pro((4R)NH₂)-Ile-Ile-Tyr-Pen-Asn-Arg-Dab-Thr-^{D}Dab-Lys-Cys-Dab-Ser-Tyr;
Val-Hyp-Ile-Ile-Tyr-Pen-Asn-Arg-Dab-Thr-^{D}Dab-Ser-Cys-Dab-Arg-Tyr;
Val-Pro((4R)NH₂)-Ile-Thr-Tyr-Pen-Asn-Arg-Dab-Thr-^{D}Dab-Cit-Cys-Dab-Ser-Tyr;
Val-Pro((4R)NH₂)-Ile-Thr-Tyr-Pen-Asn-Arg-Dab-Thr-^{D}Dab-Lys-Cys-Ser-Ser-Tyr;
Val-Hyp-Ile-Thr-Tyr-Pen-Ile-Arg-Dab-Thr-^{D}Dab-Lys-Cys-Ser-Arg-Tyr;
Val-Hyp-Ile-Ile-Tyr-Pen-Asn-Arg-Dab-Thr-^{D}Dab-Lys-Cys-Dab-Ser-Tyr;
Val-Pro((4R)NH₂)-Ile-Ile-Tyr-Pen-Asn-Arg-Dab-Thr-^{D}Dab-Cit-Cys-Dab-Ser-Tyr;
Val-Hyp-Ile-Ile-Tyr-Pen-Asn-Arg-Dab-Thr-^{D}Dab-Lys-Cys-Ser-Arg-Tyr;
Val-Hyp-Ile-Dab-Tyr-Pen-Asn-Arg-Dab-Thr-^{D}Dab-Cit-Cys-Dab-Ser-Tyr;
Val-Pro((4R)NH₂)-Ile-Ile-Tyr-Pen-Asn-Arg-Dab-Thr-^{D}Dab-Lys-Cys-Dab-Thr-Tyr;
Val-Pro((4R)NH₂)-Ile-Thr-Tyr-Pen-Asn-Arg-Dab-Thr-^{D}Dab-Lys-Cys-Ser-Arg-Tyr;
Val-Hyp-Ile-Ile-Tyr-Pen-Asn-Arg-Dab-Thr-^{D}Dab-Lys-Cys-Dab-Thr-Tyr;
Val-Hyp-Ile-Ile-Tyr-Pen-Asn-Arg-Dab-Ser-^{D}Dab-Lys-Cys-Dab-Thr-Tyr;
Val-Hyp-Ile-Dab-Tyr-Pen-Asn-Arg-Dab-Thr-^{D}Dab-Cit-Cys-Dab-Thr-Tyr;
tBuGly-Hyp-Ile-Ile-Tyr-Pen-Asn-Arg-Dab-Thr-^{D}Dab-Lys-Cys-Dab-Arg-Tyr;
Val-Pro((4R)NH₂)-Ile-Ile-Tyr-Pen-Asn-Arg-Dab-Thr-^{D}Dab-Ile-Cys-Dab-Thr-Tyr;
Val-Pro((4R)NH₂)-Ile-Thr-Tyr-Pen-Asn-Arg-Dab-Thr-^{D}Dab-Cit-Cys-Dab-Thr-Tyr;
Val-Pro((4R)NH₂)-Ile-Thr-Tyr-Pen-Asn-Arg-Dab-Thr-^{D}Dab-Ile-Cys-Dab-Thr-Tyr;
Val-Hyp-Ile-Ile-Tyr-Pen-Asn-Arg-Dab-Ser-^{D}Dab-Ile-Cys-Dab-Arg-Tyr;
Val-Pro((4R)NH₂)-Ile-Thr-Tyr-Pen-Asn-Arg-Dab-Thr-^{D}Dab-Lys-Cys-Ser-Thr-Tyr;
Val-Pro((4R)NH₂)-Ile-Thr-Tyr-Pen-Asn-Arg-Dab-Thr-^{D}Dab-Lys-Cys-Dab-Thr-Tyr;
Val-Hyp-Ile-Ile-Tyr-Pen-Asn-Arg-Orn-Thr-^{D}Dab-Ile-Cys-Dab-Arg-Tyr;
Val-Hyp-Ile-Orn-Tyr-Pen-Asn-Arg-Dab-Thr-^{D}Dab-Lys-Cys-Dab-Thr-Tyr;
Val-Hyp-Ile-Thr-Tyr-Pen-Ile-Arg-Dab-Thr-^{D}Dab-Orn-Cys-Dab-Arg-Tyr;
Val-Hyp-Ile-Ile-Tyr-Pen-Asn-Arg-Cit-Thr-^{D}Dab-Lys-Cys-Dab-Arg-Tyr;
Val-Hyp-Ile-Lys(Me)-Tyr-Pen-Asn-Arg-Dab-Thr-^{D}Dab-Lys-Cys-Dab-Arg-Tyr;
Val-Hyp-Ile-Val(3OH)-Tyr-Pen-Asn-Arg-Dab-Thr-^{D}Dab-Lys-Cys-Dab-Arg-Tyr;
Val-Pro(4,4F₂)-Ile-Thr-Tyr-Pen-Asn-Arg-Dab-Thr-^{D}Dab-Lys-Cys-Dab-Arg-Tyr;
Val-Pro(3,4dehydro)-Ile-Thr-Tyr-Pen-Asn-Arg-Dab-Thr-^{D}Dab-Lys-Cys-Dab-Arg-Tyr;
Val-Pro(4,4F₂)-Ile-Thr-Tyr-Pen-Asn-Arg-Dab-Thr-^{D}Dab-Lys-Cys-Dab-Arg-Tyr;
Val-Pro((4R)OPh)-Ile-Thr-Tyr-Pen-Asn-Arg-Dab-Thr-^{D}Dab-Lys-Cys-Dab-Arg-Tyr
Val-Pro((4R)F)-Ile-Thr-Tyr-Pen-Asn-Arg-Dab-Thr-^{D}Dab-Lys-Cys-Dab-Arg-Tyr;
Val-Pic-Ile-Thr-Tyr-Pen-Asn-Arg-Dab-Thr-^{D}Dab-Lys-Cys-Dab-Arg-Tyr;
Val-NalloThr-Ile-Thr-Tyr-Pen-Asn-Arg-Dab-Thr-^{D}Dab-Lys-Cys-Dab-Arg-Tyr;
Hyp-Ile-Thr-Tyr-Pen-Asn-Arg-Dab(iPr)-Thr-^{D}Dab-Lys-Cys-Dab-Orn(iPr)-Tyr;
Hyp-Ile-Thr-Tyr-Pen-Asn-Arg-Dab(iPr)-Thr-^{D}Dab-Lys-Cys-Dab(iPr)-Arg-Tyr;
Hyp-Ile-Thr-Tyr-Pen-Asn-Arg-Dab-Thr-^{D}Dab-Lys-Cys-Dab-Orn(iPr)-Tyr;
Hyp-Ile-Thr-Tyr-Pen-Ile-Arg-Dab-Thr-^{D}Dab-Lys-Cys-Dab-Orn(iPr)-Tyr; and
Hyp-Ile-Thr-Tyr-Pen-Asn-Arg-Dab-Thr-^{D}Dab(iPr)-Lys-Cys-Dab-Arg-Tyr;
or a tautomer, a rotamer, a salt, a hydrate or a solvate thereof,
wherein Cys or Pen at P⁶, if present, and Cys or Pen at P¹³, if present, optionally form a disulfide bridge between P⁶ and P¹³, or wherein
Asp at P⁶, if present, and Dab at P¹³, if present, optionally form a lactam bridge between P⁶ and P¹³, or wherein
Pra at P⁶, if present, and Abu(4N₃) at P¹³, if present, optionally form a 1,2,3-triazole bridge between P⁶ and P¹³.

9. The compound according to claim 8, wherein Cys or Pen at P⁶, and Cys or Pen at P¹³, form a disulfide bridge between P⁶ and P¹³.

10. The compound according to claim 9, wherein the compound is Gua-Val-Hyp-Ile-Thr-Tyr-Pen-Asn-Arg-Dab-Thr-^{D}Dab-Lys-Cys-Dab-Arg-Tyr;
or a tautomer, a rotamer, a salt, a hydrate or a solvate thereof.

11. A pharmaceutical composition containing a compound of formula (I) or a mixture of compounds of formula (I) as defined in any one of claims 1 to 10 or a pharmaceutically acceptable salt thereof and at least one pharmaceutically inert carrier, optionally in a form suitable for oral, topical, transdermal, injection, buccal, transmucosal, rectal, pulmonary or inhalation administration, especially in the form of a tablet, a dragee, a capsule, a solution, a liquid, a gel, a plaster, a cream, an ointment, a syrup, a slurry, a suspension, a spray, a nebulizer, an aerosol, or a suppository.

12. A compound of formula (I) as defined in any one of claims 1 to 10, or a pharmaceutically acceptable salt thereof, for use as a medicament.

13. A compound of formula (I) as defined in any one of the claims 1 to 10 or a pharmaceutically acceptable salt thereof for use as a pharmaceutically active substance having antibiotic activity in the treatment or prevention of an infection or disease related to such infection.

14. A compound for use according to claim 13 or a pharmaceutically acceptable salt thereof, wherein the infection or disease related to such infection is related to respiratory diseases or skin or soft tissue diseases or gastrointestinal diseases or eye diseases or ear diseases or CNS diseases or bone diseases or cardiovascular diseases or genitourinary diseases, or nosocomial infections, or catheter-related and non-catheter-related infections, or urinary tract infections, or bloodstream infections; or infection-induced sepsis.

15. A compound for use according to claim 13 or claim 14 or a pharmaceutically acceptable salt thereof, wherein the infection is selected from nosocomial infections, catheter-related and non-catheter-related infections, urinary tract infections, bloodstream infections, or a disease or disorder associated with an infection, especially diseases or disorders such as ventilator-associated pneumonia (VAP), ventilator-associated bacterial pneumonia (VABP), hospital-acquired pneumonia (HAP), hospital-acquired bacterial pneumonia (HABP), healthcare-associated pneumonia (HCAP), cystic fibrosis, emphysema, asthma, pneumonia, epidemic diarrhea, necrotizing enterocolitis, typhlitis, gastroenteritis, pancreatitis, keratitis, endophthalmitis, otitis, brain abscess, meningitis, encephalitis, osteochondritis, pericarditis, epididymitis, prostatitis, urethritis, sepsis; surgical wounds, traumatic wounds, and burns.

16. Use of a compound of formula (I) as defined in any one of claims 1 to 10 or a salt thereof, as a disinfectant or preservative for foodstuffs, cosmetics, medicaments and/or other nutrient-containing materials.

17. A process for the preparation of a compound of formula (I) as defined in any one of claims 1 to 10 or a salt thereof, which comprises the following steps:
(a) coupling an appropriately functionalized solid support with an appropriately N-protected derivative of that amino acid which in the desired end-product is in position P¹⁶; any functional group which may be present in said N-protected amino acid derivative being likewise appropriately protected;
(b) removing the N-protecting group from the product thus obtained;
(c) coupling the product thus obtained with an appropriately N-protected derivative of that amino acid which in the desired end-product is in position P¹⁵; any functional group which may be present in said N-protected amino acid derivative being likewise appropriately protected;
(d) effecting steps substantially corresponding to steps (b) and (c) using appropriately N-protected derivatives of amino acids which in the desired end-product are in positions P¹⁴ to P⁶, any functional group(s) which may be present in said N-protected amino acid derivatives being likewise appropriately protected;
(e) optionally selectively deprotecting one or several protected functional group(s) present in the molecule and chemically transforming the reactive group(s) thus liberated;
(f) effecting steps substantially corresponding to steps (b) and (c) using appropriately N-protected derivatives of amino acids which in the desired end-product are in positions P⁵ to P², any functional group(s) which may be present in said N-protected amino acid derivatives being likewise appropriately protected; and, optionally, following each coupling, selectively deprotecting one or several protected functional group(s) present in the molecule and chemically transforming the reactive group(s) thus liberated;
(g) further effecting steps substantially corresponding to steps (b) and (c) using an appropriately N-protected derivative of an amino acid, or optionally, an appropriately protected derivative of a hydroxy acid, which in the desired end-product is in position P¹, any functional group(s) which may be present in said N-protected amino acid derivative, or hydroxy acid derivative, being likewise appropriately protected; and, optionally, following the coupling, selectively deprotecting one or several protected functional group(s) present in the molecule and chemically transforming the reactive group(s) thus liberated;
(h) optionally selectively deprotecting one or several protected functional group(s) present in the molecule and chemically transforming the reactive group(s) thus liberated;
(i) optionally, removing the N-protecting group at position P¹;
(j) detaching the product thus obtained from the solid support;
(k) optionally selectively deprotecting one or several protected functional group(s) present in the molecule and chemically transforming the reactive group(s) thus liberated;
(l) removing any protecting groups present on functional groups of any members of the chain of residues and, optionally, any protecting group(s) which may in addition be present in the molecule;
(m) optionally implementing additional chemical transformations of one or more reactive group(s) present in the molecule;
(n) if required, removing any protecting groups present on functional groups of any members of the chain of residues and, optionally, any protecting group(s) which may in addition be present in the molecule; and
(o) optionally converting the product thus obtained into a pharmaceutically acceptable salt; or
optionally converting a pharmaceutically acceptable or unacceptable salt thus obtained into the corresponding free compound of formula (I); or
optionally converting a pharmaceutically acceptable or unacceptable salt thus obtained into a different, pharmaceutically acceptable salt.

## Patentansprüche

1. Peptidomimetische Verbindung der allgemeinen Formel (I),
P¹-P²-P³-P⁴-P⁵-P⁶-P⁷-P⁸-P⁹-P¹⁰-P¹¹-P¹²-P¹³-P¹⁴-P¹⁵-P¹⁶ (I)
P¹ für 2OHVal, Val, NMeVal, Abu, tBuGly steht;
wobei die Aminogruppe von P¹ gegebenenfalls durch eine Guanidinogruppe (Gua) oder durch eine Tetramethylguanidinogruppe (TMG) ersetzt ist;
P² für Pro, Pro (4R)F, Pro(4R)OMe, Pro(4R)OPhe, Pro(3,4dehydro), Pr(4,4F2), Pic;
Agb, Agp, Dab, Dab(iPr), Dap, Dap(iPr), Har, Lys, Lys(iPr), Narg, Ndab, Nlys, Norn, Orn, Orn(iPr), Pro((4R)Guanidin), Pro((4R)NH₂), Pro((4S)NH₂), Arg, NMeLys;
Hyp, NalloThr steht;
P³ für Hle, Ile, Leu, Nle, Cyg steht;
P⁴ für Ala, Ala(cPr), Ala(Tetrahydropyran4yl), Abu, alloIle, Cha, Cpa, Cpg, Cyg, Dea, Hle, Ile, Leu, Met, Nle, OctGly, tBuGly, tBuAla, Val, NMeAla, NMeVal, Nva;
Phe, His, Phe(3OH), Phe(4F), Phe(4OCF3), Trp(6Cl), Tyr(3Cl), Tyr(3F), Tyr(Phenyl), Trp, Tyr, 4Thz, Phe(4(4Hydroxyphenoxy)), Phe(4NH2), Tyr(Me), Ntyr, Nphe; Agb, Agp, Dab, Dab(iPr), Dap, Dap(iPr), Har, Lys, Lys(iPr), Narg, Ndab, Nlys, Norn, Orn, Orn(iPr), Arg, NMeLys, Lys(Me);
alloThr, Cit, Hgn, Hse, Leu((3R)OH), Asn, Gln, Ser, Thr, Asp, Glu, Hgl, Val(3OH) steht;
P⁵ für Phe, His, Trp, Tyr steht;
P⁶ für Pra, Abu(4N₃);
Dab, Dap;
Cys, Hcy, NMeCys, Pen;
Asp, Glu, Hgl steht;
P⁷ für Ala, Ala(cPr), Ala(Tetrahydropyran4yl), Abu, alloIle, Cha, Cpa, Cpg, Cyg, Dea, Hle, Ile, Leu, Met, Nle, OctGly, tBuGly, tBuAla, Val, NMeAla, NMeVal, Nva;
Phe, His, Phe(3OH), Phe(4F), Phe(4OCF3), Trp(6Cl), Tyr(3Cl), Tyr(3F), Tyr(Phenyl), Trp, Tyr, 4Thz, Phe(4(4Hydroxyphenoxy)), Phe(4NH2), Tyr(Me), Ntyr, Nphe; Agb, Agp, Dab, Dab(iPr), Dap, Dap(iPr), Har, Lys, Lys(iPr), Narg, Ndab, Nlys, Norn, Orn, Orn(iPr), Arg, NMeLys;
alloThr, Cit, Hgn, Hse, Leu((3R)OH), Asn, Gln, Ser, Thr, Asp, Glu, Hgl steht;
P⁸ für Agb, Har, Arg. Lys, Orn steht;
P⁹ für Dab, Dap, Har, Lys, Orn, Arg, Dab(iPr);
Cit steht;
P¹⁰ für alloThr, Hse, Ser, Thr, Hyp, Gln, Asn, Glu, Asp steht;
P¹¹ für ^{D}Dab, ^{D}Dap, ^{D}Lys, ^{D}Orn, ^{D}Arg, ^{D}Dab(iPr) steht;
P¹² für Ala, Ala(cPr), Ala(Tetrahydropyran4yl), Abu, alloIle, Cha, Cpa, Cpg, Cyg, Dea, Hle, Ile, Leu, Met, Nle, OctGly, tBuGly, tBuAla, Val, NMeAla, NMeVal, Nva;
Phe, His, Phe(3OH), Phe(4F), Phe(40CF3), Trp(6Cl), Tyr(3Cl), Tyr(3F), Tyr(Phenyl), Trp, Tyr, 4Thz, Phe(4(4Hydroxyphenoxy)), Phe(4NH2), Tyr(Me), Ntyr, Nphe; Agb, Agp, Dab, Dab(iPr), Dap, Dap(iPr), Har, Lys, Lys(iPr), Narg, Ndab, Nlys, Norn, Orn, Orn(iPr), Arg, NMeLys;
alloThr, Cit, Hgn, Hse, Leu((3R)OH), Asn, Gln, Ser, Thr, Asp, Glu, Hgl steht;
P¹³ für Pra, Abu(4N₃);
Dab, Dap;
Cys, Hcy, NMeCys, Pen;
Asp, Glu, Hgl steht;
P¹⁴ für Phe, His, Phe(3OH), Phe(4F), Phe(4OCF3), Trp(6Cl), Tyr(3Cl), Tyr(3F), Tyr(Phenyl), Trp, Tyr, 4Thz, Phe(4(4Hydroxyphenoxy)), Phe(4NH2), Tyr(Me), Ntyr, Nphe;
Agb, Agp, Dab, Dab(iPr); Dap, Dap(iPr), Har, Lys, Lys(iPr), Narg, Ndab, Nlys, Norn, Orn, Orn(iPr), Arg, NMeLys;
alloThr, Cit, Hgn, Hse, Leu((3R)OH), Asn, Gln, Ser, Thr, Asp, Glu, Hgl steht;
P¹⁵ für Ala, Ala(cPr), Ala(Tetrahydropyran4yl), Abu, alloIle, Cha, Cpa, Cpg, Cyg, Dea, Hle, Ile, Leu, Met, Nle, OctGly, tBuGly, tBuAla, Val, NMeAla, NMeVal, Nva;
Agb, Agp, Dab, Dab(iPr), Dap, Dap(iPr), Har, Lys, Lys(iPr), Narg, Ndab, Nlys, Norn, Orn, Orn(iPr), Arg, NMeLys;
alloThr, Cit, Hgn, Hse, Leu((3R)OH), Asn, Gln, Ser, Thr, Asp, Glu, Hgl steht;
P¹⁶ für Ala, Ala(cPr), Ala(Tetrahydropyran4yl), Abu, alloIle, Cha, Cpa, Cpg, Cyg, Dea, Hle, Ile, Leu, Met, Nle, OctGly, tBuGly, tBuAla, Val, NMeAla, NMeVal, Nva;
Phe, His, Phe(3OH), Phe(4F), Phe(4OCF3), Trp(6Cl), Tyr(3Cl), Tyr(3F), Tyr(Phenyl), Trp, Tyr, 4Thz, Phe(4(4Hydroxyphenoxy)), Phe(4NH2), Tyr(Me), Ntyr, Nphe; oder ein Tautomer, ein Rotamer, ein Salz, ein Hydrat oder ein Solvat davon,
wobei
Cys, Hcy, NMeCys oder Pen an P⁶, sofern vorhanden, und Cys, Hcy, NMeCys oder Pen an P¹³, sofern vorhanden, gegebenenfalls eine Disulfidbrücke zwischen P⁶ und P¹³ bilden; oder wobei
Dab oder Dap an P⁶, sofern vorhanden, und Asp, Glu oder Hgl an P¹³, sofern vorhanden, gegebenenfalls eine Lactambrücke zwischen P⁶ und P¹³ bilden; oder wobei
Asp, Glu oder Hgl an P⁶, sofern vorhanden, und Dab oder Dap an P¹³, sofern vorhanden, gegebenenfalls eine Lactambrücke zwischen P⁶ und P¹³ bilden; oder wobei
Pra an P⁶, sofern vorhanden, und Abu(4N₃) an P¹³, sofern vorhanden, gegebenenfalls eine 1,2,3-Triazolbrücke zwischen P⁶ und P¹³ bilden; oder wobei
Abu(4N₃) an P⁶, sofern vorhanden, und Pra an P¹³, sofern vorhanden, gegebenenfalls eine 1,2,3-Triazolbrücke zwischen P6 und P13 bilden;
mit der Maßgabe, dass mindestens drei Aminosäurereste unter den vier Aminosäureresten an den Positionen P⁹, P¹², P¹⁴ und P¹⁵ basische Aminosäurereste sind, die aus Agb, Agp, Dab, Dab(iPr), Dap, Dap(iPr), Har, Lys, Lys(iPr), Narg, Ndab, Nlys, Norn, Orn, Orn(iPr), Arg, NMeLys ausgewählt sind.

2. Verbindung nach Anspruch 1, wobei
P¹ für 2OHVal, Val, NMeVal, Abu, tBuGly steht;
wobei die Aminogruppe von P¹ gegebenenfalls durch eine Guanidinogruppe (Gua) oder durch eine Tetramethylguanidinogruppe (TMG) ersetzt ist;
P² für Pro, Pro (4R)F, Pro(4R)OMe, Pro (4R)OPhe, Pro(3,4dehydro), Pr(4,4F2), Pic;
Agb, Agp, Dab, Dab(iPr), Dap, Dap(iPr), Har, Lys, Lys(iPr), Narg, Ndab, Nlys, Norn, Orn, Orn(iPr), Pro((4R)Guanidin), Pro((4R)NH₂), Pro((4S)NH₂), Arg, NMeLys;
Hyp, NalloThr steht;
P³ für Ile steht;
P⁴ für Ala, Ala(cPr), Ala(Tetrahydropyran4yl), Abu, alloIle, Cha, Cpa, Cpg, Cyg, Dea, Hle, Ile, Leu, Met, Nle, OctGly, tBuGly, tBuAla, Val, NMeAla, NMeVal, Nva;
Phe, His, Phe(3OH), Phe(4F), Phe(4OCF3), Trp(6Cl), Tyr(3Cl), Tyr(3F), Tyr(Phenyl), Trp, Tyr, 4Thz, Phe(4(4Hydroxyphenoxy)), Phe(4NH2), Tyr(Me), Ntyr, Nphe; Agb, Agp, Dab, Dab(iPr), Dap, Dap(iPr), Har, Lys, Lys(iPr), Narg, Ndab, Nlys, Norn, Orn, Orn(iPr), Arg, NMeLys, Lys(Me);
alloThr, Cit, Hgn, Hse, Leu((3R)OH), Asn, Gln, Ser, Thr, Asp, Glu, Hgl, Val(3OH) steht;
P⁵ für Phe, His, Trp, Tyr steht;
P⁶ für Dab, Dap;
Cys, Pen;
Asp, Glu steht;
P⁷ für Ala, Ala(cPr), Ala(Tetrahydropyran4yl), Abu, alloIle, Cha, Cpa, Cpg, Cyg, Dea, Hle, Ile, Leu, Met, Nle, OctGly, tBuGly, tBuAla, Val, NMeAla, NMeVal, Nva;
Phe, His, Phe(3OH), Phe(4F), Phe(40CF3), Trp(6Cl), Tyr(3Cl), Tyr(3F), Tyr(Phenyl), Trp, Tyr, 4Thz, Phe(4(4Hydroxyphenoxy)), Phe(4NH2), Tyr(Me), Ntyr, Nphe; Agb, Agp, Dab, Dab(iPr), Dap, Dap(iPr), Har, Lys, Lys(iPr), Narg, Ndab, Nlys, Norn, Orn, Orn(iPr), Arg, NMeLys;
alloThr, Cit, Hgn, Hse, Leu((3R)OH), Asn, Gln, Ser, Thr, Asp, Glu, Hgl steht;
P⁸ für Arg steht;
P⁹ für Dab, Dap, Har, Lys, Orn, Arg, Dab(iPr);
Cit steht;
P¹⁰ für alloThr, Hse, Ser, Thr steht;
P¹¹ für ^{D}Dab, ^{D}Dap, ^{D}Lys, ^{D}Orn, ^{D}Arg, ^{D}Dab(iPr) steht;
P¹² für Ala, Ala(cPr), Ala(Tetrahydropyran4yl), Abu, alloIle, Cha, Cpa, Cpg, Cyg, Dea, Hle, Ile, Leu, Met, Nle, OctGly, tBuGly, tBuAla, Val, NMeAla, NMeVal, Nva;
Phe, His, Phe(3OH), Phe(4F), Phe(40CF3), Trp(6Cl), Tyr(3Cl), Tyr(3F), Tyr(Phenyl), Trp, Tyr, 4Thz, Phe(4(4Hydroxyphenoxy)), Phe(4NH2), Tyr(Me), Ntyr, Nphe; Agb, Agp, Dab, Dab(iPr), Dap, Dap(iPr), Har, Lys, Lys(iPr), Narg, Ndab, Nlys, Norn, Orn, Orn(iPr), Arg, NMeLys;
alloThr, Cit, Hgn, Hse, Leu((3R)OH), Asn, Gln, Ser, Thr, Asp, Glu, Hgl steht;
P¹³ für Dab, Dap;
Cys, Pen;
Asp, Glu steht;
P¹⁴ für Phe, His, Phe(3OH), Phe(4F), Phe(40CF3), Trp(6Cl), Tyr(3Cl), Tyr(3F), Tyr(Phenyl), Trp, Tyr, 4Thz, Phe(4(4Hydroxyphenoxy)), Phe(4NH2), Tyr(Me), Ntyr, Nphe; Agb, Agp, Dab, Dab(iPr); Dap, Dap(iPr), Har, Lys, Lys(iPr), Narg, Ndab, Nlys, Norn, Orn, Orn(iPr), Arg, NMeLys;
alloThr, Cit, Hgn, Hse, Leu((3R)OH), Asn, Gln, Ser, Thr, Asp, Glu oder Hgl steht;
P¹⁵ für Ala, Ala(cPr), Ala(Tetrahydropyran4yl), Abu, alloIle, Cha, Cpa, Cpg, Cyg, Dea, Hle, Ile, Leu, Met, Nle, OctGly, tBuGly, tBuAla, Val, NMeAla, NMeVal, Nva;
Agb, Agp, Dab, Dab(iPr), Dap, Dap(iPr), Har, Lys, Lys(iPr), Narg, Ndab, Nlys, Norn, Orn, Orn(iPr), Arg, NMeLys;
alloThr, Cit, Hgn, Hse, Leu((3R)OH), Asn, Gln, Ser, Thr, Asp, Glu oder Hgl steht;
P¹⁶ für Ala, Ala(cPr), Ala(Tetrahydropyran4yl), Abu, alloIle, Cha, Cpa, Cpg, Cyg, Dea, Hle, Ile, Leu, Met, Nle, OctGly, tBuGly, tBuAla, Val, NMeAla, NMeVal, Nva;
Phe, His, Phe(3OH), Phe(4F), Phe(4OCF3), Trp(6Cl), Tyr(3Cl), Tyr(3F), Tyr(Phenyl), Trp, Tyr, 4Thz, Phe(4(4Hydroxyphenoxy)), Phe(4NH2), Tyr(Me), Ntyr, Nphe; oder ein Tautomer, ein Rotamer, ein Salz, ein Hydrat oder ein Solvat davon,
wobei
Cys oder Pen an P⁶, sofern vorhanden, und Cys oder Pen an P¹³, sofern vorhanden, gegebenenfalls eine Disulfidbrücke zwischen P⁶ und P¹³ bilden; oder wobei
Dab oder Dap an P⁶, sofern vorhanden, und Asp oder Glu an P¹³, sofern vorhanden, gegebenenfalls eine Lactambrücke zwischen P⁶ und P¹³ bilden; oder wobei
Asp oder Glu an P⁶, sofern vorhanden, und Dab oder Dap an P¹³, sofern vorhanden, gegebenenfalls eine Lactambrücke zwischen P⁶ und P¹³ bilden;
mit der Maßgabe, dass mindestens drei Aminosäurereste unter den vier Aminosäureresten an den Positionen P⁹, P¹², P¹⁴ und P¹⁵ basische Aminosäurereste sind, die aus Agb, Agp, Dab, Dab(iPr), Dap, Dap(iPr), Har, Lys, Lys(iPr), Narg, Ndab, Nlys, Norn, Orn, Orn(iPr), Arg, NMeLys ausgewählt sind.

3. Verbindung nach Anspruch 1 oder Anspruch 2, wobei P¹ für 2OHVal, Val, NMeVal, Abu, tBuGly steht;
wobei die Aminogruppe von P¹ gegebenenfalls durch eine Guanidinogruppe (Gua) oder durch eine Tetramethylguanidinogruppe (TMG) ersetzt ist;
P² für Pro, Pro (4R)F, Pro(4R)OMe, Pro (4R)OPhe, Pro(3,4dehydro), Pr(4,4F2), Pic;
Agb, Agp, Dab, Dab(iPr), Dap, Dap(iPr), Lys, Lys(iPr), Narg, Ndab, Nlys, Norn, Orn, Orn(iPr), Pro((4R)NH₂), Arg, NMeLys;
Hyp, NalloThr steht;
P³ für Hle, Ile, Leu, Nle steht;
P⁴ für Ala, Ala(cPr), Ala(Tetrahydropyran4yl), Abu, alloIle, Cyg, Dea, Ile, Leu, Nle, tBuGly, tBuAla, Val, NMeAla, NMeVal, Nva;
Phe, His, Phe(3OH), Phe(4F), Trp(6Cl), Trp, Tyr, 4Thz, Phe(4(4Hydroxyphenoxy)), Phe(4NH2), Tyr(Me), Ntyr, Nphe;
Agb, Agp, Dab, Dab(iPr), Dap, Dap(iPr), Lys, Lys(iPr), Narg, Ndab, Nlys, Norn, Orn, Orn(iPr), Arg, NMeLys, Lys(Me);
alloThr, Cit, Hgn, Hse, Leu((3R)OH), Asn, Gln, Ser, Thr, Asp, Glu, Hgl, Val(3OH) steht;
P⁵ für Phe, His, Trp, Tyr steht;
P⁶ für Pra, Abu(4N₃);
Dab, Dap;
Cys, Hcy, NMeCys, Pen;
Asp, Glu, Hgl steht;
P⁷ für Ala, Ala(cPr), Ala(Tetrahydropyran4yl), Abu, alloIle, Cyg, Dea, Ile, Leu, Nle, tBuGly, tBuAla, Val, NMeAla, NMeVal, Nva;
Phe, His, Phe(3OH), Phe(4F), Trp(6Cl), Trp, Tyr, 4Thz, Phe(4(4Hydroxyphenoxy)), Phe(4NH2), Tyr(Me), Ntyr, Nphe; Agb, Agp, Dab, Dab(iPr), Dap, Dap(iPr), Lys, Lys(iPr), Narg, Ndab, Nlys, Norn, Orn, Orn(iPr), Arg, NMeLys;
alloThr, Cit, Hgn, Hse, Leu((3R)OH), Asn, Gln, Ser, Thr, Asp, Glu, Hgl steht;
P⁸ für Agb, Har, Arg steht;
P⁹ für Dab, Dap, Har, Lys, Orn, Arg, Dab(iPr);
Cit steht;
P¹⁰ für alloThr, Hse, Ser, Thr steht;
P¹¹ für ^{D}Dab, ^{D}Dap, ^{D}Lys, ^{D}Orn, ^{D}Arg, ^{D}Dab(iPr) steht;
P¹² für Ala, Ala(cPr), Ala(Tetrahydropyran4yl), Abu, alloIle, Cyg, Dea, Ile, Leu, Nle, tBuGly, tBuAla, Val, NMeAla, NMeVal, Nva;
Phe, His, Phe(3OH), Phe(4F), Trp(6Cl), Tyr(Phenyl), Trp, Tyr, 4Thz, Phe(4(4Hydroxyphenoxy)), Phe(4NH2), Tyr(Me), Ntyr, Nphe;
Agb, Agp, Dab, Dab(iPr), Dap, Dap(iPr), Lys, Lys(iPr), Narg, Ndab, Nlys, Norn, Orn, Orn(iPr), Arg, NMeLys;
alloThr, Cit, Hgn, Hse, Leu((3R)OH), Asn, Gln, Ser, Thr, Asp, Glu, Hgl steht;
P¹³ für Pra, Abu(4N₃);
Dab, Dap;
Cys, Hcy, NMeCys, Pen;
Asp, Glu, Hgl steht;
P¹⁴ für Phe, His, Phe(3OH), Phe(4F), Trp(6Cl), Trp, Tyr, 4Thz, Phe(4(4Hydroxyphenoxy)), Phe(4NH2), Tyr(Me), Ntyr, Nphe;
Agb, Agp, Dab, Dab(iPr); Dap, Dap(iPr), Lys, Lys(iPr), Narg, Ndab, Nlys, Norn, Orn, Orn(iPr), Arg, NMeLys;
alloThr, Cit, Hgn, Hse, Leu((3R)OH), Asn, Gln, Ser, Thr, Asp, Glu, Hgl steht;
P¹⁵ für Ala, Ala(cPr), Ala(Tetrahydropyran4yl), Abu, alloIle, Cyg, Dea, Ile, Leu, Nle, tBuGly, tBuAla, Val, NMeAla, NMeVal, Nva;
Agb, Agp, Dab, Dab(iPr), Dap, Dap(iPr), Lys, Lys(iPr), Narg, Ndab, Nlys, Norn, Orn, Orn(iPr), Arg, NMeLys;
alloThr, Cit, Hgn, Hse, Leu((3R)OH), Asn, Gln, Ser, Thr, Asp, Glu, Hgl steht;
P¹⁶ für Ala, Ala(cPr), Ala(Tetrahydropyran4yl), Abu, alloIle, Cyg, Dea, Ile, Leu, Nle, tBuGly, tBuAla, Val, NMeAla, NMeVal, Nva;
Phe, His, Phe(3OH), Phe(4F), Trp(6Cl), Trp, Tyr, 4Thz, Phe(4(4Hydroxyphenoxy)), Phe(4NH2), Tyr(Me), Ntyr, Nphe;
oder ein Tautomer, ein Rotamer, ein Salz, ein Hydrat oder ein Solvat davon,
wobei
Cys, Hcy, NMeCys oder Pen an P⁶, sofern vorhanden, und Cys, Hcy, NMeCys oder Pen an P¹³, sofern vorhanden, gegebenenfalls eine Disulfidbrücke zwischen P⁶ und P¹³ bilden; oder wobei
Dab oder Dap an P⁶, sofern vorhanden, und Asp, Glu oder Hgl an P¹³, sofern vorhanden, gegebenenfalls eine Lactambrücke zwischen P⁶ und P¹³ bilden; oder wobei
Asp, Glu oder Hgl an P⁶, sofern vorhanden, und Dab oder Dap an P¹³, sofern vorhanden, gegebenenfalls eine Lactambrücke zwischen P⁶ und P¹³ bilden; oder wobei
Pra an P⁶, sofern vorhanden, und Abu(4N₃) an P¹³, sofern vorhanden, gegebenenfalls eine 1,2,3-Triazolbrücke zwischen P⁶ und P¹³ bilden; oder wobei
Abu(4N₃)an P⁶, sofern vorhanden, und Pra an P¹³, sofern vorhanden, gegebenenfalls eine 1,2,3-Triazolbrücke zwischen P6 und P13 bilden;
mit der Maßgabe, dass mindestens drei Aminosäurereste unter den vier Aminosäureresten an den Positionen P⁹, P¹², P¹⁴ und P¹⁵ basische Aminosäurereste sind, die aus Agb, Agp, Dab, Dab(iPr), Dap, Dap(iPr), Lys, Lys(iPr), Narg, Ndab, Nlys, Norn, Orn, Orn(iPr), Arg, NMeLys ausgewählt sind.

4. Verbindung nach einem der Ansprüche 1 bis 3, wobei P¹ für 2OHVal, Val, NMeVal, Abu, tBuGly steht;
wobei die Aminogruppe von P¹ gegebenenfalls durch eine Guanidinogruppe (Gua) oder durch eine Tetramethylguanidinogruppe (TMG) ersetzt ist;
P² für Pro, Pro (4R)F, Pro(4R)OMe, Pro (4R)OPhe, Pro(3,4dehydro), Pr(4,4F2), Pic;
Agb, Agp, Dab, Dab(iPr), Dap, Dap(iPr), Lys, Lys(iPr), Narg, Ndab, Nlys, Norn, Orn, Orn(iPr), Pro((4R)NH₂), Arg, NMeLys;
Hyp, NalloThr steht;
P³ für Ile steht;
P⁴ für Ala, Ala(cPr), Ala(Tetrahydropyran4yl), Abu, alloIle, Cyg, Dea, Ile, Leu, Nle, tBuGly, tBuAla, Val, NMeAla, NMeVal, Nva;
Phe, His, Phe(3OH), Phe(4F), Trp(6Cl), Trp, Tyr, 4Thz, Phe(4(4Hydroxyphenoxy)), Phe(4NH2), Tyr(Me), Ntyr, Nphe;
Agb, Agp, Dab, Dab(iPr), Dap, Dap(iPr), Lys, Lys(iPr), Narg, Ndab, Nlys, Norn, Orn, Orn(iPr), Arg, NMeLys; Lys (Me) ;
alloThr, Cit, Hgn, Hse, Leu((3R)OH), Asn, Gln, Ser, Thr, Asp, Glu, Hgl, Val(3OH) steht;
P⁵ für Phe, His, Trp, Tyr steht;
P⁶ für Dab, Dap;
Cys, Pen;
Asp, Glu steht;
P⁷ für Ala, Ala(cPr), Ala(Tetrahydropyran4yl), Abu, alloIle, Cyg, Dea, Ile, Leu, Nle, tBuGly, tBuAla, Val, NMeAla, NMeVal, Nva;
Phe, His, Phe(3OH), Phe(4F), Trp(6Cl), Trp, Tyr, 4Thz, Phe(4(4Hydroxyphenoxy)), Phe(4NH2), Tyr(Me), Ntyr, Nphe; Agb, Agp, Dab, Dab(iPr), Dap, Dap(iPr), Lys, Lys(iPr), Narg, Ndab, Nlys, Norn, Orn, Orn(iPr), Arg, NMeLys;
alloThr, Cit, Hgn, Hse, Leu((3R)OH), Asn, Gln, Ser, Thr, Asp, Glu, Hgl steht;
P⁸ für Arg steht;
P⁹ für Dab, Dap, Har, Lys, Orn, Arg, Dab(iPr);
Cit steht;
P¹⁰ für alloThr, Hse, Ser, Thr steht;
P¹¹ für ^{D}Dab, ^{D}Dap, ^{D}Lys, ^{D}Orn, ^{D}Arg, ^{D}Dab(iPr) steht;
P¹² für Ala, Ala(cPr), Ala(Tetrahydropyran4yl), Abu, alloIle, Cyg, Dea, Ile, Leu, Nle, tBuGly, tBuAla, Val, NMeAla, NMeVal, Nva;
Phe, His, Phe(3OH), Phe(4F), Trp(6Cl), Tyr(Phenyl), Trp, Tyr, 4Thz, Phe(4(4Hydroxyphenoxy)), Phe(4NH2), Tyr(Me), Ntyr, Nphe;
Agb, Agp, Dab, Dab(iPr), Dap, Dap(iPr), Lys, Lys(iPr), Narg, Ndab, Nlys, Norn, Orn, Orn(iPr), Arg, NMeLys;
alloThr, Cit, Hgn, Hse, Leu((3R)OH), Asn, Gln, Ser, Thr, Asp, Glu, Hgl steht;
P¹³ für Dab, Dap;
Cys, Pen;
Asp, Glu steht;
P¹⁴ für Phe, His, Phe(3OH), Phe(4F), Trp(6Cl), Trp, Tyr, 4Thz, Phe(4(4Hydroxyphenoxy)), Phe(4NH2), Tyr(Me), Ntyr, Nphe;
Agb, Agp, Dab, Dab(iPr); Dap, Dap(iPr), Lys, Lys(iPr), Narg, Ndab, Nlys, Norn, Orn, Orn(iPr), Arg, NMeLys;
alloThr, Cit, Hgn, Hse, Leu((3R)OH), Asn, Gln, Ser, Thr, Asp, Glu oder Hgl steht;
P¹⁵ für Ala, Ala(cPr), Ala(Tetrahydropyran4yl), Abu, alloIle, Cyg, Dea, Ile, Leu, Nle, tBuGly, tBuAla, Val, NMeAla, NMeVal, Nva;
Agb, Agp, Dab, Dab(iPr), Dap, Dap(iPr), Lys, Lys(iPr), Narg, Ndab, Nlys, Norn, Orn, Orn(iPr), Arg, NMeLys;
alloThr, Cit, Hgn, Hse, Leu((3R)OH), Asn, Gln, Ser, Thr, Asp, Glu, Hgl steht;
P¹⁶ für Ala, Ala(cPr), Ala(Tetrahydropyran4yl), Abu, alloIle, Cyg, Dea, Ile, Leu, Nle, tBuGly, tBuAla, Val, NMeAla, NMeVal, Nva;
Phe, His, Phe(3OH), Phe(4F), Trp(6Cl), Trp, Tyr, 4Thz, Phe(4(4Hydroxyphenoxy)), Phe(4NH2), Tyr(Me), Ntyr, Nphe;
oder ein Tautomer, ein Rotamer, ein Salz, ein Hydrat oder ein Solvat davon,
wobei
Cys oder Pen an P⁶, sofern vorhanden, und Cys oder Pen an P¹³, sofern vorhanden, gegebenenfalls eine Disulfidbrücke zwischen P⁶ und P¹³ bilden; oder wobei
Dab oder Dap an P⁶, sofern vorhanden, und Asp oder Glu an P¹³, sofern vorhanden, gegebenenfalls eine Lactambrücke zwischen P⁶ und P¹³ bilden; oder wobei
Asp oder Glu an P⁶, sofern vorhanden, und Dab oder Dap an P¹³, sofern vorhanden, gegebenenfalls eine Lactambrücke zwischen P⁶ und P¹³ bilden;
mit der Maßgabe, dass mindestens drei Aminosäurereste unter den vier Aminosäureresten an den Positionen P⁹, P¹², P¹⁴ und P¹⁵ basische Aminosäurereste sind, die aus Agb, Agp, Dab, Dab(iPr), Dap, Dap(iPr), Lys, Lys(iPr), Narg, Ndab, Nlys, Norn, Orn, Orn(iPr), Arg, NMeLys ausgewählt sind.

5. Verbindung nach einem der Ansprüche 1 bis 4, wobei P¹ für Val, 2OHVal, NMeVal, Gua-Val, TMG-Val, Abu oder tBuGly steht;
P² für Pro, Pro (4R)F, Pro(4R)OMe, Pro (4R)OPhe, Pro(3,4dehydro), Pr(4,4F2), Pic, Pro((4R)NH₂), Ndab, NalloThr oder Hyp steht;
P³ für Ile steht;
P⁴ für Ile, Thr, Phe, Dab, Orn, Arg, Tyr, Leu, Asn, Lys, Lys(Me), Dap, Val (3OH) oder alloThr steht;
P⁵ für Trp oder Tyr steht;
P⁶ für Cys, Pen, Asp oder Pra steht;
P⁷ für Asn, Ala, Leu, Ile, Ser, Thr, Lys, Dap, Glu oder His steht;
P⁸ für Arg steht;
P⁹ für Arg, Dab, Dab(iPr), Lys oder Cit steht;
P¹⁰ für Ser oder Thr steht;
P¹¹ für ^{D}Dab, ^{D}Dap, ^{D}Orn, ^{D}Lys, ^{D}Arg oder ^{D}Dab(iPr) steht;
P¹² für Lys, Ile, Ser, Tyr, Trp, Asn, Dab, Orn oder Cit steht;
P¹³ für Cys, Pen, Dab oder Abu(4N₃) steht;
P¹⁴ für Dab, Dab(iPr), Lys, Gln, Ser oder Tyr steht;
P¹⁵ für Arg, Thr, Leu, Ser, Dab, Lys, Orn oder Orn(iPr) steht; und
P¹⁶ für Nle, Cha oder Tyr steht;
oder ein Tautomer, ein Rotamer, ein Salz, ein Hydrat oder ein Solvat davon,
wobei Cys oder Pen an P⁶, sofern vorhanden, und Cys oder Pen an P¹³, sofern vorhanden, gegebenenfalls eine Disulfidbrücke zwischen P⁶ und P¹³ bilden oder wobei
Asp an P⁶, sofern vorhanden, und Dab an P¹³, sofern vorhanden, gegebenenfalls eine Lactambrücke zwischen P⁶ und P¹³ bilden oder wobei
Pra an P⁶, sofern vorhanden, und Abu(4N₃) an P¹³, sofern vorhanden, gegebenenfalls eine 1,2,3-Triazolbrücke zwischen P⁶ und P¹³ bilden;
mit der Maßgabe, dass mindestens drei Aminosäurereste unter den vier Aminosäureresten an den Positionen P⁹, P¹², P¹⁴ und P¹⁵ basische Aminosäurereste sind, die aus Dab, Lys, Arg oder Dab (iPr) an P⁹, Lys, Dab oder Orn an P¹², Dab, Dab(iPr) oder Lys an P¹⁴ und Arg, Dab, Lys, Orn oder Orn(iPr) an P¹⁵ ausgewählt sind.

6. Verbindung nach einem der Ansprüche 1 bis 5, wobei P¹ für Val, NMeVal, Gua-Val, TMG-Val oder Abu steht;
P² für Pro, Pro(4R)OMe, Pro(3,4dehydro), Pic, Pro((4R)NH₂), Ndab, NalloThr oder Hyp steht;
P³ für Ile steht;
P⁴ für Ile, Thr, Phe, Dab, Arg, Val(3OH) oder Tyr steht;
P⁵ für Tyr steht;
P⁶ für Cys, Pen oder Asp steht;
P⁷ für Asn, Leu, Ile, Ser, Dap oder His steht;
P⁸ für Arg steht;
P⁹ für Arg, Lys, Dab oder Dab(iPr) steht;
P¹⁰ für Ser oder Thr steht;
P¹¹ für ^{D}Dab, ^{D}Dab(iPr) oder ^{D}Arg steht;
P¹² für Lys, Ile, Ser, Dab, Orn oder Cit steht;
P¹³ für Cys, Pen oder Dab steht;
P¹⁴ für Dab, Dab(iPr), Lys, Gln, Ser oder Tyr steht;
P¹⁵ für Arg, Dab, Orn, Orn(iPr), Ser oder Thr steht;
P¹⁶ für Nle, Cha oder Tyr steht;
oder ein Tautomer, ein Rotamer, ein Salz, ein Hydrat oder ein Solvat davon,
wobei Cys oder Pen an P⁶, sofern vorhanden, und Cys oder Pen an P¹³, sofern vorhanden, gegebenenfalls eine Disulfidbrücke zwischen P⁶ und P¹³ bilden oder wobei
Asp an P⁶, sofern vorhanden, und Dab an P¹³, sofern vorhanden, gegebenenfalls eine Lactambrücke zwischen P⁶ und P¹³ bilden;
mit der Maßgabe, dass mindestens drei Aminosäurereste unter den vier Aminosäureresten an den Positionen P⁹, P¹², P¹⁴ und P¹⁵ basische Aminosäurereste sind, die aus Dab, Lys, Arg oder Dab (iPr) an P⁹, Lys, Orn oder Dab an P¹², Lys, Dab oder Dab(iPr) an P¹⁴ und Arg, Dab, Orn oder Orn(iPr) an P¹⁵ ausgewählt sind.

7. Verbindung nach einem der Ansprüche 1 bis 6, wobei
P¹ für 2OHVal, Gua-Val oder TMG-Val steht;
P² für Pro, Pro((4R)NH₂) oder Hyp steht;
P³ für Ile steht;
P⁴ für Ile, Thr, Dab oder Orn steht;
P⁵ für Tyr steht;
P⁶ für Cys, Pen, Asp oder Pra steht;
P⁷ für Ile, Asn oder Thr steht;
P⁸ für Arg steht;
P⁹ für Lys, Arg, Dab oder Dab(iPr) steht;
P¹⁰ für Ser oder Thr steht;
P¹¹ für ^{D}Dab, ^{D}Dab(iPr) oder ^{D}Dap steht;
P¹² für Lys, Ile, Asn, Ser, Tyr, Orn oder Cit steht;
P¹³ für Cys, Dab oder Abu(4N₃) steht;
P¹⁴ für Dab, Dab(iPr), Lys oder Ser steht;
P¹⁵ für Arg, Dab, Orn; Orn(iPr), Ser oder Thr steht;
P¹⁶ für Nle oder Tyr steht;
oder ein Tautomer, ein Rotamer, ein Salz, ein Hydrat oder ein Solvat davon,
wobei Cys oder Pen an P⁶, sofern vorhanden, und Cys an P¹³, sofern vorhanden, gegebenenfalls eine Disulfidbrücke zwischen P⁶ und P¹³ bilden oder wobei
Asp an P⁶, sofern vorhanden, und Dab an P¹³, sofern vorhanden, gegebenenfalls eine Lactambrücke zwischen P⁶ und P¹³ bilden; oder wobei
Pra an P⁶, sofern vorhanden, und Abu(4N₃) an P¹³, sofern vorhanden, gegebenenfalls eine 1,2,3-Triazolbrücke zwischen P⁶ und P¹³ bilden;
mit der Maßgabe, dass mindestens drei Aminosäurereste unter den vier Aminosäureresten an den Positionen P⁹, P¹², P¹⁴ und P¹⁵ basische Aminosäurereste sind, die aus Dab, Lys, Arg oder Dab(iPr) an P⁹, Lys oder Orn an P¹², Lys, Dab oder Dab(iPr) an P¹⁴ und Arg, Dab, Orn oder Orn(iPr) an P¹⁵ ausgewählt sind.

8. Verbindung nach Anspruch 1, wobei die Verbindung ausgewählt ist aus der Gruppe bestehend aus:
Val-Pro-Ile-Ile-Tyr-Cys-Asn-Arg-Arg-Thr-^{D}Dab-Lys-Cys-Dab-Arg-Nle;
Val-Pro((4R)NH₂)-Ile-Ile-Tyr-Pen-Asn-Arg-Arg-Thr-^{D}Dab-Lys-Cys-Dab-Arg-Nle;
Val-Pro((4R)NH₂)-Ile-Ile-Tyr-Pen-Asn-Arg-Dab-Thr-^{D}Dab-Lys-Cys-Dab-Arg-Nle;
Val-Ndab-Ile-Ile-Tyr-Pen-Asn-Arg-Dab-Thr-^{D}Dab-Lys-Cys-Dab-Arg-Nle
Val-Pro((4R)NH₂)-Ile-Thr-Tyr-Pen-Asn-Arg-Dab-Thr-^{D}Dab-Lys-Cys-Dab-Arg-Nle;
Val-Pro-Ile-Ile-Tyr-Cys-Asn-Arg-Dab-Thr-^{D}Dab-Lys-Cys-Dab-Arg-Cha;
Val-Pro-Ile-Ile-Tyr-Pen-Asn-Arg-Dab-Thr-^{D}Dab-Lys-Cys-Dab-Arg-Tyr;
Val-Pro-Ile-Ile-Tyr-Cys-Asn-Arg-Dab-Thr-^{D}Dab-Lys-Pen-Dab-Arg-Tyr;
Val-Pro((4R)NH₂)-Ile-Ile-Tyr-Cys-Asn-Arg-Dab-Thr-^{D}Dab-Lys-Cys-Dab-Arg-Tyr;
Val-Hyp-Ile-Ile-Tyr-Cys-Asn-Arg-Dab-Thr-^{D}Dab-Lys-Cys-Dab-Arg-Tyr;
Val-Pro-Ile-Thr-Tyr-Cys-Asn-Arg-Dab-Thr-^{D}Dab-Lys-Cys-Dab-Arg-Tyr;
Val-Pro-Ile-Phe-Tyr-Cys-Asn-Arg-Dab-Thr-^{D}Dab-Lys-Cys-Dab-Arg-Tyr;
2OHVal-Pro-Ile-Ile-Tyr-Cys-Asn-Arg-Dab-Thr-^{D}Dab-Lys-Cys-Dab-Arg-Tyr;
Val-Hyp-Ile-Ile-Tyr-Pen-Asn-Arg-Dab-Thr-^{D}Dab-Lys-Cys-Dab-Arg-Tyr;
Val-Hyp-Ile-Thr-Tyr-Pen-Asn-Arg-Dab-Thr-^{D}Dab-Lys-Cys-Dab-Arg-Tyr;
Val-Hyp-Ile-Thr-Tyr-Pen-Asn-Arg-Dab-Thr-^{D}Dap-Lys-Cys-Dab-Arg-Tyr;
Val-Hyp-Ile-Thr-Tyr-Pen-Asn-Arg-Dab-Thr-^{D}Orn-Lys-Cys-Dab-Arg-Tyr;
Val-Hyp-Ile-Thr-Tyr-Pen-Asn-Arg-Dab-Thr-^{D}Lys-Lys-Cys-Dab-Arg-Tyr;
Val-Hyp-Ile-Thr-Tyr-Pen-Asn-Arg-Dab-Thr-^{D}Arg-Lys-Cys-Dab-Arg-Tyr;
Val-Hyp-Ile-Dab-Tyr-Pen-Asn-Arg-Dab-Thr-^{D}Dab-Lys-Cys-Dab-Thr-Tyr;
Val-Hyp-Ile-Arg-Tyr-Pen-Asn-Arg-Dab-Thr-^{D}Dab-Lys-Cys-Dab-Thr-Tyr;
Val-Hyp-Ile-Tyr-Tyr-Pen-Asn-Arg-Dab-Thr-^{D}Dab-Lys-Cys-Dab-Arg-Tyr;
Val-Hyp-Ile-Leu-Tyr-Pen-Asn-Arg-Dab-Thr-^{D}Dab-Lys-Cys-Dab-Arg-Tyr;
Val-Hyp-Ile-Asn-Tyr-Pen-Asn-Arg-Dab-Thr-^{D}Dab-Lys-Cys-Dab-Arg-Tyr;
Val-Hyp-Ile-Dab-Tyr-Pen-Asn-Arg-Dab-Thr-^{D}Dab-Lys-Cys-Dab-Arg-Tyr;
Val-Hyp-Ile-Lys-Tyr-Pen-Asn-Arg-Dab-Thr-^{D}Dab-Lys-Cys-Dab-Arg-Tyr;
Val-Hyp-Ile-Thr-Trp-Pen-Asn-Arg-Dab-Thr-^{D}Dab-Lys-Cys-Dab-Arg-Tyr;
Val-Hyp-Ile-Thr-Tyr-Pen-Ala-Arg-Dab-Thr-^{D}Dab-Lys-Cys-Dab-Arg-Tyr;
Val-Hyp-Ile-Thr-Tyr-Pen-Leu-Arg-Dab-Thr-^{D}Dab-Lys-Cys-Dab-Arg-Tyr;
Val-Hyp-Ile-Thr-Tyr-Pen-Ile-Arg-Dab-Thr-^{D}Dab-Lys-Cys-Dab-Arg-Tyr;
Val-Hyp-Ile-Thr-Tyr-Pen-Ser-Arg-Dab-Thr-^{D}Dab-Lys-Cys-Dab-Arg-Tyr;
Val-Hyp-Ile-Thr-Tyr-Pen-Thr-Arg-Dab-Thr-^{D}Dab-Lys-Cys-Dab-Arg-Tyr;
Val-Hyp-Ile-Thr-Tyr-Pen-Lys-Arg-Dab-Thr-^{D}Dab-Lys-Cys-Dab-Arg-Tyr;
Val-Hyp-Ile-Thr-Tyr-Pen-Asn-Arg-Lys-Thr-^{D}Dab-Lys-Cys-Dab-Arg-Tyr;
Val-Hyp-Ile-Thr-Tyr-Pen-Asn-Arg-Arg-Thr-^{D}Dab-Lys-Cys-Dab-Arg-Tyr;
Val-Hyp-Ile-Thr-Tyr-Pen-Asn-Arg-Dab-Thr-^{D}Dab-Ile-Cys-Dab-Arg-Tyr;
Val-Hyp-Ile-Thr-Tyr-Pen-Asn-Arg-Dab-Thr-^{D}Dab-Ser-Cys-Dab-Arg-Tyr;
Val-Hyp-Ile-Thr-Tyr-Pen-Asn-Arg-Dab-Thr-^{D}Dab-Tyr-Cys-Dab-Arg-Tyr;
Val-Hyp-Ile-Thr-Tyr-Pen-Asn-Arg-Dab-Thr-^{D}Dab-Trp-Cys-Dab-Arg-Tyr;
Val-Hyp-Ile-Thr-Tyr-Pen-Asn-Arg-Dab-Thr-^{D}Dab-Asn-Cys-Dab-Arg-Tyr;
Va-Hyp-Ile-Thr-Tyr-Pen-Asn-Arg-Dab-Thr-^{D}Dab-Dab-Cys-Dab-Arg-Tyr;
Val-Hyp-Ile-Thr-Tyr-Pen-Asn-Arg-Dab-Thr-^{D}Dab-Lys-Cys-Dab-Leu-Tyr
Val-Hyp-Ile-Thr-Tyr-Pen-Asn-Arg-Dab-Thr-^{D}Dab-Lys-Cys-Dab-Ser-Tyr;
Val-Hyp-Ile-Thr-Tyr-Pen-Asn-Arg-Dab-Thr-^{D}Dab-Lys-Cys-Dab-Dab-Tyr;
Val-Hyp-Ile-Thr-Tyr-Pen-Asn-Arg-Dab-Thr-^{D}Dab-Lys-Cys-Dab-Lys-Tyr;
Val-Hyp-Ile-Thr-Tyr-Pen-Asn-Arg-Dab-Thr-^{D}Dab-Lys-Cys-Dab-Orn-Tyr;
Val-Hyp-Ile-Thr-Tyr-Pen-Asn-Arg-Dab-Thr-^{D}Dab-Cit-Cys-Dab-Arg-Tyr;
NMeVal-Hyp-Ile-Thr-Tyr-Pen-Asn-Arg-Dab-Thr-^{D}Dab-Lys-Cys-Dab-Arg-Tyr;
Val-Hyp-Ile-Thr-Tyr-Pen-Dap-Arg-Dab-Thr-^{D}Dab-Lys-Cys-Dab-Arg-Tyr;
Val-Hyp-Ile-Dap-Tyr-Pen-Asn-Arg-Dab-Thr-^{D}Dab-Lys-Cys-Dab-Arg-Tyr;
Val-Hyp-Ile-Arg-Tyr-Pen-Asn-Arg-Dab-Thr-^{D}Dab-Lys-Cys-Dab-Arg-Tyr;
Val-Pro((4R)NH₂)-Ile-Thr-Tyr-Pen-Asn-Arg-Dab-Thr-^{D}Dab-Lys-Cys-Tyr-Arg-Tyr;
Gua-Val-Pro-Ile-Ile-Tyr-Cys-Asn-Arg-Dab-Thr-^{D}Dab-Lys-Cys-Dab-Arg-Tyr;
Gua-Val-Pro-Ile-Ile-Tyr-Pen-Asn-Arg-Dab-Thr-^{D}Dab-Lys-Cys-Dab-Arg-Tyr;
Gua-Val-Hyp-Ile-Thr-Tyr-Pen-Asn-Arg-Dab-Thr-^{D}Dab-Lys-Cys-Dab-Arg-Tyr;
Gua-Val-Hyp-Ile-Ile-Tyr-Pen-Asn-Arg-Dab-Thr-^{D}Dab-Lys-Cys-Dab-Arg-Tyr;
Gua-Val-Pro((4R)NH₂)-Ile-Thr-Tyr-Pen-Asn-Arg-Dab-Thr-^{D}Dab-Lys-Cys-Dab-Arg-Tyr;
Gua-Val-Pro((4R)NH₂)-Ile-Ile-Tyr-Pen-Asn-Arg-Dab-Thr-^{D}Dab-Lys-Cys-Dab-Arg-Tyr;
Gua-Val-Hyp-Ile-Tyr-Tyr-Pen-Asn-Arg-Dab-Thr-^{D}Dab-Lys-Cys-Gln-Arg-Tyr;
Gua-Val-Hyp-Ile-Thr-Tyr-Pen-Glu-Arg-Dab-Thr-^{D}Dab-Lys-Cys-Dab-Arg-Tyr;
Gua-Val-Hyp-Ile-Thr-Tyr-Pen-His-Arg-Dab-Thr-^{D}Dab-Lys-Cys-Dab-Arg-Tyr;
Gua-Val-Hyp-Ile-Thr-Tyr-Pen-Asn-Arg-Dab-Thr-^{D}Dab-Dab-Cys-Dab-Arg-Tyr;
Gua-Val-Hyp-Ile-Thr-Tyr-Pen-Asn-Arg-Dab-Ser-^{D}Dab-Lys-Cys-Dab-Arg-Tyr;
Gua-Val-Hyp-Ile-Thr-Tyr-Pen-Asn-Arg-Dab-Ser-^{D}Dab-Lys-Cys-Dab-Dab-Tyr;
Gua-Val-Hyp-Ile-Thr-Tyr-Pen-Asn-Arg-Dab-Ser-^{D}Dab-Lys-Cys-Dab-Orn-Tyr;
Gua-Val-Pro((4R)NH₂)-Ile-Thr-Tyr-Pen-Asn-Arg-Dab-Thr-^{D}Dab-Lys-Cys-Tyr-Arg-Tyr;
Gua-Val-Pro((4R)NH₂)-Ile-Ile-Tyr-Pen-Asn-Arg-Dab-Thr-^{D}Dab-Lys-Cys-Tyr-Arg-Tyr;
Gua-Val-Hyp-Ile-alloThr-Tyr-Pen-Asn-Arg-Dab-Thr-^{D}Dab-Lys-Cys-Tyr-Arg-Tyr;
TMG-Val-Pro-Ile-Ile-Tyr-Cys-Asn-Arg-Dab-Thr-^{D}Dab-Lys-Cys-Dab-Arg-Tyr;
TMG-Val-Hyp-Ile-Thr-Tyr-Pen-Asn-Arg-Dab-Thr-^{D}Dab-Lys-Cys-Dab-Arg-Tyr;
Val-Hyp-Ile-Ile-Tyr-Pra-Asn-Arg-Dab-Thr-^{D}Dab-Lys-Abu (4N₃) -Dab-Arg-Tyr;
Val-Hyp-Ile-Ile-Tyr-Asp-Asn-Arg-Dab-Thr-^{D}Dab-Lys-Dab-Dab-Arg-Tyr;
Val-Hyp-Ile-Ile-Tyr-Pen-Asn-Arg-Dab-Thr-^{D}Dab-Lys-Cys-Dab-Cit-Tyr;
Val-Hyp-Ile-Ile-Tyr-Pen-Asn-Arg-Dab-Thr-^{D}Dab-Lys-Cys-Dab-Dab-Tyr;
Val-Hyp-Ile-Ile-Tyr-Pen-Asn-Arg-Dab-Ser-^{D}Dab-Lys-Cys-Dab-Arg-Tyr;
Val-Hyp-Ile-Ile-Tyr-Pen-Asn-Arg-Dab-Ser-^{D}Dab-Lys-Cys-Dab-Dab-Tyr;
Val-Hyp-Ile-Thr-Tyr-Pen-Asn-Arg-Dab-Ser-^{D}Dab-Lys-Cys-Dab-Dab-Tyr;
Val-Hyp-Ile-Ile-Tyr-Pen-Asn-Arg-Dab-Thr-^{D}Dab-Orn-Cys-Dab-Arg-Tyr;
Abu-Hyp-Ile-Ile-Tyr-Pen-Asn-Arg-Dab-Thr-^{D}Dab-Lys-Cys-Dab-Arg-Tyr;
Val-Pro-Ile-Thr-Tyr-Pen-Asn-Arg-Dab-Thr-^{D}Dab-Lys-Cys-Dab-Arg-Tyr;
Val-Hyp-Ile-Dab-Tyr-Pen-Asn-Arg-Dab-Ser-^{D}Dab-Lys-Cys-Dab-Dab-Tyr;
Val-Hyp-Ile-Ile-Tyr-Pen-Asn-Arg-Lys-Thr-^{D}Dab-Lys-Cys-Lys-Arg-Tyr;
Val-Hyp-Ile-Ile-Tyr-Pen-Asn-Arg-Dab-Thr-^{D}Dab-Ile-Cys-Dab-Dab-Tyr;
Val-Hyp-Ile-Ile-Tyr-Pen-Asn-Arg-Dab-Ser-^{D}Dab-Ile-Cys-Dab-Dab-Tyr;
Val-Hyp-Ile-Ile-Tyr-Pen-Asn-Arg-Dab-Thr-^{D}Dab-Lys-Cys-Dab-Orn-Tyr;
Val-Hyp-Ile-Ile-Tyr-Pen-Asn-Arg-Dab-Ser-^{D}Dab-Lys-Cys-Dab-Orn-Tyr;
Val-Hyp-Ile-Ile-Tyr-Pen-Asn-Arg-Dab-Thr-^{D}Dab-Ile-Cys-Dab-Arg-Tyr;
Val-Hyp-Ile-Thr-Tyr-Pen-Ile-Arg-Dab-Thr-^{D}Dab-Lys-Cys-Dab-Orn-Tyr;
Val-Hyp-Ile-Dab-Tyr-Pen-Asn-Arg-Dab-Thr-^{D}Dab-Lys-Cys-Dab-Orn-Tyr;
Val-Pro((4R)NH₂)-Ile-Ile-Tyr-Pen-Asn-Arg-Dab-Thr-^{D}Dab-Ile-Cys-Dab-Arg-Tyr;
Val-Pro((4R)NH₂)-Ile-Ile-Tyr-Pen-Asn-Arg-Dab-Thr-^{D}Dab-Cit-Cys-Dab-Arg-Tyr;
Val-Hyp-Ile-Thr-Tyr-Pen-Ile-Arg-Dab-Thr-^{D}Dab-Lys-Cys-Ser-Orn-Tyr;
Val-Pro((4R)NH₂)-Ile-Ile-Tyr-Pen-Asn-Arg-Dab-Thr-^{D}Dab-Lys-Cys-Ser-Arg-Tyr;
Val-Hyp-Ile-Thr-Tyr-Pen-Asn-Arg-Dab-Ser-^{D}Dab-Lys-Cys-Dab-Orn-Tyr;
tBuGly-Hyp-Ile-Thr-Tyr-Pen-Ile-Arg-Dab-Thr-^{D}Dab-Lys-Cys-Dab-Orn-Tyr;
Val-Hyp-Ile-Ile-Tyr-Pen-Asn-Arg-Dab-Ser-^{D}Dab-Ile-Cys-Dab-Orn-Tyr;
Val-Hyp-Ile-Ile-Tyr-Pen-Asn-Arg-Dab-Thr-^{D}Dab-Ile-Cys-Dab-Orn-Tyr;
Val-Hyp-Ile-Ile-Tyr-Pen-Asn-Arg-Dab-Thr-^{D}Dab-Cit-Cys-Dab-Arg-Tyr;
Val-Hyp-Ile-Thr-Tyr-Pen-Ile-Arg-Dab-Thr-^{D}Dab-Lys-Cys-Dab-Ser-Tyr;
Val-Pro((4R)NH₂)-Ile-Ile-Tyr-Pen-Asn-Arg-Dab-Thr-^{D}Dab-Lys-Cys-Dab-Ser-Tyr;
Val-Hyp-Ile-Ile-Tyr-Pen-Asn-Arg-Dab-Thr-^{D}Dab-Ser-Cys-Dab-Arg-Tyr;
Val-Pro((4R)NH₂)-Ile-Thr-Tyr-Pen-Asn-Arg-Dab-Thr-^{D}Dab-Cit-Cys-Dab-Ser-Tyr;
Val-Pro((4R)NH₂)-Ile-Thr-Tyr-Pen-Asn-Arg-Dab-Thr-^{D}Dab-Lys-Cys-Ser-Ser-Tyr;
Val-Hyp-Ile-Thr-Tyr-Pen-Ile-Arg-Dab-Thr-^{D}Dab-Lys-Cys-Ser-Arg-Tyr;
Val-Hyp-Ile-Ile-Tyr-Pen-Asn-Arg-Dab-Thr-^{D}Dab-Lys-Cys-Dab-Ser-Tyr;
Val-Pro((4R)NH₂)-Ile-Ile-Tyr-Pen-Asn-Arg-Dab-Thr-^{D}Dab-Cit-Cys-Dab-Ser-Tyr;
Val-Hyp-Ile-Ile-Tyr-Pen-Asn-Arg-Dab-Thr-^{D}Dab-Lys-Cys-Ser-Arg-Tyr;
Val-Hyp-Ile-Dab-Tyr-Pen-Asn-Arg-Dab-Thr-^{D}Dab-Cit-Cys-Dab-Ser-Tyr;
Val-Pro((4R)NH₂)-Ile-Ile-Tyr-Pen-Asn-Arg-Dab-Thr-^{D}Dab-Lys-Cys-Dab-Thr-Tyr;
Val-Pro((4R)NH₂)-Ile-Thr-Tyr-Pen-Asn-Arg-Dab-Thr-^{D}Dab-Lys-Cys-Ser-Arg-Tyr;
Val-Hyp-Ile-Ile-Tyr-Pen-Asn-Arg-Dab-Thr-^{D}Dab-Lys-Cys-Dab-Thr-Tyr;
Val-Hyp-Ile-Ile-Tyr-Pen-Asn-Arg-Dab-Ser-^{D}Dab-Lys-Cys-Dab-Thr-Tyr;
Val-Hyp-Ile-Dab-Tyr-Pen-Asn-Arg-Dab-Thr-^{D}Dab-Cit-Cys-Dab-Thr-Tyr;
tBuGly-Hyp-Ile-Ile-Tyr-Pen-Asn-Arg-Dab-Thr-^{D}Dab-Lys-Cys-Dab-Arg-Tyr;
Val-Pro((4R)NH₂)-Ile-Ile-Tyr-Pen-Asn-Arg-Dab-Thr-^{D}Dab-Ile-Cys-Dab-Thr-Tyr;
Val-Pro((4R)NH₂)-Ile-Thr-Tyr-Pen-Asn-Arg-Dab-Thr-^{D}Dab-Cit-Cys-Dab-Thr-Tyr;
Val-Pro((4R)NH₂)-Ile-Thr-Tyr-Pen-Asn-Arg-Dab-Thr-^{D}Dab-Ile-Cys-Dab-Thr-Tyr;
Val-Hyp-Ile-Ile-Tyr-Pen-Asn-Arg-Dab-Ser-^{D}Dab-Ile-Cys-Dab-Arg-Tyr;
Val-Pro((4R)NH₂)-Ile-Thr-Tyr-Pen-Asn-Arg-Dab-Thr-^{D}Dab-Lys-Cys-Ser-Thr-Tyr;
Val-Pro((4R)NH₂)-Ile-Thr-Tyr-Pen-Asn-Arg-Dab-Thr-^{D}Dab-Lys-Cys-Dab-Thr-Tyr;
Val-Hyp-Ile-Ile-Tyr-Pen-Asn-Arg-Orn-Thr-^{D}Dab-Ile-Cys-Dab-Arg-Tyr;
Val-Hyp-Ile-Orn-Tyr-Pen-Asn-Arg-Dab-Thr-^{D}Dab-Lys-Cys-Dab-Thr-Tyr;
Val-Hyp-Ile-Thr-Tyr-Pen-Ile-Arg-Dab-Thr-^{D}Dab-Orn-Cys-Dab-Arg-Tyr;
Val-Hyp-Ile-Ile-Tyr-Pen-Asn-Arg-Cit-Thr-^{D}Dab-Lys-Cys-Dab-Arg-Tyr;
Val-Hyp-Ile-Lys(Me)-Tyr-Pen-Asn-Arg-Dab-Thr-^{D}Dab-Lys-Cys-Dab-Arg-Tyr;
Val-Hyp-Ile-Val(30H)-Tyr-Pen-Asn-Arg-Dab-Thr-^{D}Dab-Lys-Cys-Dab-Arg-Tyr;
Val-Pro(4,4F₂)-Ile-Thr-Tyr-Pen-Asn-Arg-Dab-Thr-^{D}Dab-Lys-Cys-Dab-Arg-Tyr;
Val-Pro(3,4dehydro)-Ile-Thr-Tyr-Pen-Asn-Arg-Dab-Thr-^{D}Dab-Lys-Cys-Dab-Arg-Tyr;
Val-Pro(4,4F₂)-Ile-Thr-Tyr-Pen-Asn-Arg-Dab-Thr-^{D}Dab-Lys-Cys-Dab-Arg-Tyr;
Val-Pro((4R)OPh)-Ile-Thr-Tyr-Pen-Asn-Arg-Dab-Thr-^{D}Dab-Lys-Cys-Dab-Arg-Tyr
Val-Pro((4R)F)-Ile-Thr-Tyr-Pen-Asn-Arg-Dab-Thr-^{D}Dab-Lys-Cys-Dab-Arg-Tyr;
Val-Pic-Ile-Thr-Tyr-Pen-Asn-Arg-Dab-Thr-^{D}Dab-Lys-Cys-Dab-Arg-Tyr;
Val-NalloThr-Ile-Thr-Tyr-Pen-Asn-Arg-Dab-Thr-^{D}Dab-Lys-Cys-Dab-Arg-Tyr;
Hyp-Ile-Thr-Tyr-Pen-Asn-Arg-Dab(iPr)-Thr-^{D}Dab-Lys-Cys-Dab-Orn(iPr)-Tyr;
Hyp-Ile-Thr-Tyr-Pen-Asn-Arg-Dab(iPr)-Thr-^{D}Dab-Lys-Cys-Dab(iPr)-Arg-Tyr;
Hyp-Ile-Thr-Tyr-Pen-Asn-Arg-Dab-Thr-^{D}Dab-Lys-Cys-Dab-Orn(iPr)-Tyr;
Hyp-Ile-Thr-Tyr-Pen-Ile-Arg-Dab-Thr-^{D}Dab-Lys-Cys-Dab-Orn(iPr)-Tyr und
Hyp-Ile-Thr-Tyr-Pen-Asn-Arg-Dab-Thr-^{D}Dab(iPr)-Lys-Cys-Dab-Arg-Tyr;
oder ein Tautomer, ein Rotamer, ein Salz, ein Hydrat oder ein Solvat davon,
wobei Cys oder Pen an P⁶, sofern vorhanden, und Cys oder Pen an P¹³, sofern vorhanden, gegebenenfalls eine Disulfidbrücke zwischen P⁶ und P¹³ bilden oder wobei
Asp an P⁶, sofern vorhanden, und Dab an P¹³, sofern vorhanden, gegebenenfalls eine Lactambrücke zwischen P⁶ und P¹³ bilden oder wobei
Pra an P⁶, sofern vorhanden, und Abu(4N₃) an P¹³, sofern vorhanden, gegebenenfalls eine 1,2,3-Triazolbrücke zwischen P⁶ und P¹³ bilden.

9. Verbindung nach Anspruch 8, wobei Cys oder Pen an P⁶ und Cys oder Pen an P¹³ eine Disulfidbrücke zwischen P⁶ und P¹³ bilden.

10. Verbindung nach Anspruch 9, wobei es sich bei der Verbindung um Folgendes handelt:
Gua-Val-Hyp-Ile-Thr-Tyr-Pen-Asn-Arg-Dab-Thr-^{D}Dab-Lys-Cys-Dab-Arg-Tyr;
oder ein Tautomer, ein Rotamer, ein Salz, ein Hydrat oder ein Solvat davon.

11. Pharmazeutische Zusammensetzung, enthaltend eine Verbindung der Formel (I) oder eine Mischung von Verbindungen der Formel (I) gemäß einem der Ansprüche 1 bis 10 oder ein pharmazeutisch unbedenkliches Salz davon und mindestens einen pharmazeutisch inerten Träger, gegebenfalls in einer zur oralen Verabreichung, topischen Verabreichung, transdermalen Verabreichung, Verabreichung durch Injektion, bukkalen Verabreichung, transmukosalen Verabreichung, rektalen Verabreichung, pulmonalen Verabreichung oder Verabreichung durch Inhalation geeigneten Form, insbesondere in Form einer Tablette, eines Dragees, einer Kapsel, einer Lösung, einer Flüssigkeit, eines Gels, eines Pflasters, einer Creme, einer Salbe, eines Sirups, einer Aufschlämmung, einer Suspension, eines Sprays, eines Verneblers, eines Aerosols oder eines Suppositoriums.

12. Verbindung der Formel (I) gemäß einem der Ansprüche 1 bis 10 oder ein pharmazeutisch unbedenkliches Salz davon zur Verwendung als Medikament.

13. Verbindung der Formel (I) gemäß einem der Ansprüche 1 bis 10 oder ein pharmazeutisch unbedenkliches Salz davon zur Verwendung als pharmazeutischer Wirkstoff mit antibiotischer Wirkung bei der Behandlung oder Prävention einer Infektion oder mit einer derartigen Infektion zusammenhängenden Erkrankung.

14. Verbindung zur Verwendung nach Anspruch 13 oder ein pharmazeutisch unbedenkliches Salz davon, wobei die Infektion oder mit einer derartigen Infektion zusammenhängende Erkrankung mit Atemwegserkrankungen oder Haut- oder Weichteilerkrankungen oder Magen-DarmErkrankungen oder Augenerkrankungen oder Ohrenerkrankungen oder ZNS-Erkrankungen oder Knochenerkrankungen oder Herz-Kreislauf-Erkrankungen oder Urogenitalerkrankungen zusammenhängenden Infektion oder nosokomialen Infektionen oder katheterbedingten und nicht katheterbedingten Infektionen oder Harnwegsinfektionen oder Blutkreislaufinfektionen oder durch Infektion induzierter Sepsis zusammenhängt.

15. Verbindung zur Verwendung nach Anspruch 13 oder Anspruch 14 oder ein pharmazeutisch unbedenkliches Salz davon, wobei die Infektion aus nosokomialen Infektionen, katheterbedingten und nicht katheterbedingten Infektionen, Harnwegsinfektionen, Blutkreislaufinfektionen oder einer mit einer Infektion assoziierten Erkrankung oder Störung, insbesondere Erkrankungen oder Störungen wie beatmungsassoziierter Pneumonie (Ventilator-Associated Pneumonia, VAP), beatmungsassoziierter bakterieller Pneumonie (Ventilator-Associated Bacterial Pneumonia, VABP), im Krankenhaus erworbener Pneumonie (Hospital-Acquired Pneumonia, HAP), im Krankenhaus erworbener bakterieller Pneumonie (Hospital-Acquired Bacterial Pneumonia, HABP), in Pflegeeinrichtungen erworbener Pneumonie (Healthcare-Associated Pneumonia, HCAP), zystischer Fibrose, Emphysem, Asthma, Pneumonie, epidemischer Diarrhoe, nekrotisierender Enterokolitis, Typhlitis, Gastroenteritis, Pankreatitis, Keratitis, Endophthalmitis, Otitis, Hirnabszess, Meningitis, Enzephalitis, Osteochondritis, Perikarditis, Epididymitis, Prostatitis, Urethritis, Sepsis; Operationswunden, traumatischen Wunden und Verbrennungen, ausgewählt ist.

16. Verwendung einer Verbindung der Formel (I) gemäß einem der Ansprüche 1 bis 10 oder eines Salzes davon als Desinfektionsmittel oder Konservierungsstoff für Lebensmittel, Kosmetika, Medikamente und/oder andere nährstoffhaltige Materialien.

17. Verfahren zur Herstellung einer Verbindung der Formel (I) gemäß einem der Ansprüche 1 bis 10 oder eines Salzes davon, das die folgenden Schritte umfasst:
(a) Kuppeln eines geeignet funktionalisierten festen Trägers mit einem geeignet N-geschützten Derivat der Aminosäure, die sich in dem gewünschten Endprodukt in Position P¹⁶ befindet; wobei jegliche funktionelle Gruppe, die in dem N-geschützten Aminosäurederivat vorliegen kann, ebenfalls geeignet geschützt ist;
(b) Entfernen der N-Schutzgruppe von dem so erhaltenen Produkt;
(c) Kuppeln des so erhaltenen Produkts mit einem geeignet N-geschützten Derivat der Aminosäure, die sich in dem gewünschten Endprodukt in Position P¹⁵ befindet; wobei jegliche funktionelle Gruppe, die in dem N-geschützten Aminosäurederivat vorliegen kann, ebenfalls geeignet geschützt ist;
(d) Durchführen von Schritten, die weitgehend den Schritten (b) und (c) entsprechen, unter Verwendung von geeignet N-geschützten Derivaten von Aminosäuren, die sich in dem gewünschten Endprodukt in den Positionen P¹⁴ bis P⁶ befinden, wobei jegliche funktionelle Gruppe bzw. jegliche funktionellen Gruppen, die in den N-geschützten Aminosäurederivaten vorliegen kann bzw. können, ebenfalls geeignet geschützt ist bzw. sind;
(e) gegebenenfalls selektives Entschützen einer oder mehrerer geschützter funktioneller Gruppen, die in dem Molekül vorliegen, und chemisches Umwandeln der so freigesetzten reaktiven Gruppe bzw. reaktiven Gruppen;
(f) Durchführen von Schritten, die weitgehend den Schritten (b) und (c) entsprechen, unter Verwendung von geeignet N-geschützten Derivaten von Aminosäuren, die sich in dem gewünschten Endprodukt in den Positionen P⁵ bis P² befinden, wobei jegliche funktionelle Gruppe bzw. jegliche funktionellen Gruppen, die in den N-geschützten Aminosäurederivaten vorliegen kann bzw. können, ebenfalls geeignet geschützt ist bzw. sind; und gegebenenfalls nach jeder Kupplung selektives Entschützen einer oder mehrerer geschützter funktioneller Gruppen, die in dem Molekül vorliegen, und chemisches Umwandeln der so freigesetzten reaktiven Gruppe bzw. reaktiven Gruppen;
(g) weiteres Durchführen von Schritten, die weitgehend den Schritten (b) und (c) entsprechen, unter Verwendung eines geeignet N-geschützten Derivats einer Aminosäure oder gegebenenfalls eines geeignet geschützten Derivats einer Hydroxysäure, die sich in dem gewünschten Endprodukt in Position P¹ befindet, wobei jegliche funktionelle Gruppe bzw. jegliche funktionellen Gruppen, die in dem N-geschützten Aminosäurederivat bzw. Hydroxysäurederivat vorliegen kann bzw. können, ebenfalls geeignet geschützt ist bzw. sind; und gegebenenfalls nach der Kupplung selektives Entschützen einer oder mehrerer geschützter funktioneller Gruppen, die in dem Molekül vorliegen, und chemisches Umwandeln der so freigesetzten reaktiven Gruppe bzw. reaktiven Gruppen;
(h) gegebenenfalls selektives Entschützen einer oder mehrerer geschützter funktioneller Gruppen, die in dem Molekül vorliegen, und chemisches Umwandeln der so freigesetzten reaktiven Gruppe bzw. reaktiven Gruppen;
(i) gegebenenfalls Entfernen der N-Schutzgruppe an Position P¹;
(j) Ablösen des so erhaltenen Produkts von dem festen Träger;
(k) gegebenenfalls selektives Entschützen einer oder mehrerer geschützter funktioneller Gruppen, die in dem Molekül vorliegen, und chemisches Umwandeln der so freigesetzten reaktiven Gruppe bzw. reaktiven Gruppen;
(l) Entfernen jeglicher Schutzgruppen, die an funktionellen Gruppen beliebiger Glieder der Kette von Resten vorliegen, und gegebenenfalls jeglicher Schutzgruppen, die zusätzlich in dem Molekül vorliegen können;
(m) gegebenenfalls Durchführen von zusätzlichen chemischen Transformationen einer oder mehrerer reaktiver Gruppen, die in dem Molekül vorliegen;
(n) falls erforderlich, Entfernen jeglicher Schutzgruppen, die an funktionellen Gruppen beliebiger Glieder der Kette von Resten vorliegen, und gegebenenfalls jeglicher Schutzgruppen, die zusätzlich in dem Molekül vorliegen können; und
(o) gegebenenfalls Umwandeln des so erhaltenen Produkts in ein pharmazeutisch unbedenkliches Salz; oder
gegebenenfalls Umwandeln eines so erhaltenen pharmazeutisch unbedenklichen oder nicht unbedenklichen Salzes in die entsprechende freie Verbindung der Formel (I); oder
gegebenenfalls Umwandeln eines so erhaltenen pharmazeutisch unbedenklichen oder nicht unbedenklichen Salzes in ein anderes, pharmazeutisch unbedenkliches Salz.

## Revendications

1. Composé peptidomimétique de la formule générale (I),
P¹-P²-P³-P⁴-P⁵-P⁶-P⁷-P⁸-P⁹-P¹⁰-P¹¹-P¹²-P¹³-P¹⁴-P¹⁵-P¹⁶ (I)
P¹ étant 2OHVal, Val, NMeVal, Abu, tBuGly ;
le groupe amino de P¹ étant éventuellement remplacé par un groupe guanidino (Gua) ou par un groupe tétraméthylguanidino (TMG) ;
P² étant Pro, Pro (4R) F, Pro(4R)OMe, Pro(4R)OPhe, Pro(3,4dehydro), Pr(4,4F2), Pic ;
Agb, Agp, Dab, Dab(iPr), Dap, Dap(iPr), Har, Lys, Lys(iPr), Narg, Ndab, Nlys, Norn, Orn, Orn(iPr), Pro((4R)guanidine), Pro((4R)NH₂), Pro((4S)NH₂), Arg, NMeLys ;
Hyp, NalloThr ;
P³ étant Hle, Ile, Leu, Nle, Cyg ;
P⁴ étant Ala, Ala(cPr), Ala(tétrahydropyran4yle), Abu, alloIle, Cha, Cpa, Cpg, Cyg, Dea, Hle, Ile, Leu, Met, Nle, OctGly, tBuGly, tBuAla, Val, NMeAla, NMeVal, Nva ;
Phe, His, Phe(3OH), Phe(4F), Phe(4OCF3), Trp(6Cl), Tyr(3Cl), Tyr(3F), Tyr(phényle), Trp, Tyr, 4Thz, Phe(4(4hydroxyphénoxy)), Phe(4NH2), Tyr(Me), Ntyr, Nphe ;
Agb, Agp, Dab, Dab(iPr), Dap, Dap(iPr), Har, Lys, Lys(iPr), Narg, Ndab, Nlys, Norn, Orn, Orn(iPr), Arg, NMeLys, Lys (Me) ;
alloThr, Cit, Hgn, Hse, Leu((3R)OH), Asn, Gln, Ser, Thr, Asp, Glu, Hgl, Val(3OH) ;
P⁵ étant Phe, His, Trp, Tyr ;
P⁶ étant Pra, Abu (4N₃) ;
Dab, Dap ;
Cys, Hcy, NMeCys, Pen ;
Asp, Glu, HgI ;
P⁷ étant Ala, Ala(cPr), Ala(tétrahydropyran4yle), Abu, alloIle, Cha, Cpa, Cpg, Cyg, Dea, Hle, Ile, Leu, Met, Nle, OctGly, tBuGly, tBuAla, Val, NMeAla, NMeVal, Nva ;
Phe, His, Phe(3OH), Phe(4F), Phe(4OCF3), Trp(6Cl), Tyr(3Cl), Tyr(3F), Tyr(phényle), Trp, Tyr, 4Thz, Phe(4(4hydroxyphénoxy)), Phe(4NH2), Tyr(Me), Ntyr, Nphe ;
Agb, Agp, Dab, Dab(iPr), Dap, Dap(iPr), Har, Lys, Lys(iPr), Narg, Ndab, Nlys, Norn, Orn, Orn(iPr), Arg, NMeLys ;
alloThr, Cit, Hgn, Hse, Leu((3R)OH), Asn, Gln, Ser, Thr, Asp, Glu, Hgl ;
P⁸ étant Agb, Har, Arg. Lys, Orn ;
P⁹ étant Dab, Dap, Har, Lys, Orn, Arg, Dab(iPr) ;
Cit ;
P¹⁰ étant alloThr, Hse, Ser, Thr, Hyp, Gln, Asn, Glu, Asp ;
P¹¹ étant ^{D}Dab, ^{D}Dap, ^{D}Lys, ^{D}Orn, ^{D}Arg, ^{D}Dab(iPr) ;
P¹² étant Ala, Ala(cPr), Ala(tétrahydropyran4yle), Abu, alloIle, Cha, Cpa, Cpg, Cyg, Dea, Hle, Ile, Leu, Met, Nle, OctGly, tBuGly, tBuAla, Val, NMeAla, NMeVal, Nva ;
Phe, His, Phe(3OH), Phe(4F), Phe(4OCF3), Trp(6Cl), Tyr(3Cl), Tyr(3F), Tyr(phényle), Trp, Tyr, 4Thz, Phe(4(4hydroxyphénoxy)), Phe(4NH2), Tyr(Me), Ntyr, Nphe ;
Agb, Agp, Dab, Dab(iPr), Dap, Dap(iPr), Har, Lys, Lys(iPr), Narg, Ndab, Nlys, Norn, Orn, Orn(iPr), Arg, NMeLys ;
alloThr, Cit, Hgn, Hse, Leu((3R)OH), Asn, Gln, Ser, Thr, Asp, Glu, Hgl ;
P¹³ étant Pra, Abu (4N₃) ;
Dab, Dap ;
Cys, Hcy, NMeCys, Pen ;
Asp, Glu, HgI ;
P¹⁴ étant Phe, His, Phe(3OH), Phe(4F), Phe(4OCF3), Trp(6Cl), Tyr(3Cl), Tyr(3F), Tyr(phényle), Trp, Tyr, 4Thz, Phe(4(4hydroxyphénoxy)), Phe(4NH2), Tyr(Me), Ntyr, Nphe ;
Agb, Agp, Dab, Dab(iPr) ; Dap, Dap(iPr), Har, Lys, Lys(iPr), Narg, Ndab, Nlys, Norn, Orn, Orn(iPr), Arg, NMeLys ;
alloThr, Cit, Hgn, Hse, Leu((3R)OH), Asn, Gln, Ser, Thr, Asp, Glu, Hgl ;
P¹⁵ étant Ala, Ala(cPr), Ala(tétrahydropyran4yle), Abu, alloIle, Cha, Cpa, Cpg, Cyg, Dea, Hle, Ile, Leu, Met, Nle, OctGly, tBuGly, tBuAla, Val, NMeAla, NMeVal, Nva ;
Agb, Agp, Dab, Dab(iPr), Dap, Dap(iPr), Har, Lys, Lys(iPr), Narg, Ndab, Nlys, Norn, Orn, Orn(iPr), Arg, NMeLys ;
alloThr, Cit, Hgn, Hse, Leu((3R)OH), Asn, Gln, Ser, Thr, Asp, Glu, Hgl ;
P¹⁶ étant Ala, Ala(cPr), Ala(tétrahydropyran4yle), Abu, alloIle, Cha, Cpa, Cpg, Cyg, Dea, Hle, Ile, Leu, Met, Nle, OctGly, tBuGly, tBuAla, Val, NMeAla, NMeVal, Nva ;
Phe, His, Phe(3OH), Phe(4F), Phe(4OCF3), Trp(6Cl), Tyr(3Cl), Tyr(3F), Tyr(phényle), Trp, Tyr, 4Thz, Phe(4(4hydroxyphénoxy)), Phe(4NH2), Tyr(Me), Ntyr, Nphe ;
ou forme tautomère, rotamère, sel, hydrate ou solvate correspondant(e),
Cys, Hcy, NMeCys ou Pen au niveau de P⁶, s'il est présent, et Cys, Hcy, NMeCys ou Pen au niveau de P¹³, s'il est présent, formant éventuellement un pont disulfure entre P⁶ et P¹³ ; ou
Dab ou Dap au niveau de P⁶, s'il est présent, et Asp, Glu ou HgI au niveau de P¹³, s'il est présent, formant éventuellement un pont lactame entre P⁶ et P¹³ ; ou
Asp, Glu ou HgI au niveau de P⁶, s'il est présent, et Dab ou Dap au niveau de P¹³, s'il est présent, formant éventuellement un pont lactame entre P⁶ et P¹³ ; ou
Pra au niveau de P⁶, s'il est présent, et Abu (4N₃) au niveau de P¹³, s'il est présent, formant éventuellement un pont 1,2,3-triazole entre P⁶ et P¹³ ; ou,
Abu (4N₃) au niveau de P⁶, s'il est présent, et Pra au niveau de P¹³, s'il est présent, formant éventuellement un pont 1,2,3-triazole entre P⁶ et P¹³ ;
à la condition qu'au moins trois résidus d'acides aminés parmi les quatre résidus d'acides aminés au niveau des positions P⁹, P¹², P¹⁴ et P¹⁵ soient des résidus d'acides aminés basiques choisis parmi Agb, Agp, Dab, Dab(iPr), Dap, Dap(iPr), Har, Lys, Lys(iPr), Narg, Ndab, Nlys, Norn, Orn, Orn(iPr), Arg, NMeLys.

2. Composé selon la revendication 1,
P¹ étant 2OHVal, Val, NMeVal, Abu, tBuGly ;
le groupe amino de P¹ étant éventuellement remplacé par un groupe guanidino (Gua) ou par un groupe tétraméthylguanidino (TMG) ;
P² étant Pro, Pro (4R) F, Pro(4R)OMe, Pro (4R) OPhe, Pro(3,4dehydro), Pr(4,4F2), Pic ;
Agb, Agp, Dab, Dab(iPr), Dap, Dap(iPr), Har, Lys, Lys(iPr), Narg, Ndab, Nlys, Norn, Orn, Orn(iPr), Pro((4R)guanidine), Pro((4R)NH₂), Pro((4S)NH₂), Arg, NMeLys ;
Hyp, NalloThr ;
P³ étant Ile ;
P⁴ étant Ala, Ala(cPr), Ala(tétrahydropyran4yle), Abu, alloIle, Cha, Cpa, Cpg, Cyg, Dea, Hle, Ile, Leu, Met, Nle, OctGly, tBuGly, tBuAla, Val, NMeAla, NMeVal, Nva ;
Phe, His, Phe(3OH), Phe(4F), Phe(4OCF3), Trp(6Cl), Tyr(3Cl), Tyr(3F), Tyr(phényle), Trp, Tyr, 4Thz, Phe(4(4hydroxyphénoxy)), Phe(4NH2), Tyr(Me), Ntyr, Nphe ;
Agb, Agp, Dab, Dab(iPr), Dap, Dap(iPr), Har, Lys, Lys(iPr), Narg, Ndab, Nlys, Norn, Orn, Orn(iPr), Arg, NMeLys, Lys (Me) ;
alloThr, Cit, Hgn, Hse, Leu((3R)OH), Asn, Gln, Ser, Thr, Asp, Glu, Hgl, Val(3OH) ;
P⁵ étant Phe, His, Trp, Tyr ;
P⁶ étant Dab, Dap ;
Cys, Pen ;
Asp, Glu ;
P⁷ étant Ala, Ala(cPr), Ala(tétrahydropyran4yle), Abu, alloIle, Cha, Cpa, Cpg, Cyg, Dea, Hle, Ile, Leu, Met, Nle, OctGly, tBuGly, tBuAla, Val, NMeAla, NMeVal, Nva ;
Phe, His, Phe(3OH), Phe(4F), Phe(4OCF3), Trp(6Cl), Tyr(3Cl), Tyr(3F), Tyr(phényle), Trp, Tyr, 4Thz, Phe(4(4hydroxyphénoxy)), Phe(4NH2), Tyr(Me), Ntyr, Nphe ;
Agb, Agp, Dab, Dab(iPr), Dap, Dap(iPr), Har, Lys, Lys(iPr), Narg, Ndab, Nlys, Norn, Orn, Orn(iPr), Arg, NMeLys ;
alloThr, Cit, Hgn, Hse, Leu((3R)OH), Asn, Gln, Ser, Thr, Asp, Glu, Hgl ;
P⁸ étant Arg ;
P⁹ étant Dab, Dap, Har, Lys, Orn, Arg, Dab(iPr) ;
Cit ;
P¹⁰ étant alloThr, Hse, Ser, Thr ;
P¹¹ étant ^{D}Dab, ^{D}Dap, ^{D}Lys, ^{D}Orn, ^{D}Arg, ^{D}Dab(iPr) ;
P¹² étant Ala, Ala(cPr), Ala(tétrahydropyran4yle), Abu, alloIle, Cha, Cpa, Cpg, Cyg, Dea, Hle, Ile, Leu, Met, Nle, OctGly, tBuGly, tBuAla, Val, NMeAla, NMeVal, Nva ;
Phe, His, Phe(3OH), Phe(4F), Phe(4OCF3), Trp(6Cl), Tyr(3Cl), Tyr(3F), Tyr(phényle), Trp, Tyr, 4Thz, Phe(4(4hydroxyphénoxy)), Phe(4NH2), Tyr(Me), Ntyr, Nphe ;
Agb, Agp, Dab, Dab(iPr), Dap, Dap(iPr), Har, Lys, Lys(iPr), Narg, Ndab, Nlys, Norn, Orn, Orn(iPr), Arg, NMeLys ;
alloThr, Cit, Hgn, Hse, Leu((3R)OH), Asn, Gln, Ser, Thr, Asp, Glu, Hgl ;
P¹³ étant Dab, Dap ;
Cys, Pen ;
Asp, Glu ;
P¹⁴ étant Phe, His, Phe(3OH), Phe(4F), Phe(4OCF3), Trp(6Cl), Tyr(3Cl), Tyr(3F), Tyr(phényle), Trp, Tyr, 4Thz, Phe(4(4hydroxyphénoxy)), Phe(4NH2), Tyr(Me), Ntyr, Nphe ;
Agb, Agp, Dab, Dab(iPr) ; Dap, Dap(iPr), Har, Lys, Lys(iPr), Narg, Ndab, Nlys, Norn, Orn, Orn(iPr), Arg, NMeLys ;
alloThr, Cit, Hgn, Hse, Leu((3R)OH), Asn, Gln, Ser, Thr, Asp, Glu, ou Hgl ;
P¹⁵ étant Ala, Ala(cPr), Ala(tétrahydropyran4yle), Abu, alloIle, Cha, Cpa, Cpg, Cyg, Dea, Hle, Ile, Leu, Met, Nle, OctGly, tBuGly, tBuAla, Val, NMeAla, NMeVal, Nva ;
Agb, Agp, Dab, Dab(iPr), Dap, Dap(iPr), Har, Lys, Lys(iPr), Narg, Ndab, Nlys, Norn, Orn, Orn(iPr), Arg, NMeLys ;
alloThr, Cit, Hgn, Hse, Leu((3R)OH), Asn, Gln, Ser, Thr, Asp, Glu, ou Hgl ;
P¹⁶ étant Ala, Ala(cPr), Ala(tétrahydropyran4yle), Abu, alloIle, Cha, Cpa, Cpg, Cyg, Dea, Hle, Ile, Leu, Met, Nle, OctGly, tBuGly, tBuAla, Val, NMeAla, NMeVal, Nva ;
Phe, His, Phe(3OH), Phe(4F), Phe(4OCF3), Trp(6Cl), Tyr(3Cl), Tyr(3F), Tyr(phényle), Trp, Tyr, 4Thz, Phe(4(4hydroxyphénoxy)), Phe(4NH2), Tyr(Me), Ntyr, Nphe ;
ou forme tautomère, rotamère, sel, hydrate ou solvate correspondant(e),
Cys ou Pen au niveau de P⁶, s'il est présent, et Cys ou Pen au niveau de P¹³, s'il est présent, formant éventuellement un pont disulfure entre P⁶ et P¹³ ; ou
Dab ou Dap au niveau de P⁶, s'il est présent, et Asp ou Glu au niveau de P¹³, s'il est présent, formant éventuellement un pont lactame entre P⁶ et P¹³ ; ou
Asp ou Glu au niveau de P⁶, s'il est présent, et Dab ou Dap au niveau de P¹³, s'il est présent, formant éventuellement un pont lactame entre P⁶ et P¹³ ;
à la condition qu'au moins trois résidus d'acides aminés parmi les quatre résidus d'acides aminés au niveau des positions P⁹, P¹², P¹⁴ et P¹⁵ soient des résidus d'acides aminés basiques choisis parmi Agb, Agp, Dab, Dab(iPr), Dap, Dap(iPr), Har, Lys, Lys(iPr), Narg, Ndab, Nlys, Norn, Orn, Orn(iPr), Arg, NMeLys.

3. Composé selon la revendication 1 ou la revendication 2,
P¹ étant 2OHVal, Val, NMeVal, Abu, tBuGly ;
le groupe amino de P¹ étant éventuellement remplacé par un groupe guanidino (Gua) ou par un groupe tétraméthylguanidino (TMG) ;
P² étant Pro, Pro (4R) F, Pro(4R)OMe, Pro (4R) OPhe, Pro(3,4dehydro), Pr(4,4F2), Pic ;
Agb, Agp, Dab, Dab(iPr), Dap, Dap(iPr), Lys, Lys(iPr), Narg, Ndab, Nlys, Norn, Orn, Orn(iPr), Pro((4R)NH₂), Arg, NMeLys ;
Hyp, NalloThr ;
P³ étant Hle, Ile, Leu, Nle ;
P⁴ étant Ala, Ala(cPr), Ala(tétrahydropyran4yle), Abu, alloIle, Cyg, Dea, Ile, Leu, Nle, tBuGly, tBuAla, Val, NMeAla, NMeVal, Nva ;
Phe, His, Phe(3OH), Phe(4F), Trp(6Cl), Trp, Tyr, 4Thz, Phe(4(4hydroxyphénoxy)), Phe(4NH2), Tyr(Me), Ntyr, Nphe ;
Agb, Agp, Dab, Dab(iPr), Dap, Dap(iPr), Lys, Lys(iPr), Narg, Ndab, Nlys, Norn, Orn, Orn(iPr), Arg, NMeLys, Lys (Me) ;
alloThr, Cit, Hgn, Hse, Leu((3R)OH), Asn, Gln, Ser, Thr, Asp, Glu, Hgl, Val(3OH) ;
P⁵ étant Phe, His, Trp, Tyr ;
P⁶ étant Pra, Abu (4N₃) ;
Dab, Dap ;
Cys, Hcy, NMeCys, Pen ;
Asp, Glu, Hgl ;
P⁷ étant Ala, Ala(cPr), Ala(tétrahydropyran4yle), Abu, alloIle, Cyg, Dea, Ile, Leu, Nle, tBuGly, tBuAla, Val, NMeAla, NMeVal, Nva ;
Phe, His, Phe(3OH), Phe(4F), Trp(6Cl), Trp, Tyr, 4Thz, Phe(4(4hydroxyphénoxy)), Phe(4NH2), Tyr(Me), Ntyr, Nphe ;
Agb, Agp, Dab, Dab(iPr), Dap, Dap(iPr), Lys, Lys(iPr), Narg, Ndab, Nlys, Norn, Orn, Orn(iPr), Arg, NMeLys ;
alloThr, Cit, Hgn, Hse, Leu((3R)OH), Asn, Gln, Ser, Thr, Asp, Glu, Hgl ;
P⁸ étant Agb, Har, Arg ;
P⁹ étant Dab, Dap, Har, Lys, Orn, Arg, Dab(iPr) ;
Cit ;
P¹⁰ étant alloThr, Hse, Ser, Thr ;
P¹¹ étant ^{D}Dab, ^{D}Dap, ^{D}Lys, ^{D}Orn, ^{D}Arg, ^{D}Dab(iPr) ;
P¹² étant Ala, Ala(cPr), Ala(tétrahydropyran4yle), Abu, alloIle, Cyg, Dea, Ile, Leu, Nle, tBuGly, tBuAla, Val, NMeAla, NMeVal, Nva ;
Phe, His, Phe(3OH), Phe(4F), Trp(6Cl), Tyr(phényle), Trp, Tyr, 4Thz, Phe(4(4hydroxyphénoxy)), Phe(4NH2), Tyr(Me), Ntyr, Nphe ;
Agb, Agp, Dab, Dab(iPr), Dap, Dap(iPr), Lys, Lys(iPr), Narg, Ndab, Nlys, Norn, Orn, Orn(iPr), Arg, NMeLys ;
alloThr, Cit, Hgn, Hse, Leu((3R)OH), Asn, Gln, Ser, Thr, Asp, Glu, Hgl ;
P¹³ étant Pra, Abu (4N₃) ;
Dab, Dap ;
Cys, Hcy, NMeCys, Pen ;
Asp, Glu, HgI ;
P¹⁴ étant Phe, His, Phe(3OH), Phe(4F), Trp(6Cl), Trp, Tyr, 4Thz, Phe(4(4hydroxyphénoxy)), Phe(4NH2), Tyr(Me), Ntyr, Nphe ;
Agb, Agp, Dab, Dab(iPr) ; Dap, Dap(iPr), Lys, Lys(iPr), Narg, Ndab, Nlys, Norn, Orn, Orn(iPr), Arg, NMeLys ;
alloThr, Cit, Hgn, Hse, Leu((3R)OH), Asn, Gln, Ser, Thr, Asp, Glu, Hgl ;
P¹⁵ étant Ala, Ala(cPr), Ala(tétrahydropyran4yle), Abu, alloIle, Cyg, Dea, Ile, Leu, Nle, tBuGly, tBuAla, Val, NMeAla, NMeVal, Nva ;
Agb, Agp, Dab, Dab(iPr), Dap, Dap(iPr), Lys, Lys(iPr), Narg, Ndab, Nlys, Norn, Orn, Orn(iPr), Arg, NMeLys ;
alloThr, Cit, Hgn, Hse, Leu((3R)OH), Asn, Gln, Ser, Thr, Asp, Glu, Hgl ;
P¹⁶ étant Ala, Ala(cPr), Ala(tétrahydropyran4yle), Abu, alloIle, Cyg, Dea, Ile, Leu, Nle, tBuGly, tBuAla, Val, NMeAla, NMeVal, Nva ;
Phe, His, Phe(3OH), Phe(4F), Trp(6Cl), Trp, Tyr, 4Thz, Phe(4(4hydroxyphénoxy)), Phe(4NH2), Tyr(Me), Ntyr, Nphe ;
ou forme tautomère, rotamère, sel, hydrate ou solvate correspondant(e),
Cys, Hcy, NMeCys ou Pen au niveau de P⁶, s'il est présent, et Cys, Hcy, NMeCys ou Pen au niveau de P¹³, s'il est présent, formant éventuellement un pont disulfure entre P⁶ et P¹³ ; ou
Dab ou Dap au niveau de P⁶, s'il est présent, et Asp, Glu ou HgI au niveau de P¹³, s'il est présent, formant éventuellement un pont lactame entre P⁶ et P¹³ ; ou
Asp, Glu ou HgI au niveau de P⁶, s'il est présent, et Dab ou Dap au niveau de P¹³, s'il est présent, formant éventuellement un pont lactame entre P⁶ et P¹³ ; ou
Pra au niveau de P⁶, s'il est présent, et Abu (4N₃) au niveau de P¹³, s'il est présent, formant éventuellement un pont 1,2,3-triazole entre P⁶ et P¹³ ; ou,
Abu (4N₃) au niveau de P⁶, s'il est présent, et Pra au niveau de P¹³, s'il est présent, formant éventuellement un pont 1,2,3-triazole entre P⁶ et P¹³ ;
à la condition qu'au moins trois résidus d'acides aminés parmi les quatre résidus d'acides aminés au niveau des positions P⁹, P¹², P¹⁴ et P¹⁵ soient des résidus d'acides aminés basiques choisis parmi Agb, Agp, Dab, Dab(iPr), Dap, Dap(iPr), Lys, Lys(iPr), Narg, Ndab, Nlys, Norn, Orn, Orn(iPr), Arg, NMeLys.

4. Composé selon l'une quelconque des revendications 1 à 3,
P¹ étant 2OHVal, Val, NMeVal, Abu, tBuGly ;
le groupe amino de P¹ étant éventuellement remplacé par un groupe guanidino (Gua) ou par un groupe tétraméthylguanidino (TMG) ;
P² étant Pro, Pro (4R) F, Pro(4R)OMe, Pro (4R) OPhe, Pro(3,4dehydro), Pr(4,4F2), Pic ;
Agb, Agp, Dab, Dab(iPr), Dap, Dap(iPr), Lys, Lys(iPr), Narg, Ndab, Nlys, Norn, Orn, Orn(iPr), Pro((4R)NH₂), Arg, NMeLys ;
Hyp, NalloThr ;
P³ étant Ile ;
P⁴ étant Ala, Ala(cPr), Ala(tétrahydropyran4yle), Abu, alloIle, Cyg, Dea, Ile, Leu, Nle, tBuGly, tBuAla, Val, NMeAla, NMeVal, Nva ;
Phe, His, Phe(3OH), Phe(4F), Trp(6Cl), Trp, Tyr, 4Thz, Phe(4(4hydroxyphénoxy)), Phe(4NH2), Tyr(Me), Ntyr, Nphe ;
Agb, Agp, Dab, Dab(iPr), Dap, Dap(iPr), Lys, Lys(iPr), Narg, Ndab, Nlys, Norn, Orn, Orn(iPr), Arg, NMeLys ; Lys (Me) ;
alloThr, Cit, Hgn, Hse, Leu((3R)OH), Asn, Gln, Ser, Thr, Asp, Glu, Hgl, Val(3OH) ;
P⁵ étant Phe, His, Trp, Tyr ;
P⁶ étant Dab, Dap ;
Cys, Pen ;
Asp, Glu ;
P⁷ étant Ala, Ala(cPr), Ala(tétrahydropyran4yle), Abu, alloIle, Cyg, Dea, Ile, Leu, Nle, tBuGly, tBuAla, Val, NMeAla, NMeVal, Nva ;
Phe, His, Phe(3OH), Phe(4F), Trp(6Cl), Trp, Tyr, 4Thz, Phe(4(4hydroxyphénoxy)), Phe(4NH2), Tyr(Me), Ntyr, Nphe ;
Agb, Agp, Dab, Dab(iPr), Dap, Dap(iPr), Lys, Lys(iPr), Narg, Ndab, Nlys, Norn, Orn, Orn(iPr), Arg, NMeLys ;
alloThr, Cit, Hgn, Hse, Leu((3R)OH), Asn, Gln, Ser, Thr, Asp, Glu, Hgl ;
P⁸ étant Arg ;
P⁹ étant Dab, Dap, Har, Lys, Orn, Arg, Dab(iPr) ;
Cit ;
P¹⁰ étant alloThr, Hse, Ser, Thr ;
P¹¹ étant ^{D}Dab, ^{D}Dap, ^{D}Lys, ^{D}Orn, ^{D}Arg, ^{D}Dab(iPr) ;
P¹² étant Ala, Ala(cPr), Ala(tétrahydropyran4yle), Abu, alloIle, Cyg, Dea, Ile, Leu, Nle, tBuGly, tBuAla, Val, NMeAla, NMeVal, Nva ;
Phe, His, Phe(3OH), Phe(4F), Trp(6Cl), Tyr(phényle), Trp, Tyr, 4Thz, Phe(4(4hydroxyphénoxy)), Phe(4NH2), Tyr(Me), Ntyr, Nphe ;
Agb, Agp, Dab, Dab(iPr), Dap, Dap(iPr), Lys, Lys(iPr), Narg, Ndab, Nlys, Norn, Orn, Orn(iPr), Arg, NMeLys ;
alloThr, Cit, Hgn, Hse, Leu((3R)OH), Asn, Gln, Ser, Thr, Asp, Glu, Hgl ;
P¹³ étant Dab, Dap ;
Cys, Pen ;
Asp, Glu ;
P¹⁴ étant Phe, His, Phe(3OH), Phe(4F), Trp(6Cl), Trp, Tyr, 4Thz, Phe(4(4hydroxyphénoxy)), Phe(4NH2), Tyr(Me), Ntyr, Nphe ;
Agb, Agp, Dab, Dab(iPr) ; Dap, Dap(iPr), Lys, Lys(iPr), Narg, Ndab, Nlys, Norn, Orn, Orn(iPr), Arg, NMeLys ;
alloThr, Cit, Hgn, Hse, Leu((3R)OH), Asn, Gln, Ser, Thr, Asp, Glu, ou Hgl ;
P¹⁵ étant Ala, Ala(cPr), Ala(tétrahydropyran4yle), Abu, alloIle, Cyg, Dea, Ile, Leu, Nle, tBuGly, tBuAla, Val, NMeAla, NMeVal, Nva ;
Agb, Agp, Dab, Dab(iPr), Dap, Dap(iPr), Lys, Lys(iPr), Narg, Ndab, Nlys, Norn, Orn, Orn(iPr), Arg, NMeLys ;
alloThr, Cit, Hgn, Hse, Leu((3R)OH), Asn, Gln, Ser, Thr, Asp, Glu, Hgl ;
P¹⁶ étant Ala, Ala(cPr), Ala(tétrahydropyran4yle), Abu, alloIle, Cyg, Dea, Ile, Leu, Nle, tBuGly, tBuAla, Val, NMeAla, NMeVal, Nva ;
Phe, His, Phe(3OH), Phe(4F), Trp(6Cl), Trp, Tyr, 4Thz, Phe(4(4hydroxyphénoxy)), Phe(4NH2), Tyr(Me), Ntyr, Nphe ;
ou forme tautomère, rotamère, sel, hydrate ou solvate correspondant(e),
Cys ou Pen au niveau de P⁶, s'il est présent, et Cys ou Pen au niveau de P¹³, s'il est présent, formant éventuellement un pont disulfure entre P⁶ et P¹³ ; ou
Dab ou Dap au niveau de P⁶, s'il est présent, et Asp ou Glu au niveau de P¹³, s'il est présent, formant éventuellement un pont lactame entre P⁶ et P¹³ ; ou
Asp ou Glu au niveau de P⁶, s'il est présent, et Dab ou Dap au niveau de P¹³, s'il est présent, formant éventuellement un pont lactame entre P⁶ et P¹³ ;
à la condition qu'au moins trois résidus d'acides aminés parmi les quatre résidus d'acides aminés au niveau des positions P⁹, P¹², P¹⁴ et P¹⁵ soient des résidus d'acides aminés basiques choisis parmi Agb, Agp, Dab, Dab(iPr), Dap, Dap(iPr), Lys, Lys(iPr), Narg, Ndab, Nlys, Norn, Orn, Orn(iPr), Arg, NMeLys.

5. Composé selon l'une quelconque des revendications 1 à 4,
P¹ étant Val, 2OHVal, NMeVal, Gua-Val, TMG-Val, Abu, ou tBuGly ;
P² étant Pro, Pro (4R) F, Pro (4R)OMe, Pro (4R) OPhe, Pro(3,4dehydro), Pr(4,4F2), Pic, Pro((4R)NH₂), Ndab, NalloThr, ou Hyp ;
P³ étant Ile ;
P⁴ étant Ile, Thr, Phe, Dab, Orn, Arg, Tyr, Leu, Asn, Lys, Lys(Me), Dap, Val(3OH), ou alloThr ;
P⁵ étant Trp ou Tyr ;
P⁶ étant Cys, Pen, Asp, ou Pra ;
P⁷ étant Asn, Ala, Leu, Ile, Ser, Thr, Lys, Dap, Glu, ou His ;
P⁸ étant Arg ;
P⁹ étant Arg, Dab, Dab(iPr), Lys, ou Cit ;
P¹⁰ étant Ser ou Thr ;
P¹¹ étant ^{D}Dab, ^{D}Dap, ^{D}Orn, ^{D}Lys, ^{D}Arg, ou ^{D}Dab(iPr) ;
P¹² étant Lys, Ile, Ser, Tyr, Trp, Asn, Dab, Orn, ou Cit ;
P¹³ étant Cys, Pen, Dab, ou Abu (4N₃) ;
P¹⁴ étant Dab, Dab(iPr), Lys, Gln, Ser, ou Tyr ;
P¹⁵ étant Arg, Thr, Leu, Ser, Dab, Lys, Orn, ou Orn(iPr) ; et
P¹⁶ étant Nle, Cha, ou Tyr ;
ou forme tautomère, rotamère, sel, hydrate ou solvate correspondant(e),
Cys ou Pen au niveau de P⁶, s'il est présent, et Cys ou Pen au niveau de P¹³, s'il est présent, formant éventuellement un pont disulfure entre P⁶ et P¹³, ou
Asp au niveau de P⁶, s'il est présent, et Dab au niveau de P¹³, s'il est présent, formant éventuellement un pont lactame entre P⁶ et P¹³, ou
Pra au niveau de P⁶, s'il est présent, et Abu(4N₃) au niveau de P¹³, s'il est présent, formant éventuellement un pont 1,2,3-triazole entre P⁶ et P¹³ ;
à la condition qu'au moins trois résidus d'acides aminés parmi les quatre résidus d'acides aminés au niveau des positions P⁹, P¹², P¹⁴ et P¹⁵ soient des résidus d'acides aminés basiques choisis parmi Dab, Lys, Arg, ou Dab(iPr) au niveau de P⁹, Lys, Dab, ou Orn au niveau de P¹², Dab, Dab(iPr), ou Lys au niveau de P¹⁴ et Arg, Dab, Lys, Orn ou Orn(iPr) au niveau de P¹⁵.

6. Composé selon l'une quelconque des revendications 1 à 5,
P¹ étant Val, NMeVal, Gua-Val, TMG-Val, ou Abu ;
P² étant Pro, Pro(4R)OMe, Pro(3,4dehydro), Pic, Pro((4R)NH₂), Ndab, NalloThr, ou Hyp ;
P³ étant Ile ;
P⁴ étant Ile, Thr, Phe, Dab, Arg, Val(3OH), ou Tyr ;
P⁵ étant Tyr ;
P⁶ étant Cys, Pen, ou Asp ;
P⁷ étant Asn, Leu, Ile, Ser, Dap, ou His ;
P⁸ étant Arg ;
P⁹ étant Arg, Lys, Dab, ou Dab(iPr) ;
P¹⁰ étant Ser ou Thr ;
P¹¹ étant ^{D}Dab, ^{D}Dab(iPr), ou ^{D}Arg ;
P¹² étant Lys, Ile, Ser, Dab, Orn, ou Cit ;
P¹³ étant Cys, Pen, ou Dab ;
P¹⁴ étant Dab, Dab(iPr), Lys, Gln, Ser, ou Tyr ;
P¹⁵ étant Arg, Dab, Orn, Orn(iPr), Ser, ou Thr ;
P¹⁶ étant Nle, Cha, ou Tyr ;
ou forme tautomère, rotamère, sel, hydrate ou solvate correspondant(e),
Cys ou Pen au niveau de P⁶, s'il est présent, et Cys ou Pen au niveau de P¹³, s'il est présent, formant éventuellement un pont disulfure entre P⁶ et P¹³, ou
Asp au niveau de P⁶, s'il est présent, et Dab au niveau de P¹³, s'il est présent, formant éventuellement un pont lactame entre P⁶ et P¹³ ;
à la condition qu'au moins trois résidus d'acides aminés parmi les quatre résidus d'acides aminés au niveau des positions P⁹, P¹², P¹⁴ et P¹⁵ soient des résidus d'acides aminés basiques choisis parmi Dab, Lys, Arg, ou Dab(iPr) au niveau de P⁹, Lys, Orn, ou Dab au niveau de P¹², Lys, Dab ou Dab(iPr) au niveau de P¹⁴ et Arg, Dab, Orn, ou Orn(iPr) au niveau de P¹⁵.

7. Composé selon l'une quelconque des revendications 1 à 6,
P¹ étant 2OHVal, Gua-Val ou TMG-Val ;
P² étant Pro, Pro((4R)NH₂), ou Hyp ;
P³ étant Ile ;
P⁴ étant Ile, Thr, Dab, ou Orn ;
P⁵ étant Tyr ;
P⁶ étant Cys, Pen, Asp ou Pra ;
P⁷ étant Ile, Asn, ou Thr ;
P⁸ étant Arg ;
P⁹ étant Lys, Arg, Dab, ou Dab(iPr) ;
P¹⁰ étant Ser, ou Thr ;
P¹¹ étant ^{D}Dab, ^{D}Dab(iPr), ou ^{D}Dap ;
P¹² étant Lys, Ile, Asn, Ser, Tyr, Orn, ou Cit ;
P¹³ étant Cys, Dab, ou Abu(4N₃) ;
P¹⁴ étant Dab, Dab(iPr), Lys, ou Ser ;
P¹⁵ étant Arg, Dab, Orn ; Orn(iPr), Ser, ou Thr ;
P¹⁶ étant Nle, ou Tyr ;
ou forme tautomère, rotamère, sel, hydrate ou solvate correspondant(e),
Cys ou Pen au niveau de P⁶, s'il est présent, et Cys au niveau de P¹³, s'il est présent, formant éventuellement un pont disulfure entre P⁶ et P¹³, ou
Asp au niveau de P⁶, s'il est présent, et Dab au niveau de P¹³, s'il est présent, formant éventuellement un pont lactame entre P⁶ et P¹³ ; ou
Pra au niveau de P⁶, s'il est présent, et Abu(4N₃) au niveau de P¹³, s'il est présent, formant éventuellement un pont 1,2,3-triazole entre P⁶ et P¹³ ;
à la condition qu'au moins trois résidus d'acides aminés parmi les quatre résidus d'acides aminés au niveau des positions P⁹, P¹², P¹⁴ et P¹⁵ soient des résidus d'acides aminés basiques choisis parmi Dab, Lys, Arg, ou Dab(iPr) au niveau de P⁹, Lys, ou Orn au niveau de P¹², Lys, Dab ou Dab(iPr) au niveau de P¹⁴ et Arg, Dab, Orn, ou Orn(iPr) au niveau de P¹⁵.

8. Composé selon la revendication 1, le composé étant choisi dans le groupe constitué par :
Val-Pro-Ile-Ile-Tyr-Cys-Asn-Arg-Arg-Thr-^{D}Dab-Lys-Cys-Dab-Arg-Nle ;
Val-Pro((4R)NH₂)-Ile-Ile-Tyr-Pen-Asn-Arg-Arg-Thr-^{D}Dab-Lys-Cys-Dab-Arg-Nle ;
Val-Pro((4R)NH₂)-Ile-Ile-Tyr-Pen-Asn-Arg-Dab-Thr-^{D}Dab-Lys-Cys-Dab-Arg-Nle ;
Val-Ndab-Ile-Ile-Tyr-Pen-Asn-Arg-Dab-Thr-^{D}Dab-Lys-Cys-Dab-Arg-Nle ;
Val-Pro((4R)NH₂)-Ile-Thr-Tyr-Pen-Asn-Arg-Dab-Thr-^{D}Dab-Lys-Cys-Dab-Arg-Nle ;
Val-Pro-Ile-Ile-Tyr-Cys-Asn-Arg-Dab-Thr-^{D}Dab-Lys-Cys-Dab-Arg-Cha ;
Val-Pro-Ile-Ile-Tyr-Pen-Asn-Arg-Dab-Thr-^{D}Dab-Lys-Cys-Dab-Arg-Tyr ;
Val-Pro-Ile-Ile-Tyr-Cys-Asn-Arg-Dab-Thr-^{D}Dab-Lys-Pen-Dab-Arg-Tyr ;
Val-Pro((4R)NH₂)-Ile-Ile-Tyr-Cys-Asn-Arg-Dab-Thr-^{D}Dab-Lys-Cys-Dab-Arg-Tyr ;
Val-Hyp-Ile-Ile-Tyr-Cys-Asn-Arg-Dab-Thr-^{D}Dab-Lys-Cys-Dab-Arg-Tyr ;
Val-Pro-Ile-Thr-Tyr-Cys-Asn-Arg-Dab-Thr-^{D}Dab-Lys-Cys-Dab-Arg-Tyr ;
Val-Pro-Ile-Phe-Tyr-Cys-Asn-Arg-Dab-Thr-^{D}Dab-Lys-Cys-Dab-Arg-Tyr ;
2OHVal-Pro-Ile-Ile-Tyr-Cys-Asn-Arg-Dab-Thr-^{D}Dab-Lys-Cys-Dab-Arg-Tyr ;
Val-Hyp-Ile-Ile-Tyr-Pen-Asn-Arg-Dab-Thr-^{D}Dab-Lys-Cys-Dab-Arg-Tyr ;
Val-Hyp-Ile-Thr-Tyr-Pen-Asn-Arg-Dab-Thr-^{D}Dab-Lys-Cys-Dab-Arg-Tyr ;
Val-Hyp-Ile-Thr-Tyr-Pen-Asn-Arg-Dab-Thr-^{D}Dap-Lys-Cys-Dab-Arg-Tyr ;
Val-Hyp-Ile-Thr-Tyr-Pen-Asn-Arg-Dab-Thr-^{D}Orn-Lys-Cys-Dab-Arg-Tyr ;
Val-Hyp-Ile-Thr-Tyr-Pen-Asn-Arg-Dab-Thr-^{D}Lys-Lys-Cys-Dab-Arg-Tyr ;
Val-Hyp-Ile-Thr-Tyr-Pen-Asn-Arg-Dab-Thr-^{D}Arg-Lys-Cys-Dab-Arg-Tyr ;
Val-Hyp-Ile-Dab-Tyr-Pen-Asn-Arg-Dab-Thr-^{D}Dab-Lys-Cys-Dab-Thr-Tyr ;
Val-Hyp-Ile-Arg-Tyr-Pen-Asn-Arg-Dab-Thr-^{D}Dab-Lys-Cys-Dab-Thr-Tyr ;
Val-Hyp-Ile-Tyr-Tyr-Pen-Asn-Arg-Dab-Thr-^{D}Dab-Lys-Cys-Dab-Arg-Tyr ;
Val-Hyp-Ile-Leu-Tyr-Pen-Asn-Arg-Dab-Thr-^{D}Dab-Lys-Cys-Dab-Arg-Tyr ;
Val-Hyp-Ile-Asn-Tyr-Pen-Asn-Arg-Dab-Thr-^{D}Dab-Lys-Cys-Dab-Arg-Tyr ;
Val-Hyp-Ile-Dab-Tyr-Pen-Asn-Arg-Dab-Thr-^{D}Dab-Lys-Cys-Dab-Arg-Tyr ;
Val-Hyp-Ile-Lys-Tyr-Pen-Asn-Arg-Dab-Thr-^{D}Dab-Lys-Cys-Dab-Arg-Tyr ;
Val-Hyp-Ile-Thr-Trp-Pen-Asn-Arg-Dab-Thr-^{D}Dab-Lys-Cys-Dab-Arg-Tyr ;
Val-Hyp-Ile-Thr-Tyr-Pen-Ala-Arg-Dab-Thr-^{D}Dab-Lys-Cys-Dab-Arg-Tyr ;
Val-Hyp-Ile-Thr-Tyr-Pen-Leu-Arg-Dab-Thr-^{D}Dab-Lys-Cys-Dab-Arg-Tyr ;
Val-Hyp-Ile-Thr-Tyr-Pen-Ile-Arg-Dab-Thr-^{D}Dab-Lys-Cys-Dab-Arg-Tyr ;
Val-Hyp-Ile-Thr-Tyr-Pen-Ser-Arg-Dab-Thr-^{D}Dab-Lys-Cys-Dab-Arg-Tyr ;
Val-Hyp-Ile-Thr-Tyr-Pen-Thr-Arg-Dab-Thr-^{D}Dab-Lys-Cys-Dab-Arg-Tyr ;
Val-Hyp-Ile-Thr-Tyr-Pen-Lys-Arg-Dab-Thr-^{D}Dab-Lys-Cys-Dab-Arg-Tyr ;
Val-Hyp-Ile-Thr-Tyr-Pen-Asn-Arg-Lys-Thr-^{D}Dab-Lys-Cys-Dab-Arg-Tyr ;
Val-Hyp-Ile-Thr-Tyr-Pen-Asn-Arg-Arg-Thr-^{D}Dab-Lys-Cys-Dab-Arg-Tyr ;
Val-Hyp-Ile-Thr-Tyr-Pen-Asn-Arg-Dab-Thr-^{D}Dab-Ile-Cys-Dab-Arg-Tyr ;
Val-Hyp-Ile-Thr-Tyr-Pen-Asn-Arg-Dab-Thr-^{D}Dab-Ser-Cys-Dab-Arg-Tyr ;
Val-Hyp-Ile-Thr-Tyr-Pen-Asn-Arg-Dab-Thr-^{D}Dab-Tyr-Cys-Dab-Arg-Tyr ;
Val-Hyp-Ile-Thr-Tyr-Pen-Asn-Arg-Dab-Thr-^{D}Dab-Trp-Cys-Dab-Arg-Tyr ;
Val-Hyp-Ile-Thr-Tyr-Pen-Asn-Arg-Dab-Thr-^{D}Dab-Asn-Cys-Dab-Arg-Tyr ;
Val-Hyp-Ile-Thr-Tyr-Pen-Asn-Arg-Dab-Thr-^{D}Dab-Dab-Cys-Dab-Arg-Tyr ;
Val-Hyp-Ile-Thr-Tyr-Pen-Asn-Arg-Dab-Thr-^{D}Dab-Lys-Cys-Dab-Leu-Tyr ;
Val-Hyp-Ile-Thr-Tyr-Pen-Asn-Arg-Dab-Thr-^{D}Dab-Lys-Cys-Dab-Ser-Tyr ;
Val-Hyp-Ile-Thr-Tyr-Pen-Asn-Arg-Dab-Thr-^{D}Dab-Lys-Cys-Dab-Dab-Tyr ;
Val-Hyp-Ile-Thr-Tyr-Pen-Asn-Arg-Dab-Thr-^{D}Dab-Lys-Cys-Dab-Lys-Tyr ;
Val-Hyp-Ile-Thr-Tyr-Pen-Asn-Arg-Dab-Thr-^{D}Dab-Lys-Cys-Dab-Orn-Tyr ;
Val-Hyp-Ile-Thr-Tyr-Pen-Asn-Arg-Dab-Thr-^{D}Dab-Cit-Cys-Dab-Arg-Tyr ;
NMeVal-Hyp-Ile-Thr-Tyr-Pen-Asn-Arg-Dab-Thr-^{D}Dab-Lys-Cys-Dab-Arg-Tyr ;
Val-Hyp-Ile-Thr-Tyr-Pen-Dap-Arg-Dab-Thr-^{D}Dab-Lys-Cys-Dab-Arg-Tyr ;
Val-Hyp-Ile-Dap-Tyr-Pen-Asn-Arg-Dab-Thr-^{D}Dab-Lys-Cys-Dab-Arg-Tyr ;
Val-Hyp-Ile-Arg-Tyr-Pen-Asn-Arg-Dab-Thr-^{D}Dab-Lys-Cys-Dab-Arg-Tyr ;
Val-Pro((4R)NH₂)-Ile-Thr-Tyr-Pen-Asn-Arg-Dab-Thr-^{D}Dab-Lys-Cys-Tyr-Arg-Tyr ;
Gua-Val-Pro-Ile-Ile-Tyr-Cys-Asn-Arg-Dab-Thr-^{D}Dab-Lys-Cys-Dab-Arg-Tyr ;
Gua-Val-Pro-Ile-Ile-Tyr-Pen-Asn-Arg-Dab-Thr-^{D}Dab-Lys-Cys-Dab-Arg-Tyr ;
Gua-Val-Hyp-Ile-Thr-Tyr-Pen-Asn-Arg-Dab-Thr-^{D}Dab-Lys-Cys-Dab-Arg-Tyr ;
Gua-Val-Hyp-Ile-Ile-Tyr-Pen-Asn-Arg-Dab-Thr-^{D}Dab-Lys-Cys-Dab-Arg-Tyr ;
Gua-Val-Pro((4R)NH₂)-Ile-Thr-Tyr-Pen-Asn-Arg-Dab-Thr-^{D}Dab-Lys-Cys-Dab-Arg-Tyr ;
Gua-Val-Pro((4R)NH₂)-Ile-Ile-Tyr-Pen-Asn-Arg-Dab-Thr-^{D}Dab-Lys-Cys-Dab-Arg-Tyr ;
Gua-Val-Hyp-Ile-Tyr-Tyr-Pen-Asn-Arg-Dab-Thr-^{D}Dab-Lys-Cys-Gln-Arg-Tyr ;
Gua-Val-Hyp-Ile-Thr-Tyr-Pen-Glu-Arg-Dab-Thr-^{D}Dab-Lys-Cys-Dab-Arg-Tyr ;
Gua-Val-Hyp-Ile-Thr-Tyr-Pen-His-Arg-Dab-Thr-^{D}Dab-Lys-Cys-Dab-Arg-Tyr ;
Gua-Val-Hyp-Ile-Thr-Tyr-Pen-Asn-Arg-Dab-Thr-^{D}Dab-Dab-Cys-Dab-Arg-Tyr ;
Gua-Val-Hyp-Ile-Thr-Tyr-Pen-Asn-Arg-Dab-Ser-^{D}Dab-Lys-Cys-Dab-Arg-Tyr ;
Gua-Val-Hyp-Ile-Thr-Tyr-Pen-Asn-Arg-Dab-Ser-^{D}Dab-Lys-Cys-Dab-Dab-Tyr ;
Gua-Val-Hyp-Ile-Thr-Tyr-Pen-Asn-Arg-Dab-Ser-^{D}Dab-Lys-Cys-Dab-Orn-Tyr ;
Gua-Val-Pro((4R)NH₂)-Ile-Thr-Tyr-Pen-Asn-Arg-Dab-Thr-^{D}Dab-Lys-Cys-Tyr-Arg-Tyr ;
Gua-Val-Pro((4R)NH₂)-Ile-Ile-Tyr-Pen-Asn-Arg-Dab-Thr-^{D}Dab-Lys-Cys-Tyr-Arg-Tyr ;
Gua-Val-Hyp-Ile-alloThr-Tyr-Pen-Asn-Arg-Dab-Thr-^{D}Dab-Lys-Cys-Tyr-Arg-Tyr ;
TMG-Val-Pro-Ile-Ile-Tyr-Cys-Asn-Arg-Dab-Thr-^{D}Dab-Lys-Cys-Dab-Arg-Tyr ;
TMG-Val-Hyp-Ile-Thr-Tyr-Pen-Asn-Arg-Dab-Thr-^{D}Dab-Lys-Cys-Dab-Arg-Tyr ;
Val-Hyp-Ile-Ile-Tyr-Pra-Asn-Arg-Dab-Thr-^{D}Dab-Lys-Abu(4N₃)-Dab-Arg-Tyr ;
Val-Hyp-Ile-Ile-Tyr-Asp-Asn-Arg-Dab-Thr-^{D}Dab-Lys-Dab-Dab-Arg-Tyr ;
Val-Hyp-Ile-Ile-Tyr-Pen-Asn-Arg-Dab-Thr-^{D}Dab-Lys-Cys-Dab-Cit-Tyr ;
Val-Hyp-Ile-Ile-Tyr-Pen-Asn-Arg-Dab-Thr-^{D}Dab-Lys-Cys-Dab-Dab-Tyr ;
Val-Hyp-Ile-Ile-Tyr-Pen-Asn-Arg-Dab-Ser-^{D}Dab-Lys-Cys-Dab-Arg-Tyr ;
Val-Hyp-Ile-Ile-Tyr-Pen-Asn-Arg-Dab-Ser-^{D}Dab-Lys-Cys-Dab-Dab-Tyr ;
Val-Hyp-Ile-Thr-Tyr-Pen-Asn-Arg-Dab-Ser-^{D}Dab-Lys-Cys-Dab-Dab-Tyr ;
Val-Hyp-Ile-Ile-Tyr-Pen-Asn-Arg-Dab-Thr-^{D}Dab-Orn-Cys-Dab-Arg-Tyr ;
Abu-Hyp-Ile-Ile-Tyr-Pen-Asn-Arg-Dab-Thr-^{D}Dab-Lys-Cys-Dab-Arg-Tyr ;
Val-Pro-Ile-Thr-Tyr-Pen-Asn-Arg-Dab-Thr-^{D}Dab-Lys-Cys-Dab-Arg-Tyr ;
Val-Hyp-Ile-Dab-Tyr-Pen-Asn-Arg-Dab-Ser-^{D}Dab-Lys-Cys-Dab-Dab-Tyr ;
Val-Hyp-Ile-Ile-Tyr-Pen-Asn-Arg-Lys-Thr-^{D}Dab-Lys-Cys-Lys-Arg-Tyr ;
Val-Hyp-Ile-Ile-Tyr-Pen-Asn-Arg-Dab-Thr-^{D}Dab-Ile-Cys-Dab-Dab-Tyr ;
Val-Hyp-Ile-Ile-Tyr-Pen-Asn-Arg-Dab-Ser-^{D}Dab-Ile-Cys-Dab-Dab-Tyr ;
Val-Hyp-Ile-Ile-Tyr-Pen-Asn-Arg-Dab-Thr-^{D}Dab-Lys-Cys-Dab-Orn-Tyr ;
Val-Hyp-Ile-Ile-Tyr-Pen-Asn-Arg-Dab-Ser-^{D}Dab-Lys-Cys-Dab-Orn-Tyr ;
Val-Hyp-Ile-Ile-Tyr-Pen-Asn-Arg-Dab-Thr-^{D}Dab-Ile-Cys-Dab-Arg-Tyr ;
Val-Hyp-Ile-Thr-Tyr-Pen-Ile-Arg-Dab-Thr-^{D}Dab-Lys-Cys-Dab-Orn-Tyr ;
Val-Hyp-Ile-Dab-Tyr-Pen-Asn-Arg-Dab-Thr-^{D}Dab-Lys-Cys-Dab-Orn-Tyr ;
Val-Pro((4R)NH₂)-Ile-Ile-Tyr-Pen-Asn-Arg-Dab-Thr-^{D}Dab-Ile-Cys-Dab-Arg-Tyr ;
Val-Pro((4R)NH₂)-Ile-Ile-Tyr-Pen-Asn-Arg-Dab-Thr-^{D}Dab-Cit-Cys-Dab-Arg-Tyr ;
Val-Hyp-Ile-Thr-Tyr-Pen-Ile-Arg-Dab-Thr-^{D}Dab-Lys-Cys-Ser-Orn-Tyr ;
Val-Pro((4R)NH₂)-Ile-Ile-Tyr-Pen-Asn-Arg-Dab-Thr-^{D}Dab-Lys-Cys-Ser-Arg-Tyr ;
Val-Hyp-Ile-Thr-Tyr-Pen-Asn-Arg-Dab-Ser-^{D}Dab-Lys-Cys-Dab-Orn-Tyr ;
tBuGly-Hyp-Ile-Thr-Tyr-Pen-Ile-Arg-Dab-Thr-^{D}Dab-Lys-Cys-Dab-Orn-Tyr ;
Val-Hyp-Ile-Ile-Tyr-Pen-Asn-Arg-Dab-Ser-^{D}Dab-Ile-Cys-Dab-Orn-Tyr ;
Val-Hyp-Ile-Ile-Tyr-Pen-Asn-Arg-Dab-Thr-^{D}Dab-Ile-Cys-Dab-Orn-Tyr ;
Val-Hyp-Ile-Ile-Tyr-Pen-Asn-Arg-Dab-Thr-^{D}Dab-Cit-Cys-Dab-Arg-Tyr ;
Val-Hyp-Ile-Thr-Tyr-Pen-Ile-Arg-Dab-Thr-^{D}Dab-Lys-Cys-Dab-Ser-Tyr ;
Val-Pro((4R)NH₂)-Ile-Ile-Tyr-Pen-Asn-Arg-Dab-Thr-^{D}Dab-Lys-Cys-Dab-Ser-Tyr ;
Val-Hyp-Ile-Ile-Tyr-Pen-Asn-Arg-Dab-Thr-^{D}Dab-Ser-Cys-Dab-Arg-Tyr ;
Val-Pro((4R)NH₂)-Ile-Thr-Tyr-Pen-Asn-Arg-Dab-Thr-^{D}Dab-Cit-Cys-Dab-Ser-Tyr ;
Val-Pro((4R)NH₂)-Ile-Thr-Tyr-Pen-Asn-Arg-Dab-Thr-^{D}Dab-Lys-Cys-Ser-Ser-Tyr ;
Val-Hyp-Ile-Thr-Tyr-Pen-Ile-Arg-Dab-Thr-^{D}Dab-Lys-Cys-Ser-Arg-Tyr ;
Val-Hyp-Ile-Ile-Tyr-Pen-Asn-Arg-Dab-Thr-^{D}Dab-Lys-Cys-Dab-Ser-Tyr ;
Val-Pro((4R)NH₂)-Ile-Ile-Tyr-Pen-Asn-Arg-Dab-Thr-^{D}Dab-Cit-Cys-Dab-Ser-Tyr ;
Val-Hyp-Ile-Ile-Tyr-Pen-Asn-Arg-Dab-Thr-^{D}Dab-Lys-Cys-Ser-Arg-Tyr ;
Val-Hyp-Ile-Dab-Tyr-Pen-Asn-Arg-Dab-Thr-^{D}Dab-Cit-Cys-Dab-Ser-Tyr ;
Val-Pro((4R)NH₂)-Ile-Ile-Tyr-Pen-Asn-Arg-Dab-Thr-^{D}Dab-Lys-Cys-Dab-Thr-Tyr ;
Val-Pro((4R)NH₂)-Ile-Thr-Tyr-Pen-Asn-Arg-Dab-Thr-^{D}Dab-Lys-Cys-Ser-Arg-Tyr ;
Val-Hyp-Ile-Ile-Tyr-Pen-Asn-Arg-Dab-Thr-^{D}Dab-Lys-Cys-Dab-Thr-Tyr ;
Val-Hyp-Ile-Ile-Tyr-Pen-Asn-Arg-Dab-Ser-^{D}Dab-Lys-Cys-Dab-Thr-Tyr ;
Val-Hyp-Ile-Dab-Tyr-Pen-Asn-Arg-Dab-Thr-^{D}Dab-Cit-Cys-Dab-Thr-Tyr ;
tBuGly-Hyp-Ile-Ile-Tyr-Pen-Asn-Arg-Dab-Thr-^{D}Dab-Lys-Cys-Dab-Arg-Tyr ;
Val-Pro((4R)NH₂)-Ile-Ile-Tyr-Pen-Asn-Arg-Dab-Thr-^{D}Dab-Ile-Cys-Dab-Thr-Tyr ;
Val-Pro((4R)NH₂)-Ile-Thr-Tyr-Pen-Asn-Arg-Dab-Thr-^{D}Dab-Cit-Cys-Dab-Thr-Tyr ;
Val-Pro((4R)NH₂)-Ile-Thr-Tyr-Pen-Asn-Arg-Dab-Thr-^{D}Dab-Ile-Cys-Dab-Thr-Tyr ;
Val-Hyp-Ile-Ile-Tyr-Pen-Asn-Arg-Dab-Ser-^{D}Dab-Ile-Cys-Dab-Arg-Tyr ;
Val-Pro((4R)NH₂)-Ile-Thr-Tyr-Pen-Asn-Arg-Dab-Thr-^{D}Dab-Lys-Cys-Ser-Thr-Tyr ;
Val-Pro((4R)NH₂)-Ile-Thr-Tyr-Pen-Asn-Arg-Dab-Thr-^{D}Dab-Lys-Cys-Dab-Thr-Tyr ;
Val-Hyp-Ile-Ile-Tyr-Pen-Asn-Arg-Orn-Thr-^{D}Dab-Ile-Cys-Dab-Arg-Tyr ;
Val-Hyp-Ile-Orn-Tyr-Pen-Asn-Arg-Dab-Thr-^{D}Dab-Lys-Cys-Dab-Thr-Tyr ;
Val-Hyp-Ile-Thr-Tyr-Pen-Ile-Arg-Dab-Thr-^{D}Dab-Orn-Cys-Dab-Arg-Tyr ;
Val-Hyp-Ile-Ile-Tyr-Pen-Asn-Arg-Cit-Thr-^{D}Dab-Lys-Cys-Dab-Arg-Tyr ;
Val-Hyp-Ile-Lys(Me)-Tyr-Pen-Asn-Arg-Dab-Thr-^{D}Dab-Lys-Cys-Dab-Arg-Tyr ;
Val-Hyp-Ile-Val(30H)-Tyr-Pen-Asn-Arg-Dab-Thr-^{D}Dab-Lys-Cys-Dab-Arg-Tyr ;
Val-Pro(4,4F₂)-Ile-Thr-Tyr-Pen-Asn-Arg-Dab-Thr-^{D}Dab-Lys-Cys-Dab-Arg-Tyr ;
Val-Pro(3,4dehydro)-Ile-Thr-Tyr-Pen-Asn-Arg-Dab-Thr-^{D}Dab-Lys-Cys-Dab-Arg-Tyr ;
Val-Pro(4,4F₂)-Ile-Thr-Tyr-Pen-Asn-Arg-Dab-Thr-^{D}Dab-Lys-Cys-Dab-Arg-Tyr ;
Val-Pro((4R)OPh)-Ile-Thr-Tyr-Pen-Asn-Arg-Dab-Thr-^{D}Dab-Lys-Cys-Dab-Arg-Tyr ;
Val-Pro((4R)F)-Ile-Thr-Tyr-Pen-Asn-Arg-Dab-Thr-^{D}Dab-Lys-Cys-Dab-Arg-Tyr ;
Val-Pic-Ile-Thr-Tyr-Pen-Asn-Arg-Dab-Thr-^{D}Dab-Lys-Cys-Dab-Arg-Tyr ;
Val-NalloThr-Ile-Thr-Tyr-Pen-Asn-Arg-Dab-Thr-^{D}Dab-Lys-Cys-Dab-Arg-Tyr ;
Hyp-Ile-Thr-Tyr-Pen-Asn-Arg-Dab(iPr)-Thr-^{D}Dab-Lys-Cys-Dab-Orn(iPr)-Tyr ;
Hyp-Ile-Thr-Tyr-Pen-Asn-Arg-Dab(iPr)-Thr-^{D}Dab-Lys-Cys-Dab(iPr)-Arg-Tyr ;
Hyp-Ile-Thr-Tyr-Pen-Asn-Arg-Dab-Thr-^{D}Dab-Lys-Cys-Dab-Orn(iPr)-Tyr ;
Hyp-Ile-Thr-Tyr-Pen-Ile-Arg-Dab-Thr-^{D}Dab-Lys-Cys-Dab-Orn(iPr)-Tyr ; et
Hyp-Ile-Thr-Tyr-Pen-Asn-Arg-Dab-Thr-^{D}Dab(iPr)-Lys-Cys-Dab-Arg-Tyr ;
ou forme tautomère, rotamère, sel, hydrate ou solvate correspondant(e),
Cys ou Pen au niveau de P⁶, s'il est présent, et Cys ou Pen au niveau de P¹³, s'il est présent, formant éventuellement un pont disulfure entre P⁶ et P¹³, ou
Asp au niveau de P⁶, s'il est présent, et Dab au niveau de P¹³, s'il est présent, formant éventuellement un pont lactame entre P⁶ et P¹³, ou
Pra au niveau de P⁶, s'il est présent, et Abu(4N₃) au niveau de P¹³, s'il est présent, formant éventuellement un pont 1,2,3-triazole entre P⁶ et P¹³.

9. Composé selon la revendication 8, Cys ou Pen au niveau de P⁶, et Cys ou Pen au niveau de P¹³, formant un pont disulfure entre P⁶ et P¹³.

10. Composé selon la revendication 9, le composé étant Gua-Val-Hyp-Ile-Thr-Tyr-Pen-Asn-Arg-Dab-Thr-^{D}Dab-Lys-Cys-Dab-Arg-Tyr ;
ou forme tautomère, rotamère, sel, hydrate ou solvate correspondant(e).

11. Composition pharmaceutique contenant un composé de formule (I) ou un mélange de composés de formule (I) tel que défini selon l'une quelconque des revendications 1 à 10 ou un sel pharmaceutiquement acceptable correspondant et au moins un support pharmaceutiquement inerte, éventuellement sous une forme appropriée pour une administration orale, topique, transdermique, par injection, buccale, transmuqueuse, rectale, pulmonaire ou par inhalation, notamment sous forme d'un comprimé, d'une dragée, d'une gélule, d'une solution, d'un liquide, d'un gel, d'un pansement, d'une crème, d'un onguent, d'un sirop, d'une bouillie, d'une suspension, d'un spray, d'un nébuliseur, d'un aérosol ou d'un suppositoire.

12. Composé de formule (I) tel que défini dans l'une quelconque des revendications 1 à 10 ou sel pharmaceutiquement acceptable correspondant, destiné à être utilisé comme médicament.

13. Composé de formule (I) tel que défini dans l'une quelconque des revendications 1 à 10 ou sel pharmaceutiquement acceptable correspondant destiné à être utilisé comme substance pharmaceutiquement active ayant une activité antibiotique dans le traitement ou la prévention d'une infection ou d'une maladie associée à une telle infection.

14. Composé pour une utilisation selon la revendication 13 ou sel pharmaceutiquement acceptable correspondant, l'infection ou la maladie liée à une telle infection étant liée à des maladies respiratoires ou des maladies de la peau ou des tissus mous ou des maladies gastro-intestinales ou des maladies oculaires ou des maladies des oreilles ou des maladies du SNC ou des maladies osseuses ou des maladies cardiovasculaires ou des maladies génito-urinaires ou des maladies nosocomiales, ou des infections liées ou non à un cathéter, ou des infections des voies urinaires, ou des infections sanguines, ou une septicémie induite par une infection.

15. Composé pour une utilisation selon la revendication 13 ou la revendication 14 ou sel pharmaceutiquement acceptable correspondant, l'infection étant choisie parmi les infections nosocomiales, les infections liées ou non à un cathéter, les infections urinaires, les infections du sang, ou une maladie ou un trouble associé à une infection, en particulier des maladies ou des troubles tel(le)s que la pneumonie associée à la ventilation mécanique (PAVM), la pneumonie bactérienne associée à la ventilation mécanique (PBAV), la pneumonie acquise à l'hôpital (PAH), la pneumonie bactérienne acquise à l'hôpital (PBAH), la pneumonie associée aux soins de santé (PASS), la fibrose kystique, l'emphysème, l'asthme, la pneumonie, la diarrhée épidémique, l'entérocolite nécrosante, la typhlite, la gastro-entérite, la pancréatite, la kératite, l'endophtalmie, l'otite, l'abcès cérébral, la méningite, l'encéphalite, l'ostéochondrite, la péricardite, l'épididymite, la prostatite, l'urétrite, la septicémie ; les plaies chirurgicales, les plaies traumatiques et les brûlures.

16. Utilisation d'un composé de formule (I) tel que défini dans l'une quelconque des revendications 1 à 10 ou d'un sel correspondant comme désinfectant ou conservateur pour des produits alimentaires, des produits cosmétiques, des médicaments et/ou d'autres matières contenant des nutriments.

17. Procédé de préparation d'un composé de formule (I) tel que défini selon l'une quelconque des revendications 1 à 10 ou d'un sel correspondant, qui comprend les étapes suivantes :
(a) couplage d'un support solide fonctionnalisé de façon appropriée avec un dérivé N-protégé approprié de l'acide aminé qui, dans le produit final souhaité, se trouve en position P¹⁶ ; tout groupe fonctionnel éventuellement présent dans ledit dérivé d'acide aminé N-protégé étant également protégé de manière appropriée ;
(b) élimination du groupe N-protecteur du produit ainsi obtenu ;
(c) couplage du produit ainsi obtenu avec un dérivé N-protégé approprié de l'acide aminé qui, dans le produit final souhaité, se trouve en position P¹⁵ ; tout groupe fonctionnel éventuellement présent dans ledit dérivé d'acide aminé N-protégé étant également protégé de manière appropriée ;
(d) réalisation des étapes correspondant sensiblement aux étapes (b) et (c) en utilisant de manière appropriée des dérivés N-protégés d'acides aminés qui, dans le produit final souhaité, se trouvent en positions P¹⁴ à P⁶, tout groupe fonctionnel éventuellement présent dans lesdits dérivés d'acides aminés N-protégés étant également protégé de manière appropriée ;
(e) éventuellement déprotection sélective d'un ou plusieurs groupe(s) fonctionnel(s) protégé(s) présent(s) dans la molécule et transformation chimique du ou des groupe(s) réactif(s) ainsi libéré(s) ;
(f) réalisation des étapes correspondant sensiblement aux étapes (b) et (c) en utilisant de manière appropriée des dérivés N-protégés d'acides aminés qui, dans le produit final souhaité, se trouvent dans les positions P⁵ à P², tout groupe fonctionnel éventuellement présent dans lesdits dérivés d'acides aminés N-protégés étant également protégé de manière appropriée ; et, éventuellement, après chaque couplage, déprotection sélective d'un ou plusieurs groupe(s) fonctionnel(s) protégé(s) présent(s) dans la molécule et transformation chimique du ou des réactif(s) ainsi libéré(s) ;
(g) réalisation en outre d'étapes correspondant sensiblement aux étapes (b) et (c) en utilisant un dérivé N-protégé approprié d'un acide aminé, ou éventuellement, un dérivé d'un hydroxyacide protégé de manière appropriée, qui, dans le produit final souhaité, se trouve en position P¹, tout groupe fonctionnel éventuellement présent dans ledit dérivé d'acide aminé N-protégé, ou dérivé d'hydroxyacide, étant également protégé de manière appropriée ; et éventuellement suite du couplage, déprotection sélective d'un ou plusieurs groupe(s) fonctionnel(s) protégé(s) présent(s) dans la molécule et transformation chimique du ou des groupe(s) réactif(s) ainsi libéré(s) ;
(h) éventuellement déprotection sélective d'un ou plusieurs groupe(s) fonctionnel(s) protégé(s) présent(s) dans la molécule et transformation chimique du ou des groupe(s) réactif(s) ainsi libéré(s) ;
(i) éventuellement, élimination du groupe protecteur de N en position P¹ ;
(j) détachement du produit ainsi obtenu du support solide ;
(k) éventuellement déprotection sélective d'un ou plusieurs groupe(s) fonctionnel(s) protégé(s) présent(s) dans la molécule et transformation chimique du ou des groupe(s) réactif(s) ainsi libéré(s) ;
(l) élimination de tout groupe protecteur présent sur des groupes fonctionnels de quelconques éléments de la chaîne de résidus et, éventuellement, tout groupe protecteur qui peut de plus être présent dans la molécule ;
(m) éventuellement implémentation de transformations chimiques supplémentaires d'un ou plusieurs groupe(s) réactif(s) présent(s) dans la molécule ;
(n) si nécessaire, élimination de tout groupe protecteur présent sur des groupes fonctionnels de quelconques éléments de la chaîne de résidus et, éventuellement, tout groupe protecteur qui peut de plus être présent dans la molécule ; et
(o) éventuellement conversion du produit ainsi obtenu en un sel pharmaceutiquement acceptable ; ou
éventuellement conversion d'un sel pharmaceutiquement acceptable ou inacceptable obtenu ainsi en le composé libre de formule (I) correspondant ; ou
éventuellement conversion d'un sel pharmaceutiquement acceptable ou inacceptable ainsi obtenu en un sel différent, pharmaceutiquement acceptable.
